(19) Europäisches Patentamt
European Patent Office
Office européen des brevets

(11) EP 3 720 857 B1

(12) # EUROPEAN PATENT SPECIFICATION

(45) Date of publication and mention of the grant of the patent:
**08.09.2021 Bulletin 2021/36**

(21) Application number: **18811770.9**

(22) Date of filing: **26.11.2018**

(51) Int Cl.:
***C07D 491/052*** *(2006.01)* ***A61P 31/20*** *(2006.01)*
***A61P 31/12*** *(2006.01)* ***A61K 31/436*** *(2006.01)*

(86) International application number:
**PCT/EP2018/082540**

(87) International publication number:
**WO 2019/110352 (13.06.2019 Gazette 2019/24)**

(54) **2-OXO-5H-CHROMENO[4,3-B]PYRIDINES FOR USE IN THE TREATMENT OF HEPATITIS B**

2-OXO-5H-CHROMENO[4,3-B]PYRIDINE ZUR VERWENDUNG BEI DER BEHANDLUNG VON HEPATITIS B

2-OXO-5H-CHROMENO[4,3-B]PYRIDINES DESTINÉES À ÊTRE UTILISÉES DANS LE TRAITEMENT DE L'HÉPATITE B

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2017 GB 201720163**

(43) Date of publication of application:
**14.10.2020 Bulletin 2020/42**

(73) Proprietor: **Galapagos NV**
**2800 Mechelen (BE)**

(72) Inventors:
- **MAMMOLITI, Oscar**
  **2800 Mechelen (BE)**
- **JOANNESSE, Caroline, Martine, Andrée, Marie**
  **2800 Mechelen (BE)**
- **PALISSE, Adeline, Marie, Elise**
  **2800 Mechelen (BE)**
- **VAN DER PLAS, Steven, Emiel**
  **2800 Mechelen (BE)**
- **CATANA, Cornel**
  **011713, Sector 1**
  **Bucharest (RO)**
- **JAUNET, Alexis, Patrick, Claude**
  **2800 Mechelen (BE)**
- **LARUELLE, Chris, Ronan**
  **2800 Mechelen (BE)**
- **DE MUNCK, Tom, Roger, Lisette**
  **9200 Dendermonde (BE)**
- **DE BLIECK, Ann**
  **9112 Sinaai (BE)**
- **DUTHION, Béranger**
  **93230 Romainville (FR)**

(74) Representative: **Bar, Grégory**
**Galapagos NV**
**IP Department**
**Generaal De Wittelaan, L11 A3**
**2800 Mechelen (BE)**

(56) References cited:
**WO-A1-2013/033228** **WO-A1-2015/113990**
**WO-A1-2016/039938** **WO-A1-2016/071215**
**WO-A1-2016/128335** **WO-A1-2017/013046**
**WO-A1-2017/017043** **WO-A1-2017/102648**
**WO-A1-2017/108630** **WO-A1-2017/114812**
**WO-A1-2017/140821** **WO-A1-2017/205115**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

## FIELD OF THE INVENTION

The present invention relates to compounds useful in the prophylaxis and/or treatment of hepatitis B virus (HBV). The present invention also provides methods for the production of the compound of the invention, pharmaceutical compositions comprising the compound of the invention. Disclosed are methods for the prophylaxis and/or treatment of hepatitis B virus by administering the compound of the invention.

## BACKGROUND OF THE INVENTION

It is estimated that 240 million people are chronically infected with HBV worldwide. More than 750,000 deaths annually worldwide are attributed to HBV-related end-stage complications including cirrhosis of the liver, liver failure, and hepatocellular carcinoma (HCC), which occur decades post-exposure and represent the second leading cause of cancer death worldwide (Dawood et al., 2017).

The hepatitis B virus (HBV) belongs to the hepadnaviridae family and is a small, enveloped partially double-stranded DNA virus, consisting of 4 overlapping open reading frames (ORF) encoding respectively for the core, polymerase, envelope and X-proteins (Gómez-Moreno and Garaigorta, 2017).

The transmission of HBV occurs usually via exposure to body fluids, blood, skin lesions, sexual relationship, or passing from the mother to baby. Upon initial exposure to HBV, the failure to induce a significant innate immune response within hepatocytes and the immunosuppressive liver microenvironment can lead to incomplete clearance of infected hepatocytes and the establishment of a chronic hepatitis B (CHB) infection which can be categorised into five clinical phases: (1) immune tolerance characterised by high HBV serum titres (~$2 \times 10^9$ IU/mL) and poorly-activated but not completely silent HBV-specific CD8+ T-cells; (2) immune reactive HBV e antigen (HBeAg)-positive characterised by observable flares of immune-mediated liver damage and fluctuations in HBV titres; (3) inactive HBV carrier characterized by low HBV titres and slower liver injury progression compared to the preceding immune reactive phase; (4) HBeAg-negative chronic hepatitis (or "functional cure") wherein disease progression is halted characterized by undetectable HBV serum DNA and HBV surface antigen recognizing antibodies; and (5) HBV surface antigen (HBsAg)-negative phases, wherein viral covalently closed circular DNA (cccDNA) remains present in the liver but is transcriptionally silent, and may be reactived, for example under immune suppressive therapy (Tu et al., 2017).

The infection starts with the HBV virion interacting with the hepatocytes via initial interaction with proteoglycans followed by entry of the virus to the hepatocyte via the sodium taurocholate transporter peptide (NTCP), the established entry factor for HBV (Huan et al, 2012). Internalisation is then followed unmasking of the outer protein coat of the viral particle to reveal the inner capsid, which is then transported to and enters the nucleus of the hepatocyte. This capsid is then disassembled, allowing the released DNA to be converted initially to relaxed circular DNA (rcDNA), which is in turn converted into the highly stable closed circular DNA (cccDNA) minichromosomal form of the virus DNA. The cccDNA then serves as the template for production of all viral RNA transcripts, which are then translated to the different viral proteins required for assembly of new infectious viral particles.

Pegylated interferon (Peg-IFN)-α is an approved therapies now in use for CHB which is intended to boost immune response resulting in HBeAg and HBsAg seroconversion, meaning the virus is then under the control of the host immune system, reducing circulating HBV DNA to undetectable levels, and placing the virus under the control of the host immune system. Unfortunately, Peg-IFN-α treatment shows limited rate of success even after years of treatment (van Zonneveld et al., 2004), moreover since IFN is associated with substantial adverse effects in many patients it is only used temporarily in a limited number of patients.

The second treatment type are nucleos(t)ide analogue reverse transcriptase inhibitors (NUCs) which act to suppressing HBV DNA formation in new viral particles, but have no effect on the cccDNA population (Sussman, 2009). Treatment with NUCs is associated with reduced liver necroinflammation, increased liver fibrosis regression rate, normalization of alanine aminotransferase (ALT) levels, reduced risk of liver cirrhosis, decompensation and hCC, and increased survival (Ruan et al., 2013).

In patients receiving NUC therapy, HBsAg decrease is generally very low; even with long-term therapy (5-7 years) (Buti et al., 2015). It has been shown that even when HBV replication is well-controlled, HBsAg clearance is unlikely to occur during a patient's lifetime (Chevaliez et al., 2013). With this very low rate of HBsAg loss and the high rate of viral rebound and biochemical relapse that commonly occurs with discontinuation, lifelong NUC therapy is generally recommended for the majority of patients.

Thus, the current drugs are not satisfactory and there is strong interest in new therapies with novel mode of action for the treatment of HBV. For example, blocking viral replication in newly infected hepatocytes and preventing cccDNA production, suppressing cccDNA, and/or blocking cccDNA transcriptional activity. Fused-tricyclic compounds for use against HBV infections have been disclosed, for example in WO 2017/205115, WO 2017/140821, WO

2017/114812, WO 2017/108630, WO 2017/102648, WO 2017/017043, WO 2017/013046 and elsewhere.

## SUMMARY OF THE INVENTION

The present invention is based on the identification of novel compounds, for the prophylaxis and/or treatment of HBV. The present invention also provides methods for the production of these compounds and pharmaceutical compositions comprising these compounds. Disclosed are methods for the prophylaxis and/or treatment of HBV by administering the compounds of the invention.

Accordingly, in a first aspect of the invention, the compounds of the invention are provided having a Formula I:

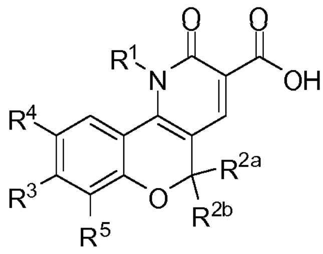

I

wherein

$R^1$ is $C_{3-6}$ cycloalkyl optionally substituted with one or more independently selected halo;
Each $R^{2a}$ and $R^{2b}$ is independently selected from:

- H,
- $C_{1-4}$ alkyl optionally substituted with one or more independently selected
    - halo,
    - -OH,
    - -CN,
        - $C_{1-4}$ alkoxy,
        - $C_{1-4}$ thioalkoxy, or
    - $-SO_2-C_1-_4$ alkyl;
- $C_{3-7}$ cycloalkyl optionally substituted with one or more independently selected
    - -CN,
    - halo,
    - -OH,
    - $-SO_2-C_1-_4$ alkyl,
    - $C_{1-4}$ alkyl optionally substituted with one or more independently selected halo, or
    - $C_{1-4}$ alkoxy optionally substituted with one or more independently selected halo; and
- 4-7 membered monocyclic heterocycloalkyl comprising one or more independently selected O, N, or S heteroatoms, optionally substituted with one or more independently selected
    - -CN,
    - halo,
    - -OH,
    - $-SO_2-C_1-_4$ alkyl,
    - =O,
    - $C_{1-4}$ alkyl optionally substituted with one or more independently selected halo, or
    - $C_{1-4}$ alkoxy optionally substituted with one or more independently selected halo;

$R^3$ is

- $C_{1-4}$ Alkyl optionally substituted with one or more independently selected
  - ◦ halo,
  - ◦ CN,
  - ◦ -OH,
  - ◦ $SO_2$-$C_{1-4}$ alkyl,
  - ◦ $C_{1-4}$ alkoxy, or
  - ◦ 4-7 membered monocyclic heterocycloalkyl comprising one or more independently selected O, N, or S heteroatoms,
- halo,
- -CN, or
- $C_{1-4}$ alkoxy optionally substituted with one or more independently selected $R^7$ groups;

$R^4$ is

- H,
- -$SO_2$-$C_1$-$_4$ alkyl,
- halo,
- CN
- $C_{1-4}$ alkyl optionally substituted with one or more independently selected halo, or
- $C_{1-4}$ alkoxy;

$R^5$ is H, -CN, halo, or $C_{1-4}$ alkyl optionally substituted with one or more independently selected halo; and each $R^{6a}$, $R^{6b}$, $R^{6c}$, and $R^{6d}$ is independently h or $C_{1-4}$ alkyl;
each $R^7$ is independently

- halo,
- -CN,
- -OH,
- -$SO_2$-$C_1$-$_4$ alkyl,
- $C_{1-4}$ alkoxy,
- phenyl,
- 5-6 membered monocyclic heteroaryl comprising one or more independently selected O, N, or S heteroatoms,
- $C_{3-7}$ cycloalkyl optionally substituted with one or more independently selected $R^{8a}$,
- monocyclic 4-7 membered heterocycloalkyl comprising one or more independently selected O, N, or S, optionally substituted with one or more independently selected $R^{8b}$, and
- fused/spiro/bridged 4-10 membered heterocycloalkyl comprising one or more independently selected O, N, or S, optionally substituted with one or more independently selected $R^{8c}$;

Each $R^{8a}$, $R^{8b}$, and $R^{8c}$ is independently selected from:

- halo,
- -CN,
- -$SO_2$-$C_1$-$_4$ alkyl,
- =O,
- alkyl optionally substituted with one or more independently selected halo, or -OH,
- -C(=O)$NR^{6a}R^{6b}$,
- -$C_{1-4}$ alkoxy, or
- -C(=O) $C_{1-4}$ alkyl.

In a particular aspect, the compounds of the invention are provided for use in the prophylaxis and / or treatment of hepatitis B.

Furthermore, it has also been unexpectedly demonstrated that the compounds of the invention exhibit good potency and exposure *in vivo*. This may result in low dosages regimen, and suitability for use alone or in combination with other medicaments.

In a further aspect, the present invention provides pharmaceutical compositions comprising a compound of the invention, and a pharmaceutical carrier, excipient or diluent. In a particular aspect, the pharmaceutical composition may additionally comprise further therapeutically active ingredients suitable for use in combination with the compounds of the invention. In a more particular aspect, the further therapeutically active ingredient is an agent for the treatment of HBV.

Moreover, the compounds of the invention, useful in the pharmaceutical compositions and treatment methods disclosed herein, are pharmaceutically acceptable as prepared and used.

In a further aspect of the disclsoure, this invention provides a method of treating a mammal, in particular humans, afflicted with a condition selected from among those listed herein, and particularly HBV, which method comprises administering an effective amount of the pharmaceutical composition or compounds of the invention as described herein.

The present invention also provides pharmaceutical compositions comprising a compound of the invention, and a suitable pharmaceutical carrier, excipient or diluent for use in medicine. In a particular aspect, the pharmaceutical composition is for use in the prophylaxis and/or treatment of HBV.

In additional aspects, this invention provides methods for synthesizing the compounds of the invention, with representative synthetic protocols and pathways disclosed later on herein.

Other objects and advantages will become apparent to those skilled in the art from a consideration of the ensuing detailed description.

It will be appreciated that compounds of the invention may be metabolized to yield biologically active metabolites.

## BRIEF DESCRIPTION OF THE DRAWING

**FIGURE 1** refers to Example 3 and shows the infected mice HBsAg blood level for the disease vehicle group (filled diamonds), the group treated with illustrative compound 46 at 0.3 mg/kg p.o. q.d. (asterisks), the group treated with illustrative compound 46 at 3 mg/kg p.o. q.d. (asterisks), the group treated with illustrative compound 46 at 30 mg/kg p.o. q.d. (filled squares), the group treated with illustrative compound 44 at 1 mg/kg p.o. q.d. (crosses), and the group treated with illustrative compound 44 at 10 mg/kg p.o. q.d. (filled circles).

## DETAILED DESCRIPTION OF THE INVENTION

### Definitions

The following terms are intended to have the meanings presented therewith below and are useful in understanding the description and intended scope of the present invention.

When describing the invention, which may include compounds, pharmaceutical compositions containing such compounds and methods of using such compounds and compositions, the following terms, if present, have the following meanings unless otherwise indicated. It should also be understood that when described herein any of the moieties defined forth below may be substituted with a variety of substituents, and that the respective definitions are intended to include such substituted moieties within their scope as set out below. Unless otherwise stated, the term "substituted" is to be defined as set out below. It should be further understood that the terms "groups" and "radicals" can be considered interchangeable when used herein.

The articles 'a' and 'an' may be used herein to refer to one or to more than one (*i.e.* at least one) of the grammatical objects of the article. By way of example 'an analogue' means one analogue or more than one analogue.

'Alkyl' means straight or branched aliphatic hydrocarbon having the specified number of carbon atoms. Particular alkyl groups have 1 to 6 carbon atoms or 1 to 4 carbon atoms. Branched means that one or more alkyl groups such as methyl, ethyl or propyl is attached to a linear alkyl chain. Particular alkyl groups are methyl ($-CH_3$), ethyl ($-CH_2-CH_3$), n-propyl ($-CH_2-CH_2-CH_3$), isopropyl ($-CH(CH_3)_2$), n-butyl ($-CH_2-CH_2-CH_2-CH_3$), tert-butyl ($-CH_2-C(CH_3)_3$), sec-butyl ($-CH_2-CH(CH_3)_2$), n-pentyl ($-CH_2-CH_2-CH_2-CH_2-CH_3$), n-hexyl ($-CH_2-CH_2-CH_2-CH_2-CH_2-CH_3$), and 1,2-dimethylbutyl ($-CHCH_3)-C(CH_3)H_2-CH_2-CH_3$). Particular alkyl groups have between 1 and 4 carbon atoms.

'Alkenyl' refers to monovalent olefinically (unsaturated) hydrocarbon groups with the number of carbon atoms specified. Particular alkenyl has 2 to 8 carbon atoms, and more particularly, from 2 to 6 carbon atoms, which can be straight-chained or branched and having at least 1 and particularly from 1 to 2 sites of olefinic unsaturation. Particular alkenyl groups include ethenyl ($-CH=CH_2$), n-propenyl ($-CH_2CH=CH_2$), isopropenyl ($-C(CH_3)=CH_2$) and the like.

'Alkylene' refers to divalent alkene radical groups having the number of carbon atoms specified, in particular having 1 to 6 carbon atoms and more particularly 1 to 4 carbon atoms which can be straight-chained or branched. This term is exemplified by groups such as methylene ($-CH_2-$), ethylene ($-CH_2-CH_2-$), or $-CH(CH_3)-$ and the like.

'Alkynylene' refers to divalent alkyne radical groups having the number of carbon atoms and the number of triple bonds specified, in particular 2 to 6 carbon atoms and more particularly 2 to 4 carbon atoms which can be straight-chained or branched. This term is exemplified by groups such as $-C\equiv C-$, $-CH_2-C\equiv C-$, and $-C(CH_3)H-C\equiv CH-$.

'Alkoxy' refers to the group O-alkyl, where the alkyl group has the number of carbon atoms specified. In particular

the term refers to the group -O-$C_{1-6}$ alkyl. Particular alkoxy groups are methoxy, ethoxy, n-propoxy, isopropoxy, n-butoxy, tert-butoxy, sec-butoxy, n-pentoxy, n-hexoxy, and 1,2-dimethylbutoxy. Particular alkoxy groups are lower alkoxy, i.e. with between 1 and 6 carbon atoms. Further particular alkoxy groups have between 1 and 4 carbon atoms.

'Amino' refers to the radical -$NH_2$.

'Aryl' refers to a monovalent aromatic hydrocarbon group derived by the removal of one hydrogen atom from a single carbon atom of a parent aromatic ring system. In particular aryl refers to an aromatic ring structure, monocyclic or fused polycyclic, with the number of ring atoms specified. Specifically, the term includes groups that include from 6 to 10 ring members. Particular aryl groups include phenyl, and naphthyl.

'Cycloalkyl'refers to a non-aromatic hydrocarbyl ring structure, monocyclic, fused polycyclic, bridged polycyclic, or spirocyclic, with the number of ring atoms specified. A cycloalkyl may have from 3 to 12 carbon atoms, in particular from 3 to 10, and more particularly from 3 to 7 carbon atoms. Such cycloalkyl groups include, by way of example, single ring structures such as cyclopropyl, cyclobutyl, cyclopentyl, cyclohexyl, and cycloheptyl.

'Cyano' refers to the radical -CN.

'Halo' or 'halogen' refers to fluoro (F), chloro (Cl), bromo (Br) and iodo (I). Particular halo groups are either fluoro or chloro.

'Hetero' when used to describe a compound or a group present on a compound means that one or more carbon atoms in the compound or group have been replaced by a nitrogen, oxygen, or sulfur heteroatom. hetero may be applied to any of the hydrocarbyl groups described above such as alkyl, e.g. heteroalkyl, cycloalkyl, e.g. heterocycloalkyl, aryl, e.g. heteroaryl, and the like having from 1 to 4, and particularly from 1 to 3 heteroatoms, more typically 1 or 2 heteroatoms, for example a single heteroatom.

'Heteroaryl' means an aromatic ring structure, monocyclic or fused polycyclic, that includes one or more heteroatoms independently selected from O, N and S and the number of ring atoms specified. In particular, the aromatic ring structure may have from 5 to 9 ring members. The heteroaryl group can be, for example, a five membered or six membered monocyclic ring or a fused bicyclic structure formed from fused five and six membered rings or two fused six membered rings or, by way of a further example, two fused five membered rings. Each ring may contain up to four heteroatoms typically selected from nitrogen, sulphur and oxygen. Typically the heteroaryl ring will contain up to 4 heteroatoms, more typically up to 3 heteroatoms, more usually up to 2, for example a single heteroatom. In one embodiment, the heteroaryl ring contains at least one ring nitrogen atom. The nitrogen atoms in the heteroaryl rings can be basic, as in the case of an imidazole or pyridine, or essentially non-basic as in the case of an indole or pyrrole nitrogen. In general the number of basic nitrogen atoms present in the heteroaryl group, including any amino group substituents of the ring, will be less than five.

Examples of five membered monocyclic heteroaryl groups include but are not limited to pyrrolyl, furanyl, thiophenyl, imidazolyl, furazanyl, oxazolyl, oxadiazolyl, oxatriazolyl, isoxazolyl, thiazolyl, isothiazolyl, pyrazolyl, triazolyl and tetrazolyl groups.

Examples of six membered monocyclic heteroaryl groups include but are not limited to pyridinyl, pyrazinyl, pyridazinyl, pyrimidinyl and triazinyl.

Particular examples of bicyclic heteroaryl groups containing a five membered ring fused to another five-membered ring include but are not limited to imidazothiazolyl and imidazoimidazolyl.

Particular examples of bicyclic heteroaryl groups containing a six membered ring fused to a five membered ring include but are not limited to benzofuranyl, benzothiophenyl, benzoimidazolyl, benzoxazolyl, isobenzoxazolyl, benzisoxazolyl, benzothiazolyl, benzoisothiazolyl, isobenzofuranyl, indolyl, isoindolyl, indolizinyl, purinyl (*e.g.* adenine, guanine), indazolyl, pyrazolopyrimidinyl, triazolopyrimidinyl, and pyrazolopyridinyl groups.

Particular examples of bicyclic heteroaryl groups containing two fused six membered rings include but are not limited to quinolinyl, isoquinolinyl, pyridopyridinyl, quinoxalinyl, quinazolinyl, cinnolinyl, phthalazinyl, naphthyridinyl, and pteridinyl groups. Particular heteroaryl groups are those derived from thiophenyl, pyrrolyl, benzothiophenyl, benzofuranyl, indolyl, pyridinyl, quinolinyl, imidazolyl, oxazolyl and pyrazinyl.

Examples of representative heteroaryls include the following:

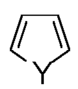 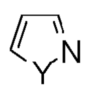 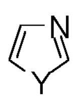 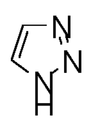 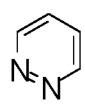 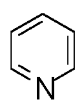 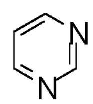 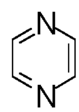

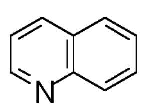 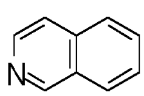 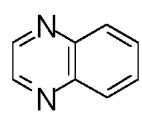 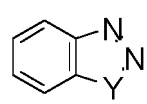 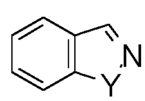 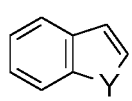

wherein each Y is selected from >C=O, NH, O and S.

'Heterocycloalkyl' means a non-aromatic fully saturated ring structure, monocyclic, fused polycyclic, spirocyclic, or bridged polycyclic, that includes one or more heteroatoms independently selected from O, N and S and the number of ring atoms specified. The heterocycloalkyl ring structure may have from 4 to 12 ring members, in particular from 4 to 10 ring members and more particularly from 4 to 7 ring members. Each ring may contain up to four heteroatoms typically selected from nitrogen, sulphur and oxygen. Typically the heterocycloalkyl ring will contain up to 4 heteroatoms, more typically up to 3 heteroatoms, more usually up to 2, for example a single heteroatom. Examples of heterocyclic rings include, but are not limited to azetidinyl, oxetanyl, thietanyl, pyrrolidinyl (e.g. 1-pyrrolidinyl, 2-pyrrolidinyl and 3-pyrrolidinyl), tetrahydrofuranyl (e.g. 1-tetrahydrofuranyl, 2-tetrahydrofuranyl and 3-tetrahydrofuranyl), tetrahydrothiophenyl (e.g. 1-tetrahydrothiophenyl, 2-tetrahydrothiophenyl and 3-tetrahydrothiophenyl), piperidinyl (e.g. 1-piperidinyl, 2-piperidinyl, 3-piperidinyl and 4-piperidinyl), tetrahydropyranyl (e.g. 4-tetrahydropyranyl), tetrahydrothiopyranyl (e.g. 4-tetrahydrothiopyranyl), morpholinyl, thiomorpholinyl, dioxanyl, or piperazinyl.

As used herein, the term 'heterocycloalkenyl' means a 'heterocycloalkyl', which comprises at least one double bond. Particular examples of heterocycloalkenyl groups are shown in the following illustrative examples:

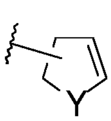 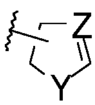 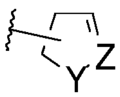 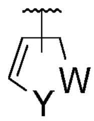 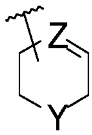 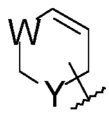 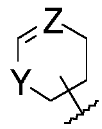 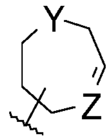

wherein each W is selected from $CH_2$, NH, O and S; each Y is selected from NH, O, C(=O),$-SO_2$, and S; and each Z is selected from N or CH.

Particular examples of monocyclic rings are shown in the following illustrative examples:

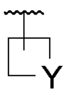 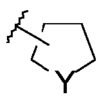 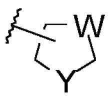 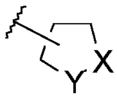 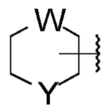 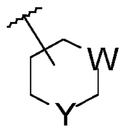 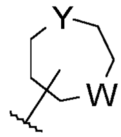

wherein each W and Y is independently selected from $-CH_2-$, -NH-, -O- and -S-.

Particular examples of fused bicyclic rings are shown in the following illustrative examples:

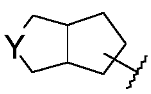 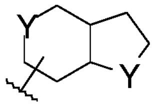 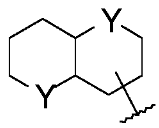 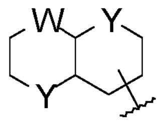

wherein each W and Y is independently selected from $-CH_2-$, -NH-, -O- and -S-.

Particular examples of bridged bicyclic rings are shown in the following illustrative examples:

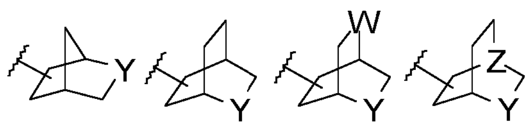

wherein each W and Y is independently selected from $-CH_2-$, -NH-, -O- and -S- and each Z is selected from N or CH.

Particular examples of spirocyclic rings are shown in the following illustrative examples:

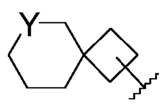 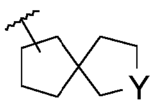 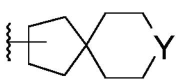

wherein each Y is selected from $-CH_2-$, -NH-, -O- and -S-.

'Hydroxyl' refers to the radical -OH.

'Oxo' refers to the radical =0.

'Substituted' refers to a group in which one or more hydrogen atoms are each independently replaced with the same or different substituent(s).

'Sulfo' or 'sulfonic acid' refers to a radical such as $-SO_3H$.

'Thiol' refers to the group -SH.

As used herein, term 'substituted with one or more' refers to one to four substituents. In one embodiment it refers to one to three substituents. In further embodiments it refers to one or two substituents. In a yet further embodiment it refers to one substituent.

'Thioalkoxy' refers to the group -S-alkyl where the alkyl group has the number of carbon atoms specified. In particular the term refers to the group $-S-C_{1-6}$ alkyl. Particular thioalkoxy groups are thiomethoxy, thioethoxy, n-thiopropoxy, isothiopropoxy, n-thiobutoxy, tert-thiobutoxy, sec-thiobutoxy, n-thiopentoxy, n-thiohexoxy, and 1,2-dimethylthiobutoxy. Particular thioalkoxy groups are lower thioalkoxy, *i.e.* with between 1 and 6 carbon atoms. Further particular alkoxy groups have between 1 and 4 carbon atoms.

One having ordinary skill in the art of organic synthesis will recognize that the maximum number of heteroatoms in a stable, chemically feasible heterocyclic ring, whether it is aromatic or non-aromatic, is determined by the size of the ring, the degree of unsaturation and the valence of the heteroatoms. In general, a heterocyclic ring may have one to four heteroatoms so long as the heteroaromatic ring is chemically feasible and stable.

'Pharmaceutically acceptable' means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

'Pharmaceutically acceptable salt' refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.* an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like. The term 'pharmaceutically acceptable cation' refers to an acceptable cationic counter-ion of an acidic functional group. Such cations are exemplified by sodium, potassium, calcium, magnesium, ammonium, tetraalkylammonium cations, and the like.

'Pharmaceutically acceptable vehicle' refers to a diluent, adjuvant, excipient or carrier with which a compound of the invention is administered.

'Prodrugs' refers to compounds, including derivatives of the compounds of the invention, which have cleavable groups and become by solvolysis or under physiological conditions the compounds of the invention which are pharmaceutically active *in vivo.* Such examples include, but are not limited to, choline ester derivatives and the like, N-alkylmorpholine esters and the like.

'Solvate' refers to forms of the compound that are associated with a solvent, usually by a solvation reaction. This physical association includes hydrogen bonding. Conventional solvents include water, EtOH, acetic acid and the like. The compounds of the invention may be prepared *e.g.* in crystalline form and may be solvated or hydrated. Suitable solvates include pharmaceutically acceptable solvates, such as hydrates, and further include both stoichiometric solvates and non-stoichiometric solvates. In certain instances the solvate will be capable of isolation, for example when one or more solvent molecules are incorporated in the crystal lattice of the crystalline solid. 'Solvate' encompasses both solution-phase and isolable solvates. Representative solvates include hydrates, ethanolates and methanolates.

'Subject' includes humans. The terms 'human', 'patient' and 'subject' are used interchangeably herein.

'Effective amount' means the amount of a compound of the invention that, when administered to a subject for treating a disease, is sufficient to effect such treatment for the disease. The "effective amount" can vary depending on the compound, the disease and its severity, and the age, weight, etc., of the subject to be treated.

'Preventing' or 'prevention' refers to a reduction in risk of acquiring or developing a disease or disorder (*i.e.* causing at least one of the clinical symptoms of the disease not to develop) in a subject that may be exposed to a disease-causing agent, or predisposed to the disease in advance of disease onset.

The term 'prophylaxis' is related to 'prevention', and refers to a measure or procedure the purpose of which is to prevent, rather than to treat or cure a disease. Non-limiting examples of prophylactic measures may include the administration of vaccines; the administration of low molecular weight heparin to hospital patients at risk for thrombosis due, for example, to immobilization; and the administration of an anti-malarial agent such as chloroquine, in advance of a visit to a geographical region where malaria is endemic or the risk of contracting malaria is high.

'Treating' or 'treatment' of any disease or disorder refers, in one embodiment, to ameliorating the disease or disorder (*i.e.* arresting the disease or reducing the manifestation, extent or severity of at least one of the clinical symptoms thereof). In another embodiment 'treating' or 'treatment' refers to ameliorating at least one physical parameter, which may not be discernible by the subject. In yet another embodiment, 'treating' or 'treatment' refers to modulating the disease or disorder, either physically, (*e.g.* stabilization of a discernible symptom), physiologically, (*e.g.* stabilization of a physical parameter), or both. In a further embodiment, "treating" or "treatment" relates to slowing the progression of the disease.

As used herein the term 'inflammatory disease(s)' refers to the group of conditions including, rheumatoid arthritis, osteoarthritis, juvenile idiopathic arthritis, psoriasis, psoriatic arthritis, ankylosing spondylitis, allergic airway disease (*e.g.* asthma, rhinitis), chronic obstructive pulmonary disease (COPD), inflammatory bowel diseases (*e.g.* Crohn's disease, ulcerative colitis), endotoxin-driven disease states (*e.g.* complications after bypass surgery or chronic endotoxin states contributing to *e.g.* chronic cardiac failure), and related diseases involving cartilage, such as that of the joints. Particularly the term refers to rheumatoid arthritis, osteoarthritis, allergic airway disease (*e.g.* asthma), chronic obstructive pulmonary disease (COPD) and inflammatory bowel diseases. More particularly the term refers to rheumatoid arthritis, chronic obstructive pulmonary disease (COPD) and inflammatory bowel diseases

As used herein the term "HBV" or "Hepatitis B virus" refers to a species of viruses of the genus Orthohepadnavirus. This virus causes hepatitis B.

As used herein the term 'HBcAg' refers to hepatitis B virus core antigen. It is an indicator of hepatitis B viral replication. This antigen forms the inner capsid of the hepatitis B virus. HBcAg may be secreted into blood as naked capsids.

As used herein the term 'HBeAg' refers to a particular secreted hepatitis B viral protein and its presence in the serum of patients can serve as a marker of active replication of the hepatitis B virus in chronic hepatitis patients.

As used herein the term 'HBsAg' refers to the hepatitis B viral surface antigen family of three proteins, which form the outer protein coat of the HBV virion. It is secreted into blood both as a part of viral particles, but also as non-infectious protein complexes containing only HBsAg, and can serve as a marker of active replication of the hepatitis B virus in chronic hepatitis patients.

As used herein the term 'HBV DNA' refers to the DNA of HBV. This DNA is often found in the blood. It is secreted into the blood only inside viral particles, and can be used as a marker of active replication of hepatitis B virus in chronically infected patients.

'Compound(s) of the invention', and equivalent expressions, are meant to embrace compounds of the Formula(e) as herein described, which expression includes the pharmaceutically acceptable salts, and the solvates, *e.g.* hydrates, and the solvates of the pharmaceutically acceptable salts where the context so permits. Similarly, reference to intermediates, whether or not they themselves are claimed, is meant to embrace their salts, and solvates, where the context so permits.

When ranges are referred to herein, for example but without limitation, $C_{1-8}$ alkyl, the citation of a range should be considered a representation of each member of said range.

Other derivatives of the compounds of this invention have activity in both their acid and acid derivative forms, but in the acid sensitive form often offers advantages of solubility, tissue compatibility, or delayed release in the mammalian organism (Bundgard, H, 1985). Prodrugs include acid derivatives well known to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this invention are particularly useful prodrugs. In some cases it is desirable to prepare double ester type prodrugs such as (acyloxy)alkyl esters or ((alkoxycarbonyl)oxy)alkylesters. Particular such prodrugs are the $C_{1-8}$ alkyl, $C_{2-8}$ alkenyl, $C_{6-10}$ optionally substituted aryl, and ($C_{6-10}$ aryl)-($C_{1-4}$ alkyl) esters of the compounds of the invention.

The present disclosure includes all isotopic forms of the compounds of the invention provided herein, whether in a form (i) wherein all atoms of a given atomic number have a mass number (or mixture of mass numbers) which predominates in nature (referred to herein as the 'natural isotopic form') or (ii) wherein one or more atoms are replaced by atoms having the same atomic number, but a mass number different from the mass number of atoms which predominates in nature (referred to herein as an 'unnatural variant isotopic form'). It is understood that an atom may naturally exists as a mixture of mass numbers. The term "unnatural variant isotopic form" also includes embodiments in which the proportion of an atom of given atomic number having a mass number found less commonly in nature (referred to herein as an 'uncommon isotope') has been increased relative to that which is naturally occurring e.g. to the level of >20%, >50%, >75%, >90%, >95% or> 99% by number of the atoms of that atomic number (the latter embodiment

referred to as an 'isotopically enriched variant form'). The term 'unnatural variant isotopic form' also includes embodiments in which the proportion of an uncommon isotope has been reduced relative to that which is naturally occurring. Isotopic forms may include radioactive forms (i.e. they incorporate radioisotopes) and non-radioactive forms. Radioactive forms will typically be isotopically enriched variant forms.

An unnatural variant isotopic form of a compound may thus contain one or more artificial or uncommon isotopes such as deuterium ($^2H$ or D), carbon-11 ($^{11}C$), carbon-13 ($^{13}C$), carbon-14 ($^{14}C$), nitrogen-13 ($^{13}N$), nitrogen-15 ($^{15}N$), oxygen-15 ($^{15}O$), oxygen-17 ($^{17}O$), oxygen-18 ($^{18}O$), phosphorus-32 ($^{32}P$), sulphur-35 ($^{35}S$), chlorine-36 ($^{36}Cl$), chlorine-37 ($^{37}Cl$), fluorine-18 ($^{18}F$) iodine-123 ($^{123}I$), iodine-125 ($^{125}I$) in one or more atoms or may contain an increased proportion of said isotopes as compared with the proportion that predominates in nature in one or more atoms.

Unnatural variant isotopic forms comprising radioisotopes may, for example, be used for drug and/or substrate tissue distribution studies. The radioactive isotopes tritium, i.e. $^3H$, and carbon-14, i.e. $^{14}C$, are particularly useful for this purpose in view of their ease of incorporation and ready means of detection. Unnatural variant isotopic forms which incorporate deuterium i.e. $^2H$ or D may afford certain therapeutic advantages resulting from greater metabolic stability, for example, increased in vivo half-life or reduced dosage requirements, and hence may be preferred in some circumstances. Further, unnatural variant isotopic forms may be prepared which incorporate positron emitting isotopes, such as $^{11}C$, $^{18}F$, $^{15}0$ and $^{13}N$, and would be useful in Positron Emission Topography (PET) studies for examining substrate receptor occupancy.

It is also to be understood that compounds that have the same molecular formula but differ in the nature or sequence of bonding of their atoms or the arrangement of their atoms in space are termed 'isomers'. Isomers that differ in the arrangement of their atoms in space are termed 'stereoisomers'.

Stereoisomers that are not mirror images of one another are termed 'diastereomers' and those that are non-superimposable mirror images of each other are termed 'enantiomers'. When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e. as (+) or (-)-isomers respectively). A chiral compound can exist as either individual enantiomer or as a mixture thereof. A mixture containing equal proportions of the enantiomers is called a 'racemic mixture'.

'Tautomers' refer to compounds that are interchangeable forms of a particular compound structure, and that vary in the displacement of hydrogen atoms and electrons. Thus, two structures may be in equilibrium through the movement of $\pi$ electrons and an atom (usually h). For example, enols and ketones are tautomers because they are rapidly interconverted by treatment with either acid or base. Another example of tautomerism is the aci- and nitro- forms of phenylnitromethane, that are likewise formed by treatment with acid or base.

Tautomeric forms may be relevant to the attainment of the optimal chemical reactivity and biological activity of a compound of interest.

The compounds of the invention may possess one or more asymmetric centers; such compounds can therefore be produced as individual (R)- or (S)- stereoisomers or as mixtures thereof.

Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art.

It will be appreciated that compounds of the invention may be metabolized to yield biologically active metabolites.

## THE INVENTION

The present invention is based on the identification of novel compounds, for the prophylaxis and/or treatment of HBV. The present invention also provides methods for the production of these compounds and pharmaceutical compositions comprising these compounds. Disclosed are methods for the prophylaxis and/or treatment of HBV by administering the compounds of the invention.

Accordingly, in a first aspect of the invention, the compounds of the invention are provided having a Formula I:

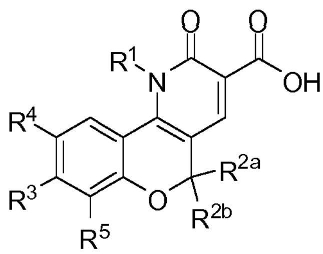

I

wherein

$R^1$ is $C_{3-6}$ cycloalkyl optionally substituted with one or more independently selected halo; Each $R^{2a}$ and $R^{2b}$ is independently selected from:

- H,
- $C_{1-4}$ alkyl optionally substituted with one or more independently selected
    - halo,
    - -OH,
    - -CN,
    - $C_{1-4}$ alkoxy,
    - $C_{1-4}$ thioalkoxy, or
    - $-SO_2-C_1-_4$ alkyl;
- $C_{3-7}$ cycloalkyl optionally substituted with one or more independently selected
    - -CN,
    - halo,
    - -OH,
    - $-SO_2-C_1-_4$ alkyl,
    - $C_{1-4}$ alkyl optionally substituted with one or more independently selected halo, or
    - $C_{1-4}$ alkoxy optionally substituted with one or more independently selected halo; and
- 4-7 membered monocyclic heterocycloalkyl comprising one or more independently selected O, N, or S heteroatoms, optionally substituted with one or more independently selected
    - -CN,
    - halo,
    - -OH,
    - $-SO_2-C_1-_4$ alkyl,
    - =O,
    - $C_{1-4}$ alkyl optionally substituted with one or more independently selected halo, or
    - $C_{1-4}$ alkoxy optionally substituted with one or more independently selected halo;

$R^3$ is

- $C_{1-4}$ alkyl optionally substituted with one or more independently selected
    - halo,
    - CN,
    - -OH,
    - $SO_2-C_{1-4}$ alkyl,
    - $C_{1-4}$ alkoxy, or
    - 4-7 membered monocyclic heterocycloalkyl comprising one or more independently selected O, N, or S heteroatoms,

- halo,
- -CN, or
- $C_{1-4}$ alkoxy optionally substituted with one or more independently selected $R^7$ groups;

$R^4$ is

- H,
- $-SO_2-C_1-_4$ alkyl,
- halo,
- CN,
- $C_{1-4}$ alkyl optionally substituted with one or more independently selected halo, or
- $C_{1-4}$ alkoxy;

$R^5$ is H, -CN, halo, or $C_{1-4}$ alkyl optionally substituted with one or more independently selected halo; and each $R^{6a}$, $R^{6b}$, $R^{6c}$, and $R^{6d}$ is independently h or $C_{1-4}$ alkyl;
each $R^7$ is independently

- halo,
- -CN,
- -OH,
- $-SO_2-C_1-_4$ alkyl,
- $C_{1-4}$ alkoxy,
- phenyl,
- 5-6 membered monocyclic heteroaryl comprising one or more independently selected O, N, or S heteroatoms,
- $C_{3-7}$ cycloalkyl optionally substituted with one or more independently selected $R^{8a}$,
- monocyclic 4-7 membered heterocycloalkyl comprising one or more independently selected O, N, or S, optionally substituted with one or more independently selected $R^{8b}$, and
- fused/spiro/bridged 4-10 membered heterocycloalkyl comprising one or more independently selected O, N, or S, optionally substituted with one or more independently selected $R^{8c}$;

each $R^{8a}$, $R^{8b}$, and $R^{8c}$ is independently selected from:

- halo,
- -CN,
- $-SO_2-C_1-_4$ alkyl,
- =O,
- alkyl optionally substituted with one or more independently selected halo, or -OH,
- $-C(=O)NR^{6a}R^{6b}$,
- $-C_{1-4}$ alkoxy, or
- -C(=O) $C_{1-4}$ alkyl.

In one embodiment, the compound of the invention is according to Formula I, wherein $R^1$ is $C_{3-6}$ cycloalkyl. In a particular embodiment, $R^1$ is cyclopropyl. In another embodiment, $R^1$ is cyclobutyl.

In one embodiment, the compound of the invention is according to Formula I, wherein $R^1$ is $C_{3-6}$ cycloalkyl substituted with one or more independently selected halo. In a particular embodiment, $R^1$ is cyclopropyl, or cyclobutyl, each of which is substituted with one or more independently selected halo. In another embodiment, $R^1$ is $C_{3-6}$ cycloalkyl substituted with one or more independently selected F. In a particular embodiment, $R^1$ is cyclopropyl, or cyclobutyl, each of which is substituted with one, two, or three F.

In one embodiment, $R^5$ is H, -CN, halo, or $C_{1-4}$ alkyl optionally substituted with one or more independently selected halo. In a particular embodiment, $R^5$ is H, -CN, $-CH_3$, $-CF_3$, F or Cl. In a more particular embodiment, $R^5$ is H.

In one embodiment, the compound of the invention is according to Formula II:

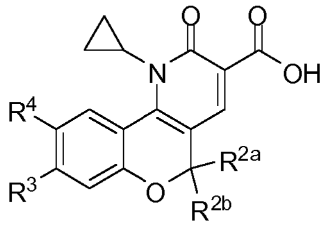

II

wherein $R^{2a}$, $R^{2b}$, $R^3$and $R^4$ are as described previously.

In one embodiment, the compound of the invention is according to Formula I or II, wherein $R^{2a}$ is H.

In one embodiment, the compound of the invention is according to Formula I or II, wherein $R^{2a}$ is $C_{1-4}$ alkyl. In a particular embodiment, $R^{2a}$ is $-CH_3$, $-CH_2CH_3$, $-CH(CH_3)_2$ or $-C(CH_3)_3$. In a more particular embodiment, $R^{2a}$ is $-CH(CH_3)_2$.

In one embodiment, the compound of the invention is according to Formula I or II, wherein $R^{2a}$ is $C_{1-4}$ alkyl substituted with one or more independently selected halo, -OH, -CN, or $C_{1-4}$ alkoxy, $-SO_2-C_{1-4}$ alkyl. In a particular embodiment, $R^{2a}$ is $-CH_3$, $CH_2CH_3$, $-CH(CH_3)_2$ or $-C(CH_3)_3$, each of which is substituted with one or more independently selected halo, -OH, -CN, $C_{1-4}$ alkoxy, $C_{1-4}$ thioalkoxy, or $-SO_2-C_1-_4$ alkyl. In another particular embodiment, $R^{2a}$ is $C_{1-4}$ alkyl substituted with one or more independently selected F, Cl, -OH, -CN, $-OCH_3$, $-OCH_2CH_3$, $-SCH_3$, or $-SO_2CH_3$. In a more particular embodiment, $R^{2a}$ is $-CH_3$, $-CH_2CH_3$, $-CH(CH_3)_2$ or $-C(CH_3)_3$, each of which is substituted with one or more independently selected F, Cl, -OH, -CN, $-OCH_3$, $-OCH_2CH_3$, $-SCH_3$, or $-SO_2CH_3$. In a most particular embodiment, $R^{2a}$ is $-CH_2OH$, or $-C(CH_3)_2CH_2OCH_3$.

In one embodiment, the compound of the invention is according to Formula I or II, wherein $R^{2a}$ is $C_{1-4}$ alkyl substituted with one or more independently selected halo, -OH, -CN, or $C_{1-4}$ alkoxy, $-SO_2-C_1-_4$ alkyl. In a particular embodiment, $R^{2a}$ is $-CH_3$, $CH_2CH_3$, $-CH(CH_3)_2$ or $-C(CH_3)_3$, each of which is substituted with one or more independently selected halo, -OH, -CN, or $C_{1-4}$ alkoxy, $-SO_2-C_1-_4$ alkyl. In another particular embodiment, $R^{2a}$ is $C_{1-4}$ alkyl substituted with one or more independently selected F, Cl, -OH, -CN, $-OCH_3$, $-OCH_2CH_3$, or $-SO_2CH_3$. In a more particular embodiment, $R^{2a}$ is $-CH_3$, $-CH_2CH_3$, $-CH(CH_3)_2$ or $-C(CH_3)_3$, each of which is substituted with one or more independently selected F, Cl, -OH, -CN, $-OCH_3$, $-OCH_2CH_3$, or $-SO_2CH_3$. In a most particular embodiment, $R^{2a}$ is $-CH_2OH$, or $-C(CH_3)_2CH_2OCH_3$.

In one embodiment, the compound of the invention is according to Formula I or II, wherein $R^{2a}$ is $C_{3-7}$ cycloalkyl. In a particular embodiment, $R^{2a}$ is cyclopropyl, cyclobutyl, cyclopentyl, or cyclohexyl. In a more particular embodiment, $R^{2a}$ is cyclopropyl or cyclobutyl.

In one embodiment, the compound of the invention is according to Formula I or II, wherein $R^{2a}$ is $C_{3-7}$ cycloalkyl substituted with one or more independently selected -CN, halo, -OH, $-SO_2-C_1-_4$ alkyl, $C_{1-4}$ alkyl optionally substituted with one or more independently selected halo, or $C_{1-4}$ alkoxy optionally substituted with one or more independently selected halo. In a particular embodiment, $R^{2a}$ is cyclopropyl, cyclobutyl, cyclopentyl, or cyclohexyl, each of which is substituted with one or more independently selected -CN, halo, -OH, $-SO_2-C_1-_4$ alkyl, $C_{1-4}$ alkyl optionally substituted with one or more independently selected halo, or $C_{1-4}$ alkoxy optionally substituted with one or more independently selected halo. In another particular embodiment, $R^{2a}$ is cyclopropyl, cyclobutyl, cyclopentyl, or cyclohexyl, each of which is substituted with one or more independently selected -CN, F, Cl, -OH, $-SO_2-CH_3$, $-CH_3$, $-CH_2CH_3$, $-CF_3$, $-CHF_2$, $-CH_2CF_3$, $-OCH_3$, $-OCH_2CH_3$, or $-OCF_3$. In a more particular embodiment, $R^{2a}$ is cyclopropyl substituted with one -CN, F, Cl, -OH, $-SO_2-CH_3$, $-CH_3$, $-CH_2CH_3$, $-CF_3$, $CHF_2$, $-CH_2CF_3$, $-OCH_3$, $-OCH_2CH_3$, or $-OCF_3$. In a most particular embodiment, $R^{2a}$ is cyclopropyl substituted with one -CN.

In one embodiment, the compound of the invention is according to Formula I or II, wherein $R^{2a}$ is 4-7 membered monocyclic heterocycloalkyl comprising one or more independently selected O, N, or S heteroatoms. In a particular embodiment, $R^{2a}$ is oxetanyl.

In one embodiment, the compound of the invention is according to Formula I or II, wherein $R^{2a}$ is 4-7 membered monocyclic heterocycloalkyl comprising one or more independently selected O, N, or S heteroatoms, substituted with one or more independently selected-CN, halo, -OH, $-SO_2-C_{1-4}$ alkyl, =O, $C_{1-4}$ alkyl optionally substituted with one or more independently selected halo, or $C_{1-4}$ alkoxy optionally substituted with one or more independently selected halo. In a particular embodiment, $R^{2a}$ is oxetanyl substituted with one or more independently selected -CN, halo, -OH, $-SO_2-C_{1-4}$ alkyl, =O, $C_{1-4}$ alkyl optionally substituted with one or more independently selected halo, or $C_{1-4}$ alkoxy optionally substituted with one or more independently selected halo. In another particular embodiment, $R^{2a}$ is 4-7 membered monocyclic heterocycloalkyl comprising one or more independently selected O, N, or S heteroatoms, substituted with one or more independently selected -CN, F, -OH, $-SO_2-CH_3$, =O, $-CH_3$, $-CH_2CH_3$, $-CF_3$, $-CHF_2$, $-CH_2CF_3$, $-OCH_3$, $-OCH_2CH_3$, or $-OCF_3$. In a more particular embodiment, $R^{2a}$ is oxetanyl substituted with one or more independently selected -CN, F,

-OH, $-SO_2-CH_3$, =O, $-CH_3$, $-CH_2CH_3$, $-CF_3$, $-CHF_2$, $-CH_2CF_3$, $-OCH_3$, $-OCH_2CH_3$, or $-OCF_3$. In a most particular embodiment, $R^{2a}$ is oxetanyl substituted with one $-CH_3$.

In one embodiment, the compound of the invention is according to Formula III:

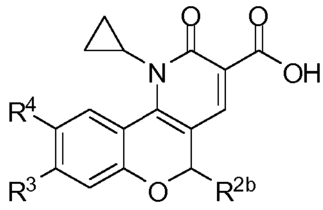

III

wherein $R^{2b}$, $R^3$ and $R^4$ are as described previously.

In one embodiment, the compound of the invention is according to Formula I, II or III, wherein $R^{2b}$ is H.

In one embodiment, the compound of the invention is according to Formula I, II or III, wherein $R^{2b}$ is $C_{1-4}$ alkyl. In a particular embodiment, $R^{2b}$ is $-CH_3$, $-CH_2CH_3$, $-CH(CH_3)_2$ or $-C(CH_3)_3$. In a more particular embodiment, $R^{2b}$ is $-CH(CH_3)_2$.

In one embodiment, the compound of the invention is according to Formula I or II, wherein $R^{2b}$ is $C_{1-4}$ alkyl substituted with one or more independently selected halo, -OH, -CN, or $C_{1-4}$ alkoxy, $-SO_2-C_1-_4$ alkyl. In a particular embodiment, $R^{2b}$ is $-CH_3$, $CH_2CH_3$, $-CH(CH_3)_2$ or $-C(CH_3)_3$, each of which is substituted with one or more independently selected halo, -OH, -CN, $C_{1-4}$ alkoxy, $C_{1-4}$ thioalkoxy, or $-SO_2-C_1-_4$ alkyl. In another particular embodiment, $R^{2b}$ is $C_{1-4}$ alkyl substituted with one or more independently selected F, Cl, -OH, -CN, $-OCH_3$, $-OCH_2CH_3$, $-SCH_3$, or $-SO_2CH_3$. In a more particular embodiment, $R^{2b}$ is $-CH_3$, $CH_2CH_3$, $-CH(CH_3)_2$ or $-C(CH_3)_3$., each of which is substituted with one or more independently selected F, Cl, -OH, -CN, $-OCH_3$, $-OCH_2CH_3$, $-SCH_3$, or $-SO_2CH_3$. In a most particular embodiment, $R^{2b}$ is $-CH_2OH$, or $-C(CH_3)_2CH_2OCH_3$.

In one embodiment, the compound of the invention is according to Formula I, II or III, wherein $R^{2b}$ is $C_{1-4}$ alkyl substituted with one or more independently selected halo, -OH, -CN, or $C_{1-4}$ alkoxy, $-SO_2-C_1-_4$ alkyl. In a particular embodiment, $R^{2b}$ is $-CH_3$, $CH_2CH_3$, $-CH(CH_3)_2$ or-$C(CH_3)_3$, each of which is substituted with one or more independently selected halo, -OH, -CN, or $C_{1-4}$ alkoxy, $-SO_2-C_{1-4}$ alkyl. In another particular embodiment, $R^{2b}$ is $C_{1-4}$ alkyl substituted with one or more independently selected F, Cl, -OH, -CN, $-OCH_3$, $-OCH_2CH_3$, or $-SO_2CH_3$. In a more particular embodiment, $R^{2b}$ is $-CH_3$, $-CH_2CH_3$, $-CH(CH_3)_2$ or $-C(CH_3)_3$, each of which is substituted with one or more independently selected F, Cl, -OH, -CN, $-OCH_3$, $-OCH_2CH_3$, or $-SO_2CH_3$. In a most particular embodiment, $R^{2b}$ is $-CH_2OH$, or $-C(CH_3)_2CH_2OCH_3$.

In one embodiment, the compound of the invention is according to Formula I, II or III, wherein $R^{2b}$ is $C_{3-7}$ cycloalkyl. In a particular embodiment, $R^{2b}$ is cyclopropyl, cyclobutyl, cyclopentyl, or cyclohexyl. In a more particular embodiment, $R^{2b}$ is cyclopropyl or cyclobutyl.

In one embodiment, the compound of the invention is according to Formula I, II or III, wherein $R^{2b}$ is $C_{3-7}$ cycloalkyl substituted with one or more independently selected -CN, halo, -OH, $-SO_2-C_{1-4}$ alkyl, $C_{1-4}$ alkyl optionally substituted with one or more independently selected halo, or $C_{1-4}$ alkoxy optionally substituted with one or more independently selected halo. In a particular embodiment, $R^{2b}$ is cyclopropyl, cyclobutyl, cyclopentyl, or cyclohexyl, each of which is substituted with one or more independently selected -CN, halo, -OH, $-SO_2-C_{1-4}$ alkyl, $C_{1-4}$ alkyl optionally substituted with one or more independently selected halo, or $C_{1-4}$ alkoxy optionally substituted with one or more independently selected halo. In another particular embodiment, $R^{2b}$ is cyclopropyl, cyclobutyl, cyclopentyl, or cyclohexyl, each of which is substituted with one or more independently selected -CN, F, Cl, -OH, $-SO_2-CH_3$, $-CH_3$, $-CH_2CH_3$, $-CF_3$, $CHF_2$, $-CH_2CF_3$, $-OCH_3$, $-OCH_2CH_3$, or $-OCF_3$. In a more particular embodiment, $R^{2b}$ is cyclopropyl substituted with one -CN, F, Cl, -OH, $-SO_2-CH_3$, $-CH_3$, $-CH_2CH_3$, $-CF_3$, $-CHF_2$, $-CH_2CF_3$, $-OCH_3$, $-OCH_2CH_3$, or $-OCF_3$. In a most particular embodiment, $R^{2b}$ is cyclopropyl substituted with one -CN.

In one embodiment, the compound of the invention is according to Formula I, II or III, wherein $R^{2b}$ is 4-7 membered monocyclic heterocycloalkyl comprising one or more independently selected O, N, or S heteroatoms. In a particular embodiment, $R^{2b}$ is oxetanyl.

In one embodiment, the compound of the invention is according to Formula I, II or III, wherein $R^{2b}$ is 4-7 membered monocyclic heterocycloalkyl comprising one or more independently selected O, N, or S heteroatoms, substituted with one or more independently selected -CN, halo, -OH, $-SO_2-C_{1-4}$ alkyl, =O, $C_{1-4}$ alkyl optionally substituted with one or more independently selected halo, or $C_{1-4}$ alkoxy optionally substituted with one or more independently selected halo. In a particular embodiment, $R^{2b}$ is oxetanyl substituted with one or more independently selected -CN, halo, -OH, $-SO_2-C_{1-4}$ alkyl, =O, $C_{1-4}$ alkyl optionally substituted with one or more independently selected halo, or $C_{1-4}$

alkoxy optionally substituted with one or more independently selected halo. In another particular embodiment, $R^{2b}$ is 4-7 membered monocyclic heterocycloalkyl comprising one or more independently selected O, N, or S heteroatoms, substituted with one or more independently selected -CN, F, -OH, $-SO_2-CH_3$, =O, $-CH_3$, $-CH_2CH_3$, $-CF_3$, $CHF_2$, $-CH_2CF_3$, $-OCH_3$, $-OCH_2CH_3$, or $-OCF_3$. In a more particular embodiment, $R^{2b}$ is oxetanyl substituted with one or more independently selected -CN, F, -OH, $-SO_2-CH_3$, =O, $-CH_3$, $-CH_2CH_3$, $-CF_3$, $-CHF_2$, $-CH_2CF_3$, $-OCH_3$, $-OCH_2CH_3$, or $-OCF_3$. In a most particular embodiment, $R^{2b}$ is oxetanyl substituted with one $-CH_3$.

In one embodiment, the compound of the invention is according to Formula IVa or IVb:

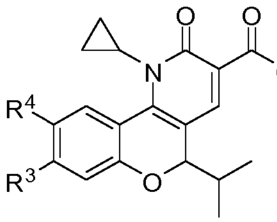

IVa

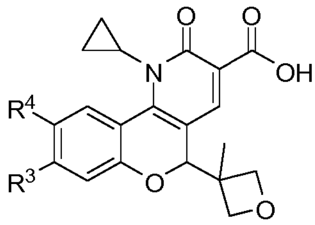

IVb

wherein $R^3$and $R^4$ are as described previously.

In one embodiment, the compound of the invention is according to Formula Va or Vb:

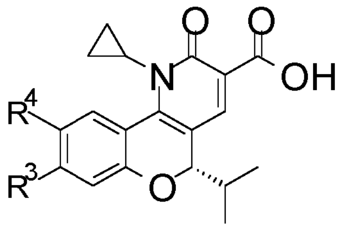

Va

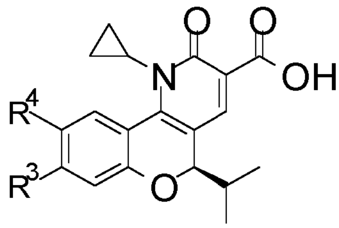

Vb

wherein $R^3$ and $R^4$ are as described previously.

In one embodiment, the compound of the invention is according to Formula VIa or VIb:

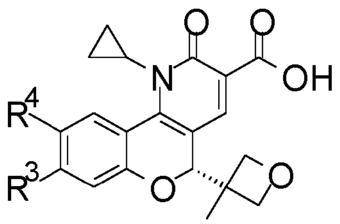

VIa

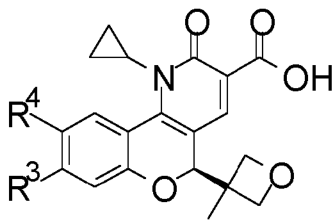

VIb

wherein $R^3$and $R^4$ are as described previously.

In one embodiment, the compound of the invention is according to any one of Formulae I-VIb, wherein $R^4$ is H.

In one embodiment, the compound of the invention is according to any one of Formulae I-VIb, wherein $R^4$ is $-SO_2-C_{1-4}$ alkyl. In a particular embodiment, $R^4$ is $-SO_2CH_3$, or $-SO_2CH_2CH_3$.

In one embodiment, the compound of the invention is according to any one of Formulae I-VIb, wherein $R^4$ is halo. In a particular embodiment, $R^4$ is F. in another particular embodiment, $R^4$ is or Cl.

In one embodiment, the compound of the invention is according to any one of Formulae I-VIb, wherein $R^4$ is -CN.

In one embodiment, the compound of the invention is according to any one of Formulae I-VIb, wherein $R^4$ is $C_{1-4}$ alkyl optionally substituted with one or more independently selected halo. In a particular embodiment, $R^4$ is $-CH_3$, $-CH_2CH_3$, $-CH(CH_3)_2$, or $-CF_3$.

In one embodiment, the compound of the invention is according to any one of Formulae I-VIb, wherein $R^4$ is $-OCH_3$, or $-OCH_2CH_3$. In a more particular embodiment, $R^4$ is $-OCH_3$.

In one embodiment, the compound of the invention is according to any one of Formulae I-VIb, wherein $R^3$ is

$C_{1-4}$ Alkyl. In a particular embodiment, $R^3$ is $-CH_3$, $-CH_2CH_3$, or $-CH_2CH(CH_3)_2$.

In one embodiment, the compound of the invention is according to any one of Formulae I-VIb, wherein $R^3$ is $C_{1-4}$ Alkyl substituted with one or more independently selected halo, -CN, -OH, $-SO_2C_{1-4}$ alkyl, $C_{1-4}$ alkoxy, or 4-7 membered monocyclic heterocycloalkyl comprising one or more independently selected O, N, or S heteroatoms. In on embodiment, $R^3$ is $-CH_3$, $-CH_2CH_3$, or $-CH_2CH(CH_3)_2$, each of which is substituted with one or more independently selected halo, -CN, -OH, $-SO_2C_{1-4}$ alkyl, $C_{1-4}$ alkoxy, or 4-7 membered monocyclic heterocycloalkyl comprising one or more independently selected O, N, or S heteroatoms. In another particular embodiment, $R^3$ is $C_{1-4}$ alkyl substituted with one or more independently selected F, -CN, $-SO_2CH_3$, $-OCH_3$, oxetanyl, tetrahydrofuranyl, or tetrahydropyranyl. In a more particular embodiment, $R^3$ is $-CH_3$, $-CH_2CH_3$, or $-CH_2CH(CH_3)_2$, each of which is substituted with one or more independently selected F,-CN, $-SO_2CH_3$, $-OCH_3$, oxetanyl, tetrahydrofuranyl, or tetrahydropyranyl.

In one embodiment, the compound of the invention is according to any one of Formulae I-VIb, wherein $R^3$ is halo. In a particular embodiment, $R^3$ is F or Cl.

In one embodiment, the compound of the invention is according to any one of Formulae I-VIb, wherein $R^3$ is -CN.

In one embodiment, the compound of the invention is according to any one of Formulae I-VIb, wherein $R^3$ is $C_{1-4}$ alkoxy. In a particular embodiment, $R^3$ is $-OCH_3$, $-OCH_2CH_3$, $-OCH_2CH_2CH_3$, $-OCH_2CH(CH_3)_2$, or $-OCH(CH_3)_2$. In a more particular embodiment, $R^3$ is $-OCH_3$.

In one embodiment, the compound of the invention is according to any one of Formulae I-VIb, wherein $R^3$ is $C_{1-4}$ alkoxy substituted with one or more independently selected $R^7$ groups. In a particular embodiment, $R^3$ is $C_{1-4}$ alkoxy substituted with one, two or three independently selected $R^7$ groups. In another particular embodiment, $R^3$ is $-OCH_3$, $-OCH_2CH_3$, $-OCH_2CH_2CH_3$, $-OCH_2CH(CH_3)_2$, or $-OCH(CH_3)_2$, each of which substituted with one, two or three independently selected $R^7$ groups. In a more particular embodiment, $R^3$ is $C_{1-4}$ alkoxy substituted with one $R^7$ group. In another more particular embodiment, $R^3$ is $-OCH_3$, $-OCH_2CH_3$, $-OCH_2CH_2CH_3$, $-OCH_2CH(CH_3)_2$, or $-OCH(CH_3)_2$, each of which substituted with one $R^7$ group. In a most particular embodiment, $R^3$ is $-OCH_2CH_2CH_3$ substituted with one $R^7$ group.

In one embodiment, the compound of the invention is according to any one of Formulae I-VIb, wherein $R^3$ is as previously described and one or more $R^7$ is halo. In a particular embodiment, $R^7$ is F.

In one embodiment, the compound of the invention is according to any one of Formulae I-VIb, wherein $R^3$ is as previously described and one or more $R^7$ is -CN.

In one embodiment, the compound of the invention is according to any one of Formulae I-VIb, wherein $R^3$ is as previously described and one or more $R^7$ is -OH.

In one embodiment, the compound of the invention is according to any one of Formulae I-VIb, wherein $R^3$ is as previously described and one or more $R^7$ is $-SO_2C_{1-4}$ alkyl. In a particular embodiment, $R^7$ is $-SO_2CH_3$, or $-SO_2CH_2CH_3$.

In one embodiment, the compound of the invention is according to any one of Formulae I-VIb, wherein $R^3$ is as previously described and one or more $R^7$ is $C_{1-4}$ alkoxy. In a particular embodiment, $R^7$ is $-OCH_3$, $-OCH_2CH_3$, $-OCH_2CH_2CH_3$, $-OCH_2CH(CH_3)_2$, or $-OCH(CH_3)_2$. In a more particular embodiment, $R^7$ is $-OCH_3$, or $-OCH_2CH_3$.

In one embodiment, the compound of the invention is according to any one of Formulae I-VIb, wherein $R^3$ is as previously described and one or more $R^7$ is phenyl.

In one embodiment, the compound of the invention is according to any one of Formulae I-VIb, wherein $R^3$ is as previously described and one or more $R^7$ is 5-6 membered monocyclic heteroaryl comprising one or more independently selected O, N, or S heteroatoms. In one embodiment, $R^7$ is imidazolyl, pyrazolyl, triazolyl, furanyl, pyridinyl, pyrazinyl, or pyrimidinyl.

In one embodiment, the compound of the invention is according to any one of Formulae I-VIb, wherein $R^3$ is as previously described and one or more $R^7$ is $C_{3-7}$ cycloalkyl. In a more particular embodiment, $R^7$ is cyclopropyl, cyclobutyl, cyclopentyl or cyclohexyl.

In one embodiment, the compound of the invention is according to any one of Formulae I-VIb, wherein $R^3$ is as previously described and one or more $R^7$ is $C_{3-7}$ cycloalkyl substituted with one or more independently selected $R^{8a}$. In a more particular embodiment, $R^7$ is cyclopropyl, cyclobutyl, cyclopentyl or cyclohexyl, each of which is substituted with one or more independently selected $R^{8a}$. In a more particular embodiment, $R^7$ is cyclopropyl, cyclobutyl, cyclopentyl or cyclohexyl, each of which is substituted with one $R^{8a}$. In a further more particular embodiment, each $R^{8a}$ is independently F, -CN, $-SO_2CH_3$, =O, $-CH_3$, $-CH_2CH_3$, $-CF_3$, $-CH_2-OCH_3$, $-C(=O)NH_2$, $-C(=O)NHCH_3$, $-C(=O)N(CH_3)_2$, $-OCH_3$, $-OCH_2CH_3$, or $-C(=O)CH_3$. In a most particular embodiment, $R^{8a}$ is -CN.

In one embodiment, the compound of the invention is according to any one of Formulae I-VIb, wherein $R^3$ is as previously described and one or more $R^7$ is monocyclic 4-7 membered heterocycloalkyl comprising one or more independently selected O, N, or S. In a particular embodiment, $R^7$ is oxetanyl, tetrahydrofuranyl, or dioxanyl.

In one embodiment, the compound of the invention is according to any one of Formulae I-VIb, wherein $R^3$ is as previously described and one or more $R^7$ is monocyclic 4-7 membered heterocycloalkyl comprising one or more independently selected O, N, or S, substituted with one or more independently selected $R^{8b}$. In a particular embodiment, $R^7$ is monocyclic 4-7 membered heterocycloalkyl comprising one or more independently selected O, N, or S, substituted

with one, two or three independently selected $R^{8b}$. In a more particular embodiment, $R^7$ is oxetanyl, oxazolidinyl, azetidinyl, or pyrrolyl, each of which is substituted with one, two or three independently selected $R^{8b}$. In a most particular embodiment, $R^{8b}$ is F, -CN, $-SO_2CH_3$, =O, $-CH_3$, $-CH_2CH_3$, $CF_3$, $-CH_2-OCH_3$, $-C(=O)NH_2$, $-C(=O)NHCH_3$, $-C(=O)N(CH_3)_2$, $-OCH_3$, $-OCH_2CH_3$, or $-C(=O)CH_3$. In a further most particular embodiment, $R^{8b}$ is -CN, $-SO_2CH_3$, =O, or $-CH_3$.

In one embodiment, the compound of the invention is according to any one of Formulae I-VIb, wherein $R^3$ is as previously described and one or more $R^7$ is fused/spiro/bridged 4-10 membered heterocycloalkyl comprising one or more independently selected O, N, or S. in a particular embodiment, $R^7$. In a particular embodiment, $R^7$ is

In one embodiment, the compound of the invention is according to any one of Formulae I-VIb, wherein $R^3$ is as previously described and one or more $R^7$ is monocyclic 4-7 membered heterocycloalkyl comprising one or more independently selected O, N, or S, substituted with one or more independently selected $R^{8b}$. In a particular embodiment, $R^7$ is monocyclic 4-7 membered heterocycloalkyl comprising one or more independently selected O, N, or S, substituted with one, two or three independently selected $R^{8b}$. In a more particular embodiment, $R^7$ is oxetanyl, oxazolidinyl, azetidinyl, or pyrrolyl, each of which is substituted with one, two or three independently selected $R^{8b}$. In a most particular embodiment, $R^{8b}$ is F,-CN, $-SO_2CH_3$, =O, $-CH_3$, $-CH_2CH_3$, $-CF_3$, $-CH_2-OCH_3$, $-C(=O)NH_2$, $-C(=O)NHCH_3$, $-C(=O)N(CH_3)_2$, $-OCH_3$, $-OCH_2CH_3$, or $-C(=O)CH_3$. In a further most particular embodiment, $R^{8b}$ is -CN, $-SO_2CH_3$, =O, or $-CH_3$.

In one embodiment, the compound of the invention is according to any one of Formulae I-VIb, wherein $R^3$ is as previously described and one or more $R^7$ is fused/spiro/bridged 4-10 membered heterocycloalkyl comprising one or more independently selected O, N, or S. In a particular embodiment, $R^7$ is 2-Oxa-6-aza-spiro[3.3]heptanyl, 6-Oxa-2-aza-spiro[3.4]octanyl, or hexahydro-furo[3,4-c]pyrrolyl,

In one embodiment, the compound of the invention is according to any one of Formulae I-VIb, wherein $R^3$ is as previously described and one or more $R^7$ is fused/spiro/bridged 4-10 membered heterocycloalkyl comprising one or more independently selected O, N, or S, substituted with one or more independently selected $R^{8c}$. in a particular embodiment, $R^7$ is 2-Oxa-6-aza-spiro[3.3]heptanyl, 6-Oxa-2-aza-spiro[3.4]octanyl, or hexahydro-furo[3,4-c]pyrrolyl,each of which is substituted with one or more independently selected $R^{8c}$. In a most particular embodiment, $R^{8c}$ is F, -CN, $-SO_2CH_3$, =O, $-CH_3$, $-CH_2CH_3$, $-CF_3$, $-CH_2-OCH_3$, $-C(=O)NH_2$, $-C(=O)NHCH_3$, $-C(=O-N(CH_3)_2$, $-OCH_3$, $-OCH_2CH_3$, or $-C(=O)CH_3$.

In one embodiment, the compound of the invention is according to any one of Formulae I-VIb, wherein $R^3$ is $-OCH_3$, or $-OCH_2CH_2CH_2OCH_3$. In a most particular embodiment, $R^3$ is $-OCH_2CH_2CH_2OCH_3$.

In one embodiment, the compound of the invention is selected from:

1-cyclopropyl-9- fluoro-5- isopropyl-8-(3-methoxypropoxy)-2-oxo-5H-chromeno[4,3-b]pyridine-3-carboxylic acid,
9-chloro-l-cyclopropyl-8-(3-methoxypropoxy)-5-(3-methyloxetan-3-yl)-2-oxo-5H-chromeno[4,3-b]pyridine-3-carboxylic acid,
1-cyclopropyl-5-isopropyl-8,9-dimethoxy-2-oxo-5H-chromeno[4,3-b]pyridine-3-carboxylic acid,
9-chloro-1-cyclopropyl-5-isopropyl-8-(3-methoxypropoxy)-2-oxo-5H-chromeno[4,3-b]pyridine-3-carboxylic acid,
9-chloro-1-cyclopropyl-5-isopropyl-8-(oxetan-3-ylmethoxy)-2-oxo-5H-chromeno[4,3-b]pyridine-3-carboxylic acid,
9-chloro-1-cyclopropyl-8-(3-methoxypropoxy)-2-oxo-5H-chromeno[4,3-b]pyridine-3-carboxylic acid, 9-chloro-1-cyclopropyl-8-(1, 4-dioxan-2+ylmethoxy)-5-isopropyl-2-oxo-5H-chromeno [4,3 -b]pyridine-3-carboxylic acid,
7-chloro-l-cyclopropyl-5-isopropyl-8-(3-methoxypropoxy)-2-oxo-5H-chromeno[4,3-b]pyridine-3-carboxylic acid,
5-tert-butyl-l-cyclopropyl-8,9-dimethoxy-2-oxo-5H-chromeno[4,3-b]pyridine-3-carboxylic acid, 1-cyclopropyl-5-isopropyl-9-methoxy-8-(3-methoxypropoxy)-2-oxo-5H-chromeno[4,3-b]pyridine-3-carboxylic acid,
9-chloro-1-cyclopropyl-5-ethyl-8-(3-methoxypropoxy)-2-oxo-5H-chromeno[4,3-b]pyridine-3-carboxylic acid,
1-cyclopropyl-5-ethyl-9-methoxy-8-(3-methoxypropoxy)-2-oxo-5H-chromeno[4,3-b]pyridine-3-carboxylic acid,
9-chloro-1-cyclopropyl-8-(3-methoxypropoxy)-5-(oxetan-3-yl)-2-oxo-5H-chromeno[4,3-b]pyridine-3-carboxylic acid,
9-chloro-1-cyclopropyl-8-(3-methoxypropoxy)-5,5-dimethyl-2-oxo-chromeno[4,3-b]pyridine-3-carboxylic acid,
1-cyclopropyl-5-ethyl-8,9-dimethoxy-2-oxo-5H-chromeno[4,3-b]pyridine-3-carboxylic acid,
1-cyclopropyl-5-ethyl-9-fluoro-8-(3-methoxypropoxy)-2-oxo-5H-chromeno[4,3-b]pyridine-3-carboxylic acid,
9-chloro-l-cyclobutyl-5-isopropyl-8-(3-methoxypropoxy)-2-oxo-5H-chromeno[4,3-b]pyridine-3-carboxylic acid,
1-cyclopropyl-5-(hydroxymethyl)-8,9-dimethoxy-2-oxo-5H-chromeno[4,3-b]pyridine-3-carboxylic acid, 9-chloro-1-cyclopropyl-5-(2-methoxy-1,1-dimethyl-ethyl)-8-(3-methoxypropoxy)-2-oxo-5H-chromeno[4,3 -b]pyridine-3-carboxylic acid,
1-cyclopropyl-5-isopropyl-9-methoxy-2-oxo-8-(trideuteriomethoxy)-5H-chromeno[4,3-b]pyridine-3-carboxylic acid,
9-chloro-1-cyclopropyl-8-methoxy-5-(oxetan-3-yl)-2-oxo-5H-chromeno[4,3-b]pyridine-3-carboxylic acid,
9-chloro-5-(1-cyanocyclopropyl)-1-cyclopropyl-8-(3-methoxypropoxy)-2-oxo-5H-chromeno[4,3-b]pyridine-3-carboxylic acid,
9-chloro-1-(3,3-difluorocyclobutyl)-5-isopropyl-8-(3-methoxypropoxy)-2-oxo-5H-chromeno[4,3-b]pyridine-3-carboxylic acid,

9-cyano-1-cyclopropyl-5-isopropyl-8-(3-methoxypropoxy)-2-oxo-5H-chromeno[4,3-b]pyridine-3-carboxylic acid,
9-chloro-1-cyclopropyl-8-(2-methoxyethoxy)-5-(3-methyloxetan-3-yl)-2-oxo-5H-chromeno[4,3-b]pyridine-3-carboxylic acid,
1-cyclobutyl-9-fluoro-8-(3-methoxypropoxy)-5-(3-methyloxetan-3-yl)-2-oxo-5H-chromeno[4,3-b]pyridine-3-carboxylic acid,
9-fluoro-8-(3-methoxypropoxy)-5-(3-methyloxetan-3-yl)-2-oxo-1-(2,2,3,3-tetradeuteriocyclopropyl)-5H-chromeno[4,3-b]pyridine-3-carboxylic acid,
1-cyclopropyl-5-isopropyl-9-methoxy-8-[(3-methyloxetan-3-yl)methoxy]-2-oxo-5H-chromeno[4,3-b]pyridine-3-carboxylic acid,
1-cyclopropyl-5-isopropyl-9-methoxy-8-[(3-methyl-2-oxo-oxazolidm-5-yl)methoxy]-2-oxo-5H-chromeno[4,3-b]pyridine-3-carboxylic acid,
8-(3-cyanopropoxy)-1-cyclopropyl-5-isopropyl-9-methoxy-2-oxo-5H-chromeno[4,3-b]pyridine-3-carboxylic acid,
1-cyclopropyl-5-isopropyl-9-methoxy-8-(2-methoxyethoxy)-2-oxo-5H-chromeno[4,3-b]pyridine-3-carboxylic acid,
1-cyclopropyl-5-isopropyl-9-methoxy-8-[(1-methylsulfonylazetidin-3-yl)methoxy]-2-oxo-5H-chromeno[4,3-b]pyridine-3-carboxylic acid,
1-cyclopropyl-8-(1,4-dioxan-2-ylmethoxy)-5-isopropyl-9-methoxy-2-oxo-5H-chromeno[4,3-b]pyridine-3-carboxylic acid,
1-cyclopropyl-5-isopropyl-9-methoxy-8-(oxetan-3-ylmethoxy)-2-oxo-5H-chromeno[4,3-b]pyridine-3-carboxylic acid,
1-cyclopropyl-5-isopropyl-9-methoxy-2-oxo-8-[(2-oxooxazolidin-5-yl)methoxy]-5H-chromeno[4,3-b]pyridine-3-carboxylic acid,
1-cyclopropyl-8-(3-hydroxypropoxy)-5-isopropyl-9-methoxy-2-oxo-5H-chromeno[4,3-b]pyridine-3-carboxylic acid,
1-cyclopropyl-8-ethoxy-5-isopropyl-9-methoxy-2-oxo-5H-chromeno[4,3-b]pyridine-3-carboxylic acid,
1-cyclopropyl-8-isobutoxy-5-isopropyl-9-methoxy-2-oxo-5H-chromeno[4,3-b]pyridine-3-carboxylic acid,
1-cyclopropyl-8-isopropoxy-5-isopropyl-9-methoxy-2-oxo-5H-chromeno[4,3-b]pyridine-3-carboxylic acid,
l-cyclopropyl-5-isopropyl-9-methoxy-2-oxo-8-propoxy-5H-chromeno[4,3-b]pyridine-3-carboxylic acid,
8-benzyloxy-1-cyclopropyl-5-isopropyl-9-methoxy-2-oxo-5H-chromeno[4,3-b]pyridine-3-carboxylic acid,
1-cyclopropyl-8-(2-ethoxyethoxy)-5-isopropyl-9-methoxy-2-oxo-5H-chromeno[4,3-b]pyridine-3-carboxylic acid,
8-[(1-cyanocyclobutyl)methoxy]-1-cyclopropyl-5-isopropyl-9-methoxy-2-oxo-5H-chromeno[4,3-b]pyridine-3-carboxylic acid,
(5S)-1-cyclopropyl-9-fluoro-5-isopropyl-8-(3-methoxypropoxy)-2-oxo-5H-chromeno[4,3-b]pyridine-3-carboxylic acid,
(5R)-1-cyclopropyl-9-fluoro-5-isopropyl-8-(3-methoxypropoxy)-2-oxo-5H-chromeno[4,3-b]pyridine-3-carboxylic acid,
(5R)-9-chloro-l-cyclopropyl-8-(3-methoxypropoxy)-5-(3-methyloxetan-3-yl)-2-oxo-5H-chromeno[4,3-b]pyridine-3-carboxylic acid,
(5S)-9-chloro-1-cyclopropyl-8-(3-methoxypropoxy)-5-(3-methyloxetan-3-yl)-2-oxo-5H-chromeno[4,3-b]pyridine-3-carboxylic acid,
9-chloro-1-cyclobutyl-8-(3-methoxypropoxy)-5-(3-methyloxetan- 3-yl)-2-oxo-5H -chromeno[4,3-b]pyridine-3-carboxylic acid,
1-cyclopropyl-9-fluoro-8-(3-methoxypropoxy)-5-(3-methyloxetan-3-yl)-2-oxo-5H-chromeno[4,3-b]pyridine-3-carboxylic acid,
9-chloro-1-cyclopropyl-8-[[(2S)-1,4-dioxan-2-yl]methoxy]-5-isopropyl-2-oxo-5H-chromeno[4,3-b]pyridine-3-carboxylic acid,
9-chloro-1-cyclopropyl-8-[[(2R)-1,4-dioxan-2-yl]methoxy]-5-isopropyl-2-oxo-5H-chromeno[4,3-b]pyridine-3-carboxylic acid,
9-chloro-1-cyclopropyl-5-isopropyl-8-[(3-methyloxetan-3-yl)methoxy]-2-oxo-5H-chromeno[4,3-b]pyridine-3-carboxylic acid,
9-chloro-1-cyclopropyl-8-(3-methoxypropoxy)-2-oxo-5-tetrahydrofuran-3-yl-5H-chromeno[4,3-b]pyridine-3-carboxylic acid,
9-chloro-1-cyclopropyl-8-(3-methoxypropoxy)-2-oxo-5-tetrahydropyran-4-yl-5H-chromeno[4,3-b]pyridine-3-carboxylic acid,
9-chloro-1-cyclopropyl-8-methoxy-5-(3-methyloxetan-3-yl)-2-oxo-5H-chromeno[4,3-b]pyridine-3-carboxylic acid,
9-chloro-1-cyclopropyl-8-ethoxy-5-(3-methyloxetan-3-yl)-2-oxo-5H-chromeno[4,3-b]pyridine-3-carboxylic acid,
9-chloro-1-cyclopropyl-8-(cyclopropylmethoxy)-5-(3-methyloxetan-3-yl)-2-oxo-5H-chromeno[4,3-b]pyridine-3-carboxylic acid,
9-chloro-1-cyclopropyl-5-(3-methyloxetan-3-yl)-2-oxo-8-(trideuteriomethoxy)-5H-chromeno[4,3-b]pyridine-3-carboxylic acid,

1-cyclopropyl-8,9-difluoro-2-oxo-5H-chromeno[4,3-b]pyridine-3-carboxylic acid,
9-chloro-1-cyclopropyl-8-fluoro-2-oxo-5H-chromeno[4,3-b]pyridine-3-carboxylic acid,
8,9-dichloro-1-cyclopropyl-2-oxo-5H-chromeno[4,3-b]pyridine-3-carboxylic acid,
1-cyclopropyl-5-isopropyl-8-(3-methoxypropoxy)-2-oxo-9-(trifluoromethyl)-5H-chromeno[4,3-b]pyridine-3-carboxylic acid,
1-cyclopropyl-5-isopropyl-8-(3-methoxypropoxy)-9-methylsulfonyl-2-oxo-5H-chromeno[4,3-b]pyridine-3-carboxylic acid,
1-cyclopropyl-9-fluoro-5-isopropyl-8-(oxetan-3-ylmethoxy)-2-oxo-5H-chromeno[4,3-b]pyridine-3-carboxylic acid,
1-cyclopropyl-9-fluoro-5-isopropyl-8-[(3-methyloxetan-3-yl)methoxy]-2-oxo-5H-chromeno[4,3-b]pyridine-3-carboxylic acid,
(5S)-9-chloro-1-cyclopropyl-8-methoxy-5-(3-methyloxetan-3-yl)-2-oxo-5H-chromeno[4,3-b]pyridine-3-carboxylic acid,
(5R)-9-chloro-1-cyclopropyl-8-methoxy-5-(3-methyloxetan-3-yl)-2-oxo-5H-chromeno[4,3-b]pyridine-3-carboxylic acid,
9-chloro-1-cyclopropyl-5-(3-methyloxetan-3-yl)-2-oxo-8-(2,2,2-trifluoroethoxy)-5H-chromeno[4,3-b]pyridine-3-carboxylic acid,
9-chloro-1-cyclopropyl-8-[[(2R)-1,4-dioxan-2-yl]methoxy]-5-(3-methyloxetan-3-yl)-2-oxo-5H-chromeno [4,3 -b]pyridine-3 -carboxylic acid,
9-chloro-1-cyclopropyl-8-(difluoromethoxy)-5-(3-methyloxetan-3-yl)-2-oxo-5H-chromeno[4,3-b]pyridine-3-carboxylic acid,
9-bromo-1-cyclopropyl-5-isopropyl-8-(3-methoxypropoxy)-2-oxo-5H-chromeno[4,3-b]pyridine-3-carboxylic acid,
9-chloro-1-cyclopropyl-5-(3-methyloxetan-3-yl)-8-[(1-methylsulfonylazetidm-3-yl)methoxy]-2-oxo-5H-chromeno[4,3-b]pyridine-3-carboxylic acid,
1-cyclopropyl-8-(3-methoxypropoxy)-5-(3-methyloxetan-3-yl)-2-oxo-5H-chromeno[4,3-b]pyridine-3-carboxylic acid,
1-cyclopropyl-8,9-dimethoxy-5-(methylsulfanylmethyl)-2-oxo-5H-chromeno[4,3-b]pyridine-3-carboxylic acid,
9-chloro-8-(3-methoxypropoxy)-5-(3-methyloxetan-3-yl)-2-oxo-1-(1,2,2,3,3-pentadeuteriocyclopropyl)-5H-chromeno[4,3-b]pyridine-3-carboxylic acid.

In a particular embodiment, the compound of the invention is

9-chloro-1-cyclopropyl-8-(3-methoxypropoxy)-5-(3-methyloxetan-3-yl)-2-oxo-5H-chromeno[4,3-b]pyridine-3-carboxylic acid,
(5R)-9-chloro-1-cyclopropyl-8-(3-methoxypropoxy)-5-(3-methyloxetan-3-yl)-2-oxo-5H-chromeno[4,3-b]pyridine-3-carboxylic acid, or
(5S)-9-chloro-1-cyclopropyl-8-(3-methoxypropoxy)-5-(3-methyloxetan-3-yl)-2-oxo-5H-chromeno[4,3-b]pyridine-3-carboxylic acid.

In another particular embodiment, the compound of the invention is not

9-chloro-1-cyclopropyl-8-(3-methoxypropoxy)-5-(3-methyloxetan-3-yl)-2-oxo-5H-chromeno[4,3-b]pyridine-3-carboxylic acid,
(5R)-9-chloro-1-cyclopropyl-8-(3-methoxypropoxy)-5-(3-methyloxetan-3-yl)-2-oxo-5H-chromeno[4,3-b]pyridine-3-carboxylic acid, or
(5S)-9-chloro-1-cyclopropyl-8-(3-methoxypropoxy)-5-(3-methyloxetan-3-yl)-2-oxo-5H-chromeno[4,3-b]pyridine-3-carboxylic acid.

In one embodiment, the compounds of the invention are provided in a natural isotopic form.

In one embodiment, the compounds of the invention are provided in an unnatural variant isotopic form. In a specific embodiment, the unnatural variant isotopic form is a form in which deuterium (i.e. $^{2}H$ or D) is incorporated where hydrogen is specified in the chemical structure in one or more atoms of a compound of the invention. In one embodiment, the atoms of the compounds of the invention are in an isotopic form which is not radioactive. In one embodiment, one or more atoms of the compounds of the invention are in an isotopic form which is radioactive. Suitably radioactive isotopes are stable isotopes. Suitably the unnatural variant isotopic form is a pharmaceutically acceptable form.

In one embodiment, a compound of the invention is provided whereby a single atom of the compound exists in an unnatural variant isotopic form. In another embodiment, a compound of the invention is provided whereby two or more atoms exist in an unnatural variant isotopic form.

Unnatural isotopic variant forms can generally be prepared by conventional techniques known to those skilled

in the art or by processes described herein e.g. processes analogous to those described in the accompanying Examples for preparing natural isotopic forms. Thus, unnatural isotopic variant forms could be prepared by using appropriate isotopically variant (or labelled) reagents in place of the normal reagents employed in the illustrative example as examples.

In one aspect a compound of the invention according to any one of the embodiments herein described is present as the free base.

In one aspect a compound of the invention according to any one of the embodiments herein described is a pharmaceutically acceptable salt.

In one aspect a compound of the invention according to any one of the embodiments herein described is a solvate of the compound.

In one aspect a compound of the invention according to any one of the embodiments herein described is a solvate of a pharmaceutically acceptable salt of a compound.

While specified groups for each embodiment have generally been listed above separately, a compound of the invention includes one in which several or each embodiment in the above Formula, as well as other formulae presented herein, is selected from one or more of particular members or groups designated respectively, for each variable. Therefore, this invention is intended to include all combinations of such embodiments within its scope.

While specified groups for each embodiment have generally been listed above separately, a compound of the invention may be one for which one or more variables (for example, R groups) is selected from one or more embodiments according to any of the Formula(e) listed above. Therefore, the present invention is intended to include all combinations of variables from any of the disclosed embodiments within its scope.

Alternatively, the exclusion of one or more of the specified variables from a group or an embodiment, or combinations thereof is also contemplated by the present invention.

In certain aspects, the present disclosure provides prodrugs and derivatives of the compounds according to the formulae above. Prodrugs are derivatives of the compounds of the invention, which have metabolically cleavable groups and become by solvolysis or under physiological conditions the compounds of the invention, which are pharmaceutically active, *in vivo*. Such examples include, but are not limited to, choline ester derivatives and the like, N-alkylmorpholine esters and the like.

Other derivatives of the compounds of this invention have activity in both their acid and acid derivative forms, but the acid sensitive form often offers advantages of solubility, tissue compatibility, or delayed release in the mammalian organism (Bundgard, H, 1985). Prodrugs include acid derivatives well known to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this invention are preferred prodrugs. In some cases it is desirable to prepare double ester type prodrugs such as (acyloxy)alkyl esters or ((alkoxycarbonyl)oxy)alkylesters. Particularly useful are the $C_1$ to $C_8$ alkyl, $C_2$-$C_8$ alkenyl, aryl, $C_7$-$C_{12}$ substituted aryl, and $C_7$-$C_{12}$ arylalkyl esters of the compounds of the invention.

## CLAUSES

1. A compound according to Formula I:

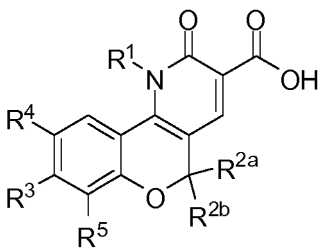

I

wherein

$R^1$ is $C_{3-6}$ cycloalkyl optionally substituted with one or more independently selected halo;
Each $R^{2a}$ and $R^{2b}$ is independently selected from:

- H,
- $C_{1-4}$ alkyl optionally substituted with one or more independently selected
    - halo,
    - -OH,
    - -CN,
    - $C_{1-4}$ alkoxy,
    - $C_{1-4}$ thioalkoxy, or
    - $-SO_2-C_1-_4$ alkyl;
- $C_{3-7}$ cycloalkyl optionally substituted with one or more independently selected
    - -CN,
    - halo,
    - -OH,
    - $-SO_2-C_1-_4$ alkyl,
    - $C_{1-4}$ alkyl optionally substituted with one or more independently selected halo, or
    - $C_{1-4}$ alkoxy optionally substituted with one or more independently selected halo; and
- 4-7 membered monocyclic heterocycloalkyl comprising one or more independently selected O, N, or S heteroatoms, optionally substituted with one or more independently selected
    - -CN,
    - halo,
    - -OH,
    - $-SO_2-C_1-_4$ alkyl,
    - =O,
    - $C_{1-4}$ alkyl optionally substituted with one or more independently selected halo, or
    - $C_{1-4}$ alkoxy optionally substituted with one or more independently selected halo;

$R^3$ is

- $C_{1-4}$ Alkyl optionally substituted with one or more independently selected
    - halo,
    - CN,
    - -OH,
    - $-SO_2-C_1-_4$ alkyl,
    - $C_{1-4}$ alkoxy, or
    - 4-7 membered monocyclic heterocycloalkyl comprising one or more independently selected O, N, or S heteroatoms,
- halo,
- -CN;
- $C_{1-4}$ alkoxy optionally substituted with one or more independently selected $R^7$ groups.

$R^4$ is

- H,
- $-SO_2-C_1-_4$ alkyl,
- halo,
- CN
- $C_{1-4}$ Alkyl optionally substituted with one or more independently selected halo,
- $C_{1-4}$ alkoxy

$R^5$ is H, -CN, halo, or $C_{1-4}$ Alkyl optionally substituted with one or more independently selected halo; and Each $R^{6a}$, $R^{6b}$, $R^{6c}$, and $R^{6d}$ is independently H or $C_{1-4}$ alkyl.
Each $R^7$ is independently

- halo,
- -CN,
- -OH,
- $-SO_2-C_{1-4}$ alkyl,
- $C_{1-4}$ alkoxy,
- phenyl,
- 5-6 membered monocyclic heteroaryl comprising one or more independently selected O, N, or S heteroatoms,
- $C_{3-7}$ cycloalkyl optionally substituted with one or more independently selected $R^{8a}$,
- monocyclic 4-7 membered heterocycloalkyl comprising one or more independently selected O, N, or S, optionally substituted with one or more independently selected $R^{8b}$, and
- fused/spiro/bridged 4-10 membered heterocycloalkyl comprising one or more independently selected O, N, or S, optionally substituted with one or more independently selected $R^{8c}$;

Each $R^{8a}$, $R^{8b}$, and $R^{8c}$ is independently selected from:

- halo,
- -CN,
- $-SO_2-C_{1-4}$ alkyl,
- =O,
- alkyl optionally substituted with one or more independently selected halo, or-OH,
- $-C(=O)NR^{6a}R^{6b}$,
- $-C_{1-4}$ alkoxy, or
- $-C(=O)\ C_{1-4}$ alkyl;

or a pharmaceutically acceptable salt thereof, solvate, or pharmaceutically acceptable salt of the solvate.

2. A compound or pharmaceutically acceptable salt according to clause 1, wherein $R^1$ is cyclopropyl, or cyclobutyl.

3. A compound or pharmaceutically acceptable salt according to clause 1, wherein $R^1$ is cyclopropyl, or cyclobutyl, each of which is substituted with one or more independently selected halo.

4. A compound or pharmaceutically acceptable salt according to clause 1, wherein $R^1$ is cyclopropyl or cyclobutyl, each of which is substituted with one two or three F.

5. A compound or pharmaceutically acceptable salt according to any one of clauses 1-4, wherein $R^5$ is H, -CN, halo, or $C_{1-4}$ Alkyl optionally substituted with one or more independently selected halo.

6. A compound or pharmaceutically acceptable salt according to any one of clauses 1-4, wherein $R^5$ is H, -CN, $-CH_3$, $-CF_3$, F or Cl.

7. A compound or pharmaceutically acceptable salt according to any one of clauses 1-4, wherein $R^5$ is H.

8. A compound or pharmaceutically acceptable salt according to clause 1, wherein the compound of the invention is according to Formula II:

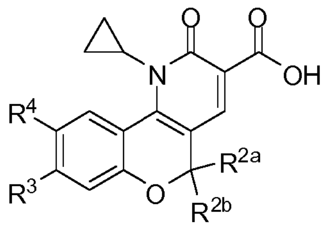

II

9. A compound or pharmaceutically acceptable salt according to any one of clauses 1-8, wherein $R^{2a}$ is H.

10. A compound or pharmaceutically acceptable salt according to any one of clauses 1-8, wherein $R^{2a}$ is $-CH_3$, $CH_2CH_3$, $-CH(CH_3)_2$ or $-C(CH_3)_3$.

11. A compound or pharmaceutically acceptable salt according to any one of clauses 1-8, wherein $R^{2a}$ is $-CH(CH_3)_2$.

12. A compound or pharmaceutically acceptable salt according to any one of clauses 1-8, wherein $R^{2a}$ is $-CH_3$, $CH_2CH_3$, $-CH(CH_3)_2$ or $-C(CH_3)_3$, each of which is substituted with one or more independently selected halo, -OH, -CN, $C_{1-4}$ alkoxy, $C_{1-4}$ thioalkoxy, or $-SO_2-C_{1-4}$ alkyl.

13. A compound or pharmaceutically acceptable salt according to any one of clauses 1-8, wherein $R^{2a}$ is $-CH_3$, $-CH_2CH_3$, $-CH(CH_3)_2$ or $-C(CH_3)_3$., each of which is substituted with one or more independently selected F, Cl, -OH,

-CN, $-OCH_3$, $-OCH_2CH_3$, $-SCH_3$, or $-SO_2CH_3$.

14. A compound or pharmaceutically acceptable salt according to any one of clauses 1-8, wherein $R^{2a}$ is $-CH_2OH$, or $-C(CH_3)_2CH_2OCH_3$.

15. A compound or pharmaceutically acceptable salt according to any one of clauses 1-8, wherein $R^{2a}$ is $C_{3-7}$ cycloalkyl.

16. A compound or pharmaceutically acceptable salt according to any one of clauses 1-8, wherein $R^{2a}$ is cyclopropyl, cyclobutyl, cyclopentyl, or cyclohexyl.

17. A compound or pharmaceutically acceptable salt according to any one of clauses 1-8, wherein $R^{2a}$ is cyclopropyl, cyclobutyl, cyclopentyl, or cyclohexyl, each of which is substituted with one or more independently selected -CN, halo, -OH, $-SO_2-C_{1-4}$ alkyl, $C_{1-4}$ alkyl optionally substituted with one or more independently selected halo, or $C_{1-4}$ alkoxy optionally substituted with one or more independently selected halo.

18. A compound or pharmaceutically acceptable salt according to any one of clauses 1-8, wherein $R^{2a}$ is cyclopropyl, cyclobutyl, cyclopentyl, or cyclohexyl, each of which is substituted with one or more independently selected -CN, F, Cl, -OH, $-SO_2-CH_3$, $-CH_3$, $-CH_2CH_3$, $-CF_3$, $-CHF_2$, $-CH_2CF_3$, $-OCH_3$, $-OCH_2CH_3$, or $-OCF_3$.

19. A compound or pharmaceutically acceptable salt according to any one of clauses 1-8, wherein $R^{2a}$ is cyclopropyl substituted with one -CN, F, Cl, -OH, $-SO_2-CH_3$, $-CH_3$, $-CH_2CH_3$, $-CF_3$, $-CHF_2$, $-CH_2CF_3$, $-OCH_3$, $-OCH_2CH_3$, or $-OCF_3$.

20. A compound or pharmaceutically acceptable salt according to any one of clauses 1-8, wherein $R^{2a}$ is 4-7 membered monocyclic heterocycloalkyl comprising one or more independently selected O, N, or S heteroatoms.

21. A compound or pharmaceutically acceptable salt according to any one of clauses 1-8, wherein $R^{2a}$ is oxetanyl.

22. A compound or pharmaceutically acceptable salt according to any one of clauses 1-8, wherein $R^{2a}$ is 4-7 membered monocyclic heterocycloalkyl comprising one or more independently selected O, N, or S heteroatoms, substituted with one or more independently selected -CN, halo, -OH, $-SO_2-C_{1-4}$ alkyl, =O, $C_{1-4}$ alkyl optionally substituted with one or more independently selected halo, or $C_{1-4}$ alkoxy optionally substituted with one or more independently selected halo.

23. A compound or pharmaceutically acceptable salt according to any one of clauses 1-8, wherein $R^{2a}$ is oxetanyl substituted with one or more independently selected -CN, halo, -OH, $-SO_2-C_{1-4}$ alkyl, =O, $C_{1-4}$ alkyl optionally substituted with one or more independently selected halo, or $C_{1-4}$ alkoxy optionally substituted with one or more independently selected halo.

24. A compound or pharmaceutically acceptable salt according to any one of clauses 1-8, wherein $R^{2a}$ is oxetanyl substituted with one $-CH_3$.

25. A compound or pharmaceutically acceptable salt according to clause 1, wherein the compound is according to Formula III:

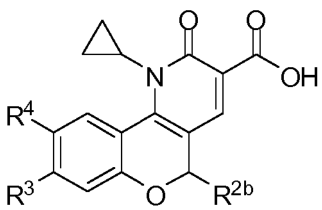

III

26. A compound or pharmaceutically acceptable salt according to any one of clauses 1-25, wherein $R^{2b}$ is H.

27. A compound or pharmaceutically acceptable salt according to any one of clauses 1-25, wherein $R^{2b}$ is $C_{1-4}$ alkyl.

28. A compound or pharmaceutically acceptable salt according to any one of clauses 1-25, wherein $R^{2b}$ is $-CH_3$, $-CH_2CH_3$, $-CH(CH_3)_2$ or $-C(CH_3)_3$.

29. A compound or pharmaceutically acceptable salt according to any one of clauses 1-25, wherein $R^{2b}$ is $-CH(CH_3)_2$.

30. A compound or pharmaceutically acceptable salt according to any one of clauses 1-25, wherein $R^{2b}$ is $C_{1-4}$ alkyl substituted with one or more independently selected halo, -OH, -CN, $C_{1-4}$ alkoxy, $C_{1-4}$ thioalkoxy, or $-SO_2-C_{1-4}$ alkyl.

31. A compound or pharmaceutically acceptable salt according to any one of clauses 1-25, wherein $R^{2b}$ is $-CH_3$, $CH_2CH_3$, $-CH(CH_3)_2$ or $-C(CH_3)_3$, each of which is substituted with one or more independently selected halo, -OH, -CN, $C_{1-4}$ alkoxy, $C_{1-4}$ thioalkoxy, or $-SO_2-C_{1-4}$ alkyl.

32. A compound or pharmaceutically acceptable salt according to any one of clauses 1-25, wherein $R^{2b}$ is $-CH_3$, $CH_2CH_3$, $-CH(CH_3)_2$ or $-C(CH_3)_3$, each of which is substituted with one or more independently selected F, Cl, -OH, -CN, $-OCH_3$, $-OCH_2CH_3$, $-SCH_3$, or $-SO_2CH_3$.

33. A compound or pharmaceutically acceptable salt according to any one of clauses 1-25, wherein $R^{2b}$ is $C_{3-7}$

cycloalkyl.

34. A compound or pharmaceutically acceptable salt according to any one of clauses 1-25, wherein $R^{2b}$ is cyclopropyl, cyclobutyl, cyclopentyl, or cyclohexyl.

35. A compound or pharmaceutically acceptable salt according to any one of clauses 1-25, wherein $R^{2b}$ is $C_{3-7}$ cycloalkyl substituted with one or more independently selected -CN, halo, -OH, $-SO_2-C_{1-4}$ alkyl, $C_{1-4}$ alkyl optionally substituted with one or more independently selected halo, or $C_{1-4}$ alkoxy optionally substituted with one or more independently selected halo.

36. A compound or pharmaceutically acceptable salt according to any one of clauses 1-25, wherein $R^{2b}$ is cyclopropyl, cyclobutyl, cyclopentyl, or cyclohexyl, each of which is substituted with one or more independently selected -CN, halo, -OH, $-SO_2-C_{1-4}$ alkyl, $C_{1-4}$ alkyl optionally substituted with one or more independently selected halo, or $C_{1-4}$ alkoxy optionally substituted with one or more independently selected halo.

37. A compound or pharmaceutically acceptable salt according to any one of clauses 1-25, wherein $R^{2b}$ is cyclopropyl, cyclobutyl, cyclopentyl, or cyclohexyl, each of which is substituted with one or more independently selected -CN, F, Cl, -OH, $-SO_2-CH_3$, $-CH_3$, $-CH_2CH_3$, $-CF_3$, $CHF_2$, $-CH_2CF_3$, $-OCH_3$, $-OCH_2CH_3$, or $-OCF_3$.

38. A compound or pharmaceutically acceptable salt according to any one of clauses 1-25, wherein $R^{2b}$ is cyclopropyl substituted with one -CN, F, Cl, -OH, $-SO_2-CH_3$, $-CH_3$, $-CH_2CH_3$, $-CF_3$, $CHF_2$, $-CH_2CF_3$, $-OCH_3$, $-OCH_2CH_3$, or $-OCF_3$.

39. A compound or pharmaceutically acceptable salt according to any one of clauses 1-25, wherein $R^{2b}$ is 4-7 membered monocyclic heterocycloalkyl comprising one or more independently selected O, N, or S heteroatoms.

40. A compound or pharmaceutically acceptable salt according to any one of clauses 1-25, wherein $R^{2b}$ is oxetanyl.

41. A compound or pharmaceutically acceptable salt according to any one of clauses 1-25, wherein $R^{2b}$ is 4-7 membered monocyclic heterocycloalkyl comprising one or more independently selected O, N, or S heteroatoms, substituted with one or more independently selected -CN, halo, -OH, $-SO_2-C_{1-4}$ alkyl, =O, $C_{1-4}$ alkyl optionally substituted with one or more independently selected halo, or $C_{1-4}$ alkoxy optionally substituted with one or more independently selected halo.

42. A compound or pharmaceutically acceptable salt according to any one of clauses 1-25, wherein $R^{2b}$ is oxetanyl substituted with one or more independently selected -CN, halo, -OH, $-SO_2-C_{1-4}$ alkyl, =O, $C_{1-4}$ alkyl optionally substituted with one or more independently selected halo, or $C_{1-4}$ alkoxy optionally substituted with one or more independently selected halo.

43. A compound or pharmaceutically acceptable salt according to any one of clauses 1-25, wherein $R^{2b}$ is oxetanyl substituted with one or more independently selected -CN, F, -OH, $-SO_2-CH_3$, =O, $-CH_3$, $-CH_2CH_3$, $-CF_3$, $CHF_2$, $-CH_2CF_3$, $-OCH_3$, $-OCH_2CH_3$, or $-OCF_3$.

44. A compound or pharmaceutically acceptable salt according to clause 1, wherein the compound is according to Formula IVa or IVb:

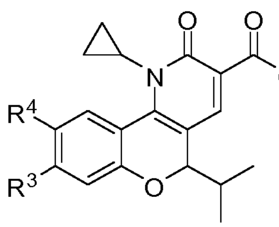

IVa

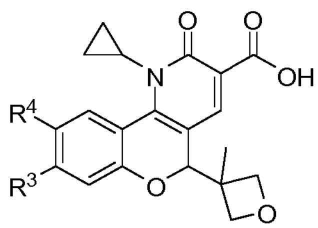

IVb

45. A compound or pharmaceutically acceptable salt according to any one of clauses 1-44, wherein $R^4$ is H.

46. A compound or pharmaceutically acceptable salt according to any one of clauses 1-44, wherein $R^4$ is $-SO_2-C_{1-4}$ alkyl.

47. A compound or pharmaceutically acceptable salt according to any one of clauses 1-44, wherein $R^4$ is $-SO_2CH_3$, or $-SO_2CH_2CH_3$.

48. A compound or pharmaceutically acceptable salt according to any one of clauses 1-44, wherein $R^4$ is halo.

49. A compound or pharmaceutically acceptable salt according to any one of clauses 1-44, wherein $R^4$ is F or Cl.

50. A compound or pharmaceutically acceptable salt according to any one of clauses 1-44, wherein $R^4$ is -CN.

51. A compound or pharmaceutically acceptable salt according to any one of clauses 1-44, wherein $R^4$ is $C_{1-4}$ alkyl optionally substituted with one or more independently selected halo.

52. A compound or pharmaceutically acceptable salt according to any one of clauses 1-44, wherein $R^4$ is $-CH_3$, $-CH_2CH_3$, $-CH(CH_3)_2$, or $-CF_3$.

53. A compound or pharmaceutically acceptable salt according to any one of clauses 1-44, wherein $R^4$ is $-OCH_3$,

or $-OCH_2CH_3$.

54. A compound or pharmaceutically acceptable salt according to any one of clauses 1-53, wherein $R^3$ is $C_{1-4}$ alkyl.

55. A compound or pharmaceutically acceptable salt according to any one of clauses 1-53, wherein $R^3$ is $-CH_3$, $-CH_2CH_3$, or $-CH_2CH(CH_3)_2$.

56. A compound or pharmaceutically acceptable salt according to any one of clauses 1-53, wherein $R^3$ is $C_{1-4}$ alkyl substituted with one or more independently selected halo,-CN, -OH, $-SO_2-C_{1-4}$ alkyl, $C_{1-4}$ alkoxy, or 4-7 membered monocyclic heterocycloalkyl comprising one or more independently selected O, N, or S heteroatoms.

57. A compound or pharmaceutically acceptable salt according to any one of clauses 1-53, wherein $R^3$ is $-CH_3$, $-CH_2CH_3$, or $-CH_2CH(CH_3)_2$, each of which is substituted with one or more independently selected halo, -CN, -OH, $-SO_2-C_{1-4}$ alkyl, $C_{1-4}$ alkoxy, or 4-7 membered monocyclic heterocycloalkyl comprising one or more independently selected O, N, or S heteroatoms.

58. A compound or pharmaceutically acceptable salt according to any one of clauses 1-53, wherein $R^3$ is $C_{1-4}$ alkyl substituted with one or more independently selected F, -CN, $-SO_2CH_3$, $-OCH_3$, oxetanyl, tetrahydrofuranyl, or tetrahydropyranyl.

59. A compound or pharmaceutically acceptable salt according to any one of clauses 1-53, wherein $R^3$ is - $CH_3$, $-CH_2CH_3$, or $-CH_2CH(CH_3)_2$, each of which is substituted with one or more independently selected F, -CN, $-SO_2CH_3$, $-OCH_3$, oxetanyl, tetrahydrofuranyl, or tetrahydropyranyl.

60. A compound or pharmaceutically acceptable salt according to any one of clauses 1-53, wherein $R^3$ is F or Cl.

61. A compound or pharmaceutically acceptable salt according to any one of clauses 1-53, wherein $R^3$ is -CN.

62. A compound or pharmaceutically acceptable salt according to any one of clauses 1-53, wherein $R^3$ is $-OCH_3$, $-OCH_2CH_3$, $-OCH_2CH_2CH_3$, $-OCH_2CH(CH_3)_2$, or $-OCH(CH_3)_2$.

63. A compound or pharmaceutically acceptable salt according to any one of clauses 1-53, wherein $R^3$ is $C_{1-4}$ alkoxy substituted with one, two or three independently selected $R^7$ groups.

64. A compound or pharmaceutically acceptable salt according to any one of clauses 1-53, wherein $R^3$ is $-OCH_3$, $-OCH_2CH_3$, $-OCH_2CH_2CH_3$, $-OCH_2CH(CH_3)_2$, or $-OCH(CH_3)_2$, each of which substituted with one, two or three independently selected $R^7$ groups.

65. A compound or pharmaceutically acceptable salt according to any one of clauses 1-53, wherein $R^3$ is $-OCH_3$, $-OCH_2CH_3$, $-OCH_2CH_2CH_3$, $-OCH_2CH(CH_3)_2$, or $-OCH(CH_3)_2$, each of which substituted with one $R^7$ group.

66. A compound or pharmaceutically acceptable salt according to clauses 63, 64, or 65, wherein $R^7$ is F.

67. A compound or pharmaceutically acceptable salt according to clauses 63, 64, or 65, wherein $R^7$ is -CN.

68. A compound or pharmaceutically acceptable salt according to clauses 63, 64, or 65, wherein $R^7$ is -OH.

69. A compound or pharmaceutically acceptable salt according to clauses 63, 64, or 65, wherein $R^7$ is $-SO_2CH_3$, or $-SO_2CH_2CH_3$.

70. A compound or pharmaceutically acceptable salt according to clauses 63, 64, or 65, wherein $R^7$ is $-OCH_3$, $-OCH_2CH_3$, $-OCH_2CH_2CH_3$, $-OCH_2CH(CH_3)_2$, or $-OCH(CH_3)_2$.

71. A compound or pharmaceutically acceptable salt according to clauses 63, 64, or 65, wherein $R^7$ is phenyl.

72. A compound or pharmaceutically acceptable salt according to clauses 63, 64, or 65, wherein $R^7$ is imidazolyl, pyrazolyl, triazolyl, furanyl, pyridinyl, pyrazinyl, or pyrimidinyl.

73. A compound or pharmaceutically acceptable salt according to clauses 63, 64, or 65, wherein $R^7$ is cyclopropyl, cyclobutyl, cyclopentyl or cyclohexyl.

74. A compound or pharmaceutically acceptable salt according to clauses 63, 64, or 65, wherein $R^7$ is cyclopropyl, cyclobutyl, cyclopentyl or cyclohexyl, each of which is substituted with one or more independently selected $R^{8a}$.

75. A compound or pharmaceutically acceptable salt according to clause 74, wherein $R^{8a}$ is F, -CN, $-SO_2CH_3$, =O, $-CH_3$, $-CH_2CH_3$, $-CF_3$, $-CH_2-OCH_3$, $-C(=O)NH_2$, $-C(=O)NHCH_3$, $-C(=O)N(CH_3)_2$, $-OCH_3$, $-OCH_2CH_3$, or $-C(=O)CH_3$.

76. A compound or pharmaceutically acceptable salt according to clauses 63, 64, or 65, wherein $R^7$ is oxetanyl, tetrahydrofuranyl, or dioxanyl.

77. A compound or pharmaceutically acceptable salt according to clauses 63, 64, or 65, wherein $R^7$ is monocyclic 4-7 membered heterocycloalkyl comprising one or more independently selected O, N, or S, substituted with one, two or three independently selected $R^{8b}$.

78. A compound or pharmaceutically acceptable salt according to clauses 63, 64, or 65, wherein $R^7$ is oxetanyl, oxazolidinyl, azetidinyl, or pyrrolyl, each of which is substituted with one, two or three independently selected $R^{Sb}$.

79. A compound or pharmaceutically acceptable salt according to clauses 77 or 78, wherein $R^{8b}$ is F,-CN, $-SO_2CH_3$, =O, $-CH_3$, $-CH_2CH_3$, $-CF_3$, $-CH_2-OCH_3$, $-C(=O)NH_2$, $-C(=O)NHCH_3$, $-C(=O)N(CH_3)_2$, $-OCH_3$, $-OCH_2CH_3$, or $-C(=O)CH_3$.

80. A compound or pharmaceutically acceptable salt according to any one of clauses 1-53, wherein $R^3$ is $-OCH_3$, or $-OCH_2CH_2CH_2OCH_3$.

81. A compound or pharmaceutically acceptable salt according to any one of clauses 1-53, wherein $R^3$ is $-OCH_2CH_2CH_2OCH_3$.

82. A pharmaceutical composition comprising a pharmaceutically acceptable carrier and a pharmaceutically effective amount of a compound or pharmaceutically acceptable salt thereof according to any one of clauses 1-81.

83. A pharmaceutical composition comprising a pharmaceutically acceptable carrier and a pharmaceutically effective amount of a compound or pharmaceutically acceptable salt thereof according to any one of clauses 1-81 and a further therapeutic agent.

84. A compound or pharmaceutically acceptable salt thereof according to any one of clauses 1-81, or a pharmaceutical composition according to clauses 82 or 83, for use in medicine.

85. A compound or pharmaceutically acceptable salt thereof according to any one of clauses 1-81, or a pharmaceutical composition according to clauses 82 or 83, for use in the prophylaxis and/or treatment of hepatitis B.

## PHARMACEUTICAL COMPOSITIONS

When employed as a pharmaceutical, a compound of the invention is typically administered in the form of a pharmaceutical composition. Such compositions can be prepared in a manner well known in the pharmaceutical art and comprise at least one active compound of the invention according to Formula I. generally, a compound of the invention is administered in a pharmaceutically effective amount. The amount of compound of the invention actually administered will typically be determined by a physician, in the light of the relevant circumstances, including the condition to be treated, the chosen route of administration, the actual compound of the invention administered, the age, weight, and response of the individual patient, the severity of the patient's symptoms, and the like.

The pharmaceutical compositions of this invention can be administered by a variety of routes including oral, rectal, transdermal, subcutaneous, intra-articular, intravenous, intramuscular, and intranasal. Depending on the intended route of delivery, a compound of the invention is preferably formulated as either injectable or oral compositions or as salves, as lotions or as patches all for transdermal administration.

The compositions for oral administration can take the form of bulk liquid solutions or suspensions, or bulk powders. More commonly, however, the compositions are presented in unit dosage forms to facilitate accurate dosing. The term 'unit dosage forms' refers to physically discrete units suitable as unitary dosages for human subjects and other mammals, each unit containing a predetermined quantity of active material calculated to produce the desired therapeutic effect, in association with a suitable pharmaceutical excipient, vehicle or carrier. Typical unit dosage forms include prefilled, premeasured ampules or syringes of the liquid compositions or pills, tablets, capsules or the like in the case of solid compositions. In such compositions, the compound of the invention according to Formula I is usually a minor component (from about 0.1 to about 50% by weight or preferably from about 1 to about 40% by weight) with the remainder being various vehicles or carriers and processing aids helpful for forming the desired dosing form.

Liquid forms suitable for oral administration may include a suitable aqueous or non-aqueous vehicle with buffers, suspending and dispensing agents, colorants, flavors and the like. Solid forms may include, for example, any of the following ingredients, or compound of the inventions of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint or orange flavoring.

Injectable compositions are typically based upon injectable sterile saline or phosphate-buffered saline or other injectable carriers known in the art. As before, the active compound of the invention according to Formula I in such compositions is typically a minor component, often being from about 0.05 to 10% by weight with the remainder being the injectable carrier and the like.

Transdermal compositions are typically formulated as a topical ointment or cream containing the active ingredient(s), generally in an amount ranging from about 0.01 to about 20% by weight, preferably from about 0.1 to about 20% by weight, preferably from about 0.1 to about 10% by weight, and more preferably from about 0.5 to about 15% by weight. When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or the formulation. All such known transdermal formulations and ingredients are included within the scope of this invention.

A compound of the invention can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania.

A compound of the invention can also be administered in sustained release forms or from sustained release drug delivery systems. A description of representative sustained release materials can be found in Remington's Pharmaceutical Sciences.

The following formulation examples illustrate representative pharmaceutical compositions that may be prepared in accordance with this invention. The present invention, however, is not limited to the following pharmaceutical compositions.

**Formulation 1 - Tablets**

A compound of the invention according to Formula I may be admixed as a dry powder with a dry gelatin binder in an approximate 1:2 weight ratio. A minor amount of magnesium stearate may be added as a lubricant. The mixture may be formed into 240-270 mg tablets (80-90 mg of active compound of the invention according to Formula I per tablet) in a tablet press.

**Formulation 2 - Capsules**

A compound of the invention according to Formula I may be admixed as a dry powder with a starch diluent in an approximate 1:1 weight ratio. The mixture may be filled into 250 mg capsules (125 mg of active compound of the invention according to Formula I per capsule).

**Formulation 3 - Liquid**

A compound of the invention according to Formula I (125 mg), may be admixed with sucrose (1.75 g) and xanthan gum (4 mg) and the resultant mixture may be blended, passed through a No. 10 mesh U.S. sieve, and then mixed with a previously made solution of microcrystalline cellulose and sodium carboxymethyl cellulose (11:89, 50 mg) in water. Sodium benzoate (10 mg), flavor, and color may be diluted with water and added with stirring. Sufficient water may then be added with stirring. Further sufficient water may be then added to produce a total volume of 5 mL.

**Formulation 4 - Tablets**

A compound of the invention according to Formula I may be admixed as a dry powder with a dry gelatin binder in an approximate 1:2 weight ratio. A minor amount of magnesium stearate may be added as a lubricant. The mixture may be formed into 450-900 mg tablets (150-300 mg of active compound of the invention according to Formula I) in a tablet press.

**Formulation 5 - Injection**

A compound of the invention according to Formula I may be dissolved or suspended in a buffered sterile saline injectable aqueous medium to a concentration of approximately 5 mg/mL.

**Formulation 6 - Topical**

Stearyl alcohol (250 g) and a white petrolatum (250 g) may be melted at about 75°C and then a mixture of A compound of the invention according to Formula I (50 g) methylparaben (0.25 g), propylparaben (0.15 g), sodium lauryl sulfate (10 g), and propylene glycol (120 g) dissolved in water (about 370 g) may be added and the resulting mixture may be stirred until it congeals.

**METHODS OF TREATMENT**

In one embodiment, the present invention provides compounds of the invention, or pharmaceutical compositions comprising a compound of the invention, for use in medicine. In a particular embodiment, the present invention provides compounds of the invention or pharmaceutical compositions comprising a compound of the invention, for use in the prophylaxis and/or treatment of hepatitis B.

In another embodiment, the present invention provides compounds of the invention, or pharmaceutical compositions comprising a compound of the invention for use in the manufacture of a medicament for use in the prophylaxis and/or treatment of hepatitis B.

In additional method of treatment aspects, this disclosure provides methods of prophylaxis and/or treatment of a mammal afflicted with hepatitis B, which methods comprise the administration of an effective amount of a compound of the invention or one or more of the pharmaceutical compositions herein described for the treatment or prophylaxis of said condition.

In one embodiment, the present invention provides pharmaceutical compositions comprising a compound of the invention, and another therapeutic agent. In a particular embodiment, the other therapeutic agent is a hepatitis B treatment agent.

Injection dose levels range from about 0.1 mg/kg/h to at least 10 mg/kg/h, all for from about 1 to about 120 h and especially 24 to 96 h. A preloading bolus of from about 0.1 mg/kg to about 10 mg/kg or more may also be administered to achieve adequate steady state levels. The maximum total dose is not expected to exceed about 1 g/day for a 40 to 80 kg human patient.

For the prophylaxis and/or treatment of long-term conditions, such as degenerative conditions, the regimen for treatment usually stretches over many months or years so oral dosing is preferred for patient convenience and tolerance. With oral dosing, one to four (1-4) regular doses daily, especially one to three (1-3) regular doses daily, typically one to two (1-2) regular doses daily, and most typically one (1) regular dose daily are representative regimens. Alternatively for long lasting effect drugs, with oral dosing, once every other week, once weekly, and once a day are representative regimens. In particular, dosage regimen can be every 1-14 days, more particularly 1-10 days, even more particularly 1-7 days, and most particularly 1-3 days.

Using these dosing patterns, each dose provides from about 1 to about 1000 mg of a compound of the invention, with particular doses each providing from about 10 to about 500 mg and especially about 30 to about 250 mg.

Transdermal doses are generally selected to provide similar or lower blood levels than are achieved using injection doses.

When used to prevent the onset of a condition, a compound of the invention will be administered to a patient at risk for developing the condition, typically on the advice and under the supervision of a physician, at the dosage levels described above. Patients at risk for developing a particular condition generally include those that have a family history of the condition, or those who have been identified by genetic testing or screening to be particularly susceptible to developing the condition.

A compound of the invention can be administered as the sole active agent or it can be administered in combination with other therapeutic agents, including other compound of the inventions that demonstrate the same or a similar therapeutic activity and that are determined to be safe and efficacious for such combined administration. In a specific embodiment, co-administration of two (or more) agents allows for significantly lower doses of each to be used, thereby reducing the side effects seen.

In one embodiment, a compound of the invention or a pharmaceutical composition comprising a compound of the invention is administered as a medicament. In a specific embodiment, said pharmaceutical composition additionally comprises a further active ingredient.

In one embodiment, a compound of the invention is co-administered with another therapeutic agent to stimulate immune response, particular agents include, but are not limited to PEG-IFN, TLR agonists, therapeutic vaccine and immune checkpoint blockers.

In one embodiment, a compound of the invention is co-administered with another therapeutic agent to repress HBV DNA levels, particular agents include, but are not limited to nucleoside analogues like lamivudine, entecavir, tenofovir, tenofovir alafenamide

In one embodiment, a compound of the invention is co-administered with another therapeutic agent to block the HBV entry receptor NTCP, particular agents include, but are not limited to Myrcludex

In one embodiment, a compound of the invention is co-administered with another therapeutic agent preventing capsid assembly. Particular agent include but are not limited to AB-506, and/or AB-423.

In one embodiment, a compound of the invention is co-administered with another therapeutic agent to repress HBsAg levels. In a particular embodiment, the HBsAg level repressing agent is a RNA interference agent. Particular agents include, but are not limited to ARB-1467.

By co-administration is included any means of delivering two or more therapeutic agents to the patient as part of the same treatment regime, as will be apparent to the skilled person. Whilst the two or more agents may be administered simultaneously in a single formulation, *i.e.* as a single pharmaceutical composition, this is not essential. The agents may be administered in different formulations and at different times.

## CHEMICAL SYNTHETIC PROCEDURES

### General

The compound of the invention can be prepared from readily available starting materials using the following general methods and procedures. It will be appreciated that where typical or preferred process conditions (*i.e.* reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art (Greene, T W; Wuts, P G M;, 1991).

The following methods are presented with details as to the preparation of a compound of the invention as defined hereinabove and the comparative examples. A compound of the invention may be prepared from known or commercially available starting materials and reagents by one skilled in the art of organic synthesis.

All reagents were of commercial grade and were used as received without further purification, unless otherwise stated. Commercially available anhydrous solvents were used for reactions conducted under inert atmosphere. Reagent grade solvents were used in all other cases, unless otherwise specified. Column chromatography was performed on silica gel 60 (35-70 μm). Thin layer chromatography was carried out using pre-coated silica gel F-254 plates (thickness 0.25 mm). $^1$H NMR spectra were recorded on a Bruker DPX 400 NMR spectrometer (400 MHz or a Bruker Advance 300 NMR spectrometer (300 MHz). Chemical shifts (δ) for 1H NMR spectra are reported in parts per million (ppm) relative to tetramethylsilane (δ 0.00) or the appropriate residual solvent peak, i.e. $CHCl_3$ (δ 7.27), as internal reference. Multiplicities are given as singlet (s), doublet (d), triplet (t), quartet (q), quintuplet (quin), multiplet (m) and broad (br). Electrospray MS spectra were obtained on a Waters platform LC/MS spectrometer or with Waters Acquity H-Class UPLC coupled to a Waters Mass detector 3100 spectrometer. Columns used: Waters Acquity UPLC BEH C18 1.7μm, 2.1mm ID x 50mm L, Waters Acquity UPLC BEH C18 1.7 μm, 2.1mm ID x 30 mm L, or Waters Xterra MS 5μm C18, 100 x 4.6mm. The methods are using either MeCN/$H_2O$ gradients ($H_2O$ contains either 0.1% TFA or 0.1% $NH_3$) or MeOH /$H_2O$ gradients ($H_2O$ contains 0.05% TFA). Microwave heating was performed with a Biotage Initiator.

**Table I. List of abbreviations used in the experimental section:**

| Abbreviation | Definition |
|---|---|
| HBV | Hepatite B Virus |
| DNA | Deoxyribonucleic acid |
| UPLC | Ultra Performance Liquid Chromatography |
| NMR | >Nuclear Magnetuic Resonance |
| TFA | Trifluoroacetic acid |
| MHz | MegaHertz |
| NaOAc | Sodium acetate |
| DMF | Dimethyl formamide |
| °C | Degree celsius |
| eq. | equivalents |
| h | hours |
| min | minute |
| CAS | Chemical Abstract number |
| THF | tetrahydrofuran |
| $PPh_3$ | triphenylphosphine |
| HPLC | High Pressure Liquid Chromatography |
| MeCN | acetonitrile |
| FBS | fetal bovine serum |
| MEM NEAA | Minimum Essential Medium non-Essential Amino Acids |
| DMSO | Dimethylsulfoxide |
| PBST | phosphate buffered saline + Tween 20 |
| DAPI | 4',6-diamidino-2-phenylindole |

## SYNTHETIC PREPARATION OF THE COMPOUNDS OF THE INVENTION

### Example 1. General synthetic methods

***1.1. Intermediates towards illustrative compounds of the invention***

***General method A: acetylation of 1,3-bis-hydroxyl benzenes***

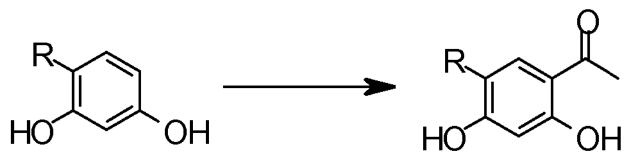

In a typical procedure, acetic acid (2 eq) is added dropwise to a mixture of 1,3-bis-hydroxy benzene (1 eq) in $BF_3{\cdot}O(C_2H_5)_2$ (1.3 M). The mixture is stirred at 90°C for 16 h. The mixture is allowed to cool and poured in a cold 10% solution of NaOAc in water. The mixture is stirred for 2 h and then extracted with ethyl acetate. The organic layer is washed (sat. $NaHCO_3$, $H_2O$), dried ($Na_2SO_4$) and concentrated to obtain the desired product after trituration.

***Illustrative example of method A: synthesis of intermediate 1, 1-(5-fluoro-2,4-dihydroxy-phenyl)ethanone***

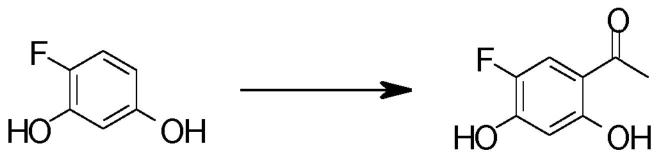

Acetic acid (13.4 mL, 234 mmol, 2 eq) is added dropwise to a mixture of 4-fluorobenzene-1,3-diol (CAS: 103068-41-3, 15 g, 117 mmol, 1 eq) in $BF_3{\cdot}O(C_2H_5)_2$ (90 mL). The mixture is stirred at 90 °C for 16 h. The mixture is allowed to cool and poured in a cold 10% solution of NaOAc in water (300 mL). The mixture is stirred for 2 h and extracted with ethyl acetate. The organic layer is washed (sat. $NaHCO_3$, $H_2O$), dried ($Na_2SO_4$) and concentrated. The residue is triturated in a methyl tert-butyl ether /petroleum ether mixture to obtain the desired product.

***General method B: mono-alkylation of 1,3-bis-hydroxyl benzene derivatives***

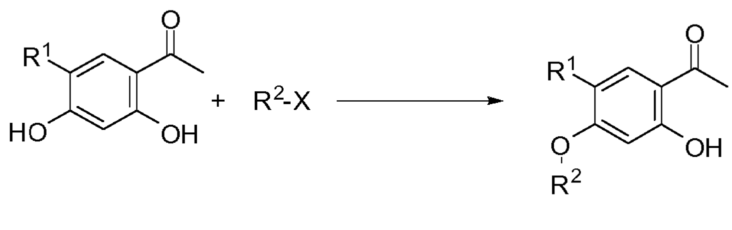

In a typical procedure, a mixture of 1,3-bis-hydroxyl benzene derivative (1 eq), alkyl halide (0.9 to 1 eq) and $K_2CO_3$ (1 eq) in DMF is stirred at room temperature for 18 to 24 h. An additional amount of alkylating agent is added (0.2 to 0.5 eq) and the mixture is stirred for a further 24 to 72 h. The mixture is diluted with water and an organic solvent. The two layers are separated. The organic layer is dried and concentrated to afford the desired product.

***Illustrative example of method B: synthesis of intermediate 3, 1-[5-chloro-2-hydroxy-4-(3-methoxypropoxy)phenyl]ethanone***

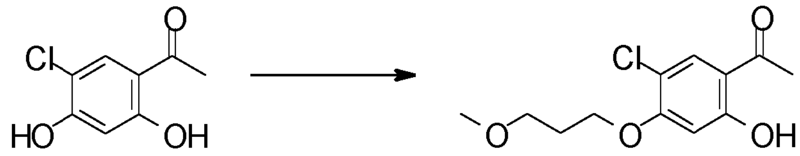

A mixture of 1-(5-chloro-2,4-dihydroxy-phenyl)ethanone (1 g, 5.4 mmol, 1 eq), 1-bromo-3-methoxy-propane (CAS: 36865-41-5, 0.6 mL, 5.4 mmol, 1 eq) and $K_2CO_3$ (741 mg, 5.4 mmol, 1 eq) in DMF (8 mL) is stirred at room temperature for 24 h. A further 0.5 eq of 1-bromo-3-methoxy-propane are added. The reaction mixture is stirred for a further 24 h. The mixture is diluted with water and dichloromethane. The two layers are separated. The organic layer is dried (filtered through phase separator) and concentrated to afford the desired product.

***Illustrative example of method B: synthesis of intermediate 4, 1-[5-fluoro-2-hydroxy-4-(3-methoxypropoxy)phenyl]ethanone***

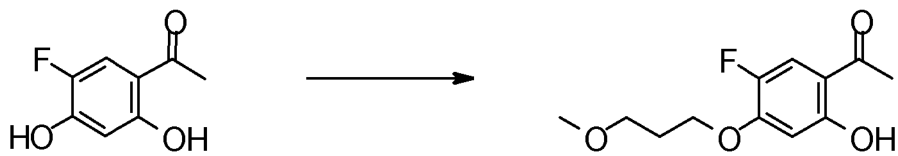

A mixture of 1-(5-fluoro-2,4-dihydroxy-phenyl)ethanone (5.8 g, 34 mmol, 1 eq), 1-bromo-3-methoxy-propane (CAS: 36865-41-5, 3.45 mL, 30.7 mmol, 0.9 eq) and $K_2CO_3$ (4.7 g, 34 mmol, 1 eq) in DMF (50 mL) is stirred at room temperature for 18 h. A further 0.2 eq of 1-bromo-3-methoxy-propane are added. The reaction mixture is stirred for a further 72 h. The mixture is diluted with water and dichloromethane. The two layers are separated. The organic layer is dried (filtered through phase separator) and concentrated to afford the desired product.

***General method C: synthesis of chromanone derivatives***

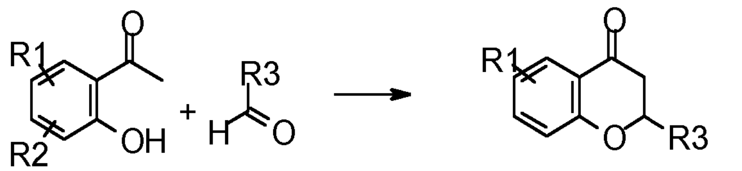

In a typical procedure, a mixture of 1-(2-hydroxyphenyl)-ethanone derivative (1eq), aldehyde (0.9 to 10 eq) and pyrrolidine (0.2 to 5 eq) in methanol is stirred at 60 to 75°C for 18 to 30 h. The mixture is diluted with an organic solvent and undergoes an aqueous work up. The organic layer is dried, concentrated and the residue may be further purified by flash column chromatography.

***Illustrative example of method C: synthesis of intermediate 7, 6-fluoro-2-isopropyl-7-(3-methoxypropoxy)chroman-4-one***

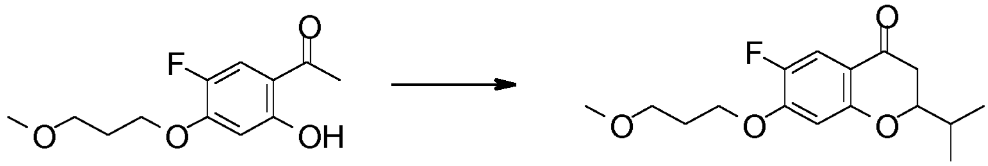

A mixture of 1-[5-fluoro-2-hydroxy-4-(3-methoxypropoxy)phenyl]ethanone (10 g, 41.3 mmol, 1eq), 2-methylpropanal (CAS: 78-84-2, 3.77 mL, 41.3 mmol, 1 eq) and pyrrolidine (CAS: 123-75-1, 0.68 mL, 8.26 mmol, 0.2 eq) in methanol (59 mL) is stirred at 60°C for 18 h. The mixture is diluted with water and dichloromethane and the two layers are separated. The organic layer is washed (saturated $NH_4Cl$ and saturated $NaHCO_3$), dried (filtered through a phase separator) and concentrated. The residue is purified by flash column chromatography ($SiO_2$, petroleum ether/ethyl acetate 80:20) to afford the desired product.

***Illustrative example of method C: synthesis of intermediate 8, 6-chloro-7-(3-methoxypropoxy)-2-(3-methyloxetan-3-yl)chroman-4-one***

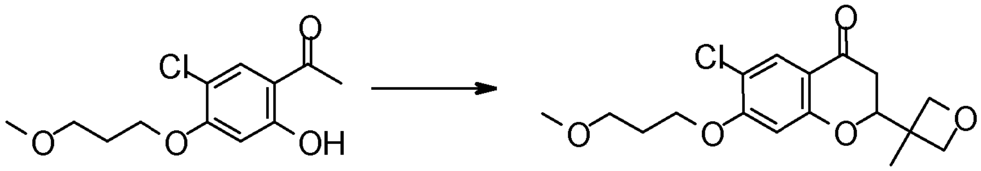

A mixture of 1-[5-chloro-2-hydroxy-4-(3-methoxypropoxy)phenyl]ethanone (2.5 g, 9.66 mmol, 1eq), 3-methyloxetane-3-carbaldehyde (CAS: 99419-31-5, 767 mg, 10.63 mmol, 1.1 eq) and pyrrolidine (CAS: 123-75-1, 0.79 mL, 9.66 mmol, 1 eq) in methanol (24 mL) is stirred at 60°C for 22 h. A further 0.3 eq of 3-methyloxetane-3-carbaldehyde are added and the mixture is stirred for 6 h. The reaction mixture is concentrated to dryness and the residue is partitioned between ethyl acetate and 1 N HCl. The two phases are separated and the organic layer is washed (brined), dried ($Na_2SO_4$) and concentrated. The residue is purified by flash column chromatography ($SiO_2$, petroleum ether/ethyl acetate 100:0 to 40:60) to afford the desired product.

***Synthesis of intermediate 23, 6-chloro-7-(3-methoxypropoxy)-2,2-dimethyl-chroman-4-one***

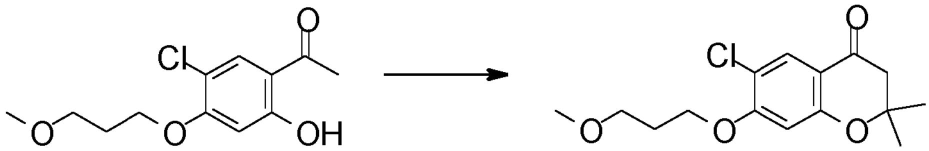

A mixture of 1-[5-chloro-2-hydroxy-4-(3-methoxypropoxy)phenyl]ethanone (1g, 3.87 mmol, 1eq), acetone (2.5 mL, 38.7 mmol, 10 eq) and pyrrolidine (CAS: 123-75-1, 0.64 mL, 7.73 mmol, 2 eq) in ethanol (4 mL) is stirred at 75 °C for 3 h in a sealed tube. The mixture is diluted with water and dichloromethane and the two layers are separated. The organic layer is washed (brine), dried ($MgSO_4$) and concentrated. The residue is purified by flash column chromatography ($SiO_2$, petroleum ether/ethyl acetate 95:5 to 0:100) to afford the desired product.

***General method D: synthesis of chromanone derivatives bearing an hydroxyl group***

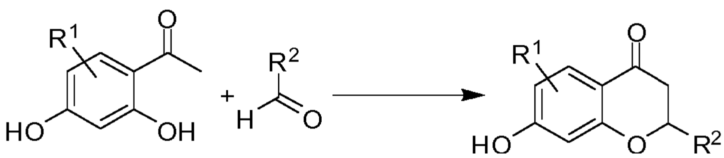

In a typical procedure, a mixture of 2,4-dihydroxy-phenyl ethanone derivative (1eq), aldehyde (2 to 18 eq) and pyrrolidine (1.2 to 5 eq) in methanol is stirred at 60 to 75 °C for 18 to 48 h. The mixture is diluted with an organic solvent and undergoes an aqueous work up. The organic layer is dried, concentrated and the residue may be further purified by flash column chromatography.

***Illustrative example of method D: synthesis of intermediate 24, 6-chloro-7-hydroxy-2-isopropyl-chroman-4-one***

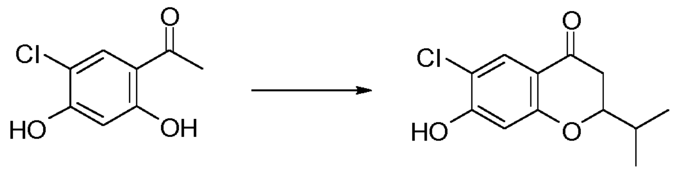

A mixture of 1-(5-chloro-2,4-dihydroxy-phenyl)ethanone (5 , 26.8 mmol, 1eq), 3- 2-methylpropanal (CAS: 78-84-2, 4.9 mL, 53.6 mmol, 2 eq) and pyrrolidine (2.2 mL, 32.2 mmol, 1.2 eq) in methanol (55 mL) is stirred at 60 °C for 22 h. The mixture is diluted with dichloromethane and the organic layer is washed (saturated $NaHCO_3$), dried ($MgSO_4$) and concentrated. The residue is purified by flash column chromatography ($SiO_2$, petroleum ether/ethyl acetate 90:10 to 50:50) to afford the desired product.

***General method E: alkylation of chromanone derivatives bearing a hydroxyl group***

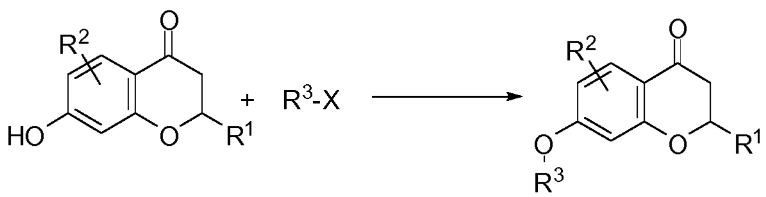

In a typical procedure, a mixture of 7-hydroxy-chroman-4-one derivative (1 eq), alkylating agent (1 to 1.5 eq) and $K_2CO_3$ (1 to 2 eq) in DMF or acetonitrile is stirred at 60 to 80 °C for 15 h. The mixture undergoes an aqueous work up and the residue is further purified by flash column chromatography.

***Illustrative example of method E: synthesis of intermediate 28, 6-chloro-2-isopropyl-7-(3-methoxypropoxy)chroman-4-one***

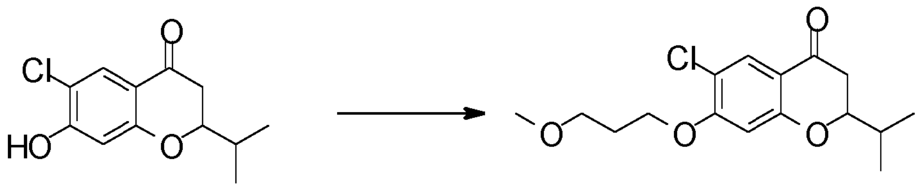

A mixture of 6-chloro-7-hydroxy-2-isopropyl-chroman-4-one (18.6 mmol, 1eq), 1-bromo-3-methoxy-propane (CAS: 36865-41-5, 4.3 g, 27.9 mmol, 1.5 eq) and $K_2CO_3$ (3.2 g, 22.6 mmol, 1.2 eq) in acetonitrile (40 mL) is stirred at 80 °C for 15 h. The mixture is diluted with dichloromethane. The organic mixture is washed with water. The two phases are separated and the organic layer is concentrated. The residue is purified by flash column chromatography ($SiO_2$, petroleum ether/ethyl acetate 100:0 to 60:40) to afford the desired product.

***Synthesis of intermediate 34, 1-(3-chloro-2,4-dihydroxy-phenyl)ethanone***

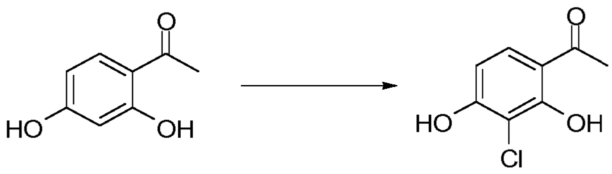

10% aqueous NaClO (13.3 mL, 21 mmol, 1.3 eq) is added dropwise to a mixture of 1-(2,4-dihydroxyphenyl)ethanone (CAS: 89-84-9, 2.5 g, 16.4 mmol, 1 eq) and aqueous NaOH (1 M, 17.3 mL. 17.3 mmol, 1.05 eq) in $H_2O$ (82 mL). The mixture is stirred at room temperature for 16 h. A further 14 mL of 10% aqueous NaClO are added and the reaction is stirred at room temperature for 2 h. A further 3 mL of 10% aqueous NaClO are added and the reaction is stirred at room temperature for 2 h. The mixture is acidified to pH 1 and the solid is filtered off. The filtrated is extracted with ethyl acetate. The two layers are separated. The organic layer is combined with the solid and concentrated. The residue is purified by flash column chromatography ($SiO_2$, petroleum ether/ethyl acetate 100:0 to 75:25) to afford the desired product.

***Synthesis of intermediate 35, 1-(2,4,5-trihydroxyphenyl)ethanone***

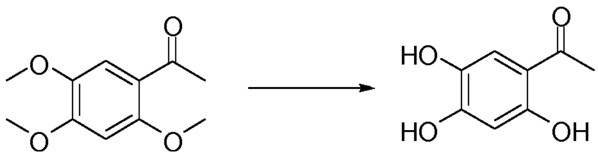

A mixture of 1-(2,4,5-trimethoxyphenyl)ethanone (10 g, 47.6 mmol, 1 eq) and $AlCl_3$ (16.5 g, 124 mmol, 2.6 eq) in toluene (48 mL) is stirred at reflux for 24 h. The mixture is concentrated to dryness and quenched with cooled 1 N HCl. The mixture is extracted with ethyl acetate. The organic layer is dried ($Na_2SO_4$) and concentrated. The residue is purified by flash column chromatography ($SiO_2$, dichloromethane/methanol 100:0 to 95:5) to afford the desired product.

***Synthesis of intermediate 36, 1-(4-benzyloxy-2,5-dihydroxy-phenyl)ethanone***

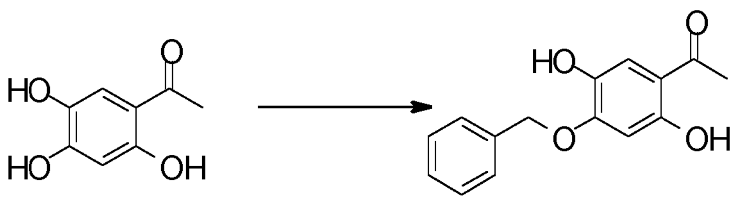

A mixture of 1-(2,4,5-trihydroxyphenyl)ethanone (6.44 g, 38.3 mmol, 1 eq), bromomethylbenzene (CAS: 100-39-0, 4.56 mL, 38.3 mmol, 1 eq) and $K_2CO_3$ (5.3 g, 38.3 mmol, 1 eq) in acetonitrile (103 mL) is stirred at 90 °C for 1 h and at room temperature for 16 h. The mixture is concentrated and the residue is purified by flash column chromatography ($SiO_2$, petroleum ether/ethyl acetate 100:0 to 0:100) to afford the desired product.

***Synthesis of intermediate 37, 1-(4-benzyloxy-2-hydroxy-5-methoxy-phenyl)ethanone***

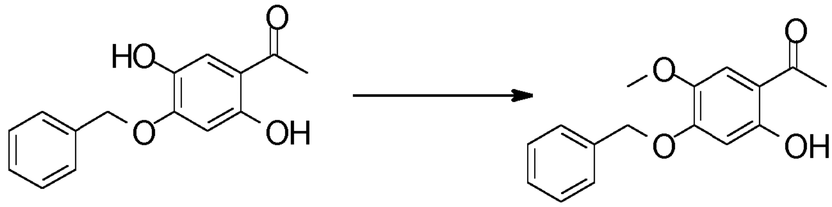

A mixture of 1-(4-benzyloxy-2,5-dihydroxy-phenyl)ethanone (7.1 g, 27.5 mmol, 1eq), iodomethane (CAS: 74-88-4, 1.71 mL, 27.5 mmol, 1 eq) and $K_2CO_3$ (3.8 g, 27.5 mmol, 1 eq) in acetonitrile (74 mL) is stirred at 90 °C for 15 h. 0.3 mL of iodomethane are added and the mixture is stirred for additional 3 h. 0.3 mL of iodomethane are added and the mixture is stirred for 2 additional h. The mixture is concentrated and the residue is purified by flash column chromatography ($SiO_2$, petroleum ether/ethyl acetate 100:0 to 50:50) to afford the desired product.

***Synthesis of intermediate 38, 6-chloro-2-isopropyl-7-(2-trimethylsilylethoxymethoxy)chroman-4-one***

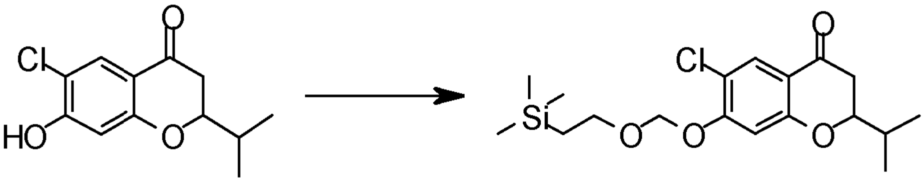

2-(Trimethylsilyl)ethoxymethyl chloride (2.4 mL, 13.6 mmol, 1.2 eq) is added very slowly over an h to a mixture of 6-chloro-7-hydroxy-2-isopropyl-chroman-4-one (2.74 g, 11.4 mmol, 1 eq), and *N,N*-diisopropylethylamine (4.36 mL, 25 mmol, 2.2 eq) in dry dichloromethane (56 mL) at 0 °C. The reaction mixture is stirred for 1 h at 0 °C and at room temperature for an additional h. The mixture is diluted with dichloromethane. The organic mixture is washed ($H_2O$), dried and concentrated to obtain the desired product.

***General method F: enamine formation from chromanone derivatives and primary amines***

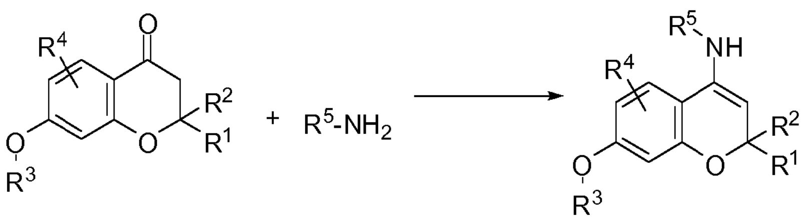

In a typical procedure, a mixture of chromanone derivative (1 eq), the primary amine (2 to 4 eq) and $Ti(OiPr)_4$ (1.2 to 2 eq) in 1,2-dichloroethane is stirred at room temperature to 60 °C for 4 to 18 h. The mixture is diluted with dichloromethane. Aqueous NaOH is added and the resulting mixture is filtered through celite. The two phases are separated. The organic layer is dried and concentrated to afford the desired product.

***Illustrative example of method F: synthesis of intermediate 39, N-cyclopropyl-6-fluoro-2-isopropyl-7-(3-methoxypropoxy)-2H-chromen-4-amine***

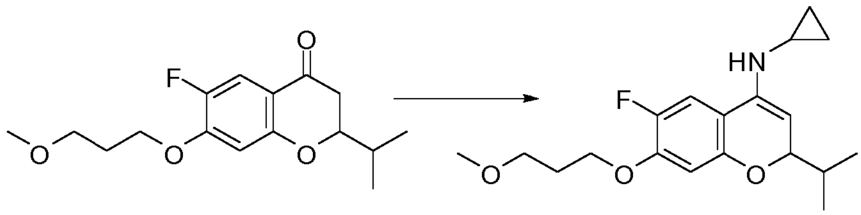

$Ti(OiPr)_4$ (3.6 mL, 12.1 mmol, 1.2 eq) is added to a mixture of 6-fluoro-2-isopropyl-7-(3-methoxypropoxy)chroman-4-one (3 g, 10.1 mmol, 1 eq) and cyclopropanamine (CAS: 765-30-0, 1.16 g, 20.2 mmol, 2 eq) in 1,2-dichloroethane (50 mL). The mixture is stirred at 50 °C for 4 h. The mixture is diluted with dichloromethane. 1 N NaOH is added and the resulting mixture is filtered through celite. The two phases are separated. The organic layer is dried (filtered through phase separator) and concentrated to afford the desired product.

***Illustrative example of method F: synthesis of intermediate 40, 6-chloro-N-cyclopropyl-7-(3-methoxypropoxy)-2-(3-methyloxetan-3-yl)-2H-chromen-4-amine***

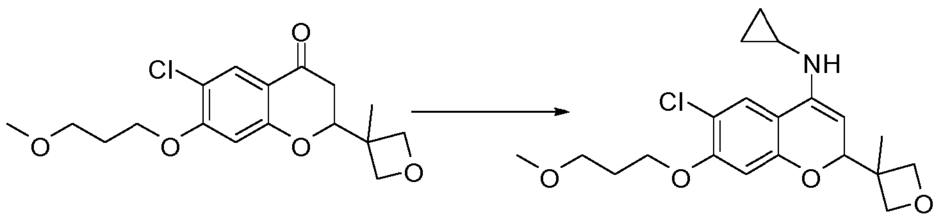

$Ti(OiPr)_4$ (1.4 mL, 4.9 mmol, 1.2 eq) is added to a mixture of 6-chloro-7-(3-methoxypropoxy)-2-(3-methyloxetan-3-yl)chroman-4-one (1.39 g, 4.1 mmol, 1 eq) and cyclopropanamine (CAS: 765-30-0, 0.465 g, 8.2 mmol, 2 eq) in 1,2-dichloroethane (41 mL). The mixture is stirred at room temperature for 2 h and at 60 °C for 1 h. An additional 0.3 mL of cyclopropanamine (1.06 eq) and 0.7 mL of $Ti(OiPr)_4$ (0.8 eq) are added and the mixture is stirred at 60 °C for 16 h. 11 mL of 2 M NaOH are added and the mixture is stirred for 1 h. The mixture is filtered and the filtrate is washed with brine. The two phases are separated. The organic layer is dried ($Na_2SO_4$) and concentrated to afford the desired product.

***General method G: condensation of enamines with diethyl 2-(ethoxymethylene)propanedioate***

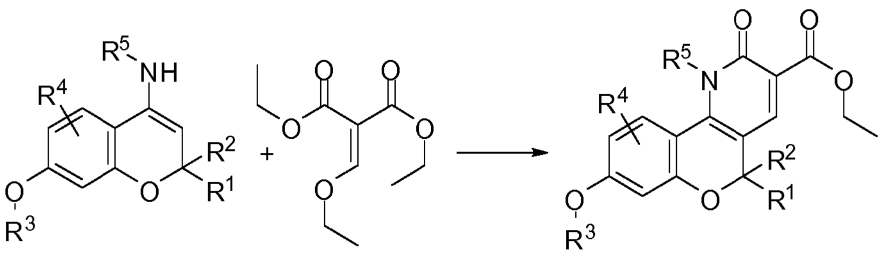

In a typical procedure, a mixture of the enamine derivative (1 eq) and diethyl 2-(ethoxymethylene)propanedioate (4 to 5 eq) is stirred at 130 to 140°C for 16 to 90 h. The mixture is adsorbed on silica and purified by flash column chromatography to yield the desired product.

***Illustrative example of method G: synthesis of intermediate 64, ethyl 1-cyclopropyl-9-fluoro-5-isopropyl-8-(3-methoxypropoxy)-2-oxo-5H-chromeno[4,3-b]pyridine-3-carboxylate***

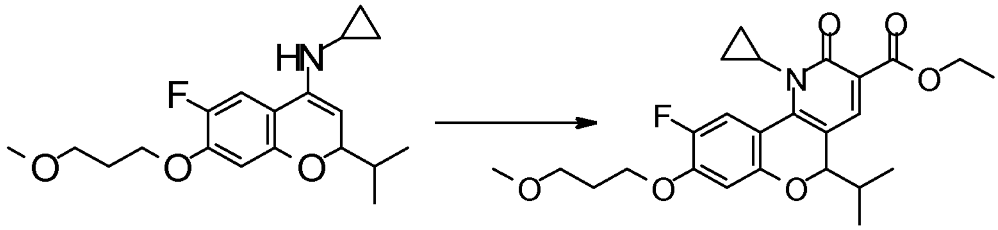

A mixture of N-cyclopropyl-6-fluoro-2-isopropyl-7-(3-methoxypropoxy)-2H-chromen-4-amine (10.1 mmol) and diethyl 2-(ethoxymethylene)propanedioate (CAS: 87-13-8, 13 mL, 45 mmol, 4.5 eq) is stirred at 130 °C for 16h. After cooling, the mixture is adsorbed on silica and purified by flash column chromatography ($SiO_2$, dichloromethane/methanol 100:0 to 98:2) to yield the desired product.

***Illustrative example of method G: synthesis of intermediate 65, ethyl 9-chloro-1-cyclopropyl-8-(3-methoxypropoxy)-5-(3-methyloxetan-3-yl)-2-oxo-5H-chromeno[4,3-b]pyridine-3-carboxylate***

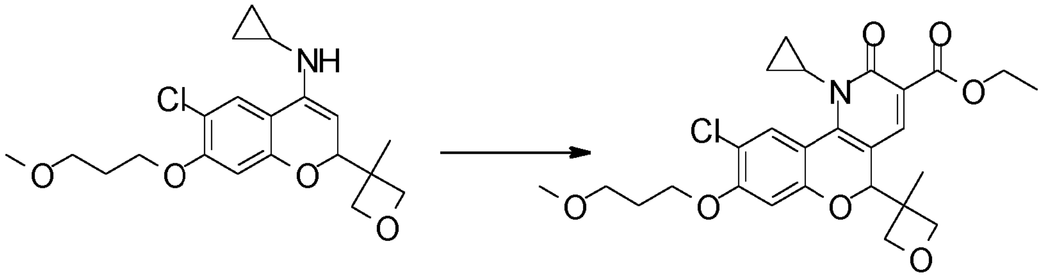

A mixture of 6-chloro-N-cyclopropyl-7-(3-methoxypropoxy)-2-(3-methyloxetan-3-yl)-2H-chromen-4-amine (4.1 mmol) and diethyl 2-(ethoxymethylene)propanedioate (CAS: 87-13-8, 3.3 mL, 16.3 mmol, 4 eq) is stirred at 130 °C for 16 h and at 140 °C for 24 h. After cooling, the mixture is diluted (dichloromethane), adsorbed on silica and purified by flash column chromatography ($SiO_2$, column eluted first with petroleum ether/ethyl acetate 100:0 to 50:50 and then with dichloromethane/methanol 100:0 to 97:3) to yield the desired product.

***General method h: benzyl group deprotection by catalyzed hydrogenation***

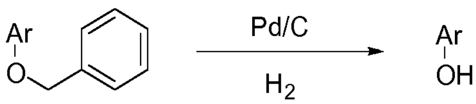


A mixture of the benzyl protected aryloxy derivative (1 eq) and palladium on carbon (10% weight, 0.1 eq) in ethyl acetate or methanol is stirred at room temperature for 18 h. The mixture is filtered and concentrated to afford the desired product.

***Illustrative example of method H: synthesis of intermediate 88, ethyl 1-cyclopropyl-8-hydroxy-5-isopropyl-9-methoxy-2-oxo-5H-chromeno[4,3-b]pyridine-3-carboxylate***

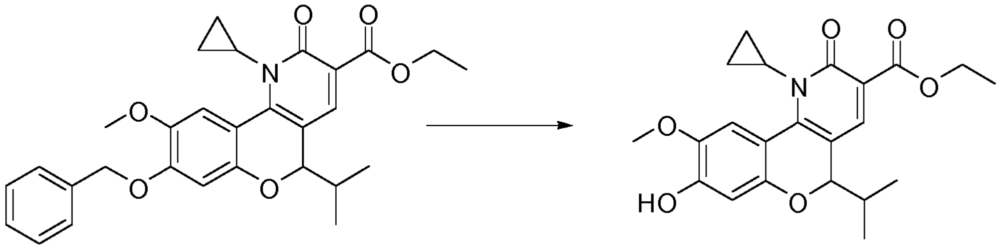

A mixture of ethyl 8-benzyloxy-1-cyclopropyl-5-isopropyl-9-methoxy-2-oxo-5H-chromeno[4,3-b]pyridine-3-carboxylate (5.8 g, 11.9 mmol, 1 eq) and 10% w/w palladium on carbon (1.3 g, 1.2 mmol, 0.1 eq) in ethyl acetate (59 mL) is stirred under 1 atm of $h_2$ at room temperature for 18 h. The mixture is flushed with a stream of $N_2$ and filtered on a celite pad. The filtrate is concentrated and taken up in dichloromethane. The organic mixture is dried (filtered on a phase separator) and concentrated. The residue is taken up in a small volume of dichloromethane and the desired product is precipitated by adding petroleum ether. The solid is further dried in a vacuum oven under reduced pressure.

***Synthesis of intermediate 90, ethyl 9-chloro-1-cyclopropyl-8-hydroxy-5-isopropyl-2-oxo-5H-chromeno[4,3-b]pyridine-3-carboxylate***

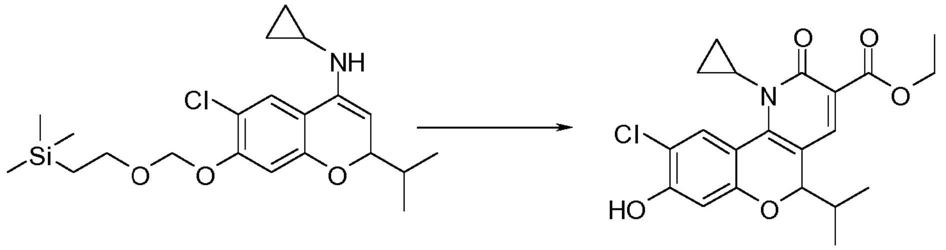

A mixture of 6-chloro-N-cyclopropyl-2-isopropyl-7-(2-trimethylsilylethoxymethoxy)-2H-chromen-4-amine (10.8 mmol) and diethyl 2-(ethoxymethylene)propanedioate (CAS: 87-13-8, 14 mL, 43.2 mmol, 4 eq) is stirred at 130 °C for 14.5 h and at 135 °C for 60 h. After cooling, the mixture is diluted (dichloromethane), adsorbed on silica and purified by flash column chromatography ($SiO_2$, petroleum ether/ethyl acetate 90:10 to 0:100). The resulting solid is triturated in acetonitrile/MTBE to afford ethyl 9-chloro-1-cyclopropyl-8-hydroxy-5-isopropyl-2-oxo-5H-chromeno[4,3-b]pyridine-3-carboxylate.

***Synthesis of intermediate 91, ethyl 1-cyclopropyl-5-isopropyl-9-methoxy-8-(3-methoxypropoxy)-2-oxo-5H-chromeno[4,3-b]pyridine-3-carboxylate***

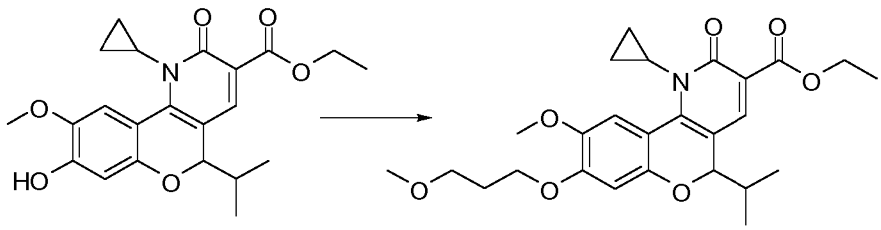

A mixture of ethyl 1-cyclopropyl-8-hydroxy-5-isopropyl-9-methoxy-2-oxo-5H-chromeno[4,3-b]pyridine-3-carboxylate (315 mg, 0.79 mmol, 1 eq), 1-bromo-3-methoxy-propane (CAS: 36865-41-5, 144 mg, 0.95 mmol, 1.2 eq) and $K_2CO_3$ (218 mg, 1.58 mmol, 2 eq) in DMF (2 mL) is stirred at room temperature for 96 h. The mixture is diluted with water and extracted with dichloromethane and ethyl acetate. The aqueous layer is concentrated and taken up in dichloromethane. All solids are filtered off and the filtrate is combined with the other organic layers. The combined organic layers are concentrated and the residue is purified by flash column chromatography ($SiO_2$, dichloromethane/methanol

100:0 to 97:3) to afford the desired product.

***Synthesis of intermediate 92, ethyl 1-cyclopropyl-5-(hydroxymethyl)-8,9-dimethoxy-2-oxo-5H-chromeno[4,3-b]pyridine-3-carboxylate***

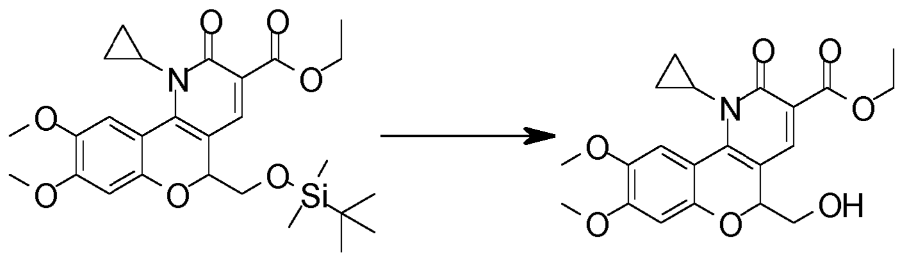

A solution of 1 M tetra-n-butylammonium fluoride in THF (CAS: 429-41-4, 0.15 mL, 0.15 mmol, 1.2 eq) is added to a solution of ethyl 5-[[tert-butyl(dimethyl)silyl]oxymethyl]-1-cyclopropyl-8,9-dimethoxy-2-oxo-5H-chromeno[4,3-b]pyridine-3-carboxylate (64 mg, 0.124 mmol, 1 eq) in dry THF at 0 °C. The mixture is stirred at room temperature for 3 h. The mixture is quenched with saturated aqueous $NH_4Cl$. The mixture is extracted with ethyl acetate. The two phases are separated and the organic layer is washed (saturated aqueous $NH_4Cl$ and brine), dried ($MgSO_4$) and concentrated. The residue is purified by flash column chromatography ($SiO_2$, column eluted first with petroleum ether/ethyl acetate 50:50 to 0:100 and then with ethyl acetate/methanol 100:0 to 95:5) to afford the desired product.

***Synthesis of intermediate 93, ethyl 1-cyclopropyl-5-isopropyl-9-methoxy-2-oxo-8-(trideuteriomethoxy)-5H-chromeno[4,3-b]pyridine-3-carboxylate***

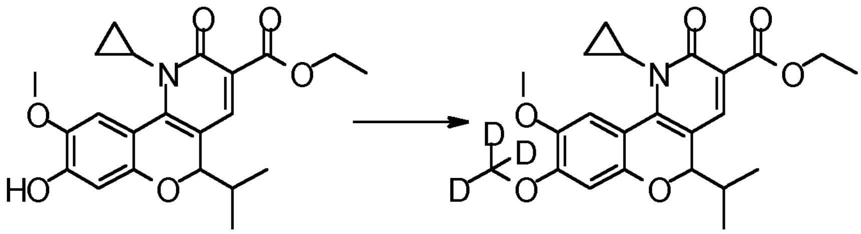

A mixture of ethyl 1-cyclopropyl-8-hydroxy-5-isopropyl-9-methoxy-2-oxo-5H-chromeno[4,3-b]pyridine-3-carboxylate (50 mg, 0.125 mmol, 1 eq), iodomethane-$d_3$ (CAS: 865-50-9, 36 mg, 0.25 mmol, 2 eq) and $K_2CO_3$ (35 mg, 0.25 mmol, 2 eq) in DMF (1.5 mL) is stirred at 70 °C for 2 h. The mixture is partitioned between dichlormethane and water. The two phases are separated. The organic layer is dried (filtered through phase separator) and concentrated to afford the desired product.

***Synthesis of intermediate 94, ethyl 9-cyano-1-cyclopropyl-5-isopropyl-8-(3-methoxypropoxy)-2-oxo-5H-chromeno[4,3-b]pyridine-3-carboxylate***

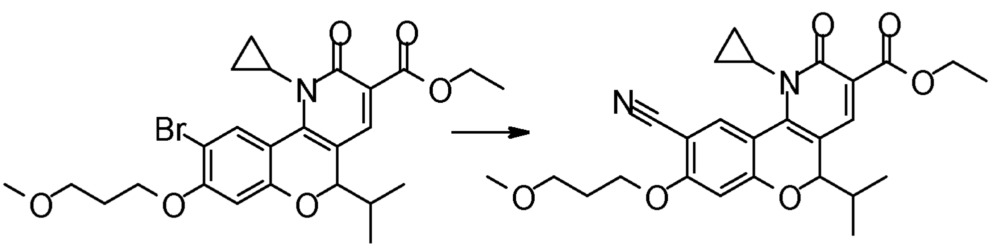

A mixture of ethyl 9-bromo-1-cyclopropyl-5-isopropyl-8-(3-methoxypropoxy)-2-oxo-5H-chromeno[4,3-b]pyridine-3-carboxylate (100 mg, 0.19 mmol, 1 eq), $Zn(CN)_2$ (23 mg, 0.19 mmol, 1 eq) and $Pd(PPh_3)_4$ (23 mg, 0.02 mmol, 0.1 eq) in a sealed tube is flushed with $N_2$. DMF (1 mL) is added and the mixture is stirred at 100 °C for 16 h. The mixture is partitioned between dichloromethane and water at basic pH. The two phases are separated and the organic layer is concentrated. The residue is purified by flash column chromatography ($SiO_2$, petroleum ether/ethyl acetate 90:10 to 0:100) to afford the desired product.

***1.2. Illustrative Compounds of the invention***

***General method I: ester hydrolysis***

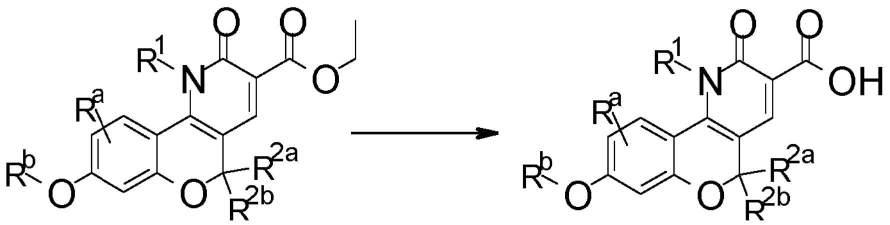

In a typical procedure, a mixture of the ester derivative (1 eq) and LiOH•$H_2O$ (2.2 to 5 eq) in 2:1 1,4-dioxane/$H_2O$ or 2:1 to 4:1 THF/$H_2O$ is stirred at room temperature to 50 °C for 1 to 16 h. The mixture is acidified with 1 N HCl and extracted with an organic solvent. The organic layer is concentrated to afford the desired product. Alternatively, the product may precipitate before or after work up and it may be further purified by washing or additional work up. In all cases, the final product may be further purified by flash column chromatography of preparative HPLC.

***Illustrative example of method I: synthesis of compound 1, 1-cyclopropyl-9-fluoro-5-isopropyl-8-(3-methoxypropoxy)-2-oxo-5H-chromeno[4,3-b]pyridine-3-carboxylic acid***

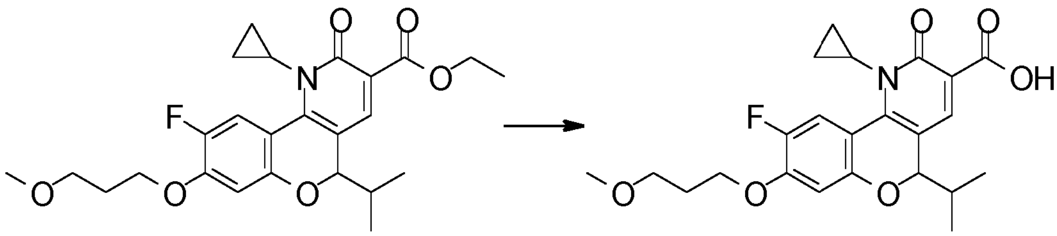

A mixture of ethyl 1-cyclopropyl-9-fluoro-5-isopropyl-8-(3-methoxypropoxy)-2-oxo-5H-chromeno[4,3-b]pyridine-3-carboxylate (13 g, 28.3 mmol, 1 eq) and LiOH•$H_2O$ (2.72 g, 64.8 mmol, 2.3 eq) in 2:1 THF/$H_2O$ (140 mL) is stirred at room temperature for 2 h. The mixture is acidified with 1 M HCl and extracted with dichloromethane. The two phases are separated. The organic layer is dried (filtered through phase separator) and concentrated. The residue is triturated with acetonitrile/MTBE to afford the desired product.

***Illustrative example of method I: synthesis of compound 2, 9-chloro-1-cyclopropyl-8-(3-methoxypropoxy)-5-(3-methyloxetan-3-yl)-2-oxo-5H-chromeno[4,3-b]pyridine-3-carboxylic acid***

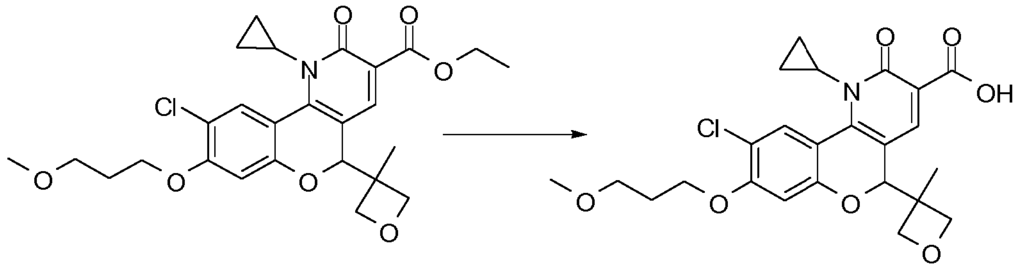

A mixture of ethyl 9-chloro-1-cyclopropyl-8-(3-methoxypropoxy)-5-(3-methyloxetan-3-yl)-2-oxo-5H-chromeno[4,3-b]pyridine-3-carboxylate (1.47 g, 2.92 mmol, 1 eq) and LiOH•$H_2O$ (0.49 g, 11.7 mmol, 4 eq) in 4:1 THF/$H_2O$ (41 mL) is stirred at room temperature for 1.5 h. A solid is formed and filtered out. The solid is washed with petroleum ether and partitioned between dichloromethane and 1 M HCl. The two phases are separated and the organic layer is washed (brine), dried ($Na_2SO_4$) and concentrated. The residue is further dried in an oven under reduced pressure to afford the desired compound.

***Alternative synthesis of compound 2, 9-chloro-1-cyclopropyl-8-(3-methoxypropoxy)-5-(3-methyloxetan-3-yl)-2-oxo-5H-chromeno[4,3-b]pyridine-3-carboxylic acid***

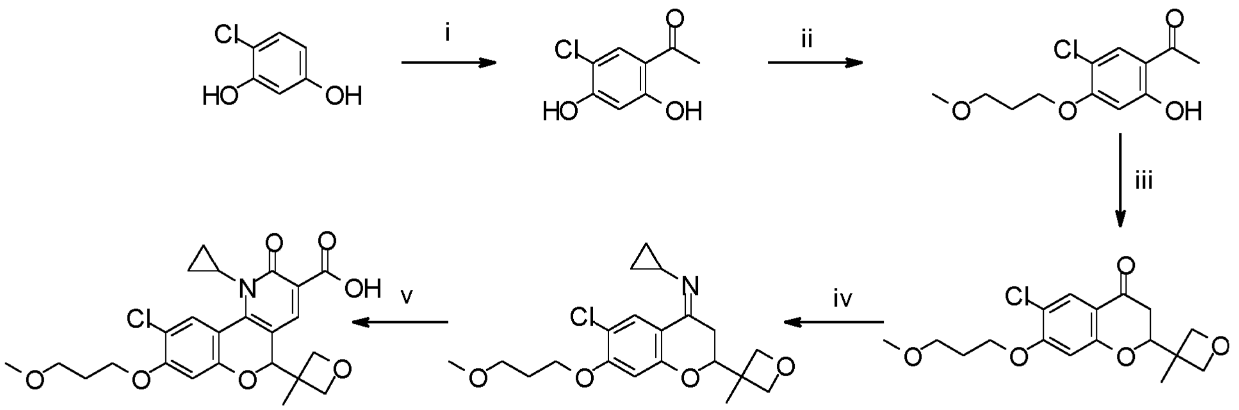


***Step i: 1-(5-chloro-2,4-dihydroxy-phenyl)ethanone***

Boron trifluoride etherate (512mL, 4.15 mol, 1.2 eq) was added to a solution of 4-chlororesorcinol (500g, 3.46 mol, 1.0 eq), acetic anhydride (490mL, 5.19 mol, 1.5 eq) in EtOAc (1500 mL). The reaction mixture was stirred at reflux for 17h. The reaction mixture was then cooled to room temperature and neutralized with 8% aqueous $NaHCO_3$ to pH between 3 and 4. The aqueous phase was extracted with EtOAc. The combined organic phases were washed successively with water and 20% aqueous NaCl and then partially concentrated such as to leave around 500mL of EtOAc. Following addition of heptane to the solution, the expected product crystallized and two crops were obtained. The two crops were combined and further dried in an oven under reduced pressure to afford the desired compound.

***Step ii: 1-[5-chloro-2-hydroxy-4-(3-methoxypropoxy)phenyl]ethanone***

1-bromo-3-methoxypropane (643mL, 5.71 mol, 1.05 eq) was added to a suspension of 1-(5-chloro-2,4-dihydroxy-phenyl)ethanone (1015g, 5.44 mol, 1.0 eq) and $Na_2CO_3$ (605g, 5.71 mol, 1.05 eq) in butyronitrile (7.5L). The reaction mixture was then stirred at reflux for 60h and at room temperature for 12h. The reaction mixture was cooled to room temperature and filtered; the cake was washed with MTBE. The filtrate was extracted several times with aqueous NaOH 2M. The combined aqueous phases were then neutralized to pH around 7 with concentrated aqueous HCl to induce crystallization of the product. The suspension was filtered and the cake washed with successively water and heptane. The solid was dried in an oven under reduced pressure to afford the desired compound.

***Step iii: 6-chloro-7-(3-methoxypropoxy)-2-(3-methyloxetan-3-yl)chroman-4-one***

Pyrrolidine (97mL, 1.16 mol, 0.25 eq) was added to a suspension of 1-[5-chloro-2-hydroxy-4-(3-methoxypropoxy)phenyl]ethanone (1200 g, 4.64 mol, 1.0 eq) and 3-methyloxetane-3-carbaldehyde (604 g, 6.03 mol, 1.3 eq) in MeOH (4000 mL). The reaction mixture was stirred at reflux for 18h and cooled to room temperature. Water (2000 mL) was slowly added to induce crystallization of the product. The suspension was filtered and the cake was washed with MeOH/water 1:1 (2000 mL). The solid was collected and dried in an oven under reduced pressure to afford the desired compound.

***Step iv: 6-chloro-N-cyclopropyl-7-(3-methoxypropoxy)-2-(3-methyloxetan-3-yl)chroman-4-imine***

Cyclopropylamine (870 mL, 12.5 mol, 4.0 eq) and acetic acid (36 mL, 0.63 mol, 0.2 eq) were successively added to a suspension of 6-chloro-7-(3-methoxypropoxy)-2-(3-methyloxetan-3-yl)chroman-4-one (1069 g, 3.14 mol, 1.0 eq) in EtOH (5.3 L). The reaction mixture was stirred at reflux for 3h30 and cooled to room temperature. Water (2.5 L) was slowly added to the reaction mixture to induce crystallization of the product. The suspension was filtered and the cake washed with EtOH/water 1:1 (2 L). The solid was collected and dried in an oven under reduced pressure to afford the desired compound.

***Step v: 9-chloro-1-cyclopropyl-8-(3-methoxypropoxy)-5-(3-methyloxetan-3-yl)-2-oxo-5H-chromeno [4,3-b]pyridine-3-carboxylic acid***

5-(Methoxymethylene)-2,2-dimethyl-1,3-dioxane-4,6-dione (363g, 1.95 mol, 1.3 eq) was added to a suspension of 6-chloro-N-cyclopropyl-7-(3-methoxypropoxy)-2-(3-methyloxetan-3-yl)chroman-4-imine (570 g, 1.5 mol, 1.0 eq) in DMSO (1200 mL). The reaction mixture was stirred at 50°C for 1h30. Aqueous NaOH 2M (1200mL, 2.4 mol, 1.6 eq) was added to the reaction mixture and this latter was stirred at 50°C for 45min. Methanol (3500 mL) was added to the reaction mixture which was then cooled to room temperature. The reaction mixture was slowly neutralized with aqueous HCl 2M (1200 mL, 2.4 mol, 1.6 eq). Crystallization occurred. The suspension was filtered and the cake was washed with MeOH (1500 mL). The solid was collected and dried in an oven under reduced pressure to afford the desired compound.

***Synthesis of deuterated compound 2, 9-chloro-8-(3-methoxypropoxy)-5-(3-methyloxetan-3-yl)-2-oxo-1-(1,2,2,3,3-pentadeuteriocyclopropyl)-5H-chromeno[4,3-b]pyridine-3-carboxylic acid***

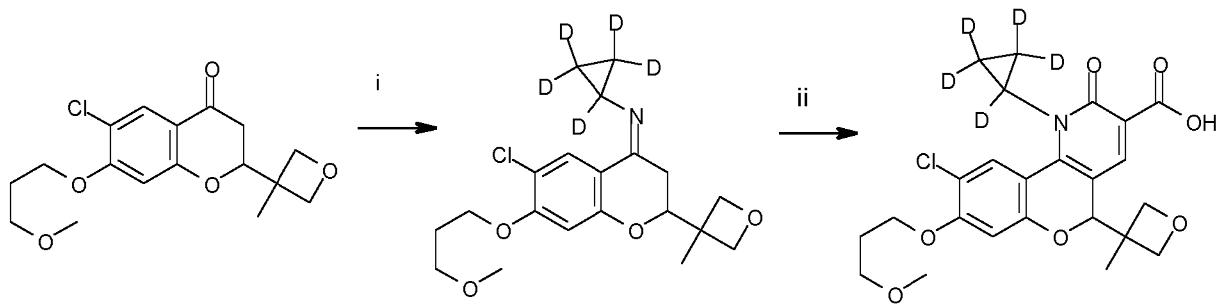


*Step i: 6-chloro-7-(3-methoxypropoxy)-2-(3-methyloxetan-3-yl)-N-(1,2,2,3,3-pentadeuteriocyclopropyl) chroman-4-imine*

$TiCl_4$ 1M in DCM (1.95 mL, 1.95 mmol, 0.5 eq) was added to a solution of 6-chloro-7-(3-methoxypropoxy)-2-(3-methyloxetan-3-yl)chroman-4-one (1.3 g, 3.82 mol, 1.0 eq) and cyclopropylamine D5 (CAS#53557-90-0, 0.52g, 8.39 mmol, 2.2 eq) in DCM (5 mL) cooled with an ice bath. The reaction mixture was stirred for 1h and MTBE (10 mL) was added. The reaction mixture was filtered on dicalite and the cake of dicalite was washed with MTBE. The filtrate was concentrated to afford the desired crude compound which was used as such.

*Step ii: 9-chloro-8-(3-methoxypropoxy)-5-(3-methytoxetan-3-yl)-2-oxo-1-(1,2,2,3,3-pentadeuterio cyclopropyl)-5H-chromeno[4,3-b]pyridine-3-carboxylic acid*

5-(Methoxymethylene)-2,2-dimethyl-1,3-dioxane-4,6-dione (428mg, 2.3 mmol, 1.7 eq) was added to a suspension of 6-chloro-7-(3-methoxypropoxy)-2-(3-methyloxetan-3-yl)-N-(1,2,2,3,3-pentadeuteriocyclopropyl)chroman-4-imine (520mg, 1.35 mol, 1.0 eq) in DMSO (1mL). The reaction mixture was stirred at 50°C for 3h. Aqueous NaOH 2M (1mL, 2.0 mmol, 1.6 eq) was added to the reaction mixture and this latter was stirred at 50°C for 45min. Methanol (5mL) was added to the reaction mixture which was then cooled to room temperature. The reaction mixture was slowly neutralized with aqueous HCl 2M (1mL, 2.0 mmol, 1.6 eq). Crystallization occurred. The suspension was filtered and the cake was washed with MeOH (2mL). The solid was purified by preparative HPLC to afford the desired compound.

***Illustrative example of method I: synthesis of compound 3, 1-cyclopropyl-5-isopropyl-8,9-dimethoxy-2-oxo-5H-chromeno[4,3-b]pyridine-3-carboxylic acid***

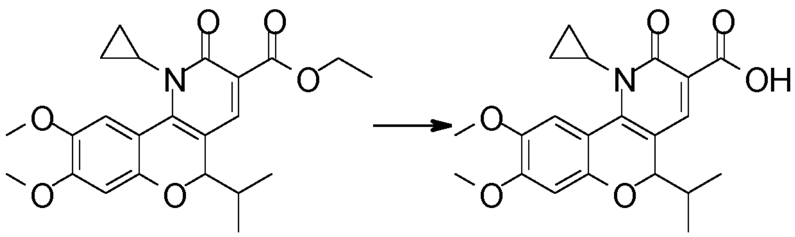

A mixture of ethyl 1-cyclopropyl-5-isopropyl-8,9-dimethoxy-2-oxo-5H-chromeno[4,3-b]pyridine-3-carboxylate (103 mg, 0.25 mmol, 1 eq) and LiOH•$H_2O$ (52 mg, 1.25 mmol, 5 eq) in 2:1 1,4-dioxane/$H_2O$ (1 mL) is stirred at 50 °C for 1 h. The mixture is diluted with $H_2O$ and acidified with 1 N HCl. A precipitate is formed and filtered off. The excess of solvent is removed by freeze-drying to obtain the desired product.

***General method J: alkylation of aryloxy derivatives by Mitsunobu reaction followed by hydrolysis***

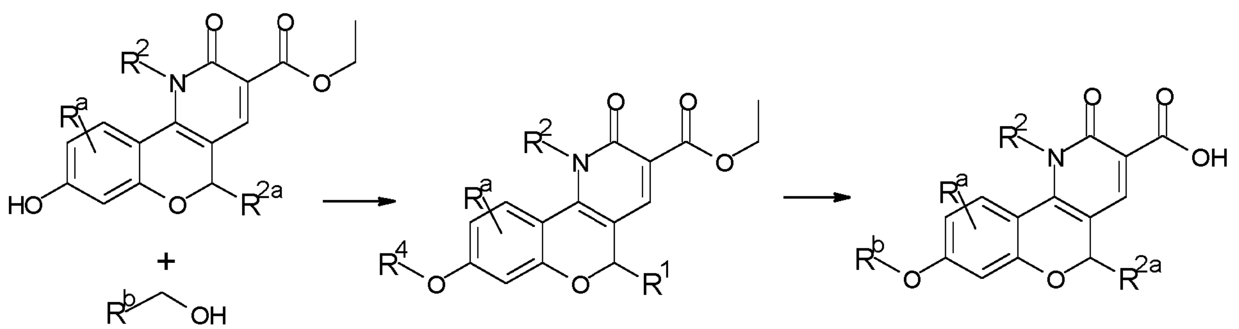

In a typical procedure, di-tert-butyl azodicarboxylate (2.4 eq) is added to a solution of the aryloxy derivative (1 eq), the alcohol (1.2 eq) and $PPh_3$ (2.4 eq) in THF under $N_2$ at 0°C. The mixture is stirred at room temperature for 18 h. The mixture is diluted with THF and $H_2O$ to reach a total of 2:1 THF/$H_2O$. LiOH•$H_2O$ (3 eq) is added and the mixture is stirred at room temperature for 40 min to 1 h. The mixture is partitioned between 1 N HCl and dichloromethane. The two phases are separated and the organic layer is washed with brine, dried and concentrated to afford the desired product.

***Illustrative example of method J: synthesis of compound 5, 9-chloro-1-cyclopropyl-5-isopropyl-8-(oxetan-3-ylmethoxy)-2-oxo-5H-chromeno[4,3-b]pyridine-3-carboxylic acid***

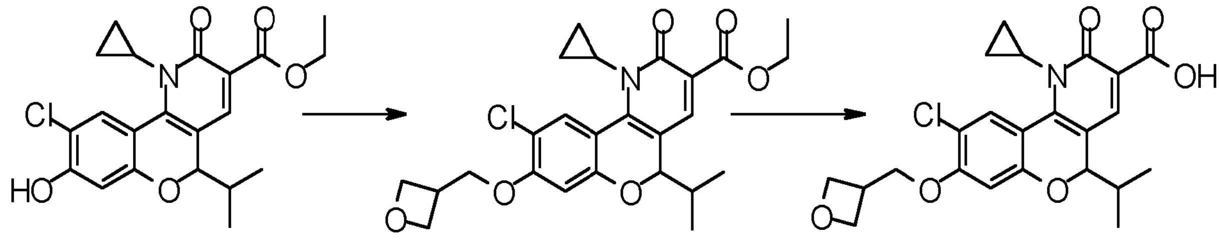

Di-tert-butyl azodicarboxylate (CAS: 870-50-8, 136 mg, 0.6 mmol, 2.4 eq) is added to a solution of ethyl 9-chloro-1-cyclopropyl-8-hydroxy-5-isopropyl-2-oxo-5H-chromeno[4,3-b]pyridine-3-carboxylate (100 mg, 0.25 mmol, 1 eq), oxetan-3-ylmethanol (CAS: 6246-06-6, 28 mg, 0.3 mmol, 1.2 eq) and $PPh_3$ (156 mg, 0.6 mmol, 2.4 eq) in THF under $N_2$ at 0 °C. The mixture is stirred at room temperature for 18 h. A second reaction using the same quantities is started and stirred at room temperature for 18 h. Additional 3-oxetanemethanol (0.2 eq), $PPh_3$ (0.4 eq) and di-tert-butyl azodicarboxylate (0.4 eq) are added. The mixture is stirred for a further 3 h to reach completion. The two reactions are mixed together and diluted with THF and $H_2O$ to reach a total of 2:1 THF/$H_2O$. LiOH•$H_2O$ (62 mg, 1.5 mmol, 3 eq) is added and the mixture is stirred for 40 min. The mixture is partitioned between 1 N HCl and dichloromethane. The two phases are separated and the organic layer is washed with brine, dried and concentrated. The residue is triturated with methanol and petroleum ether. The solid is dried in an oven under reduced pressure to afford the desired product.

***General method K: alkylation of aryloxy derivatives by reaction with alkyl halides followed by hydrolysis***

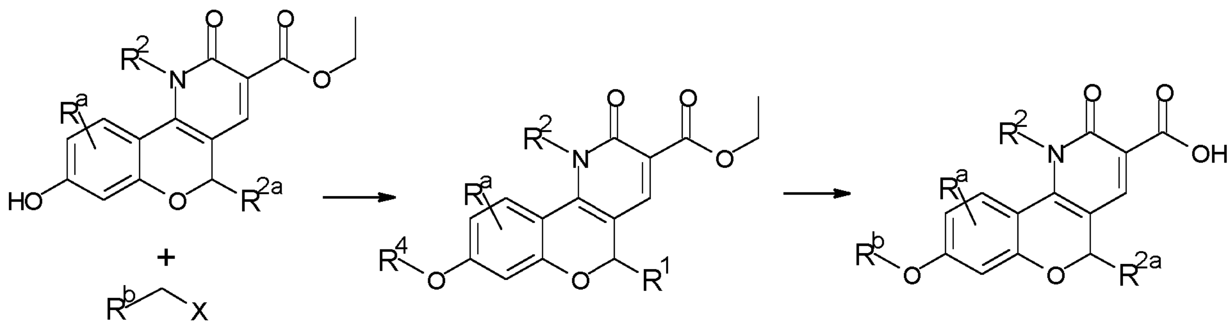

In a typical procedure, a mixture of the aryloxy derivative (1 eq), the alkylating agent (1.1 to 2 eq) and $K_2CO_3$ (2 eq) in DMF is stirred at 70 °C for 18 h. The mixture is diluted with 4:1 THF/$H_2O$ and LiOH•$H_2O$ (5 eq) is added. The mixture is stirred at room temperature for 18 to 70 h. The mixture is partitioned between ethyl acetate and HCl 1 N. The two phases are separated. The organic layer is washed (brine), dried and concentrated. The residue is triturated with methanol and petroleum ether to obtain the desired product, which may be further purified by preparative HPLC. The filtrate of the trituration may be recovered and purified by preparative HPLC.

***Illustrative example of method K: synthesis of compound 28, 1-cyclopropyl-5-isopropyl-9-methoxy-8-[(3-methyloxetan-3-yl)methoxy]-2-oxo-5H-chromeno[4,3-b]pyridine-3-carboxylic acid***

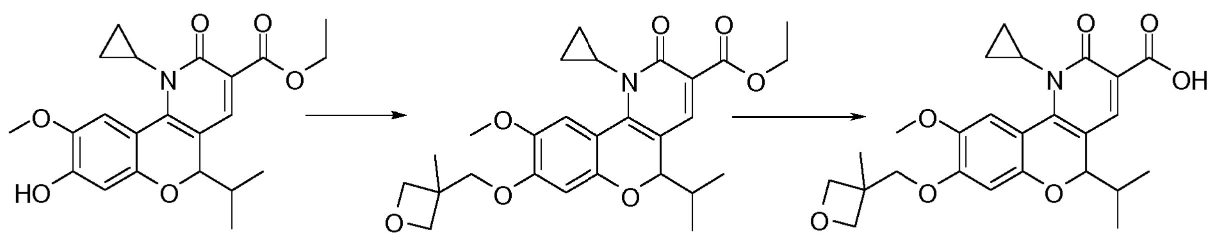

A mixture of ethyl 1-cyclopropyl-8-hydroxy-5-isopropyl-9-methoxy-2-oxo-5H-chromeno[4,3-b]pyridine-3-carboxylate (50 mg, 0.125 mmol, 1 eq), 3-(bromomethyl)-3-methyloxetane (CAS: 78385-26-9,41 mg, 0.25 mmol, 2 eq) and $K_2CO_3$ (35 mg, 0.25 mmol, 2 eq) in in DMF is stirred at 70 °C for 18 h. The mixture is diluted with 4:1 THF/$H_2O$ (5 mL) and LiOH•$H_2O$ (5 eq) is added. The mixture is stirred at room temperature for 70 h. The mixture is partitioned between ethyl acetate and HCl 1 N. The two phases are separated. The organic layer is washed (brine), dried ($Na_2SO_4$) and concentrated. The residue is triturated with methanol and petroleum ether to obtain the desired product

***Synthesis of compound 43, 8-[(1-cyanocyclobutyl)methoxy]-1-cyclopropyl-5-isopropyl-9-methoxy-2-oxo-5H-chromeno[4,3-b]pyridine-3-carboxylic acid***

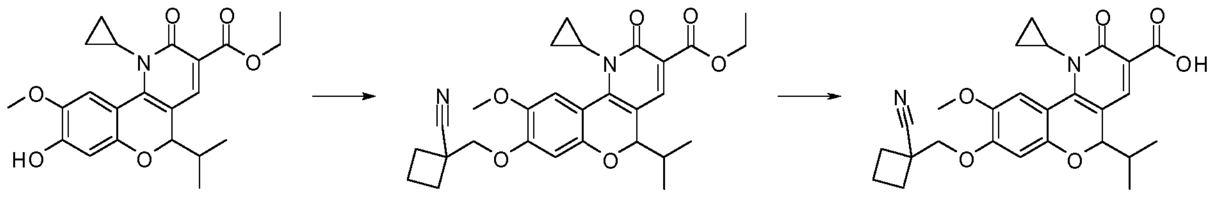

Di-tert-butyl azodicarboxylate (CAS: 870-50-8, 69 mg, 0.3 mmol, 2.4 eq) is added to a solution of ethyl 9-chloro-1-cyclopropyl-8-hydroxy-5-isopropyl-2-oxo-5H-chromeno[4,3-b]pyridine-3-carboxylate (50 mg, 0.125 mmol, 1 eq), 1-(hydroxymethyl) cyclobutanecarbonitrile (CAS: 956531-83-2, 17 mg, 0.2 mmol, 1.2 eq) and $PPh_3$ (79 mg, 0.3 mmol, 2.4 eq) in THF under $N_2$ at 0 °C. The mixture is stirred at room temperature for 4.5 h. A solution of trimethylphosphoranylidene)acetonitrile in THF (0.5 M, CAS: 176325-83-0, 0.4 mL, 0.2 mmol, 1.6 eq) is added and the resulting mixture is stirred at 40 °C for 16 h. The mixture is diluted with 4:1 THF/$H_2O$ (5 mL) and LiOH•$H_2O$ (15 eq) is added. The mixture is stirred at 40 °C for 6 h. The mixture is partitioned between 1 N HCl and dichloromethane. The two phases are separated and the organic layer is washed (brine), dried ($MgSO_4$ and filtered through phase separator) and concentrated. The residue is triturated with methanol and dried in an oven under reduced pressure to afford the desired product.

***Illustrative compounds 44, (5S)-1-cyclopropyl-9-fluoro-5-isopropyl-8-(3-methoxypropoxy)-2-oxo-5H-chromeno[4,3-b]pyridine-3-carboxylic acid, and 45, (5R)-1-cyclopropyl-9-fluoro-5-isopropyl-8-(3-methoxypropoxy)-2-oxo-5H-chromeno[4,3-b]pyridine-3-carboxylic acid***

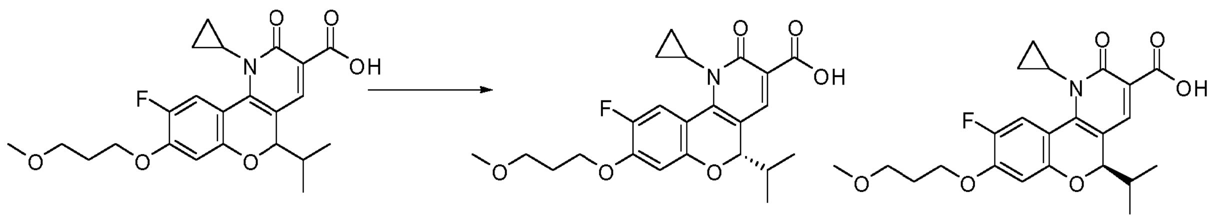

1-cyclopropyl-9-fluoro-5-isopropyl-8-(3-methoxypropoxy)-2-oxo-5H-chromeno[4,3-b]pyridine-3-carboxylic acid (270 mg, 0.63 mmol) undergoes a chiral separation (column: Lux® C4, 21.2 mm x 250 mm, 5 µm; mobile phase: isocratic, MeCN (0.1% v/v TFA)) to afford (5S)-1-cyclopropyl-9-fluoro-5-isopropyl-8-(3-methoxypropoxy)-2-oxo-5H-chromeno[4,3-b]pyridine-3-carboxylic acid and (5R)-1-cyclopropyl-9-fluoro-5-isopropyl-8-(3-methoxypropoxy)-2-oxo-5H-chromeno[4,3-b]pyridine-3-carboxylic acid.

***Illustrative compounds 46, (5S)-1-cyclopropyl-9-fluoro-5-isopropyl-8-(3-methoxypropoxy)-2-oxo-5H-chromeno[4,3-b]pyridine-3-carboxylic acid, and 47,(5R)-1-cyclopropyl-9-fluoro-5-isopropyl-8-(3-methoxypropoxy)-2-oxo-5H-chromeno[4,3-b]pyridine-3-carboxylic acid***

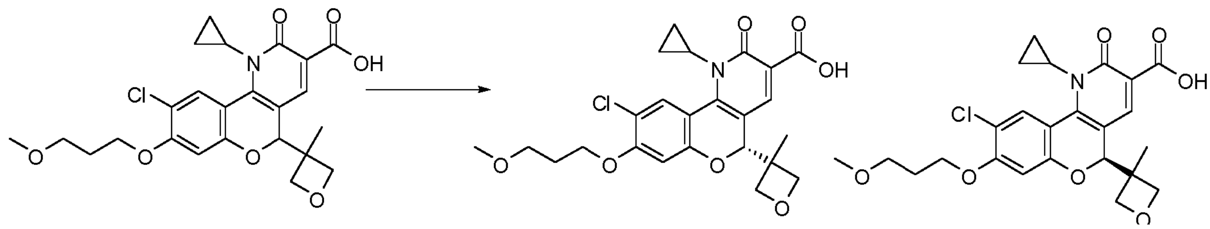

9-chloro-1-cyclopropyl-8-(3-methoxypropoxy)-5-(3-methyloxetan-3-yl)-2-oxo-5H-chromeno [4,3-b]pyridine-3-carboxylic acid (809 mg, 0.63 mmol) undergoes a chiral separation (column: Lux® C4, 21.2 mm x 250 mm, 5 μm; mobile phase: isocratic, MeCN (0.2% v/v TFA)) to afford (5R)-9-chloro-1-cyclopropyl-8-(3-methoxypropoxy)-5-(3-methyloxetan-3-yl)-2-oxo-5H-chromeno[4,3-b]pyridine-3-carboxylic acid and (5S)-9-chloro-1-cyclopropyl-8-(3-methoxypropoxy)-5-(3-methyloxetan-3-yl)-2-oxo-5H-chromeno[4,3-b]pyridine-3-carboxylic acid. Both products are further purified by preparative HPLC using the following conditions: column: Xselect CSH (30 mm x 150 mm, 5 μm); mobile phase: gradient, water (0.1% v/v Formic Acid)/acetonitrile.

***Synthesis of intermediate 107, 6-chloro-2-(3-methyloxetan-3-yl)-7-(2-trimethylsilylethoxy methoxy)chroman-4-one***

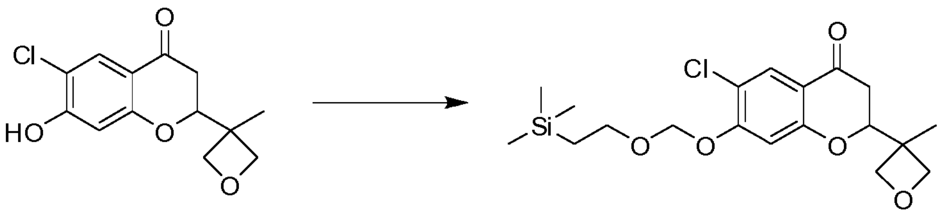

2-(Trimethylsilyl)ethoxymethyl chloride (3.7 mL, 20.7 mmol, 1.2 eq) is added very slowly over an h to a mixture of 6-chloro-7-hydroxy-2-(3-methyloxetan-3-yl)chroman-4-one (4.6 g, 17.3 mmol, 1 eq), and N,N-diisopropylethylamine (6.8 mL, 38.0 mmol, 2.2 eq) in dry dichloromethane (85 mL) at 0 °C. The reaction mixture is stirred for 1 h at 0°C and at room temperature for an additional h. The mixture is diluted with dichloromethane. The organic mixture is washed ($H_2O$), dried and concentrated to obtain the desired product.

***Synthesis of intermediate 114 ethyl 1-cyclopropyl-5-isopropyl-8-(3-methoxypropoxy)-2-oxo-9-(trifluoromethyl)-5H-chromeno[4,3-b]pyridine-3-carboxylate***

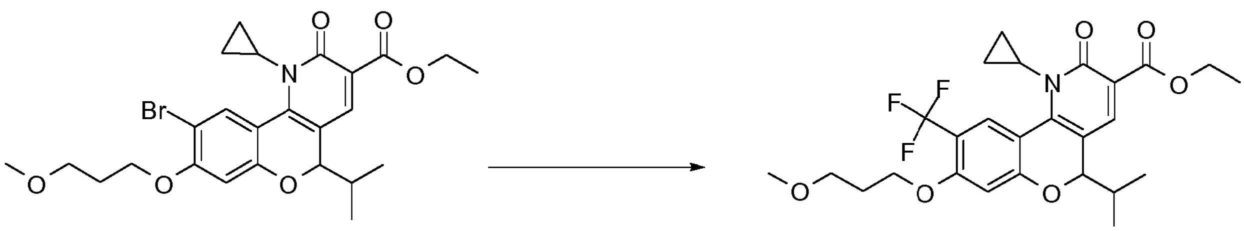

: Under $N_2$, a mixture of ethyl 9-bromo-1-cyclopropyl-5-isopropyl-8-(3-methoxypropoxy)-2-oxo-5H-chromeno[4,3-b]pyridine-3-carboxylate (30 mg, 0.058 mmol, 1 eq), potassium trifluoroacetate (53 mg, 0.30 mmol, 6 eq) and cupper iodine (66 mg, 0.30 mmol, 6 eq) in NMP (0.4 mL, 0.14 M) is stirred at 150°C for 18 hrs. The mixture is filtered over celite with dichloromethane. The filtrate is partitioned between ethyl acetate and water. The two phases are separated. The organic layer is washed (brine), dried ($Na_2SO_4$) and concentrated. The residue is purified by preparative HPLC to afford the desired product.

***Synthesis of intermediate 115 ethyl 1-cyclopropyl-5-isopropyl-8-(3-methoxypropoxy)-9-methylsulfonyl-2-oxo-5H-chromeno[4,3-b]pyridine-3-carboxylate***

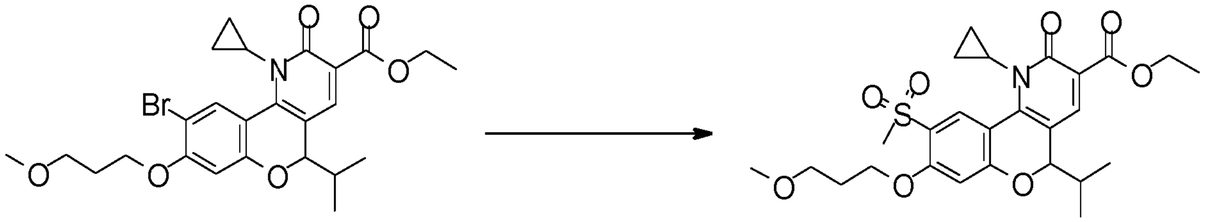

Under $N_2$, a mixture of ethyl 9-bromo-1-cyclopropyl-5-isopropyl-8-(3-methoxypropoxy)-2-oxo-5H-chromeno[4,3-b]pyridine-3-carboxylate (100 mg, 0.192 mmol, 1.0 eq), potassium metabisulfite (85 mg, 0.384 mmol, 2.0 eq), tetrabutylammonium bromide (68 mg, 0.21 mmol, 1.1 eq), sodium formate (29 mg, 0.42 mmol, 2.2 eq), palladium acetate (2.2 mg, 0.0096 mmol, 0.05 eq), triphenylphosphine (7.6 mg, 0.029 mmol, 0.15 eq) 1,10-phenanthroline (5.2 mg, 0.029 mmol, 0.15 eq) in acetonitrile (1 mL, 0.3 M) is stirred at 70°C for 2 hrs. Methyl iodide is added (41 mg, 0.29 mmol, 1.5 eq). The mixture is stirred at 70C° for 18 hrs.. The mixture is partitioned between dichloromethane and water. The two phases are separated. The organic layer is dried (filtered through phase separator) and concentrated. The residue is purified by preparative HPLC to afford the desired product.

***Synthesis of intermediate 123*** methyl 1-cyclopropyl-8-(3-methoxypropoxy)-5-(3-methyloxetan-3-yl)-2-oxo-5H-chromeno[4,3-b]pyridine-3-carboxylate

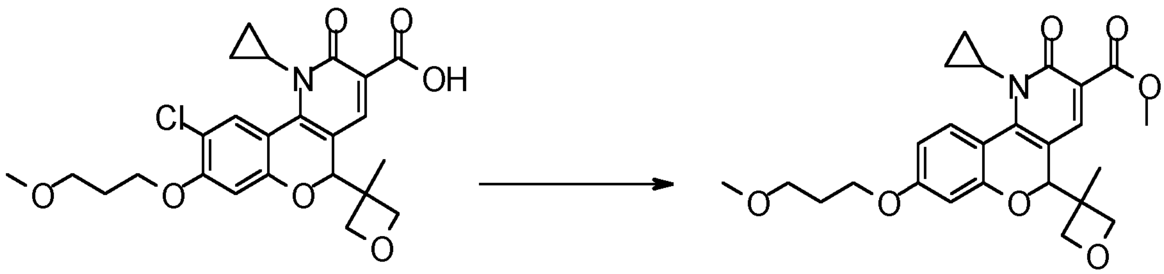

Under $N_2$, palladium on charcoal (45 mg, 0.04 mmol, 0.1 eq) is added to 9-chloro-1-cyclopropyl-8-(3-methoxypropoxy)-5-(3-methyloxetan-3-yl)-2-oxo-5H-chromeno[4,3-b]pyridine-3-carboxylic acid (200 mg, 0.42 mmol, 1.0 eq) in methanol (10 mL, 0.04 M). The mixture is stirred under $h_2$ 1 atm at room temperature for 18 hrs. The mixture is filtered over celite with dichloromethane and concentrated to afford the desired product.

***Synthesis of intermediate 124*** ethyl 9-chloro-1-cyclopropyl-5-(3-methyloxetan-3-yl)-2-oxo-8-(2,2,2-trifluoroethoxy)-5H-chromeno[4,3-b]pyridine-3-carboxylate

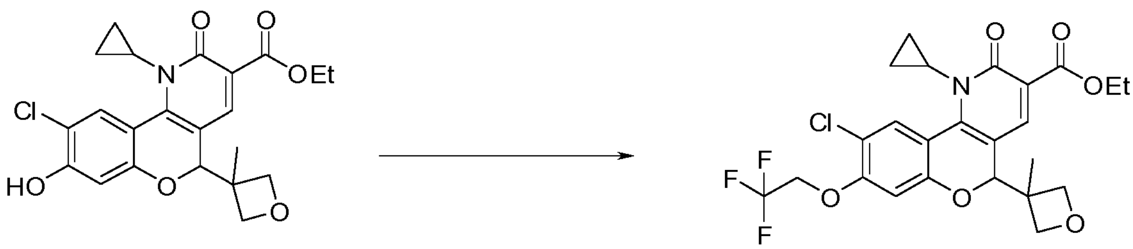

At 0°C, 2,2,2-trifluoroethyltrifluoromethane sulfonate (37 μL, 0.26 mmol, 1.2 eq) is added to a mixture of ethyl

9-chloro-1-cyclopropyl-8-hydroxy-5-(3-methyloxetan-3-yl)-2-oxo-5H-chromeno[4,3-b]pyridine-3-carboxylate (92 mg, 0.21 mmol, 1.0 eq) and cesium carbonate (140 mg, 0.43 mmol, 2.0 eq) in dry DMF (1.6 mL, 0.1 M). After 10 min, the ice bath is removed. The reaction mixture is stirred under $N_2$ atmosphere and at room temperature for 1 hr. The mixture is partitioned between dichloromethane and water. The two phases are separated. The organic layer is dried over sodium sulfate and concentrated to afford the desired product.

***Synthesis of intermediate 127 6-chloro-2-(3-methyloxetan-3-yl)-7-tetrahydropyran-2-yloxy-chroman-4-one***

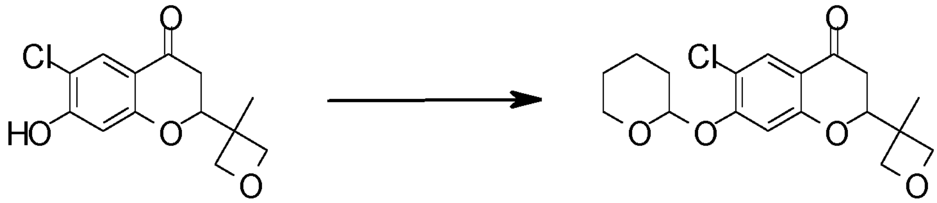

*p*-Toluenesulfonic acid monohydrate (14 mg, 0.04 mmol, 0.02 eq) is added at room temperature to a suspension of 6-chloro-7-hydroxy-2-(3-methyloxetan-3-yl)chroman-4-one (1.0 g, 3.72 mmol, 1.0 eq) and 3,4-dihydro-2H-pyran (1.02 mL, 11.2 mmol, 3.0 eq) in dry dichloromethane (8.0 mL, 0.5 M). The mixture is stirred at room temperature for 2 hrs. The mixture is partitioned between dichloromethane and water. The two phases are separated. The organic layer is washed (sat. $NaHCO_3$ solution and brine), dried ($Na_2SO_4$) and concentrated. The residue is purified by flash column chromatography ($SiO_2$, petroleum ether/ethyl acetate 75:25 to 0:100) to afford the desired product.

***Synthesis of intermediate 130*** 6-chloro-7-[[(2R)-1,4-dioxan-2-yl]methoxy]-2-(3-methyloxetan-3-yl)chroman-4-one.

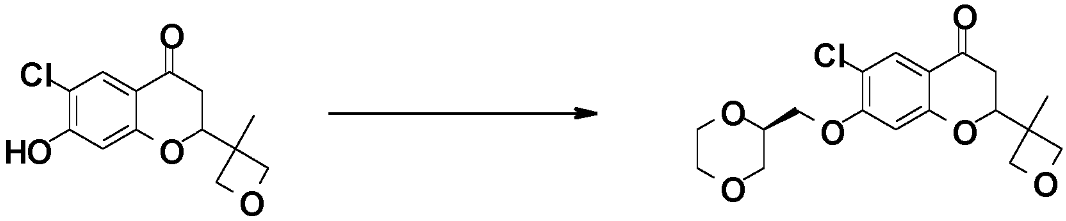

Di-tert-butyl azodicarboxylate (CAS: 870-50-8, 411 mg, 1.79 mmol, 2.4 eq) is added to a solution of 6-chloro-7-hydroxy-2-(3-methyloxetan-3-yl)chroman-4-one (200 mg, 0.74 mmol, 1.0 eq), (S)-(1,4-dioxan-2-yl)methanol (151 μL, 1.49 mmol, 2.0 eq) and $PPh_3$ (468 mg, 1.79 mmol, 2.4 eq) in THF (8.0 mL, 0.1 M) under $N_2$. The mixture is stirred at room temperature for 18 hrs. The mixture is partitioned between dichloromethane and water. The two phases are separated. The organic layer is dried over sodium sulfate and concentrated. The residue is purified by flash column chromatography ($SiO_2$, petroleum ether/ethyl acetate 50:50 to 0:100) to afford the desired product.

*Synthesis of intermediate 133* 6-chloro-7-(difluoromethoxy)-2-(3 -methyloxetan-3 -yl)chroman-4-one

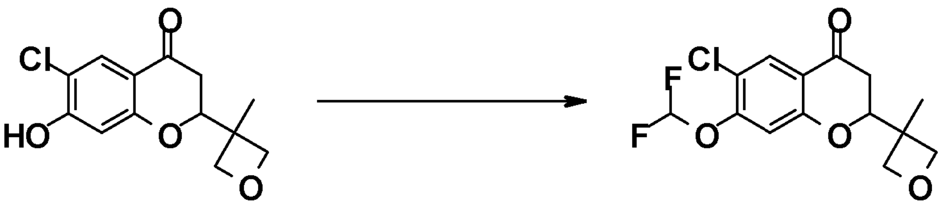

Under $N_2$, sodium chlorodifluoroacetate (227 mg, 1.49 mmol, 2.0 eq) is added at room temperature to a mixture

of 6-chloro-7-hydroxy-2-(3-methyloxetan-3-yl)chroman-4-one (200 mg, 0.74 mmol, 1.0 eq) and potassium carbonate (154 mg, 1.11 mmol, 1.5 eq) dry DMF (5.6 mL, 0.13 M). The reaction mixture is stirred at 70°C under $N_2$ atmosphere for 4 hrs. The mixture is partitioned between dichloromethane and water. The two phases are separated. The organic layer is dried over sodium sulfate and concentrated. The residue is purified by flash column chromatography ($SiO_2$, petroleum ether/ethyl acetate 100:0 to 50:50) to afford the desired product.

***Illustrative compounds 66,* (5S)-9-chloro-1-cyclopropyl-8-methoxy-5-(3-methyloxetan-3-yl)-2-oxo-5H-chromeno[4,3-b]pyridine-3-carboxylic acid *and 67,* (5R)-9-chloro-1-cyclopropyl-8-methoxy-5-(3-methyloxetan-3-yl)-2-oxo-5H-chromeno[4,3-b]pyridine-3-carboxylic acid.**

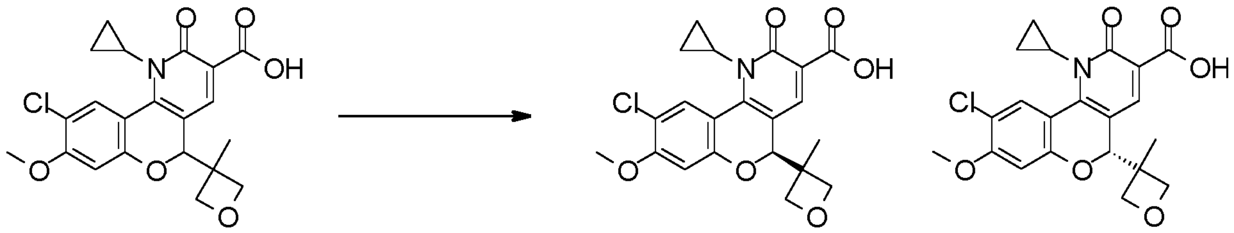

9-chloro-1-cyclopropyl-8-methoxy-5-(3-methyloxetan-3-yl)-2-oxo-5H-chromeno[4,3-b]pyridine-3-carboxylic acid (185 mg, 0.44 mmol) undergoes a chiral separation (column: Lux® C4, 21.2 mm x 250 mm, 5 µm; mobile phase: isocratic, MeCN/isopropanol 9:1 (0.2% v/v formic acid)) to afford (5S)-9-chloro-1-cyclopropyl-8-methoxy-5-(3-methyloxetan-3-yl)-2-oxo-5H-chromeno[4,3-b]pyridine-3-carboxylic acid and (5R)-9-chloro-1-cyclopropyl-8-methoxy-5-(3-methyloxetan-3-yl)-2-oxo-5H-chromeno[4,3-b] pyridine-3-carboxylic acid.

**Table II. Intermediates table**

| Int | Structure | Name | Starting material | Mtd | MW (calc) | MW (obs) |
|---|---|---|---|---|---|---|
| 1 | 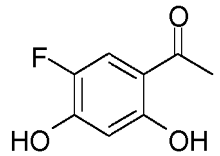 | 1-(5-fluoro-2,4-dihydroxy-phenyl)ethanone | 4-fluoro benzene-1,3-diol, CAS: 103068-41-3 | A | 170.1 | 170.9 |
| 2 | 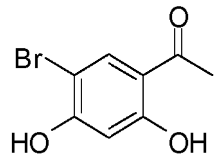 | 1-(5-bromo-2,4-dihydroxy-phenyl)ethanone | 4-bromobenze ne-1,3-diol, CAS: 6626-15-9 | A | 231.0 | NA |
| 3 | 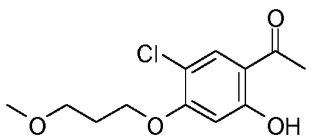 | 1-[5-chloro-2-hydroxy-4-(3-methoxypropoxy)phe nyl]ethanone | 1-(5-chloro-2,4-dihydroxy-phenyl)ethan one, CAS: 90110-32-0 | B | 258.7 | 258.9 |
| 4 | 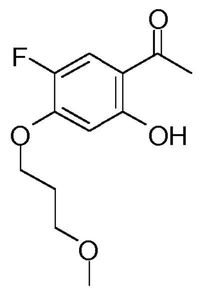 | 1-[5-fluoro-2-hydroxy-4-(3- methoxypropoxy)phe nyl]ethanone | Int. 1 | B | 242.2 | 243.0 |
| 5 | 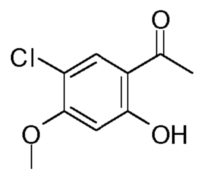 | 1-(5-chloro-2-hydroxy-4-methoxyphenyl)ethanone | 1-(5-chloro-2,4-dihydro xy-phenyl) ethanone, CAS: 90110-32-0 | B | 200.6 | 201.0 |

(continued)

| Int | Structure | Name | Starting material | Mtd | MW (calc) | MW (obs) |
|---|---|---|---|---|---|---|
| 6 | 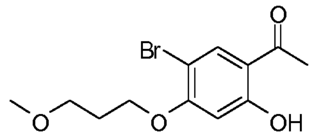 | 1-[5-bromo-2-hydroxy-4-(3-methoxypropoxy)phe nyl] ethanone | Int. 2 | B | 303.2 | 302.9 |
| 7 | 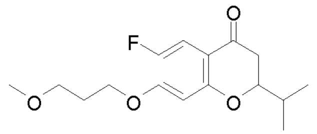 | 6-fluoro-2-isopropyl-7-(3-methoxypropo xy)chroman-4-one | Int. 4 | C | 296.3 | 297.1 |
| 8 | 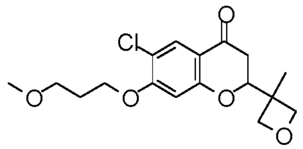 | 6-chloro-7-(3-methoxypropoxy)-2-(3-methyloxetan-3-yl) chroman-4-one | Int. 3 | C | 340.8 | 341.0 |
| 9 | 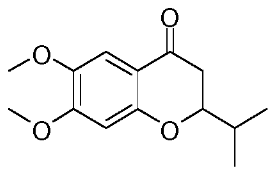 | 2-isopropyl-6,7-dimethoxy-chroman-4-one | 1-(2-hydroxy-4,5-dimethoxyphenyl)ethan one, CAS: 20628-06-2 | C | 250.3 | 251.1 |
| 10 | 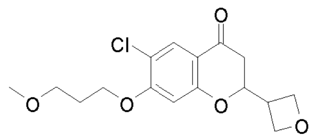 | 6-chloro-7-(3-methoxypropoxy)-2-(oxetan-3-yl)chroman-4-one | Int. 3 | C | 326.8 | 327.0 |
| 11 | 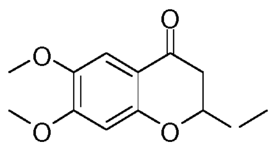 | 2-tert-butyl-6,7-dimethoxy-chroman-4-one | 1-(2-hydroxy-4,5-dimethoxyphenyl)ethan one, CAS: 20628-06-2 | C | 264.3 | 265.0 |

(continued)

| Int | Structure | Name | Starting material | Mtd | MW (calc) | MW (obs) |
|---|---|---|---|---|---|---|
| 12 | 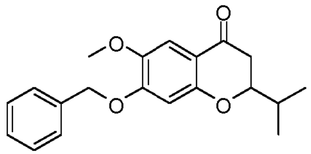 | 7-benzyloxy-2-isopropyl-6-methoxy-chroman-4-one | Int. 37 | C | 326.4 | 327.1 |
| 13 | 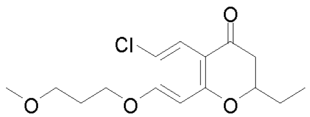 | 6-chloro-2-ethyl-7-(3-methoxypropoxy)chro man-4-one | Int. 3 | C | 298.8 | 299.0 |
| 14 | 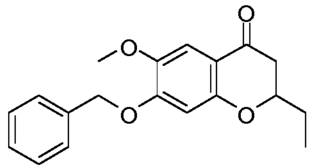 | 7-benzyloxy-2-ethyl-6-methoxy-chroman-4-one | Int. 37 | C | 312.4 | 313.1 |
| 15 | 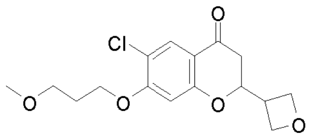 | 6-chloro-7-(3-methoxypropoxy)-2-(oxetan-3-yl)chroman-4-one | Int. 3 | C | 326.8 | 327.0 |
| 16 | 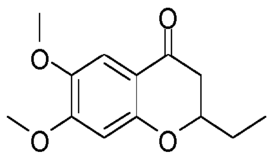 | 2-ethyl-6,7-dimethoxy-chroman-4-one | 1-(2-hydroxy-4,5-dimethoxyphenyl)ethan one, CAS: 20628-06-2 | C | 236.3 | 237.1 |
| 17 | 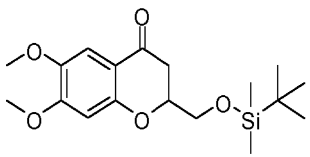 | 2-[[tert-butyl(dimethyl)silyl]o xymethyl]-6,7-dimethoxy-chroman-4-one | 1-(2-hydroxy-4,5-dimethoxyphenyl)ethan one, CAS: 20628-06-2 | C | 352.5 | 353.1 |

(continued)

| Int | Structure | Name | Starting material | Mtd | MW (calc) | MW (obs) |
|---|---|---|---|---|---|---|
| 18 | 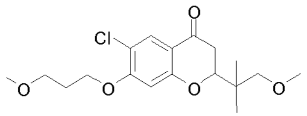 | 6-chloro-2-(2-methoxy-1,1-dimethyl-ethyl)-7-(3-methoxypropoxy)chro man-4-one | Int. 3 | C | 356.8 | 357.1 |
| 19 | 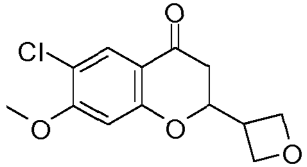 | 6-chloro-7-methoxy-2-(oxetan-3-yl)chroman-4-one | Int. 5 | C | 268.7 | 268.9 |
| 20 | 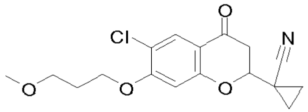 | 1-[6-chloro-7-(3-methoxypropoxy)-4-oxo-chroman-2-yl] cyclopropanecarbo nitrile | Int. 3 | C | 335.8 | 336.0 |
| 21 | 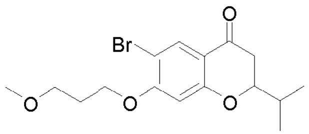 | 6-bromo-2-isopropyl-7-(3-methoxypropoxy)chro man-4-one | Int. 6 | C | 357.2 | 359.0 |
| 22 | 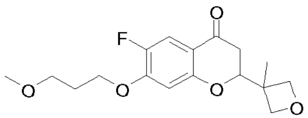 | 6-fluoro-7-(3-methoxypropoxy)-2-(3-methyloxetan-3-yl) chroman-4-one | Int. 4 | C | 324.3 | 325.0 |
| 23 | 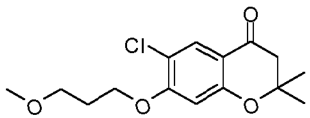 | 6-chloro-7-(3-methoxypropoxy)-2,2-dimethyl-chroman-4-one | Int. 3 | NA | 298.8 | 299.1 |
| 24 | 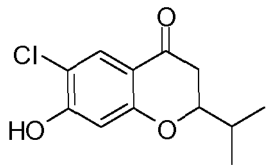 | 6-chloro-7-hydroxy-2-isopropyl-chroman-4-one | 1-(5-chloro-2,4-dihydroxy-phenyl)ethan one, CAS: 90110-32-0 | D | 240.7 | 241.1 |

(continued)

| Int | Structure | Name | Starting material | Mtd | MW (calc) | MW (obs) |
|---|---|---|---|---|---|---|
| 25 | 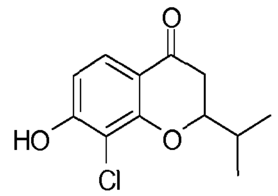 | 8-chloro-7-hydroxy-2-isopropyl-chroman-4-one | Int. 34 | D | 240.7 | 241.0 |
| 26 | 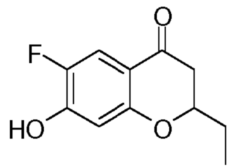 | 2-ethyl-6-fluoro-7-hydroxy-chroman-4-one | Int. 1 | D | 210.2 | 211.0 |
| 27 | 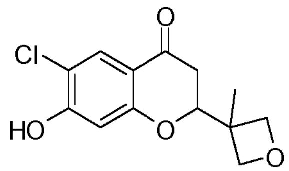 | 6-chloro-7-hydroxy-2-(3-methyloxetan-3-yl)chroman-4-one | 1-(5-chloro-2,4-dihydroxy-phenyl)ethan one, CAS: 90110-32-0 | D | 268.7 | 269.0 |
| 28 | 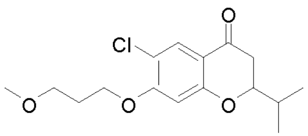 | 6-chloro-2-isopropyl-7-(3-methoxypropoxy)chro man-4-one | Int. 24 | E | 312.8 | 313.1 |
| 29 | 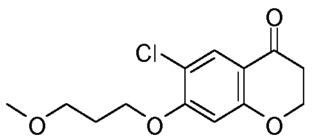 | 6-chloro-7-(3-methoxypropoxy)chro man-4-one | 6-chloro-7-hydroxy-chroman-4-one, CAS: 74277-66-0 | E | 270.7 | 271.0 |
| 30 | 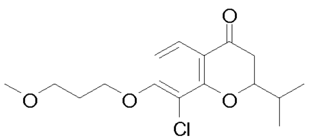 | 8-chloro-2-isopropyl-7-(3-methoxypropoxy)chro man-4-one | Int. 25 | E | 312.8 | 313.1 |

(continued)

| Int | Structure | Name | Starting material | Mtd | MW (calc) | MW (obs) |
|---|---|---|---|---|---|---|
| 31 | 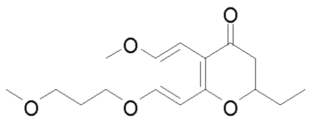 | 2-ethyl-6-methoxy-7-(3-methoxy propoxy)chroman-4-one | Int. 89 | E | 294.3 | 295.0 |
| 32 | 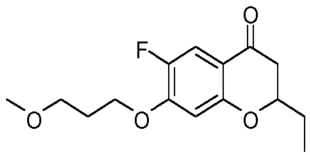 | 2-ethyl-6-fluoro-7-(3-methoxy propoxy)chroman-4-one | Int. 26 | E | 282.3 | 283.0 |
| 33 | 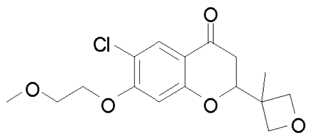 | 6-chloro-7-(2-methoxyethoxy)-2-(3-methyloxetan-3-yl) chroman-4-one | Int. 27 | E | 326.8 | 327.1 |
| 34 | 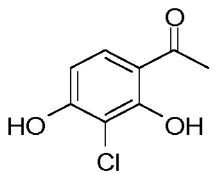 | 1-(3-chloro-2,4-dihydroxy-phenyl)ethanone | 1-(2,4-dihydroxyph enyl) ethanon e, CAS: 89-84-9 | NA | 186.6 | No ionisa tion |
| 35 | 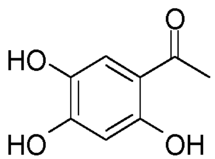 | 1-(2,4,5-trihydroxyphenyl)etha none | 1-(2,4,5-trimethoxyp henyl) ethano ne, CAS: 1136-86-3 | NA | 168.1 | 169.0 |
| 36 | 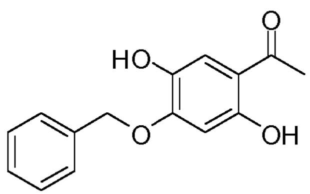 | 1 -(4-benzyloxy-2,5-dihydroxy-phenyl)ethanone | Int. 35 | NA | 258.3 | 259.0 |

(continued)

| Int | Structure | Name | Starting material | Mtd | MW (calc) | MW (obs) |
|---|---|---|---|---|---|---|
| 37 | 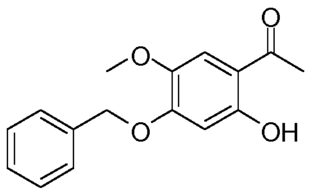 | 1 -(4-benzyloxy-2-hydroxy-5-methoxyphenyl)ethanone | Int. 36 | NA | 272.3 | 273.0 |
| 38 | 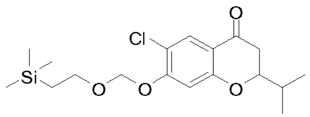 | 6-chloro-2-isopropyl-7-(2-trimethylsilylethoxym ethoxy) chroman-4-one | Int. 24 | NA | 370.9 | NA |
| 39 | 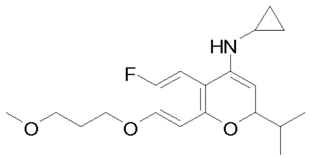 | N-cyclopropyl-6-fluoro-2-isopropyl-7-(3-methoxypropoxy)-2H-chromen-4-amine | Int. 7 | F | 335.4 | 336.1 |
| 40 | 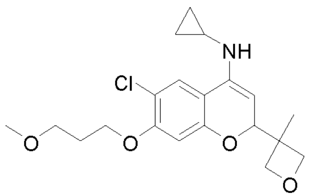 | 6-chloro-N-cyclopropyl-7-(3-methoxypropoxy)-2-(3-methyloxetan-3-yl)-2H-chromen-4-amine | Int. 8 | F | 379.9 | 380.1 |
| 41 | 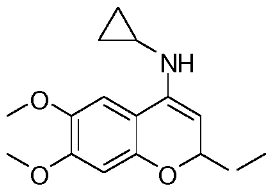 | 2-tert-butyl-N-cyclopropyl-6,7-dimethoxy-2H-chromen-4-amine | Int. 11 | F | 303.4 | 304.2 |

(continued)

| Int | Structure | Name | Starting material | Mtd | MW (calc) | MW (obs) |
|---|---|---|---|---|---|---|
| 42 | 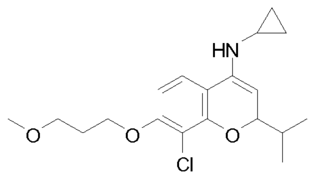 | 8-chloro-N-cyclopropyl-2-isopropyl-7-(3-methoxypropoxy)-2H-chromen-4-amine | Int. 30 | F | 351.9 | 352.1 |
| 43 | 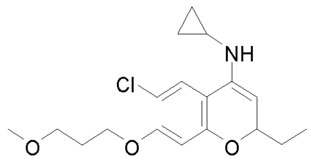 | 6-chloro-N-cyclopropyl-2-ethyl-7-(3-methoxypropoxy)- 2H-chromen-4-amine | Int. 13 | F | 337.8 | 338.1 |
| 44 | 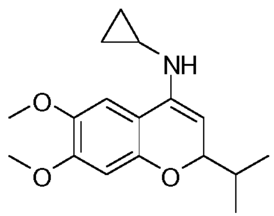 | N-cyclopropyl-2-isopropyl-6,7-dimethoxy-2H-chromen-4-amine | Int. 9 | F | 289.4 | 290.1 |
| 45 | 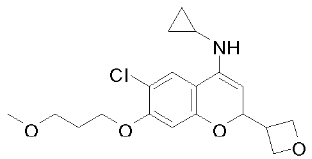 | 6-chloro-N-cyclopropyl-7-(3-methoxypropoxy)-2-(oxetan-3-yl)-2H-chromen-4-amine | Int. 10 | F | 365.9 | 366.1 |
| 46 | 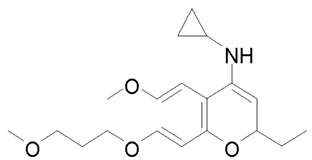 | N-cyclopropyl-2-ethyl-6-methoxy-7-(3-methoxypropoxy)-2H-chromen-4-amine | Int. 31 | F | 333.4 | 334.1 |

(continued)

| Int | Structure | Name | Starting material | Mtd | MW (calc) | MW (obs) |
|---|---|---|---|---|---|---|
| 47 | 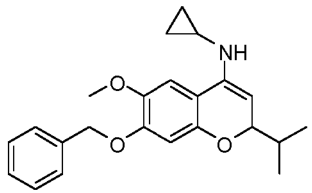 | 7-benzyloxy-N-cyclopropyl-2-isopropyl-6-methoxy-2H-chromen-4-amine | Int. 12 | F | 365.5 | 366.1 |
| 48 | 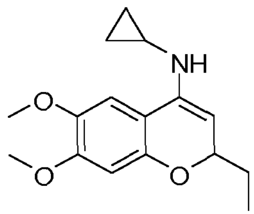 | N-cyclopropyl-2-ethyl-6,7-dimethoxy-2H-chromen-4-amine | Int. 16 | F | 275.3 | 276.1 |
| 49 | 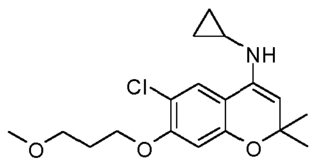 | 6-chloro-N-cyclopropyl-7-(3-methoxypropoxy)-2,2-dimethyl-chromen-4-amine | Int. 23 | F | 337.8 | 338.1 |
| 50 | 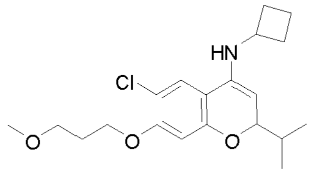 | 6-chloro-N-cyclobutyl-2-isopropyl-7-(3-methoxypropoxy)-2H-chromen-4-amine | Int. 28 | F | 365.9 | 366.1 |
| 51 | 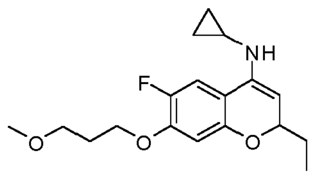 | N-cyclopropyl-2-ethyl-6-fluoro-7-(3-methoxypropoxy)-2H-chromen-4-amine | Int. 32 | F | 321.4 | 322.1 |

(continued)

| Int | Structure | Name | Starting material | Mtd | MW (calc) | MW (obs) |
|---|---|---|---|---|---|---|
| 52 | 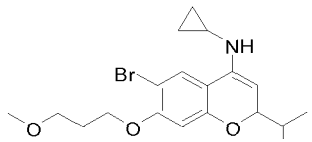 | 6-bromo-N-cyclopropyl-2-isopropyl-7-(3-methoxypropoxy)-2H-chromen-4-amine | Int. 21 | F | 396.3 | 396.0 |
| 53 | 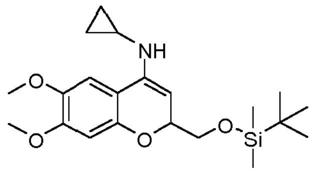 | 2-[[tert-butyl(dimethyl)silyl]o xymethyl]-N-cyclopropyl-6,7-dimethoxy-2H-chromen-4-amine | Int. 17 | F | 391.6 | 392.2 |
| 54 | 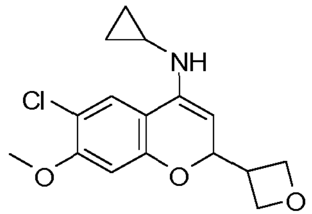 | 6-chloro-N-cyclopropyl-7-methoxy-2-(oxetan-3-yl)-2H-chromen-4-amine | Int. 19 | F | 307.8 | 308.0 |
| 55 | 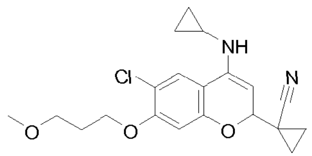 | 1-[6-chloro-4-(cyclopropylamino)-7-(3-methoxypropoxy)-2H-chromen-2-yl]cyclopropanecarbo nitrile | Int. 20 | F | 374.9 | 375.1 |
| 56 | 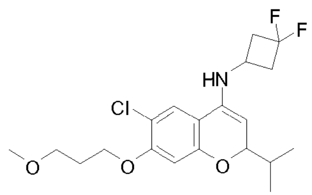 | 6-chloro-N-(3,3-difluorocyclobutyl)-2-isopropyl-7-(3-methoxypropoxy)-2H-chromen-4-amine | Int. 28 | F | 401.9 | 402.1 |

(continued)

| Int | Structure | Name | Starting material | Mtd | MW (calc) | MW (obs) |
|---|---|---|---|---|---|---|
| 57 | 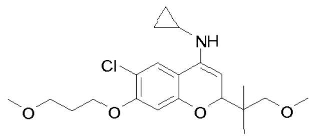 | 6-chloro-N-cyclopropyl-2-(2-methoxy-1,1-dimethyl-ethyl)-7-(3-methoxypropoxy)-2H-chromen-4-amine | Int. 18 | F | 395.9 | 396.1 |
| 58 | 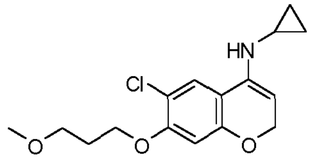 | 6-chloro-N-cyclopropyl-7-(3-methoxypropoxy)-2H-chromen-4-amine | Int. 29 | F | 309.8 | 310.0 |
| 59 | 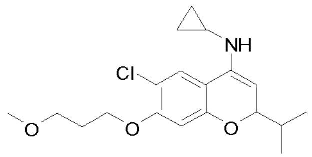 | 6-chloro-N-cyclopropyl-2-isopropyl-7-(3-methoxypropoxy)-2H-chromen-4-amine | Int. 28 | F | 351.9 | 352.2 |
| 60 | 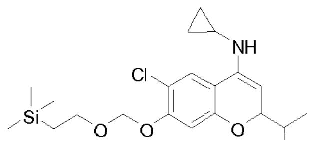 | 6-chloro-N-cyclopropyl-2-isopropyl-7-(2-trimethylsilylethoxymethoxy)-2H-chromen-4-amine | Int. 38 | F | 410.0 | 410.2 |
| 61 | 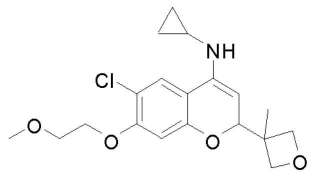 | 6-chloro-N-cyclopropyl-7-(2-methoxyethoxy)-2-(3-methyloxetan-3-yl)-2H-chromen-4-amine | Int. 33 | F | 365.9 | 366.2 |
| 62 | 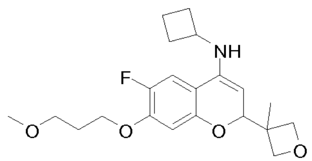 | N-cyclobutyl-6-fluoro-7-(3-methoxypropoxy)-2-(3-methyloxetan-3-yl)-2H-chromen-4-amine | Int. 22 | F | 377.5 | 378.1 |

(continued)

| Int | Structure | Name | Starting material | Mtd | MW (calc) | MW (obs) |
|---|---|---|---|---|---|---|
| 63 | 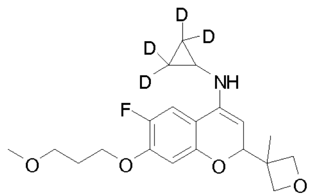 | 6-fluoro-7-(3-methoxypropoxy)-2-(3-methyloxetan-3-yl)-N-(2,2,3,3-tetradeuteriocycloprop yl)-2H-chromen-4-amine | Int. 22 | F | 367.5 | 368.2 |
| 64 | 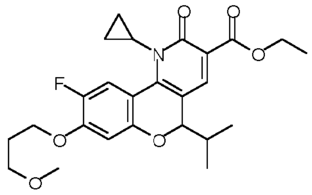 | ethyl 1-cyclopropyl-9-fluoro-5-isopropyl-8-(3-methoxypropoxy)-2-oxo-5H-chromeno[4,3-b]pyridine-3-carboxylate | Int. 39 | G | 459.5 | 460.2 |
| 65 | 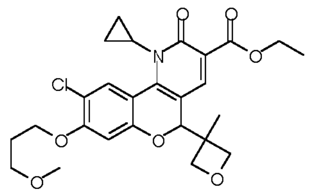 | ethyl 9-chloro-1-cyclopropyl-8-(3-methoxypropoxy)-5-(3-methyloxetan-3-yl)-2-oxo-5H-chromeno[4,3-b]pyridine-3-carboxylate | Int. 40 | G | 504.0 | 504.2 |
| 66 | 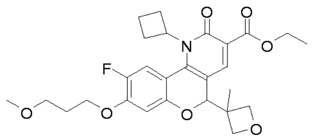 | ethyl 1-cyclobutyl-9-fluoro-8-(3-methoxypropoxy)-5-(3-methyloxetan-3-yl)-2-oxo-5H-chromeno[4,3-b]pyridine-3-carboxylate | Int. 62 | G | 501.6 | 502.1 |
| 67 | 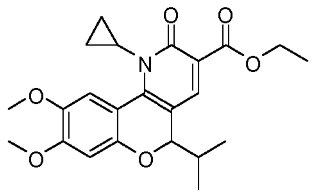 | ethyl 1-cyclopropyl-5-isopropyl-8,9-dimethoxy-2-oxo-5H-chromeno[4,3-b]pyridine-3-carboxylate | Int. 44 | G | 413.5 | 414.2 |

(continued)

| Int | Structure | Name | Starting material | Mtd | MW (calc) | MW (obs) |
|---|---|---|---|---|---|---|
| 68 | 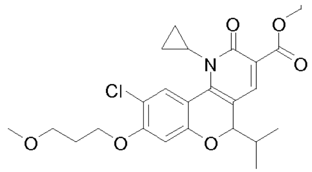 | ethyl 9-chloro-1-cyclopropyl-5-isopropyl-8-(3-methoxypropoxy)-2-oxo-5H-chromeno[4,3-b]pyridine-3-carboxylate | Int. 59 | G | 476.0 | 476.2 |
| 69 | 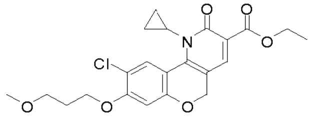 | ethyl 9-chloro-1-cyclopropyl-8-(3-methoxypropoxy)-2-oxo-5H-chromeno[4,3-b]pyridine-3-carboxylate | Int. 58 | G | 433.9 | 434.1 |
| 70 | 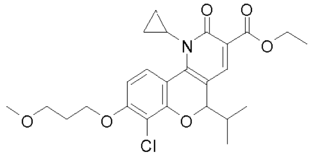 | ethyl 7-chloro-1-cyclopropyl-5-isopropyl-8-(3-methoxypropoxy)-2-oxo-5H-chromeno[4,3-b]pyridine-3-carboxylate | Int. 42 | G | 476.0 | 476.1 |
| 71 | 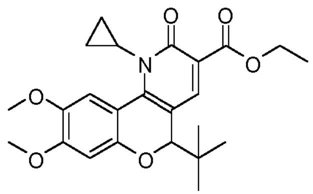 | ethyl 5-tert-butyl-1-cyclopropyl-8,9-dimethoxy-2-oxo-5H-chromeno[4,3-b]pyridine-3-carboxylate | Int. 41 | G | 427.5 | 428.2 |
| 72 | 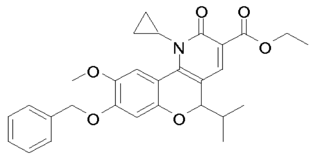 | ethyl 8-benzyloxy-1-cyclopropyl-5-isopropyl-9-methoxy-2-oxo-5H-chromeno[4,3-b]pyridine-3-carboxylate | Int. 47 | G | 489.6 | 490.2 |
| 73 | 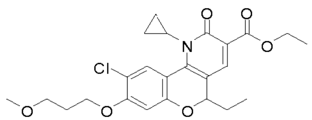 | ethyl 9-chloro-1-cyclopropyl-5-ethyl-8-(3-methoxypropoxy)-2-oxo-5H-chromeno[4,3-b]pyridine-3-carboxylate | Int. 43 | G | 461.9 | 462.1 |

(continued)

| Int | Structure | Name | Starting material | Mtd | MW (calc) | MW (obs) |
|---|---|---|---|---|---|---|
| 74 | 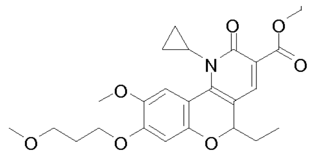 | ethyl 1-cyclopropyl-5-ethyl-9-methoxy-8-(3-methoxypropoxy)-2-oxo-5H-chromeno[4,3-b]pyridine-3-carboxylate | Int. 46 | G | 457.5 | 458.2 |
| 75 | 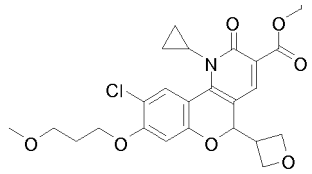 | ethyl 9-chloro-1-cyclopropyl-8-(3-methoxypropoxy)-5-(oxetan-3-yl)-2-oxo-5H-chromeno[4,3-b]pyridine-3-carboxylate | Int. 45 | G | 490.0 | 490.1 |
| 76 | 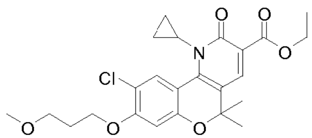 | ethyl 9-chloro-1-cyclopropyl-8-(3-methoxypropoxy)-5,5-dimethyl-2-oxo-chromeno[4,3-b]pyridine-3-carboxylate | Int. 49 | G | 461.9 | 462.1 |
| 77 | 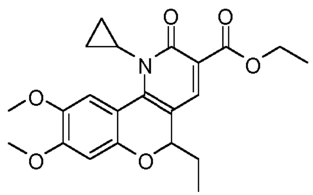 | ethyl 1-cyclopropyl-5-ethyl-8,9-dimethoxy-2-oxo-5H-chromeno[4,3-b]pyridine-3-carboxylate | Int. 48 | G | 399.4 | 400.1 |
| 78 | 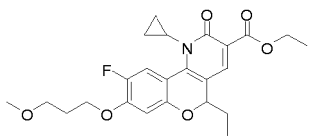 | ethyl 1-cyclopropyl-5-ethyl-9-fluoro-8-(3-methoxypropoxy)-2-oxo-5H-chromeno[4,3-b]pyridine-3-carboxylate | Int. 51 | G | 445.5 | 446.1 |
| 79 | 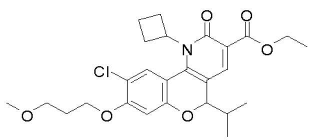 | ethyl 9-chloro-1-cyclobutyl-5-isopropyl-8-(3-methoxypropoxy)-2-oxo-5H-chromeno[4,3-b]pyridine-3-carboxylate | Int. 50 | G | 490.0 | 490.1 |

(continued)

| Int | Structure | Name | Starting material | Mtd | MW (calc) | MW (obs) |
|---|---|---|---|---|---|---|
| 80 | 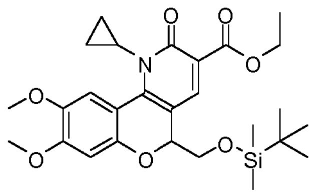 | ethyl 5-[[tert-butyl(dimethyl)silyl]o xymethyl]-1-cyclopropyl-8,9-dimethoxy-2-oxo-5H-chromeno[4,3-b]pyridine-3-carboxylate | Int. 53 | G | 515.7 | 516.3 |
| 81 | 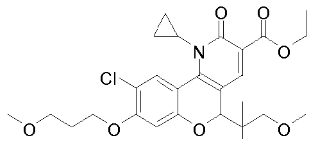 | ethyl 9-chloro-1-cyclopropyl-5-(2-methoxy-1,1-dimethyl-ethyl)-8-(3-methoxypropoxy)-2-oxo-5H-chromeno[4,3-b]pyridine-3-carboxylate | Int. 57 | G | 520.0 | 520.2 |
| 82 | 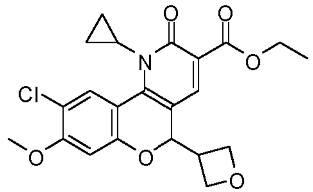 | ethyl 9-chloro-1-cyclopropyl-8-methoxy-5-(oxetan-3-yl)-2-oxo-5H-chromeno[4,3-b]pyridine-3-carboxylate | Int. 54 | G | 431.9 | 432.1 |
| 83 | 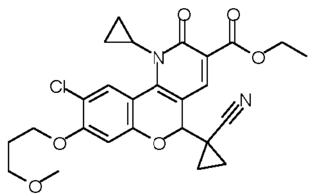 | ethyl 9-chloro-5-(1-cyanocyclopropyl)-1-cyclopropyl-8-(3-methoxypropoxy)-2-oxo-5H-chromeno[4,3-b]pyridine-3-carboxylate | Int. 55 | G | 499.0 | 499.1 |
| 84 | 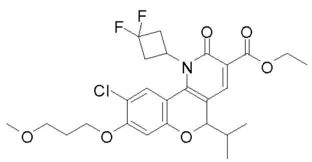 | ethyl 9-chloro-1-(3,3-difluorocyclobutyl)-5-isopropyl-8-(3-methoxypropoxy)-2-oxo-5H-chromeno[4,3-b]pyridine-3-carboxylate | Int. 56 | G | 526.0 | 526.1 |

(continued)

| Int | Structure | Name | Starting material | Mtd | MW (calc) | MW (obs) |
|---|---|---|---|---|---|---|
| 85 | 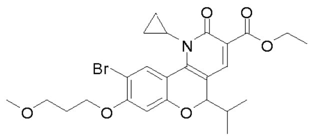 | ethyl 9-bromo-1-cyclopropyl-5-isopropyl-8-(3-methoxypropoxy)-2-oxo-5H-chromeno[4,3-b]pyridine-3-carboxylate | Int. 52 | G | 520.4 | 522.1 |
| 86 | 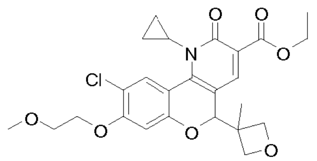 | ethyl 9-chloro-1-cyclopropyl-8-(2-methoxyethoxy)-5-(3-methyloxetan-3-yl)-2-oxo-5H-chromeno[4,3-b]pyridine-3-carboxylate | Int. 61 | G | 490.0 | 490.2 |
| 87 | 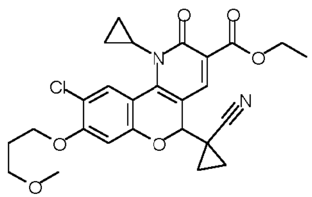 | ethyl 9-fluoro-8-(3-methoxypropoxy)-5-(3-methyloxetan-3-yl)-2-oxo-1-(2,2,3,3-tetradeuteriocycloprop yl)-5H-chromeno[4,3-b]pyridine-3-carboxylate | Int. 63 | G | 491.5 | 492.1 |
| 88 | 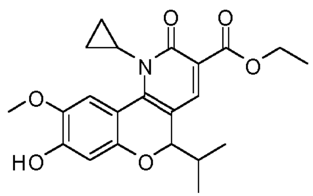 | ethyl 1-cyclopropyl-8-hydroxy-5-isopropyl-9-methoxy-2-oxo-5H-chromeno[4,3-b]pyridine-3-carboxylate | Int. 72 | H | 399.4 | 400.1 |
| 89 | 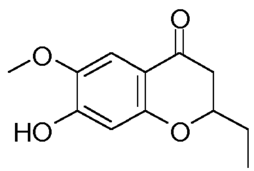 | 2-ethyl-7-hydroxy-6-methoxy-chroman-4-one | Int. 14 | H | 222.2 | 223.0 |

(continued)

| Int | Structure | Name | Starting material | Mtd | MW (calc) | MW (obs) |
|---|---|---|---|---|---|---|
| 90 | 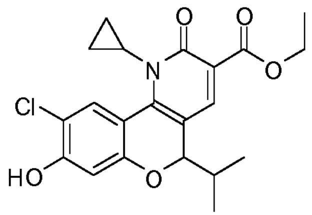 | ethyl 9-chloro-1-cyclopropyl-8-hydroxy-5-isopropyl-2-oxo-5H-chromeno[4,3-b]pyridine-3-carboxylate | Int. 60 | NA | 403.9 | 404.1 |
| 91 | 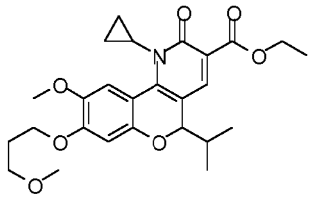 | ethyl 1-cyclopropyl-5-isopropyl-9-methoxy-8-(3-methoxypropoxy)-2-oxo-5H-chromeno[4,3-b]pyridine-3-carboxylate | Int. 88 | NA | 471.5 | 472.2 |
| 92 | 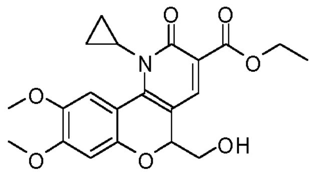 | ethyl 1-cyclopropyl-5-(hydroxymethyl)-8,9-dimethoxy-2-oxo-5H-chromeno[4,3-b]pyridine-3-carboxylate | Int. 80 | NA | 401.4 | 402.1 |
| 93 | 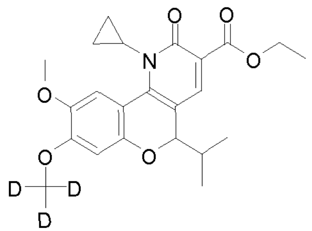 | ethyl 1-cyclopropyl-5-isopropyl-9-methoxy-2-oxo-8-(trideuteriomethoxy)-5H-chromeno[4,3-b]pyridine-3-carboxylate | Int. 88 | NA | 416.5 | 417.1 |

(continued)

| Int | Structure | Name | Starting material | Mtd | MW (calc) | MW (obs) |
|---|---|---|---|---|---|---|
| 94 | 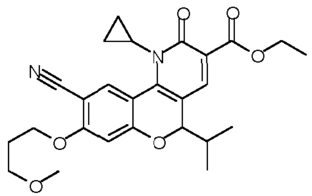 | ethyl 9-cyano-1-cyclopropyl-5-isopropyl-8-(3-methoxypropoxy)-2-oxo-5H-chromeno[4,3-b]pyridine-3-carboxylate | Int. 85 | NA | 466.5 | 467.2 |
| 95 | 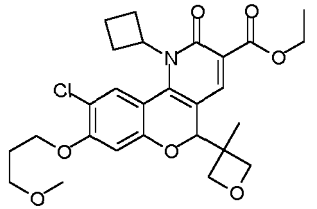 | ethyl 9-chloro-1-cyclobutyl-8-(3-methoxypropoxy)-5-(3-methyloxetan-3-yl)-2-oxo-5H-chromeno[4,3-b]pyridine-3-carboxylate | Int. 96 | G | 518.0 | 518.1 |
| 96 | 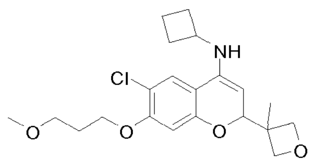 | 6-chloro-N-cyclobutyl-7-(3-methoxypropoxy)-2-(3-methyloxetan-3-yl)-2H-chromen-4-amine | Int. 8 | F | 393.9 | 394.1 |
| 97 | 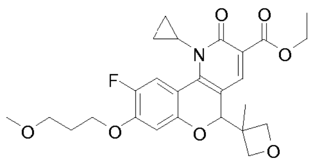 | ethyl 1-cyclopropyl-9-fluoro-8-(3-methoxypropoxy)-5-(3-methyloxetan-3-yl)-2-oxo-5H-chromeno[4,3-b]pyridine-3-carboxylate | Int. 98 | G | 487.5 | 488.1 |
| 98 | 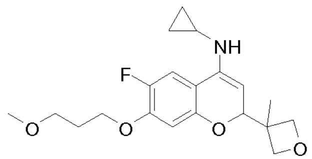 | N-cyclopropyl-6-fluoro-7-(3-methoxypropoxy)-2-(3-methyloxetan-3-yl)-2H-chromen-4-amine | Int. 22 | F | 363.4 | 364.1 |

(continued)

| Int | Structure | Name | Starting material | Mtd | MW (calc) | MW (obs) |
|---|---|---|---|---|---|---|
| 99 | 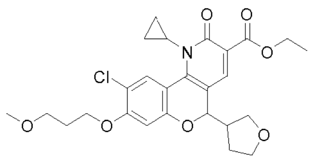 | ethyl 9-chloro-1-cyclopropyl-8-(3-methoxypropoxy)-2-oxo-5-tetrahydrofuran-3-yl-5H-chromeno[4,3-b]pyridine-3-carboxylate | Int. 101 | G | 503.9 | 504.1 |
| 100 | 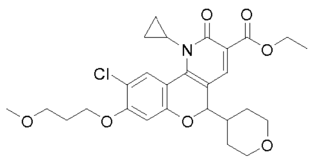 | ethyl 9-chloro-1-cyclopropyl-8-(3-methoxypropoxy)-2-oxo-5-tetrahydropyran-4-yl-5H-chromeno[4,3-b]pyridine-3-carboxylate | Int. 102 | G | 518.0 | 518.2 |
| 101 | 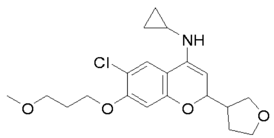 | 6-chloro-N-cyclopropyl-7-(3-methoxypropoxy)-2-tetrahydrofuran-3-yl-2H-chromen-4-amine | Int. 103 | F | 379.8 | 380.1 |
| 102 | 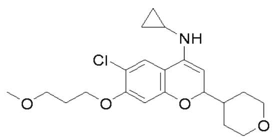 | 6-chloro-N-cyclopropyl-7-(3-methoxypropoxy)-2-tetrahydropyran-4-yl-2H-chromen-4-amine | Int. 104 | F | 393.9 | 394.1 |
| 103 | 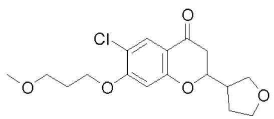 | 6-chloro-7-(3-methoxypropoxy)-2-tetrahydrofuran-3-yl-chroman-4-one | Int. 3 | C | 340.9 | 341.0 |
| 104 | 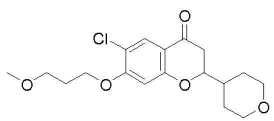 | 6-chloro-7-(3-methoxypropoxy)-2-tetrahydropyran-4-yl-chroman-4-one | Int. 3 | C | 354.8 | 355.1 |

(continued)

| Int | Structure | Name | Starting material | Mtd | MW (calc) | MW (obs) |
|---|---|---|---|---|---|---|
| 105 | 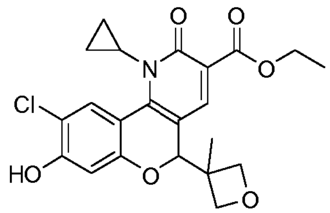 | ethyl 9-chloro-1-cyclopropyl-8-hydroxy-5-(3-methyloxetan-3-yl)-2-oxo-5H-chromeno[4,3-b]pyridine-3-carboxylate | Int. 106 | G | 431.9 | 432.1 |
| 106 | 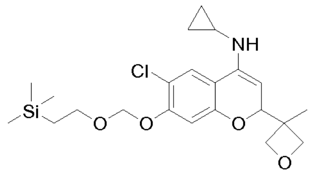 | 6-chloro-N-cyclopropyl-2-(3-methyloxetan-3-yl)-7-(2-trimethylsilylethoxym ethoxy)-2H-chromen-4-amine | Int. 107 | F | 438.0 | 438.1 |
| 107 | 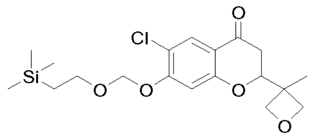 | 6-chloro-2-(3-methyloxetan-3-yl)-7 - (2-trimethylsilylethoxym ethoxy)chroman-4-one | Int. 27 | NA | 399.0 | 399.1 |
| 108 | 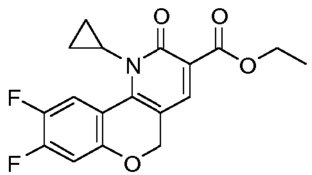 | ethyl 1-cyclopropyl-8,9-difluoro-2-oxo-5H-chromeno[4,3-b] pyridine-3-carboxylate | Int. 109 | G | 347.3 | 348.0 |
| 109 | 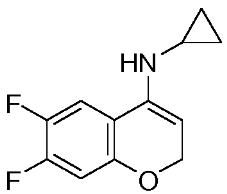 | N-cyclopropyl-6,7-difluoro-2H-chromen-4-amine | 6,7-difluorochro man-4-one | F | 223.2 | 224.0 |

(continued)

| Int | Structure | Name | Starting material | Mtd | MW (calc) | MW (obs) |
|---|---|---|---|---|---|---|
| 110 | 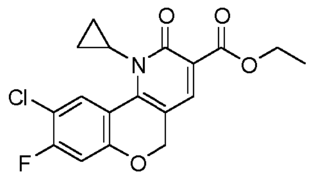 | ethyl 9-chloro-1-cyclopropyl-8-fluoro-2-oxo-5H-chromeno[4,3-b]pyridine-3-carboxylate | Int. 111 | G | 363.8 | 364.0 |
| 111 | 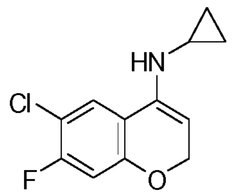 | 6-chloro-N-cyclopropyl-7-fluoro-2H-chromen-4-amine | 6-chloro-7-fluorochroman-4-one | F | 239.7 | 239.9 |
| 112 | 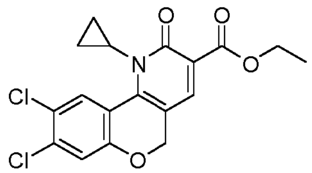 | ethyl 8,9-dichloro-1-cyclopropyl-2-oxo-5H-chromeno[4,3-b] pyridine-3-carboxylate | Int. 113 | G | 380.2 | 380.0 |
| 113 | 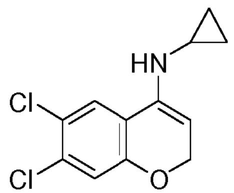 | 6,7-dichloro-N-cyclopropyl-2H-chromen-4-amine | 6,7-dichlorochro man-4-one | F | 256.1 | 255.9 |
| 114 | 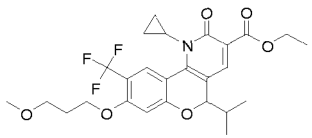 | ethyl 1-cyclopropyl-5-isopropyl-8-(3-methoxypropoxy)-2-oxo-9-(trifluoromethyl)-5H-chromeno[4,3-b]pyridine-3-carboxylate | Int. 85 | NA | 509.2 | 510.2 |

(continued)

| Int | Structure | Name | Starting material | Mtd | MW (calc) | MW (obs) |
|---|---|---|---|---|---|---|
| 115 | 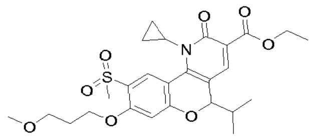 | ethyl 1-cyclopropyl-5-isopropyl-8-(3-methoxypropoxy)-9-methylsulfonyl-2-oxo-5H-chromeno[4,3-b]pyridine-3-carboxylate | Int. 85 | NA | 519.6 | 520.2 |
| 116 | 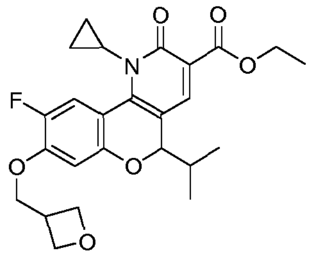 | ethyl 1-cyclopropyl-9-fluoro-5-isopropyl-8-(oxetan-3-ylmethoxy)-2-oxo-5H-chromeno[4,3-b]pyridine-3-carboxylate | Int. 117 | G | 457.5 | 430.1 |
| 117 | 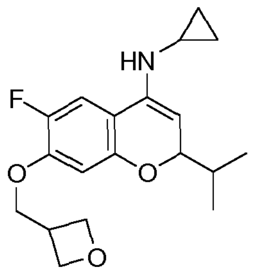 | N-cyclopropyl-6-fluoro-2-isopropyl- 7-(oxetan-3-ylmethoxy)-2H-chromen-4-amine | Int. 118 | F | 333.4 | 334.1 |
| 118 | 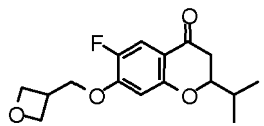 | 6-fluoro-2-isopropyl-7-(oxetan-3-ylmethoxy)chroman-4-one | Int. 119 | E | 294.3 | 295.1 |
| 119 | 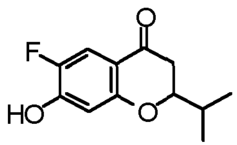 | 6-fluoro-7-hydroxy-2-isopropyl-chroman-4-one | Int. 1 | D | 224.2 | 225.0 |

(continued)

| Int | Structure | Name | Starting material | Mtd | MW (calc) | MW (obs) |
|---|---|---|---|---|---|---|
| 120 | 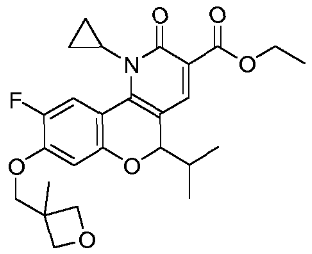 | ethyl 1-cyclopropyl-9-fluoro-5-isopropyl-8-[(3-methyloxetan-3-yl)methoxy]-2-oxo-5H-chromeno[4,3-b]pyridine-3-carboxylate | Int. 121 | G | 471.5 | 472.2 |
| 121 | 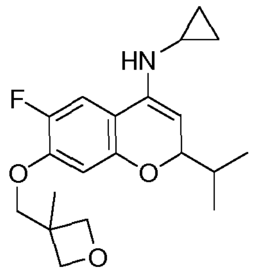 | N-cyclopropyl-6-fluoro-2-isopropyl-7-[(3-methyloxetan-3-yl) methoxy]-2H-chromen-4-amine | Int. 122 | F | 347.4 | 348.1 |
| 122 | 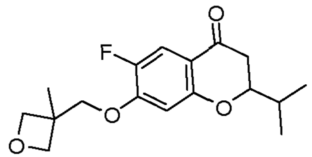 | 6-fluoro-2-isopropyl-7-[(3-methyloxetan-3-yl)methoxy] chroman-4-one | Int. 119 | E | 308.3 | 308.1 |
| 123 | 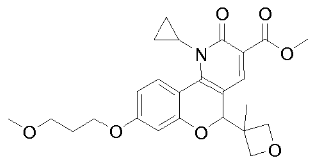 | methyl 1-cyclopropyl-8-(3-methoxypropoxy)-5-(3-methyloxetan-3-yl)-2-oxo-5H-chromeno[4,3-b]pyridine-3-carboxylate | Comp. 2 | NA | 455.1 | 456.1 |

(continued)

| Int | Structure | Name | Starting material | Mtd | MW (calc) | MW (obs) |
|---|---|---|---|---|---|---|
| 124 | 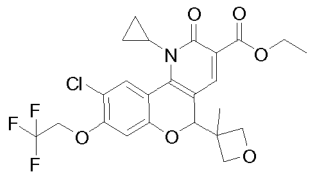 | ethyl 9-chloro-1-cyclopropyl-5-(3-methyloxetan-3-yl)-2-oxo-8-(2,2,2-trifluoroethoxy)-5H-chromeno[4,3-b]pyridine-3-carboxylate | Int. 125 | NA | 513.9 | 514.1 |
| 125 | 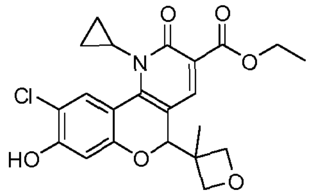 | ethyl 9-chloro-1-cyclopropyl-8-hydroxy-5-(3-methyloxetan-3-yl)-2-oxo-5H-chromeno[4,3-b]pyridine-3-carboxylate | Int. 126 | G | 431.9 | 432.0 |
| 126 | 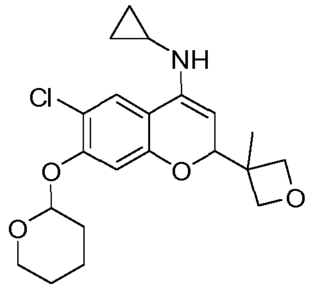 | 6-chloro-N-cyclopropyl-2-(3-methyloxetan-3-yl)-7-tetrahydropyran-2-yloxy-2H-chromen-4-amine | Int. 127 | F | 391.9 | 308.1 |
| 127 | 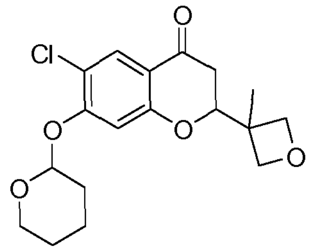 | 6-chloro-2-(3-methyloxetan-3-yl)-7-tetrahydropyran-2-yloxy-chroman-4-one | Int. 27 | NA | 352.8 | 268.9 |

(continued)

| Int | Structure | Name | Starting material | Mtd | MW (calc) | MW (obs) |
|---|---|---|---|---|---|---|
| 128 | 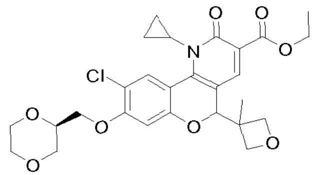 | ethyl 9-chloro-1-cyclopropyl-8-[[(2R)-1,4-dioxan-2-yl] methoxy]-5-(3-methyloxetan-3-yl)-2-oxo-5H-chromeno[4,3-b] pyridine-3-carboxylate | Int. 129 | G | 532.0 | 532.2 |
| 129 | 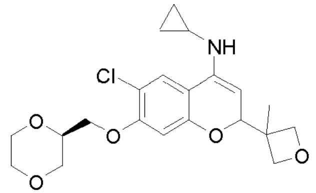 | 6-chloro-N-cyclopropyl-7-[[(2R)-1,4-dioxan-2-yl]methoxy]-2-(3-methyloxetan-3-yl)-2H-chromen-4-amine | Int. 130 | F | 407.9 | 408.1 |
| 130 | 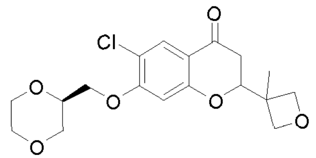 | 6-chloro-7- [[(2R)-1,4-dioxan-2-yl] methoxy]-2-(3-methyloxetan-3-yl)chroman-4-one | Int. 27 | NA | 368.8 | 369.1 |
| 131 | 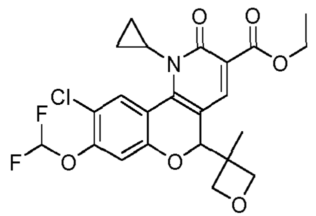 | ethyl 9-chloro-1-cyclopropyl-8-(difluoromethoxy)-5-(3-methyloxetan-3-yl)-2-oxo-5H-chromeno[4,3-b]pyridine-3-carboxylate | Int. 132 | G | 481.9 | 482.1 |
| 132 | 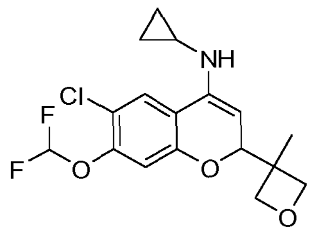 | 6-chloro-N-cyclopropyl-7-(difluoromethoxy)-2-(3-methyloxetan-3-yl)-2H-chromen-4-amine | Int. 133 | F | 357.8 | 358.1 |

(continued)

| Int | Structure | Name | Starting material | Mtd | MW (calc) | MW (obs) |
|---|---|---|---|---|---|---|
| 133 | 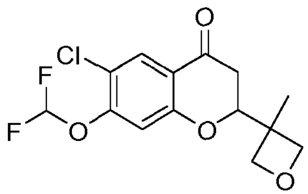 | 6-chloro-7-(difluoromethoxy)-2-(3-methyloxetan-3-yl) chroman-4-one | Int. 27 | NA | 318.7 | - |
| 134 | 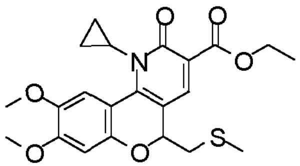 | ethyl 1-cyclopropyl-8,9-dimethoxy-5-(methylsulfanylmethy 1)-2-oxo-5H-chromeno[4,3-b]pyridine-3-carboxylate | Int. 135 | G | 431.5 | 432.1 |
| 135 | 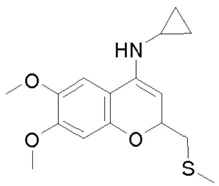 | N-cyclopropyl-6,7-dimethoxy-2-(methylsulfanylmethy 1)-2H-chromen-4-amine | Int. 136 | F | 307.4 | 308.0 |
| 136 | 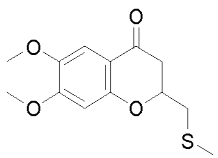 | 6,7-dimethoxy-2-(methylsulfanylmethy 1)chroman-4-one | 1-(2-hydroxy-4,5-dimethoxyphenyl)ethan one, CAS: 20628-06-2 | C | 268.3 | 269.0 |

**Table III. Illustrative compounds of the invention**

| Cpd | Structure | Name | Int | Mtd | MW (calc) | MW (obs) |
|---|---|---|---|---|---|---|
| 1 | 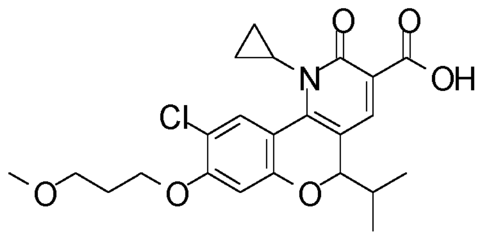 | 1-cyclopropyl-9-fluoro-5-isopropyl-8-(3-methoxypropoxy) -2-oxo-5H-chromeno [4,3-b]pyridine-3-carboxylic acid | Int. 64 | I | 431.5 | 432.1 |
| 2 | 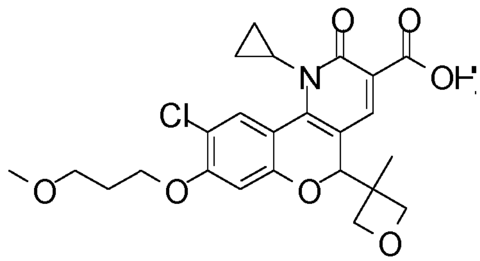 | 9-chloro-1-cyclopropyl-8-(3-methoxypropoxy) -5-(3-methyloxetan-3-yl)-2-oxo-5H-chromeno[4,3-b] pyridine-3-carboxylic acid | Int. 65 | I | 475.9 | 476.1 |
| 3 | 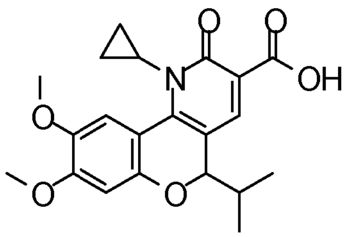 | 1-cyclopropyl-5-isopropyl-8,9-dimethoxy-2-oxo-5H-chromeno[4,3-b] pyridine-3-carboxylic acid | Int. 67 | I | 385.4 | 386.1 |
| 4 | 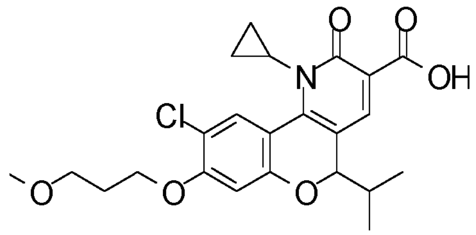 | 9-chloro-1-cyclopropyl-5-isopropyl-8-(3-methoxypropoxy) -2-oxo-5H-chromeno [4,3-b]pyridine-3-carboxylic acid | Int. 68 | I | 447.9 | 448.1 |
| 5 | 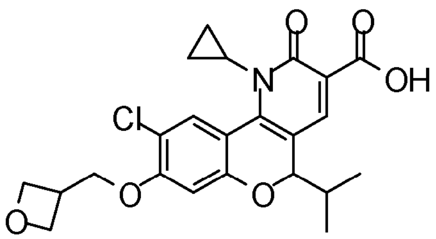 | 9-chloro-1-cyclopropyl-5-isopropyl-8-(oxetan-3-ylmethoxy)-2-oxo-5H-chromeno[4,3-b] pyridine-3-carboxylic acid | Int. 90 | J | 445.9 | 446.1 |
| 6 | 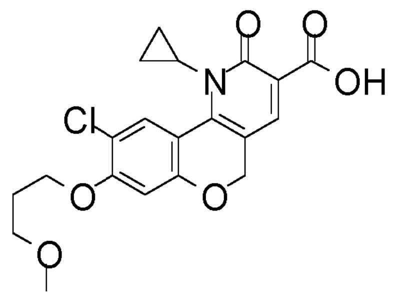 | 9-chloro-1-cyclopropyl-8-(3-methoxypropoxy) -2-oxo-5H-chromeno [4,3-b]pyridine-3-carboxylic acid | Int. 69 | I | 405.8 | 406 |

(continued)

| Cpd | Structure | Name | Int | Mtd | MW (calc) | MW (obs) |
|---|---|---|---|---|---|---|
| 7 | 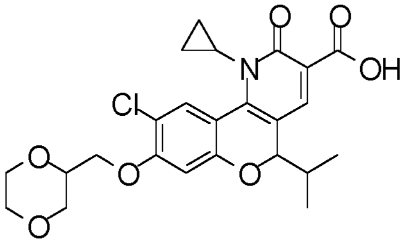 | 9-chloro-1-cyclopropyl-8-(1,4-dioxan-2-ylmethoxy)-5-isopropyl-2-oxo-5H-chromeno[4,3-b] pyridine-3-carboxylic acid | Int. 90 | J | 475.9 | 476.1 |
| 8 | 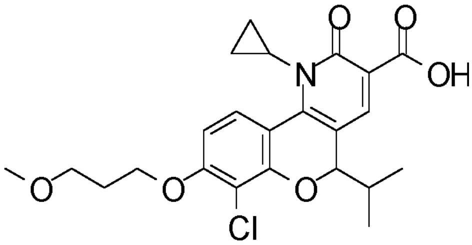 | 7-chloro-1-cyclopropyl-5-isopropyl-8-(3-methoxypropoxy) -2-oxo-5H-chromeno [4,3-b]pyridine-3-carboxylic acid | Int. 70 | I | 447.9 | 448.1 |
| 9 | 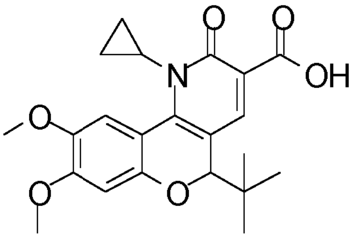 | 5-tert-butyl-1-cyclopropyl-8,9-dimethoxy-2-oxo-5H-chromeno[4,3-b] pyridine-3-carboxylic acid | Int. 71 | I | 399.4 | 400.1 |
| 10 | 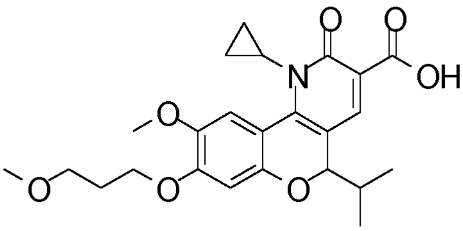 | 1-cyclopropyl-5-isopropyl-9-methoxy-8-(3-methoxypropoxy) -2-oxo-5H-chromeno [4,3-b]pyridine-3-carboxylic acid | Int. 91 | I | 443.5 | 444.1 |
| 11 | 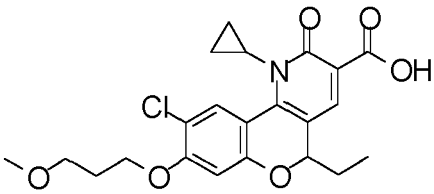 | 9-chloro-1-cyclopropyl-5-ethyl-8-(3-methoxypropoxy) -2-oxo-5H-chromeno [4,3-b]pyridine-3-carboxylic acid | Int. 73 | I | 433.9 | 434.1 |
| 12 | 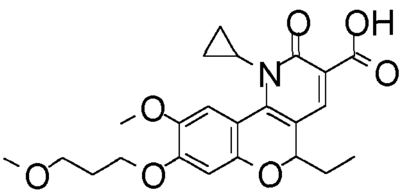 | 1-cyclopropyl-5-ethyl-9-methoxy-8-(3-methoxypropoxy) -2-oxo-5H-chromeno [4,3-b]pyridine-3-carboxylic acid | Int. 74 | I | 429.5 | 430.1 |

(continued)

| Cpd | Structure | Name | Int | Mtd | MW (calc) | MW (obs) |
|---|---|---|---|---|---|---|
| 13 | 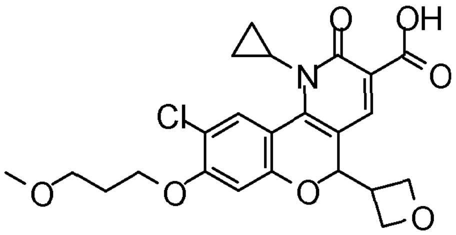 | 9-chloro-1-cyclopropyl-8-(3-methoxypropoxy) -5-(oxetan-3-yl)-2-oxo-5H-chromeno [4,3-b] pyridine-3-carboxylic acid | Int. 75 | I | 461.9 | 462.1 |
| 14 | 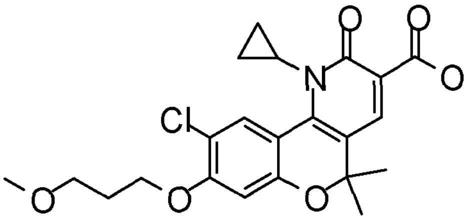 | 9-chloro-1-cyclopropyl-8-(3-methoxypropoxy) -5,5-dimethyl-2-oxo-chromeno [4,3-b] pyridine-3-carboxylic acid | Int. 76 | I | 433.9 | 434.1 |
| 15 | 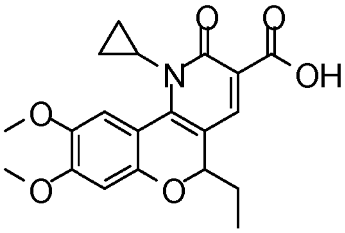 | 1-cyclopropyl-5-ethyl-8,9-dimethoxy-2-oxo-5H-chromeno[4,3-b] pyridine-3-carboxylic acid | Int. 77 | I | 371.4 | 372.1 |
| 16 | 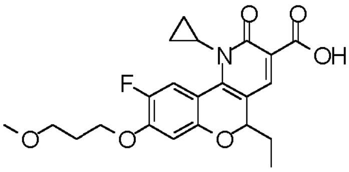 | 1-cyclopropyl-5-ethyl-9-fluoro-8-(3-methoxypropoxy) -2-oxo-5H-chromeno [4,3-b]pyridine-3-carboxylic acid | Int. 78 | I | 417.4 | 418.1 |
| 17 | 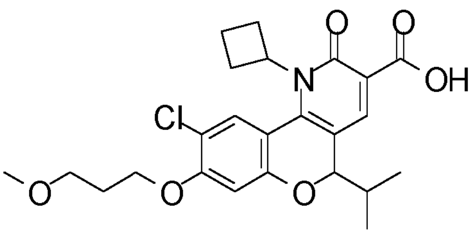 | 9-chloro-1-cyclobutyl-5-isopropyl-8-(3-methoxypropoxy) -2-oxo-5H-chromeno [4,3-b]pyridine-3-carboxylic acid | Int. 79 | I | 461.9 | 462.1 |
| 18 | 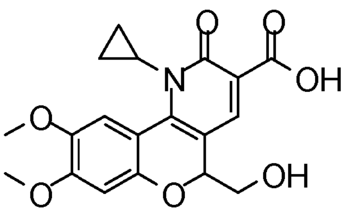 | 1-cyclopropyl-5-(hydroxymethyl)-8,9-dimethoxy-2-oxo-5H-chromeno[4,3-b] pyridine-3-carboxylic acid | Int. 92 | I | 373.4 | 374.1 |

(continued)

| Cpd | Structure | Name | Int | Mtd | MW (calc) | MW (obs) |
|---|---|---|---|---|---|---|
| 19 | 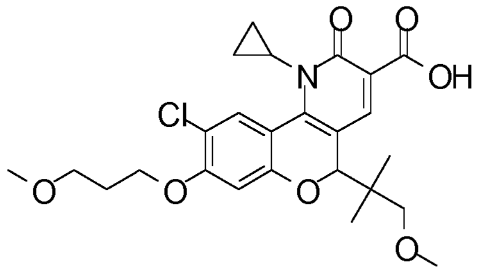 | 9-chloro-1-cyclopropyl-5-(2-methoxy-1,1-dimethyl-ethyl)-8-(3-methoxypropoxy) -2-oxo-5H-chromeno [4,3-b]pyridine-3-carboxylic acid | Int. 81 | I | 492.0 | 492.2 |
| 20 | 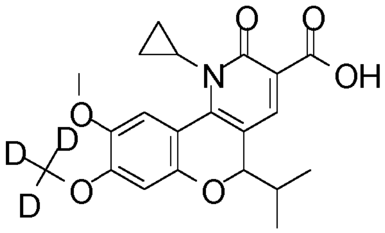 | 1-cyclopropyl-5-isopropyl-9-methoxy-2-oxo-8-(trideuteriomethoxy)-5H-chromeno [4,3-b]pyridine-3-carboxylic acid | Int. 93 | I | 388.4 | 389.1 |
| 21 | 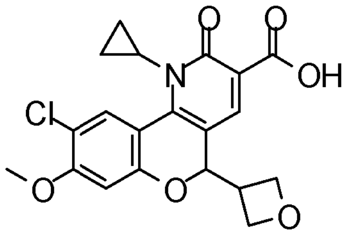 | 9-chloro-1-cyclopropyl-8-methoxy-5-(oxetan-3-yl)-2-oxo-5H-chromeno[4,3-b] pyridine-3-carboxylic acid | Int. 82 | I | 403.8 | 404 |
| 22 | 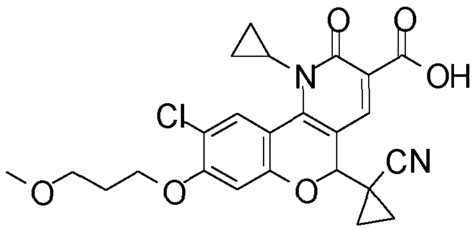 | 9-chloro-5-(1-cyanocyclopropyl )-1-cyclopropyl-8-(3-methoxypropoxy) -2-oxo-5H-chromeno [4,3-b]pyridine-3-carboxylic acid | Int. 83 | I | 470.9 | 471.1 |
| 23 | 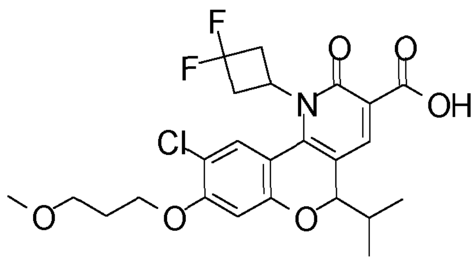 | 9-chloro-1-(3,3-difluorocyclobuty 1)-5-isopropyl-8-(3-methoxypropoxy) -2-oxo-5H-chromeno [4,3-b]pyridine-3-carboxylic acid | Int. 84 | I | 497.9 | 498.1 |
| 24 | 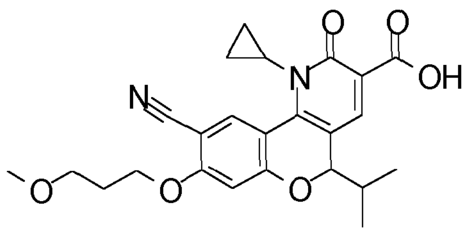 | 9-cyano-1-cyclopropyl-5-isopropyl-8-(3-methoxypropoxy) -2-oxo-5H-chromeno [4,3-b]pyridine-3-carboxylic acid | Int. 94 | I | 438.5 | 439.2 |

(continued)

| Cpd | Structure | Name | Int | Mtd | MW (calc) | MW (obs) |
|---|---|---|---|---|---|---|
| 25 | 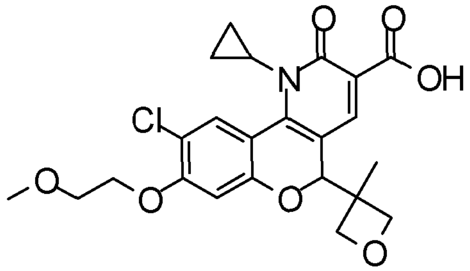 | 9-chloro-1-cyclopropyl-8-(2-methoxyethoxy)-5-(3-methyloxetan-3-yl)-2-oxo-5H-chromeno [4,3-b]pyridine-3-carboxylic acid | Int. 86 | I | 461.9 | 462.2 |
| 26 | 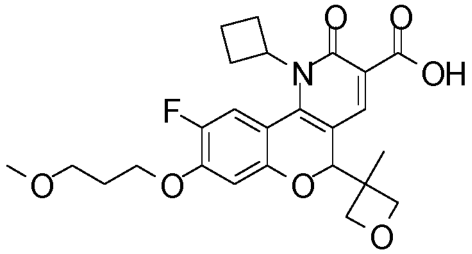 | 1-cyclobutyl-9-fluoro-8-(3-methoxypropoxy) -5-(3-methyloxetan-3-yl)-2-oxo-5H-chromeno[4,3-b] pyridine-3-carboxylic acid | Int. 66 | I | 473.5 | 474.1 |
| 27 | 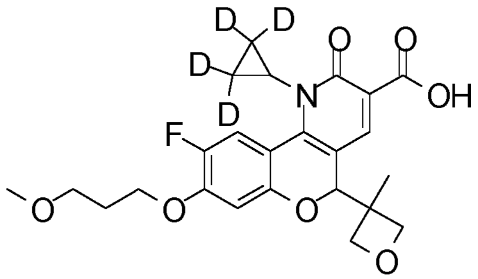 | 9-fluoro-8-(3-methoxypropoxy) -5-(3-methyloxetan-3-yl)-2-oxo-1-(2,2,3,3-tetradeuteriocycl opropyl)-5H-chromeno[4,3-b] pyridine-3-carboxylic acid | Int. 87 | I | 463.5 | 464.1 |
| 28 | 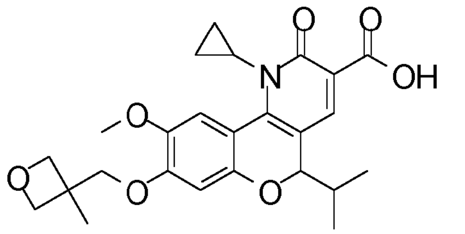 | 1-cyclopropyl-5-isopropyl-9-methoxy-8-[(3-methyloxetan-3-yl)methoxy]-2-oxo-5H-chromeno[4,3-b] pyridine-3-carboxylic acid | Int. 88 | K | 455.5 | 456.2 |
| 29 | 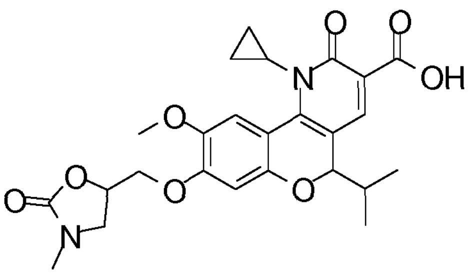 | 1-cyclopropyl-5-isopropyl-9-methoxy-8-[(3-methyl-2-oxooxazolidin-5-yl) methoxy]-2-oxo-5H-chromeno[4,3-b] pyridine-3-carboxylic acid | Int. 88 | K | 484.5 | 485.2 |

(continued)

| Cpd | Structure | Name | Int | Mtd | MW (calc) | MW (obs) |
|---|---|---|---|---|---|---|
| 30 | 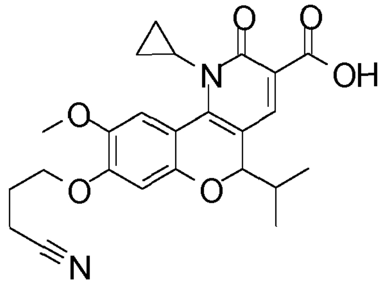 | 8-(3-cyanopropoxy)-1-cyclopropyl-5-isopropyl-9-methoxy-2-oxo-5H-chromeno [4,3-b]pyridine-3-carboxylic acid | Int. 88 | K | 438.5 | 439.2 |
| 31 | 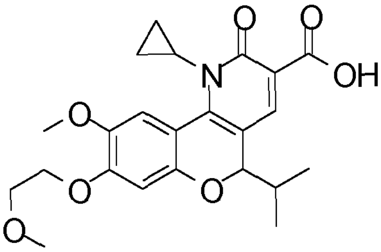 | 1-cyclopropyl-5-isopropyl-9-methoxy-8-(2-methoxyethoxy)-2-oxo-5H-chromeno [4,3-b]pyridine-3-carboxylic acid | Int. 88 | K | 429.5 | 430.2 |
| 32 | 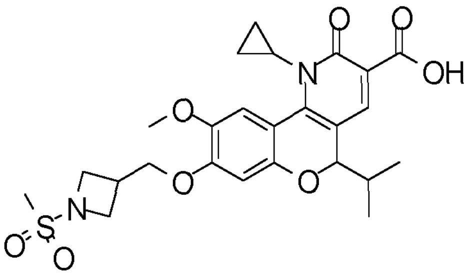 | 1-cyclopropyl-5-isopropyl-9-methoxy-8-[(1-methylsulfonylazetidin-3-yl)methoxy]-2-oxo-5H-chromeno [4,3-b]pyridine-3-carboxylic acid | Int. 88 | K | 518.6 | 519.2 |
| 33 | 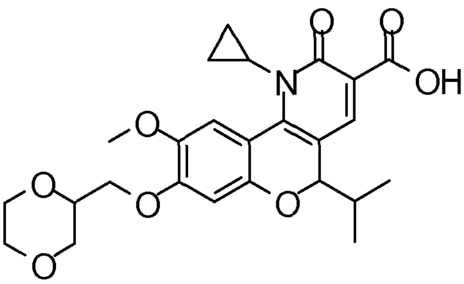 | 1-cyclopropyl-8-(1,4-dioxan-2-ylmethoxy)-5-isopropyl-9-methoxy-2-oxo-5H-chromeno[4,3-b] pyridine-3-carboxylic acid | Int. 88 | K | 471.5 | 472.2 |
| 34 | 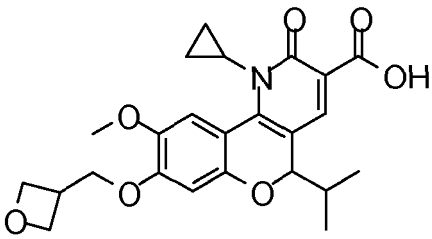 | 1-cyclopropyl-5-isopropyl-9-methoxy-8-(oxetan-3-ylmethoxy)-2-oxo-5H-chromeno[4,3-b] pyridine-3-carboxylic acid | Int. 88 | K | 441.5 | 442.2 |

(continued)

| Cpd | Structure | Name | Int | Mtd | MW (calc) | MW (obs) |
|---|---|---|---|---|---|---|
| 35 | 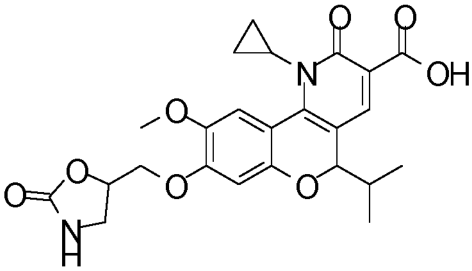 | 1-cyclopropyl-5-isopropyl-9-methoxy-2-oxo-8-[(2-oxooxazolidin-5-yl) methoxy]-5H-chromeno[4,3-b] pyridine-3-carboxylic acid | Int. 88 | K | 470.5 | 471.2 |
| 36 | 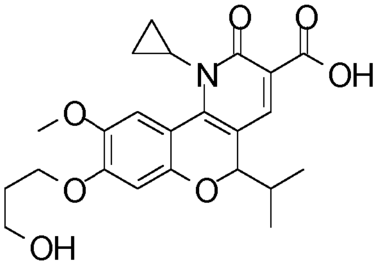 | 1-cyclopropyl-8-(3-hydroxypropoxy) -5-isopropyl-9-methoxy-2-oxo-5H-chromeno [4,3-b]pyridine-3-carboxylic acid | Int. 88 | K | 475.9 | 430.2 |
| 37 | 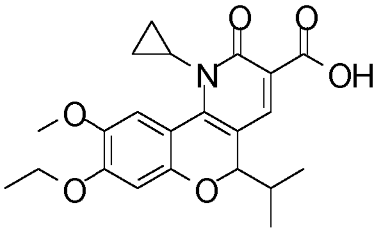 | 1-cyclopropyl-8-ethoxy-5-isopropyl-9-methoxy-2-oxo-5H-chromeno[4,3-b] pyridine-3-carboxylic acid | Int. 88 | K | 399.4 | 400.1 |
| 38 | 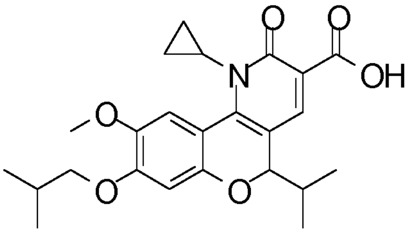 | 1-cyclopropyl-8-isobutoxy-5-isopropyl-9-methoxy-2-oxo-5H-chromeno [4,3-b]pyridine-3-carboxylic acid | Int. 88 | K | 427.5 | 428.2 |
| 39 | 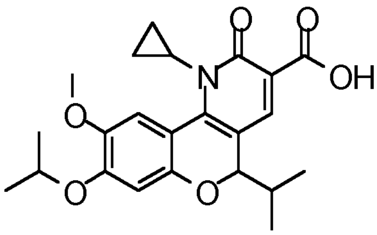 | 1-cyclopropyl-8-isopropoxy-5-isopropyl-9-methoxy-2-oxo-5H-chromeno [4,3-b]pyridine-3-carboxylic acid | Int. 88 | K | 413.5 | 414.1 |
| 40 | 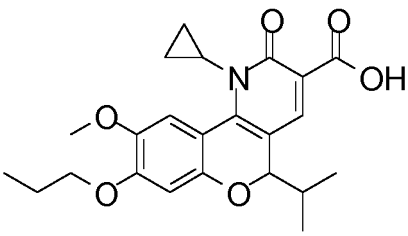 | 1-cyclopropyl-5-isopropyl-9-methoxy-2-oxo-8-propoxy-5H-chromeno[4,3-b] pyridine-3-carboxylic acid | Int. 88 | K | 413.5 | 414.1 |

(continued)

| Cpd | Structure | Name | Int | Mtd | MW (calc) | MW (obs) |
|---|---|---|---|---|---|---|
| 41 | 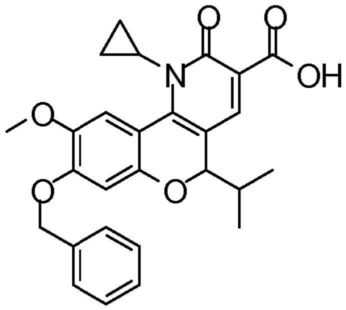 | 8-benzyloxy-1-cyclopropyl-5-isopropyl-9-methoxy-2-oxo-5H-chromeno [4,3-b]pyridine-3-carboxylic acid | Int. 88 | K | 461.5 | 462.1 |
| 42 | 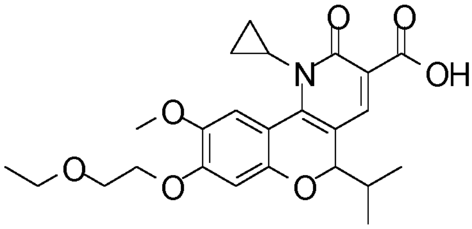 | 1-cyclopropyl-8-(2-ethoxyethoxy)-5-isopropyl-9-methoxy-2-oxo-5H-chromeno [4,3-b]pyridine-3-carboxylic acid | Int. 88 | K | 443.5 | 444.1 |
| 43 | 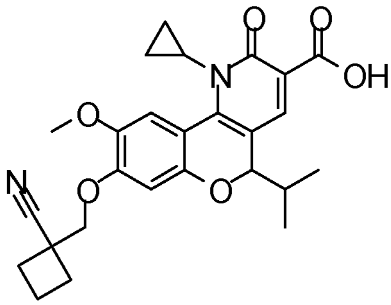 | 8-[(1-cyanocyclobutyl) methoxy]-1-cyclopropyl-5-isopropyl-9-methoxy-2-oxo-5H-chromeno [4,3-b]pyridine-3-carboxylic acid | Int. 88 | NA | 464.5 | 465.2 |
| 44 | 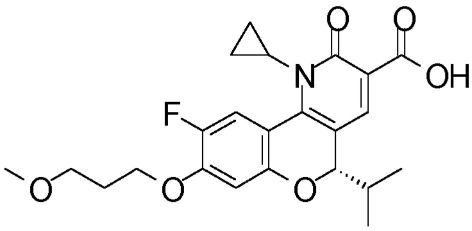 | (5S)-1-cyclopropyl-9-fluoro-5-isopropyl-8-(3-methoxypropoxy) -2-oxo-5H-chromeno [4,3-b]pyridine-3-carboxylic acid | Cpd. 1 | NA | 431.5 | 432.1 |
| 45 | 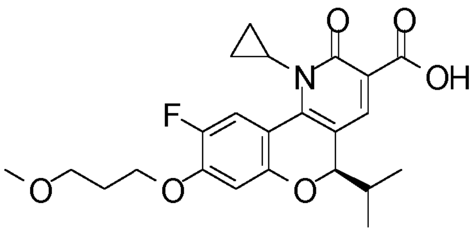 | (5R)-1-cyclopropyl-9-fluoro-5-isopropyl-8-(3-methoxypropoxy) -2-oxo-5H-chromeno [4,3-b]pyridine-3-carboxylic acid | Cpd. 1 | NA | 431.5 | 432.1 |

(continued)

| Cpd | Structure | Name | Int | Mtd | MW (calc) | MW (obs) |
|---|---|---|---|---|---|---|
| 46 | 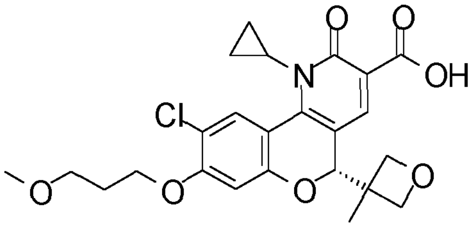 | (5R)-9-chloro-1-cyclopropyl-8-(3-methoxypropoxy) -5-(3-methyloxetan-3-yl)-2-oxo-5H-chromeno[4,3-b] pyridine-3-carboxylic acid | Cp d. 2 | NA | 475.9 | 476.1 |
| 47 | 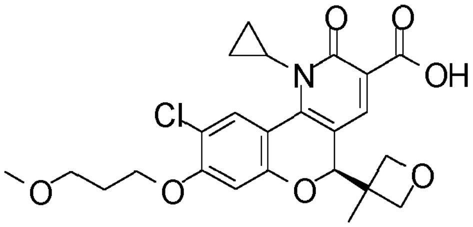 | (5S)-9-chloro-1-cyclopropyl-8-(3-methoxypropoxy) -5-(3-methyloxetan-3-yl)-2-oxo-5H-chromeno[4,3-b] pyridine-3-carboxylic acid | Cp d. 2 | NA | 475.9 | 476.1 |
| 48 | 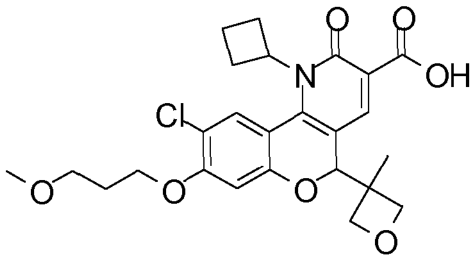 | 9-chloro-1-cyclobutyl-8-(3-methoxypropoxy) -5-(3-methyloxetan-3-yl)-2-oxo-5H-chromeno[4,3-b] pyridine-3-carboxylic acid | Int. 95 | I | 490.0 | 490 |
| 49 | 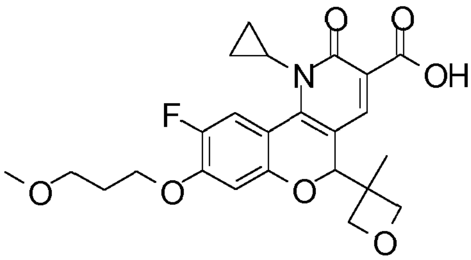 | 1-cyclopropyl-9-fluoro-8-(3-methoxypropoxy) -5-(3-methyloxetan-3-yl)-2-oxo-5H-chromeno[4,3-b] pyridine-3-carboxylic acid | Int. 97 | I | 459.5 | 460.1 |
| 50 | 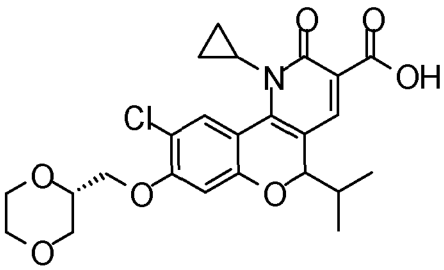 | 9-chloro-1-cyclopropyl-8-[[(2S)-1,4-dioxan-2-yl] methoxy]-5-isopropyl-2-oxo-5H-chromeno [4,3-b]pyridine-3-carboxylic acid | Int. 90 | J | 475.9 | 476.1 |
| 51 | 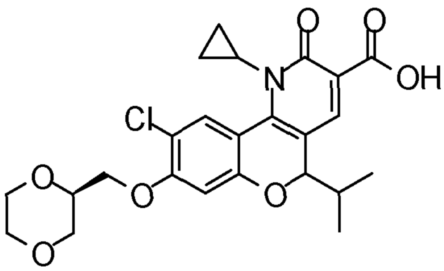 | 9-chloro-1-cyclopropyl-8-[[(2R)-1,4-dioxan-2-yl] methoxy]-5-isopropyl-2-oxo-5H-chromeno [4,3-b]pyridine-3-carboxylic acid | Int. 90 | J | 475.9 | 476.1 |

(continued)

| Cpd | Structure | Name | Int | Mtd | MW (calc) | MW (obs) |
|---|---|---|---|---|---|---|
| 52 | 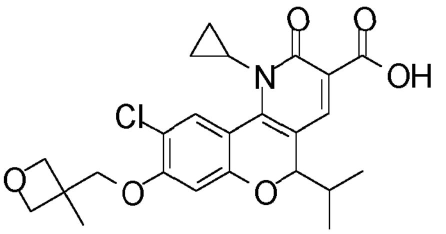 | 9-chloro-1-cyclopropyl-5-isopropyl-8-[(3-methyloxetan-3-yl) methoxy]-2-oxo-5H-chromeno[4,3-b] pyridine-3-carboxylic acid | Int. 90 | J | 459.9 | 460.1 |
| Cpd | Structure | Name | Int | Mtd | MW (calc) | MW (obs) |
| 53 | 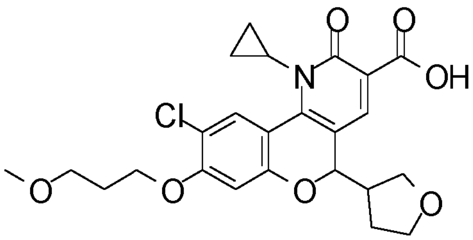 | 9-chloro-1-cyclopropyl-8-(3-methoxypropoxy) -2-oxo-5-tetrahydrofuran-3-yl-5H-chromeno[4,3-b] pyridine-3-carboxylic acid | 99 | I | 475.9 | 476.1 |
| 54 | 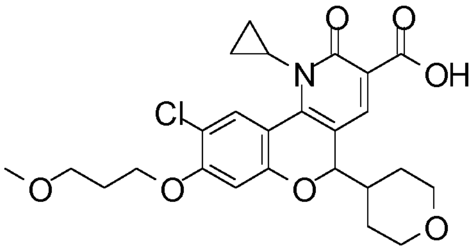 | 9-chloro-1-cyclopropyl-8-(3-methoxypropoxy) -2-oxo-5-tetrahydropyran-4-yl-5H-chromeno[4,3-b] pyridine-3-carboxylic acid | 100 | I | 489.9 | 490.1 |
| 55 | 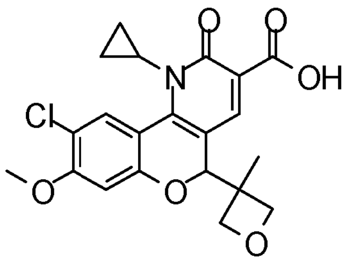 | 9-chloro-1-cyclopropyl-8-methoxy-5-(3-methyloxetan-3-yl)-2-oxo-5H- chromeno [4,3-b]pyridine-3-carboxylic acid | 105 | K | 417.8 | 418.0 |
| 56 | 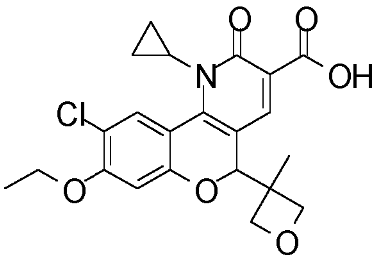 | 9-chloro-1-cyclopropyl-8-ethoxy-5-(3-methyloxetan-3-yl)-2-oxo-5H-chromeno[4,3-b] pyridine-3-carboxylic acid | 105 | K | 431.9 | 432.1 |

(continued)

| Cpd | Structure | Name | Int | Mtd | MW (calc) | MW (obs) |
|---|---|---|---|---|---|---|
| 57 | 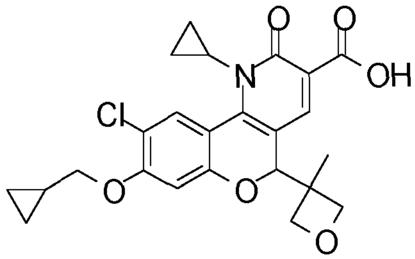 | 9-chloro-1-cyclopropyl-8-(cyclopropylmethoxy)-5-(3-methyloxetan-3-yl)-2-oxo-5H-chromeno [4,3-b]pyridine-3-carboxylic acid | 105 | K | 457.9 | 458.1 |
| 58 | 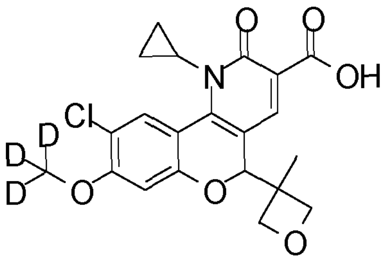 | 9-chloro-1-cyclopropyl-5-(3-methyloxetan-3-yl)-2-oxo-8-(trideuteriomethoxy)-5H-chromeno [4,3-b]pyridine-3-carboxylic acid | 105 | K | 420.9 | 421.0 |
| 59 | 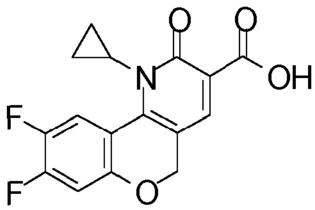 | 1-cyclopropyl-8,9-difluoro-2-oxo-5H-chromeno[4,3-b] pyridine-3-carboxylic acid | Int. 108 | I | 319.3 | 320.0 |
| 60 | 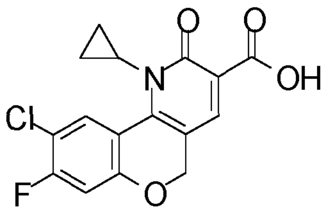 | 9-chloro-1-cyclopropyl-8-fluoro-2-oxo-5H-chromeno [4,3-b]pyridine-3-carboxylic acid | Int. 110 | I | 335.7 | 336.0 |
| 61 | 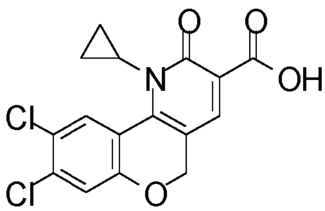 | 8,9-dichloro-1-cyclopropyl-2-oxo-5H-chromeno[4,3-b] pyridine-3-carboxylic acid | Int. 112 | I | 352.2 | 352.0 |
| 62 | 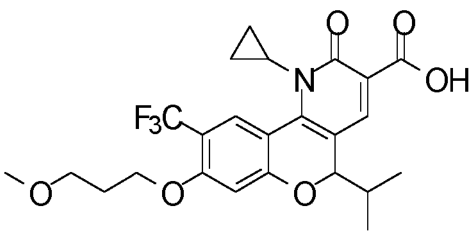 | 1-cyclopropyl-5-isopropyl-8-(3-methoxypropoxy) -2-oxo-9-(trifluoromethyl)-5H-chromeno[4,3-b] pyridine-3-carboxylic acid | Int. 114 | I | 481.5 | 482.2 |

(continued)

| Cpd | Structure | Name | Int | Mtd | MW (calc) | MW (obs) |
|---|---|---|---|---|---|---|
| 63 | 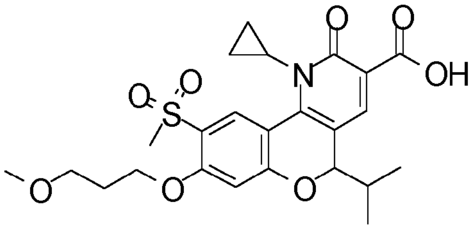 | 1-cyclopropyl-5-isopropyl-8-(3-methoxypropoxy) -9-methylsulfonyl-2-oxo-5H-chromeno[4,3-b] pyridine-3-carboxylic acid | Int. 115 | I | 491.6 | 492.2 |
| 64 | 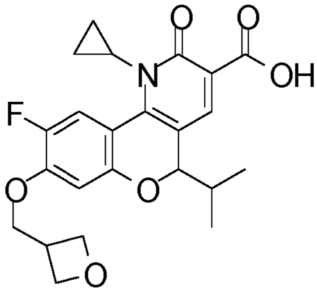 | 1-cyclopropyl-9-fluoro-5-isopropyl-8-(oxetan-3-ylmethoxy)-2-oxo-5H-chromeno[4,3-b] pyridine-3-carboxylic acid | Int. 116 | I | 429.4 | 430.2 |
| 65 | 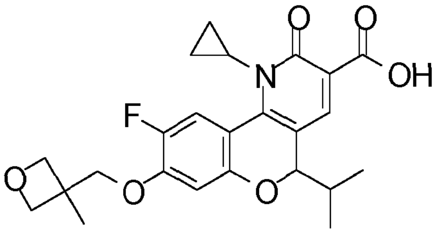 | 1-cyclopropyl-9-fluoro-5-isopropyl-8-[(3-methyloxetan-3-yl)methoxy]-2-oxo-5H-chromeno[4,3-b] pyridine-3-carboxylic acid | Int. 120 | I | 443.5 | 444.2 |
| 66 | 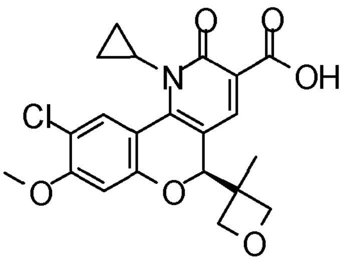 | (5S)-9-chloro-1-cyclopropyl-8-methoxy-5-(3-methyloxetan-3-yl)-2-oxo-5H-chromeno [4,3-b]pyridine-3-carboxylic acid | Cp d. 55 | NA | 417.8 | 418.0 |
| 67 | 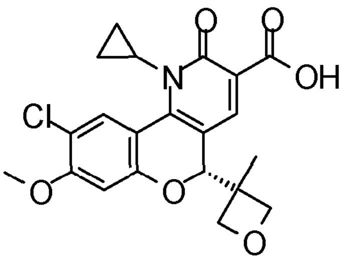 | (5R)-9-chloro-1-cyclopropyl-8-methoxy-5-(3-methyloxetan-3-yl)-2-oxo-5H-chromeno [4,3-b]pyridine-3-carboxylic acid | Cp d. 55 | NA | 417.8 | 418.1 |
| 68 | 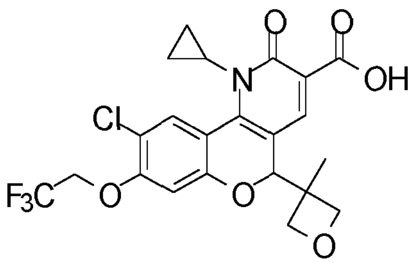 | 9-chloro-1-cyclopropyl-5-(3-methyloxetan-3-yl)-2-oxo-8-(2,2,2-trifluoroethoxy)-5H-chromeno[4,3-b] pyridine-3-carboxylic acid | Int. 124 | I | 485.8 | 486.1 |

(continued)

| Cpd | Structure | Name | Int | Mtd | MW (calc) | MW (obs) |
|---|---|---|---|---|---|---|
| 69 | 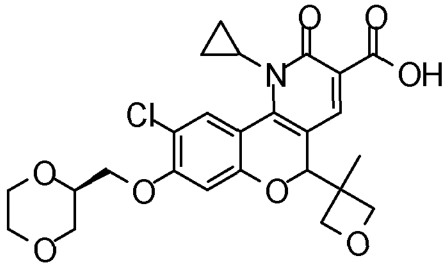 | 9-chloro-1-cyclopropyl-8-[[(2R)-1,4-dioxan-2-yl] methoxy]-5-(3-methyloxetan-3-yl)-2-oxo-5H-chromeno [4,3-b]pyridine-3-carboxylic acid | Int. 128 | I | 503.9 | 504.1 |
| 70 | 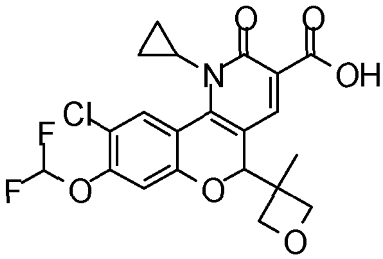 | 9-chloro-1-cyclopropyl-8-(difluoromethoxy )-5-(3-methyloxetan-3-yl)-2-oxo-5H-chromeno[4,3-b] pyridine-3-carboxylic acid | Int. 131 | I | 453.8 | 454.0 |
| 71 | 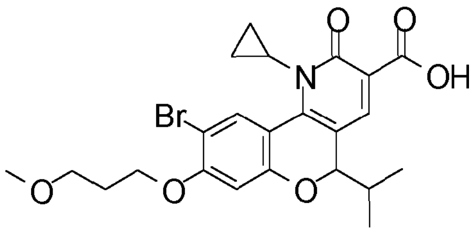 | 9-bromo-1-cyclopropyl-5-isopropyl-8-(3-methoxypropoxy) -2-oxo-5H-chromeno [4,3-b]pyridine-3-carboxylic acid | Int. 85 | I | 492.4 | 494.1 |
| 72 | 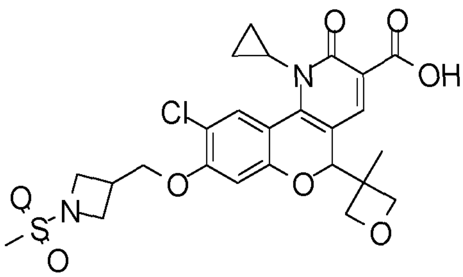 | 9-chloro-1-cyclopropyl-5-(3-methyloxetan-3-yl)-8-[(1-methylsulfonylaz etidin-3-yl)methoxy]-2-oxo-5H-chromeno [4,3-b]pyridine-3-carboxylic acid | Int. 125 | K | 551.0 | 551.1 |
| 73 | 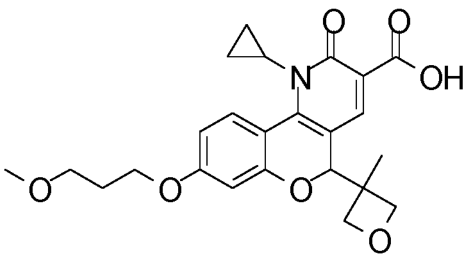 | 1-cyclopropyl-8-(3-methoxypropoxy) -5-(3-methyloxetan-3-yl)-2-oxo-5H-chromeno[4,3-b] pyridine-3-carboxylic acid | Int. 123 | I | 441.5 | 442.1 |
| 74 | 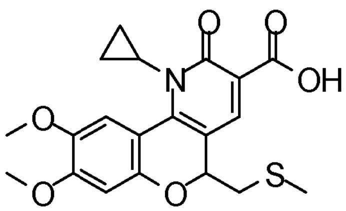 | 1 -cyclopropyl-8,9-dimethoxy-5-(methylsulfanylm ethyl)-2-oxo-5H-chromeno[4,3-b] pyridine-3-carboxylic acid | Int. 134 | I | 403.5 | 404.1 |

(continued)

| Cpd | Structure | Name | Int | Mtd | MW (calc) | MW (obs) |
|---|---|---|---|---|---|---|
| 75 | 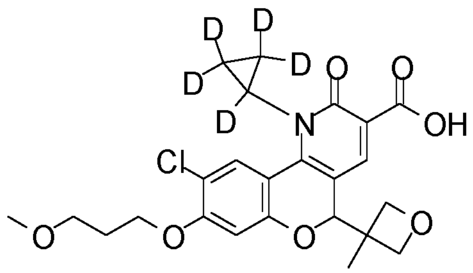 | 9-chloro-8-(3-methoxypropoxy) -5-(3-methyloxetan-3-yl)-2-oxo-1-(1,2,2,3,3-pentadeuteriocyclopropyl)-5H-chromeno[4,3-b] pyridine-3-carboxylic acid | Described above | - | 481.0 | 481.2 |

**Table IV. NMR table: final compounds**

| Cpd. | NMR |
|---|---|
| 1 | 1H NMR (400 MHz, DMSO-d6) δ 14.38 (s, 1H), 7.95 (s, 1H), 7.78 (d, 1H), 6.73 (d, 1H), 4.66 (d, 1H), 4.03 - 3.87 (m, 2H), 3.51 (dd, 1H), 3.24 (t, 2H), 3.02 (s, 3H), 1.75 (p, 2H), 1.64 - 1.50 (m, 1H), 1.11 - 0.98 (m, 1H), 0.77 (p, 1H), 0.69 (d, 3H), 0.57 (d, 3H), 0.48 - 0.37 (m, 1H), 0.03 (dq, 1H) |
| 2 | 1H NMR (400 MHz, DMSO-d6-, ppm) δ 14.57 (s, 1H), 8.21 (s, 1H), 8.06 (s, 1H), 6.93 (s, 1H), 5.47 (s, 1H), 4.54 (d, 1H), 4.48 (d, 1H), 4.24 (d, 1H), 4.23 - 4.12 (m, 2H), 4.04 (d, 1H), 3.82 - 3.72 (m, 1H), 3.50 (t, 2H), 3.26 (s, 3H), 2.00 (p, 2H), 1.32 (s, 3H), 1.28 -1.18 (m, 1H), 1.12 - 1.02 (m, 1H), 0.62 - 0.52 (m, 1H), 0.48 - 0.38 (m, 1H). |
| 3 | 1H NMR (400 MHz, DMSO-d6, ppm) δ 14.68 (s, 1H), 8.18 (s, 1H), 7.62 (s, 1H), 6.79 (s, 1H), 4.84 (d, 1H), 3.86 (s, 3H), 3.85 - 3.79 (m, 1H), 3.78 (s, 3H), 1.88 -1.74 (m, 1H), 1.37 - 1.25 (m, 1H), 1.12 -1.00 (m, 1H), 0.93 (d, 3H), 0.81 (d, 3H), 0.70 - 0.59 (m, 1H), 0.34 - 0.22 (m, 1H). |
| 4 | 1H NMR (400 MHz, DMSO-d6, ppm) δ 14.63 (s, 1H), 8.20 (d, 2H), 6.95 (s, 1H), 4.94 (d, 1H), 4.28 - 4.12 (m, 2H), 3.74 (tt, 1H), 3.51 (t, 2H), 3.26 (s, 3H), 2.00 (p, 2H), 1.88 -1.75 (m, 1H), 1.38 -1.26 (m, 1H), 1.03 - 0.94 (m, 1H), 0.93 (d, 3H), 0.81 (d, 3H), 0.73 - 0.63 (m, 1H), 0.33 - 0.22 (m, 1H). |
| 5 | 1H NMR (400 MHz, DMSO-d6, ppm) δ 14.63 (s, 1H), 8.21 (d, 2H), 7.02 (s, 1H), 4.96 (d, 1H), 4.73 (dd, 2H), 4.48 (td, 2H), 4.46 - 4.32 (m, 2H), 3.80 - 3.68 (m, 1H), 3.51 - 3.39 (m, 1H), 2.06 - 1.73 (m, 1H), 1.40 - 1.26 (m, 1H), 1.02 - 0.96 (m, 1H), 0.94 (d, 3H), 0.82 (d, 3H), 0.72 - 0.62 (m, 1H), 0.35 - 0.22 (m, 1H). |
| 6 | 1H NMR (400 MHz, DMSO-d6, ppm) δ 14.62 (s, 1H), 8.33 (s, 1H), 8.22 (s, 1H), 6.96 (s, 1H), 5.02 (s, 2H), 4.20 (t, 2H), 3.80 - 3.74 (m, 1H), 3.51 (t, 3H), 3.26 (s, 3H), 2.00 (p, 2H), 1.19 - 1.08 (m, 2H), 0.52 - 0.43 (m, 2H). |
| 7 | 1H NMR (400 MHz, DMSO-d6, ppm) δ 14.63 (s, 1H), 8.21 (d, 2H), 6.99 (s, 1H), 4.95 (d, 1H), 4.26 - 4.17 (m, 1H), 4.17 - 4.09 (m, 1H), 3.94 - 3.88 (m, 1H), 3.88 - 3.82 (m, 1H), 3.82 - 3.59 (m, 4H), 3.55 - 3.42 (m, 2H), 1.87 - 1.74 (m, 1H), 1.38 - 1.27 (m, 1H), 1.01 - 0.95 (m, 1H), 0.93 (d, 3H), 0.81 (d, 3H), 0.72 - 0.64 (m, 1H), 0.31 - 0.21 (m, 1H). |
| 8 | 1H NMR (400 MHz, DMSO-d6, ppm) δ 14.63 (s, 1H), 8.24 (s, 1H), 8.10 (d, 1H), 6.94 (d, 1H), 5.08 (d, 1H), 4.23 (t, 2H), 3.74 - 3.64 (m, 1H), 3.52 (t, 2H), 3.26 (s, 3H), 2.02 (p, 2H), 1.80 - 1.68 (m, 1H), 1.35 - 1.27 (m, 1H), 1.01 - 0.95 (m, 1H), 0.92 (d, 3H), 0.83 (d, 3H), 0.74 - 0.60 (m, 1H), 0.29 - 0.24 (m, 1H). |
| 9 | 1H NMR (400 MHz, DMSO-d6, ppm) δ 14.65 (s, 1H), 8.12 (s, 1H), 7.58 (s, 1H), 6.75 (s, 1H), 5.01 (s, 1H), 3.85 (s, 3H), 3.84 - 3.79 (m, 1H), 3.77 (s, 3H), 1.40 - 1.29 (m, 1H), 1.08 - 0.98 (m, 1H), 0.76 (s, 9H), 0.72 - 0.62 (m, 1H), 0.31 - 0.20 (m, 1H). |
| 10 | 1H NMR (400 MHz, DMSO-d6, ppm) δ 14.68 (s, 1H), 8.18 (s, 1H), 7.62 (s, 1H), 6.78 (s, 1H), 4.84 (d, 1H), 4.11 (m, 2H), 3.87 -3.80 (m, 1H), 3.79 (s, 3H), 3.48 (t, 2H), 3.26 (s, 3H), 1.98 (p, 2H), 1.85 - 1.75 (m, 1H), 1.36 - 1.26 (m, 1H), 1.13 - 1.01 (m, 1H), 0.93 (d, 3H), 0.80 (d, 3H), 0.71 - 0.59 (m, 1H), 0.71 - 0.59 (m, 1H). |
| 11 | 1H NMR (400 MHz, DMSO-d6, ppm) δ 14.39 (s, 1H), 8.23 (s, 1H), 8.20 (s, 1H), 6.94 (s, 1H), 5.17 (dd, 1H), 4.27 - 4.12 (m, 2H), 3.79 - 3.72 (m, 1H), 3.51 (t, 2H), 3.26 (s, 3H), 2.00 (p, 2H), 1.75 - 1.59 (m, 2H), 1.32 - 1.20 (m, 1H), 1.08 - 0.97 (m, 1H), 0.93 (t, 3H), 0.66 - 0.54 (m, 1H), 0.41 - 0.29 (m, 1H). |

(continued)

| Cpd. | NMR |
|---|---|
| 12 | 1H NMR (400 MHz, DMSO-d6, ppm) δ 14.68 (s, 1H), 8.21 (s, 1H), 7.61 (s, 1H), 6.77 (s, 1H), 5.08 (dd, 1H), 4.10 (tt, 2H), 3.88 - 3.80 (m, 1H), 3.79 (s, 3H), 3.48 (t, 2H), 3.25 (s, 3H), 1.98 (p, 2H), 1.76 - 1.57 (m, 2H), 1.31 - 1.21 (m, 1H), 1.17 - 1.05 (m, 1H), 0.93 (t, 3H), 0.63 - 0.52 (m, 1H), 0.40 - 0.32 (m, 1H). |
| 13 | 1H NMR (400 MHz, DMSO-d6, ppm) δ 8.21 (s, 1H), 8.14 (s, 1H), 6.93 (s, 1H), 5.53 (d, 1H), 4.65 - 4.47 (m, 4H), 4.19 (td, 2H), 3.78 - 3.72 (m, 1H), 3.54 - 3.43 (m, 3H), 3.26 (s, 3H), 2.00 (p, 2H), 1.25 - 1.16 (m, 1H), 1.13 - 1.01 (m, 1H), 0.57 - 0.50 (m, 1H), 0.45 - 0.38 (m, 1H). |
| 14 | 1H NMR (400 MHz, DMSO-d6, ppm) δ 14.67 (s, 1H), 8.25 (s, 1H), 8.20 (s, 1H), 6.88 (s, 1H), 4.19 (t, 2H), 3.82 - 3.73 (m, 1H), 3.51 (t, 2H), 3.26 (s, 3H), 2.00 (p, 2H), 1.55 (s, 6H), 1.19 - 1.10 (m, 2H), 0.52 - 0.43 (m, 2H). |
| 15 | 1H NMR (400 MHz, DMSO-d6, ppm) δ 14.68 (s, 1H), 8.22 (s, 1H), 7.62 (s, 1H), 6.78 (s, 1H), 5.08 (dd, 1H), 3.86 (s, 3H), 3.85 - 3.80 (m, 1H), 3.79 (s, 3H), 1.75 - 1.60 (m, 2H), 1.30 - 1.20 (m, 1H), 1.17 - 1.06 (m, 1H), 0.94 (t, 3H), 0.60 - 0.53 (m, 1H), 0.41 - 0.34 (m, 1H). |
| 16 | 1H NMR (400 MHz, DMSO-d6, ppm) δ 14.63 (s, 1H), 8.25 (s, 1H), 8.03 (d, 1H), 6.96 (d, 1H), 5.14 (dd, 1H), 4.26 - 4.12 (m, 2H), 3.78 - 3.74 (m, 1H), 3.48 (t, 2H), 3.26 (s, 3H), 2.00 (p, 2H), 1.75 - 1.60 (m, 2H), 1.30 - 1.21 (m, 1H), 1.12 - 1.02 (m, 1H), 0.94 (t, 3H), 0.63 - 0.59 (m, 1H), 0.40 - 0.30 (m, 1H). |
| 17 | 1H NMR (400 MHz, DMSO-d6, ppm) δ 14.47 (s, 1H), 8.15 (s, 1H), 7.66 (s, 1H), 6.98 (s, 1H), 5.26 - 5.15 (t, 1H), 4.94 (d, 1H), 4.27 - 4.11 (m, 2H), 3.50 (t, 2H), 3.26 (s, 3H), 2.66 - 2.56 (m, 1H), 2.31 - 2.19 (m, 1H), 2.10 - 1.96 (m, 4H), 1.83 - 1.71 (m, 1H), 1.68 - 1.54 (m, 2H), 0.90 (d, 3H), 0.82 (d, 3H). |
| 19 | 1H NMR (400 MHz, CDCl3 , ppm) δ 14.21 (s, 1H), 8.19 (s, 1H), 7.86 (s, 1H), 6.62 (s, 1H), 5.20 (s, 1H), 4.26 - 4.10 (m, 2H), 3.67 - 3.57 (m, 2H), 3.46 - 3.40 (m, 1H), 3.39 (s, 3H), 3.37 (s, 3H), 3.05 - 2.96 (m, 2H), 2.15 (p, 2H), 1.61 - 1.52 (m, 1H), 1.19 - 1.09 (m, 1H), 0.95 (s, 3H), 0.80 - 0.70 (m, 1H), 0.56 (s, 3H), 0.47 - 0.38 (m, 1H). |
| 20 | 1H NMR (400 MHz, DMSO-d6, ppm) δ 14.67 (s, 1H), 8.18 (s, 1H), 7.62 (s, 1H), 6.78 (s, 1H), 4.84 (d, 1H), 3.87 - 3.79 (m, 1H), 3.78 (s, 3H), 1.88 - 1.75 (m, 1H), 1.35 -1.26 (m, 1H), 1.12 -1.00 (m, 1H), 0.93 (d, 3H), 0.81 (d, 3H), 0.70 - 0.59 (m, 1H), 0.34 - 0.22 (m, 1H). |
| 21 | 1H NMR (400 MHz, DMSO-d6, ppm) δ 14.55 (s, 1H), 8.22 (s, 1H), 8.16 (s, 1H), 6.94 (s, 1H), 5.54 (d, 1H), 4.65 - 4.50 (m, 4H), 3.94 (s, 3H), 3.81 - 3.71 (m, 1H), 3.51 - 3.44 (m, 1H), 1.26 - 1.14 (m, 1H), 1.14 - 1.03 (m, 1H), 0.58 - 0.48 (m, 1H), 0.47 - 0.37 (m, 1H). |
| 22 | 1H NMR (400 MHz, DMSO-d6, ppm) δ 14.46 (s, 1H), 8.35 (s, 1H), 8.26 (s, 1H), 7.04 (s, 1H), 4.97 (s, 1H), 4.32 - 4.14 (m, 2H), 3.89 - 3.77 (m, 1H), 3.57 - 3.48 (m, 2H), 3.27 (s, 3H), 2.16 - 1.90 (m, 2H), 1.55 - 1.32 (m, 3H), 1.25 - 1.14 (m, 2H), 1.13 - 1.03 (m, 1H), 0.65 - 0.53 (m, 1H), 0.53 - 0.41 (m, 1H). |
| 23 | 1H NMR (400 MHz, DMSO-d6, ppm) δ 14.18 (s, 1H), 8.21 (s, 1H), 7.69 (s, 1H), 7.01 (s, 1H), 5.28 - 5.11 (m, 1H), 4.97 (d, 1H), 4.28 - 4.12 (m, 2H), 3.50 (t, 2H), 3.26 (s, 3H), 3.19 - 3.08 (m, 2H), 3.08 - 2.94 (m, 1H), 2.74 - 2.59 (m, 1H), 1.99 (p, 2H), 1.83 - 1.70 (m, 1H), 0.91 (d, 3H), 0.83 (d, 3H). |
| 24 | 1H NMR (400 MHz, DMSO-d6, ppm) δ 14.55 (s, 1H), 8.51 (s, 1H), 8.18 (s, 1H), 7.04 (s, 1H), 5.06 (d, 1H), 4.35 - 4.18 (m, 2H), 3.79 - 3.75 (m, 1H), 3.51 (t, 2H), 3.27 (s, 3H), 2.01 (p, 2H), 1.88 - 1.75 (m, 1H), 1.37 - 1.27 (m, 1H), 1.02 - 0.94 (m, 1H), 0.92 (d, 3H), 0.82 (d, 3H), 0.68 - 0.63 (m, 1H), 0.29 - 0.21 (m, 1H). |
| 25 | 1H NMR (400 MHz, CDCl3, ppm) δ 14.15 (s, 1H), 7.92 (s, 1H), 7.85 (s, 1H), 6.66 (s, 1H), 5.24 (s, 1H), 4.72 (d, 1H), 4.67 (d, 1H), 4.52 (d, 1H), 4.48 (d, 1H), 4.28 - 4.21 (m, 2H), 3.88 - 3.81 (m, 2H), 3.50 (s, 3H), 3.48 - 3.38 (m, 1H), 1.48 - 1.36 (m, 4H), 1.36 - 1.19 (m, 1H), 0.70 - 0.60 (m, 1H), 0.56 - 0.46 (m, 1H). |
| 26 | 1H NMR (400 MHz, CDCl3, ppm) δ 14.26 (s, 1H), 7.79 (s, 1H), 7.13 (d, 1H), 6.70 (d, 1H), 5.20 (s, 1H), 5.01 (p, 1H), 4.74 (d, 1H), 4.67 (d, 1H), 4.55 (d, 1H), 4.50 (d, 1H), 4.25 - 4.12 (m, 2H), 3.58 (t, 2H), 3.37 (s, 3H), 2.64 - 2.54 (m, 2H), 2.54 - 2.44 (m, 1H), 2.39 - 2.28 (m, 1H), 2.13 (p, 2H), 1.90 - 1.82 (m, 1H), 1.81 - 1.69 (m, 1H), 1.43 (s, 3H). |
| 27 | 1H NMR (400 MHz, CDCl3, ppm) δ 14.16 (s, 1H), 7.83 (s, 1H), 7.63 (d, 1H), 6.69 (d, 1H), 5.22 (s, 1H), 4.73 (d, 1H), 4.67 (d, 1H), 4.54 (d, 1H), 4.49 (d, 1H), 4.26 - 4.13 (m, 2H), 3.59 (t, 2H), 3.39 (s, 1H), 3.38 (s, 3H), 2.14 (p, 2H), 1.42 (s, 3H). |

(continued)

| Cpd. | NMR |
|---|---|
| 28 | 1H NMR (400 MHz, DMSO-d6, ppm) δ 14.68 (s, 1H), 8.18 (s, 1H), 7.64 (s, 1H), 6.87 (s, 1H), 4.85 (d, 1H), 4.51 (d, 2H), 4.32 (d, 2H), 4.24 - 4.10 (m, 2H), 3.86 - 3.81 (m, 1H), 3.79 (s, 3H), 1.88 - 1.72 (m, 1H), 1.38 (s, 3H), 1.36 - 1.27 (m, 1H), 1.13 - 1.01 (m, 1H), 0.94 (d, 3H), 0.81 (d, 3H), 0.72 - 0.59 (m, 1H), 0.35 - 0.21 (m, 1H). |
| 30 | 1H NMR (400 MHz, DMSO-d6, ppm) δ 14.67 (s, 1H), 8.18 (s, 1H), 7.64 (s, 1H), 6.82 (s, 1H), 4.84 (d, 1H), 4.19 - 4.04 (m, 2H), 3.86 - 3.81 (m, 1H), 3.80 (s, 3H), 2.66 (t, 2H), 2.07 (p, 2H), 2.03 -1.74 (m, 1H), 1.36 - 1.26 (m, 1H), 1.05 (dt, J = 9.3, 6.9 hz, 1H), 0.93 (d, 3H), 0.81 (d, 3H), 0.70 -0.60 (m, 1H), 0.34 - 0.21 (m, 1H). |
| 35 | 1H NMR (400 MHz, DMSO-d6, ppm) δ 14.68 (s, 1H), 8.19 (s, 1H), 7.64 (d, 2H), 6.86 (s, 1H), 5.00 - 4.90 (m, 1H), 4.85 (d, 1H), 4.34 - 4.15 (m, 2H), 3.89 - 3.81 (m, 1H), 3.80 (s, 3H), 3.62 (t, 1H), 3.39 - 3.35 (m, 1H), 1.86 - 1.73 (m, 1H), 1.36 -1.26 (m, 1H), 1.11 - 1.01 (m, 1H), 0.93 (d, 3H), 0.81 (d, 3H), 0.70 - 0.60 (m, 1H), 0.33 - 0.23 (m, 1H). |
| 36 | 1H NMR (400 MHz, DMSO-d6, ppm) δ 8.13 (s, 1H), 7.61 (s, 1H), 6.77 (s, 1H), 4.82 (d, 1H), 4.59 (t, 1H), 4.19 - 4.05 (m, 2H), 3.84 - 3.79 (m, 1H), 3.78 (s, 3H), 3.57 (q, 2H), 1.89 (p, 2H), 1.85 - 1.73 (m, 1H), 1.36 -1.26 (m, 1H), 1.11 - 1.00 (m, 1H), 0.92 (d, 3H), 0.80 (d, 3H), 0.68 - 0.59 (m, 1H), 0.33 - 0.23 (m, 1H). |
| 39 | 1H NMR (400 MHz, DMSO-d6, ppm) δ 14.68 (s, 1H), 8.17 (s, 1H), 7.60 (s, 1H), 6.78 (s, 1H), 4.83 (d, 1H), 4.78 - 4.68 (m 1H), 3.86 - 3.79 (m, 1H), 3.77 (s, 3H), 2.06 - 1.71 (m, 1H), 1.30 (d, 6H), 1.27 - 1.20 (m, 1H), 1.11 -1.00 (m, 1H), 0.92 (d, 3H), 0.80 (d, 3H), 0.71 - 0.55 (m, 1H), 0.36 - 0.21 (m, 1H). |
| 41 | 1H NMR (400 MHz, DMSO-d6, ppm) δ 14.68 (s, 1H), 8.18 (s, 1H), 7.63 (s, 1H), 7.56 - 7.32 (m, 5H), 6.90 (s, 1H), 5.26 - 5.06 (m, 2H), 4.83 (d, 1H), 3.87 - 3.80 (m, 1H), 3.78 (s, 3H), 1.87 - 1.69 (m, 1H), 1.38 - 1.26 (m, 1H), 1.13 -1.00 (m, 1H), 0.89 (d, 3H), 0.80 (d, 3H), 0.71 - 0.58 (m, 1H), 0.35 - 0.20 (m, 1H). |
| 43 | 1H NMR (400 MHz, DMSO-d6, ppm) δ 14.67 (s, 1H), 8.19 (s, 1H), 7.67 (s, 1H), 6.89 (s, 1H), 4.86 (d, 1H), 4.46 - 4.29 (dd, 2H), 3.86 (s, 1H), 3.81 (s, 3H), 2.59 - 2.51 (m, 2H), 2.36-2.23 (m, 2H), 2.23 - 2.02 (m, 2H), 2.02-1.75 (m, 1H), 1.38 -1.26 (m, 1H), 1.13 - 1.02 (m, 1H), 0.94 (d, 3H), 0.82 (d, 3H), 0.71 - 0.60 (m, 1H), 0.34 - 0.22 (m, 1H). |
| 44 | 1H NMR (400 MHz, DMSO-d6, ppm) δ 14.63 (s, 1H), 8.20 (s, 1H), 8.02 (d, 1H), 6.98 (d, 1H), 4.91 (d, 1H), 4.27 - 4.12 (m, 2H), 3.79 - 3.69 (m, 1H), 3.48 (t, 2H), 3.26 (s, 3H), 2.00 (p, 2H), 1.87 -1.74 (m, 1H), 1.35 - 1.25 (m, 1H), 1.06 - 0.97 (m, 1H), 0.93 (d, 3H), 0.81 (d, 3H), 0.72 - 0.62 (m, 1H), 0.32 - 0.23 (m, 1H). |
| 45 | 1H NMR (400 MHz, DMSO-d6, ppm) δ 14.63 (s, 1H), 8.20 (s, 1H), 8.02 (d, 1H), 6.98 (d, 1H), 4.91 (d, 1H), 4.27 - 4.12 (m, 2H), 3.79 - 3.69 (m, 1H), 3.49 (t, 2H), 3.26 (s, 3H), 2.00 (p, 2H), 1.88 - 1.73 (m, 1H), 1.37 - 1.25 (m, 1H), 1.07 - 0.96 (m, 1H), 0.93 (d, 3H), 0.81 (d, 3H), 0.73 - 0.61 (m, 1H), 0.34 - 0.22 (m, 1H). |
| 46 | 1H NMR (400 MHz, DMSO-d6, ppm) δ 14.57 (s, 1H), 8.21 (s, 1H), 8.06 (s, 1H), 6.94 (s, 1H), 5.47 (s, 1H), 4.54 (d, 1H), 4.48 (d, 1H), 4.27 - 4.14 (m, 3H), 4.04 (d, 1H), 3.82 - 3.71 (m, 1H), 3.51 (t, 2H), 3.26 (s, 3H), 2.00 (p, 2H), 1.32 (s, 3H), 1.27 -1.18 (m, 1H), 1.11 - 1.03 (m, 1H), 0.62 - 0.51 (m, 1H), 0.47 - 0.38 (m, 1H). |
| 47 | 1H NMR (400 MHz, DMSO-d6, ppm) δ 14.55 (s, 1H), 8.20 (s, 1H), 8.05 (s, 1H), 6.93 (s, 1H), 5.46 (s, 1H), 4.54 (d, 1H), 4.47 (d, 1H), 4.27 - 4.12 (m, 3H), 4.04 (d, 1H), 3.82 - 3.71 (m, 1H), 3.50 (t, 2H), 3.26 (s, 3H), 2.00 (p, 2H), 1.32 (s, 3H), 1.26 -1.22 (m, 1H), 1.11 - 1.01 (m, 1H), 0.61 - 0.51 (m, 1H), 0.48 - 0.38 (m, 1H). |
| 48 | 1H NMR (400 MHz, Chloroform-d) δ 14.26 (s, 1H), 7.80 (s, 1H), 7.39 (s, 1H), 6.66 (s, 1H), 5.22 (s, 1H), 5.02 (p, 1H), 4.74 (d, 1H), 4.68 (d, 1H), 4.55 (d, 1H), 4.49 (d, 1H), 4.25 - 4.11 (m, 2H), 3.60 (t, 2H), 3.37 (s, 3H), 2.65 - 2.58 (m, 1H), 2.58 - 2.50 (m, 1H), 2.50 - 2.39 (m, 1H), 2.39 - 2.28 (m, 1H), 2.14 (p, 2H), 1.90 - 1.72 (m, 2H), 1.42 (s, 3H). |
| 49 | 1H NMR (400 MHz, Chloroform-d) δ 14.16 (s, 1H), 7.83 (s, 1H), 7.63 (d, 1H), 6.68 (d, 1H), 5.22 (s, 1H), 4.73 (d, 1H), 4.67 (d, 1H), 4.54 (d, 1H), 4.49 (d, 1H), 4.26 - 4.13 (m, 2H), 3.59 (t, 2H), 3.46 - 3.40 (m, 1H), 3.37 (s, 3H), 2.14 (p, 2H), 1.49 - 1.37 (m, 4H), 1.36 - 1.20 (m, 2H), 0.73 - 0.64 (m, 1H), 0.58 - 0.49 (m, 1H). |
| 50 | 1H NMR (400 MHz, DMSO-*d*6, ppm) δ 14.64 (s, 1H), 8.23 (s, 1H), 8.21 (s, 1H), 7.00 (s, 1H), 4.96 (d, 1H), 4.27 - 4.18 (m, 1H), 4.18 - 4.08 (m, 1H), 3.96 - 3.89 (m, 1H), 3.89 - 3.83 (m, 1H), 3.83 - 3.60 (m, 4H), 3.56 - 3.43 (m, 2H), 1.88 - 1.75 (m, 1H), 1.39 - 1.27 (m, 1H), 1.03 - 0.95 (m, 1H), 0.94 (d, 3H), 0.82 (d, 3H), 0.75 - 0.63 (m, 1H), 0.33 - 0.22(m, 1H). |

(continued)

| Cpd. | NMR |
|---|---|
| 51 | 1H NMR (400 MHz, DMSO-*d*6, ppm) δ 14.63 (s, 1H), 8.22 (s, 1H), 8.20 (s, 1H), 6.99 (s, 1H), 4.95 (d, 1H), 4.26 - 4.17 (m, 1H), 4.17 - 4.10 (m, 1H), 3.95 - 3.88 (m, 1H), 3.88 - 3.82 (m, 1H), 3.82 - 3.59 (m, 4H), 3.55 - 3.42 (m, 2H), 1.86 - 1.74 (m, 1H), 1.36 - 1.27 (m, 1H), 1.02 - 0.95(m, 1H), 0.93 (d, 3H), 0.81 (d, 3H), 0.72 - 0.62 (m, 1H), 0.32 - 0.22 (m, 1H). |
| 52 | 1H NMR (400 MHz, DMSO-*d*6, ppm) δ 14.64 (s, 1H), 8.23 (s, 1H), 8.23 (s, 1H), 7.03 (s, 1H), 4.97 (d, 1H), 4.55 (d, 2H), 4.34 (d, 2H), 4.30 (d, 1H), 4.23 (d, 1H), 3.81 - 3.71 (m, 1H), 1.90 - 1.77 (m, 1H), 1.42 (s, 3H), 1.38 - 1.28 (m, 1H), 1.05 - 0.97 (m, 1H), 0.95 (d, 3H), 0.83 (d, 3H), 0.75 - 0.64 (m, 1H), 0.34 - 0.24 (m, 1H). |
| 53 | 1H NMR (400 MHz, DMSO-d6, ppm) δ 14.58 (s, 1H), 8.33 (s, 1H), 8.25 (s, 1H), 8.23 (s, 1H), 6.96 (dd, *J* = 295.1, 7.6 hz, 1H), 5.26 - 5.16 (m, 1H), 4.29 - 4.14 (m, 2H), 3.83 - 3.69 (m, 3H), 3.69 - 3.46 (m, 5H), 3.27 (s, 3H), 2.01 (p, *J* = 5.7 hz, 2H), 1.88 (dt, *J* = 25.7, 6.2 hz, 1H), 1.76 - 1.58 (m, 1H), 1.31 (dd, *J* = 17.9, 8.8 hz, 1H), 1.24 (s, 0H), 1.02 - 0.94 (m, 1H), 0.68 (s, 1H), 0.31 (s, 1H). |
| 54 | H NMR (400 MHz, DMSO-$d_6$) δ 14.62 (s, 1H), 8.21 (d, *J* = 5.4 hz, 2H), 6.97 (s, 1H), 5.09 (d, *J* = 8.8 hz, 1H), 4.21 (d, *J* = 30.5 hz, 2H), 3.78 (t, *J* = 15.2 hz, 3H), 3.52 (t, *J* = 6.3 hz, 2H), 3.27 (s, 3H), 3.09 (q, *J* = 12.3 hz, 2H), 2.01 (t, *J* = 6.3 hz, 2H), 1.74 (d, *J* = 13.6 hz, 2H), 1.46 (t, *J* = 12.6 hz, 1H), 1.39 - 1.24 (m, 2H), 1.13 (d, *J* = 12.3 hz, 1H), 0.96 (s, 1H), 0.71 (s, 1H), 0.27 (s, 1H). |
| 55 | $^1$H NMR (400 MHz, DMSO-$d_6$) δ 14.56 (s, 1H), 8.21 (s, 1H), 8.06 (s, 1H), 6.94 (s, 1H), 5.48 (s, 1H), 4.56 (d, *J* = 6.3 hz, 1H), 4.48 (d, *J* = 6.0 hz, 1H), 4.25 (d, *J* = 6.3 hz, 1H), 4.05 (d, *J* = 5.9 hz, 1H), 3.94 (s, 3H), 3.81 - 3.73 (m, 1H), 1.33 (s, 3H), 1.29 - 1.14 (m, 1H), 1.08 (q, *J* = 8.7, 7.6 hz, 1H), 0.59 - 0.52 (m, 1H), 0.49 - 0.40 (m, 1H). |
| 56 | 1H NMR (400 MHz, DMSO-d6, ppm) δ 14.55 (s, 1H), 8.19 (s, 1H), 8.02 (s, 1H), 6.91 (s, 1H), 5.45 (s, 1H), 4.54 (d, *J* = 6.3 hz, 1H), 4.47 (d, *J* = 6.0 hz, 1H), 4.29 - 4.14 (m, 3H), 4.03 (d, *J* = 5.9 hz, 1H), 3.75 (td, *J* = 7.1, 3.6 hz, 1H), 1.38 (t, *J* = 6.9 hz, 3H), 1.33 (s, 3H), 1.26-1.19 (m, 1H), 1.12 - 1.00 (m, 1H), 0.58 - 0.51 (m, 1H), 0.47 - 0.37 (m, 1H). |
| 57 | 1H NMR (400 MHz, DMSO-d6, ppm) δ 14.55 (s, 1H), 8.20 (s, 1H), 8.03 (s, 1H), 6.88 (s, 1H), 5.45 (s, 1H), 4.54 (d, *J* = 6.3 hz, 1H), 4.47 (d, *J* = 6.0 hz, 1H), 4.23 (d, *J* = 6.3 hz, 1H),4.07 - 3.95 (m, 3H), 3.81 - 3.72 (m, 1H), 1.32 (s, 3H), 1.30 - 1.17 (m, 3H), 1.13 - 1.01 (m, 1H), 0.64 - 0.58 (m, 2H), 0.58 - 0.51 (m, 1H), 0.58 - 0.51 (m, 1H), 0.48 - 0.34 (m,1H) |
| 58 | $^1$H NMR (400 MHz, DMSO-$d_6$) δ 14.53 (s, 1H), 8.20 (s, 1H), 7.99 (s, 1H), 6.93 (s, 1H), 5.46 (s, 1H), 4.56 (d, *J* = 6.3 hz, 1H), 4.48 (d, *J* = 6.0 hz, 1H), 4.25 (d, *J* = 6.2 hz, 1H), 4.05 (d, *J* = 5.9 hz, 1H), 3.75 (tt, *J* = 7.0, 4.3 hz, 1H), 1.33 (s, 3H), 1.21 (dt, *J* = 15.9, 6.3 hz, 1H), 1.12 - 1.01 (m, 1H), 0.53 (dd, *J* = 11.3, 6.2 hz, 1H), 0.43 (p, *J* = 5.2 hz, 1H). |
| 59 | $^1$H NMR (400 MHz, DMSO-*d6*) δ 14.56 (s, 1H), 8.39 (s, 1H), 8.28 (dd, 1H), 7.40 (dd, 1H), 5.06 (s, 2H), 3.80 (tt, 1H), 1.18 (t, 2H), 0.51 (dd, 2H). |
| 60 | $^1$H NMR (400 MHz, DMSO-$d_6$) δ 14.56 (s, 1H), 8.39 (t, 2H), 7.38 (d, 1H), 5.09 (s, 2H), 3.82 (tt, 1H), 1.15 (d, 2H), 0.54 - 0.46 (m, 2H). |
| 61 | $^1$H NMR (400 MHz, DMSO-$d_6$) δ 14.54 (s, 1H), 8.39 (s, 2H), 7.55 (s, 1H), 5.08 (s, 2H), 3.80 (d, 1H), 1.14 (d, 2H), 0.51 (d, 2H). |
| 62 | $^1$H NMR (400 MHz, DMSO-$d_6$) δ 14.61 (s, 1H), 8.35 (s, 1H), 8.24 (s, 1H), 7.04 (s, 1H), 5.05 (d, 1H), 4.24 (ddt, 2H), 3.70 (td, 1H), 3.48 (t, 2H), 3.24 (s, 3H), 1.97 (p, 2H), 1.83 (q, 1H), 1.33 (dd, 1H), 0.94 (d, 3H), 0.91 - 0.84 (m, 1H), 0.82 (d, 3H), 0.68 (dt, 1H), 0.29 (dq, 1H). |
| 63 | $^1$H NMR (400 MHz, chloroform-*d*) δ 14.23 (s, 1H), 8.63 (s, 1H), 8.21 (s, 1H), 6.73 (s, 1H), 4.72 (d, 1H), 4.39 - 4.25 (m, 2H), 3.63 (t, 2H), 3.49 (ddd, 1H), 3.38 (s, 3H), 3.24 (s, 3H), 2.19 (p, h), 1.98 (dq, 1H), 1.55 - 1.45 (m, 1H), 1.28 - 1.15 (m, 1H), 1.00 (d, 3H), 0.94 (d, 3H), 0.76 - 0.64 (m, 1H), 0.47 - 0.35 (m, 1H). |
| 64 | $^1$H NMR (400 MHz, DMSO-$d_6$) δ 14.62 (s, 1H), 8.20 (s, 1H), 8.03 (d, 1H), 7.04 (d, 1H), 4.91 (d, 1H), 4.73 (dd, 2H), 4.49 - 4.32 (m, 4H), 3.74 (s, 1H), 3.47 - 3.39 (m, 1H), 1.83 (d, 1H), 1.30 (t, 1H), 1.01 (t, 1H), 0.94 (d, 3H), 0.82 (d, 3H), 0.67 (s, 1H), 0.28 (s, 1H). |

(continued)

| Cpd. | NMR |
|---|---|
| 65 | $^1H$ NMR (400 MHz, DMSO-$d_6$) δ 14.62 (s, 1H), 8.21 (s, 1H), 8.04 (d, 1H), 7.04 (d, 1H), 4.91 (d, 1H), 4.51 (d, 2H), 4.33 (d, 2H), 4.28 (d, 1H), 4.22 (d, 1H), 3.75 (s, 1H), 1.82 (q, 1H), 1.38 (s, 3H), 1.31 (t, 1H), 1.06 - 0.98 (m, 1H), 0.94 (d, 3H), 0.82 (d, 3H), 0.67 (d, 1H), 0.28 (d, 1H). |
| 66 | $^1H$ NMR (400 MHz, DMSO-*d6*) δ 14.48 (s, 1H), 8.21 (s, 1H), 8.05 (s, 1H), 6.94 (s, 1H), 5.48 (s, 1H), 4.56 (d, 1H), 4.48 (d, 1H), 4.25 (d, 1H), 4.05 (d, 1H), 3.94 (s, 3H), 3.81 - 3.72 (m, 1H), 1.33 (s, 3H), 1.26 - 1.18 (m, 1H), 1.07 (p, 1H), 0.55 (s, 1H), 0.44 (dt, 1H). |
| 67 | $^1H$ NMR (400 MHz, DMSO-*d6*) δ 14.54 (s, 1H), 8.21 (s, 1H), 8.05 (s, 1H), 6.94 (s, 1H), 5.48 (s, 1H), 4.56 (d, 1H), 4.48 (d, 1H), 4.25 (d, 1H), 4.05 (d, 1H), 3.94 (s, 3H), 3.77 (t, 1H), 1.33 (s, 3H), 1.23 (s, 1H), 1.07 (p, 1H), 0.56 (d, 1H), 0.47 - 0.40 (m, 1H). |
| 68 | $^1H$ NMR (400 MHz, DMSO-*d6*) δ 14.54 (s, 1H), 8.26 (s, 1H), 8.10 (s, 1H), 7.12 (s, 1H), 5.51 (s, 1H), 5.10 - 4.90 (m, 2H), 4.55 (d, 1H), 4.46 (d, 1H), 4.24 (d, 1H), 4.02 (d, 1H), 3.83 - 3.74 (m, 1H), 1.33 (s, 3H), 1.28 - 1.19 (m, 1H), 1.13 - 1.01 (m, 1H), 0.63 - 0.54 (m,1H), 0.46 - 0.38 (m, 1H). |
| 69 | $^1H$ NMR (400 MHz, DMSO-*d6*) δ 14.56 (s, 1H), 8.21 (s, 1H), 8.08 (s, 1H), 6.96 (s, 1H), 5.47 (s, 1H), 4.54 (d, 1H), 4.46 (d, 1H), 4.26 - 4.09 (m,3H), 4.02 (d, 1H), 3.95 - 3.74 (m, 4H), 3.72 - 3.59 (m, 2H), 3.55 - 3.42 (m, 2H), 1.32 (s, 3H), 1.28 -1.18 (m, 1H), 1.12 - 1.01 (m, 1H), 0.63 - 0.51 (m, 1H),0.46 - 0.38 (m, 1H). |
| 70 | $^1H$ NMR (400 MHz, DMSO-*d6*) δ 14.50 (s, 1H), 8.35 (s, 1H), 8.10 (s, 1H), 7.50 (t, 1H), 7.18 (s, 1H), 5.55 (s, 1H), 4.55 (d, 1H), 4.47 (d, 1H), 4.24 (d, 1H), 4.04 (d, 1H), 3.85 - 3.75 (m, 1H), 1.32 (s, 3H), 1.29 - 1.17 (m, 1H), 1.13 -1.01 (m, 1H), 0.63 - 0.53 (m, 1H), 0.50 - 0.40 (m, 1H). |
| 71 | $^1H$ NMR (400 MHz, DMSO-$d_6$) δ 14.40 (s, 1H), 8.08 (s, 1H), 7.97 (s, 1H), 6.68 (s, 1H), 4.71 (d, 1H), 3.95 (ddt, 2H), 3.49 (d, 1H), 3.28 (t, 2H), 3.02 (s, 3H), 1.76 (p, 2H), 1.58 (q, 1H), 1.15 -1.03 (m, 1H), 0.77 - 0.66 (m, 4H), 0.57 (d, 3H), 0.49 - 0.38 (m, 1H), 0.09-0.02 (m, 1H). |
| 72 | $^1H$ NMR (400 MHz, DMSO-*d6*) δ 14.55 (s, 1H), 8.23 (s, 1H), 8.07 (s, 1H), 6.98 (s, 1H), 5.48 (s, 1H), 4.55 (d, 1H), 4.48 (d, 1H), 4.36 - 4.26 (m, 2H), 4.245(d, 1H), 4.07 - 3.96 (m, 3H), 3.80 (ddd, 3H), 3.14 - 3.05 (m, 1H),3.04 (s, 3H), 2.07 (s, 1H), 1.33 (s, 3H), 1.28 - 1.19 (m, 1H), 1.12 -1.02 (m, 1H), 0.62 -0.53 (m, 1H), 0.47 - 0.39 (m, 1H). |
| 73 | $^1H$ NMR (400 MHz, DMSO-*d6*, ppm) δ 14.62 (s, 1H), 8.07 (d, 1H), 8.05 (s, 1H), 6.72 (dd, 1H), 6.69 (d, 1H), 5.41 (s, 1H), 4.53 (d, 1H), 4.45 (d, 1H), 4.23 (d, 1H), 4.11 (t, 2H), 4.00 (d, 1H), 3.74 - 3.66 (m, 1H), 3.48 (t, 2H), 3.26 (s, 3H), 1.97 (p, 2H), 1.31 (s, 3H), 1.27 - 1.18 (m, 1H), 1.13 - 1.03 (m, 1H), 0.59 - 0.51 (m, 1H), 0.44 - 0.34 (m, 1H). |
| 74 | $^1H$ NMR (400 MHz, DMSO-$d_6$) δ 14.62 (s, 1H), 8.25 (s, 1H), 7.61 (s, 1H), 6.75 (s, 1H), 5.32 (t, 1H), 3.85 (s, 3H), 3.79 (s, 4H), 2.77 (dd, 1H), 2.68 (dd, 1H), 2.11 (s, 3H), 1.27 (dd, 2H), 1.08 (t, 1H), 0.58 (s, 1H), 0.31 (s, 1H). |

## BIOLOGICAL EXAMPLES

### Example 2. In vitro assays

#### *2.1. Hepg2 NTCP assay*

#### *2.1.1. Assay*

The principle of the hepG2-NTCP assay is to measure the effect of a test compound on the inhibition of the hepatitis virus B (HBV) infection compared to a reference compound. To be able to infect the hepatocellular carcinoma cells (HepG2) with HBV, HepG2 cells were stably transfected with the Na+-taurocholate co-transporting polypeptide (NTCP) receptor, also referred to as Sodium/bile acid cotransporter (SLC10A1), which is necessary for HBV infection. In this assay the HBV core antigen (HBcAg) is used as a marker of HBV infection. Nuclei count is used as an indication for compound toxicity.

### *2.1.2. Procedural Description*

On day one, HepG2-NTCP cells ((Ni et al., 2014)) are seeded at 5000 cells in 50 μL per well in assay medium (DMEM high glucose supplemented with 10% non-heat inactivated (non HI) fetal bovine serum (FBS), 1% P/S, 1% L-glutamine and 1% MEM NEAA) in 384 well plate and incubated at 37°C, 5% $CO_2$ overnight.

Compounds are prepared as 10 point serial dilutions using 1/3 dilution steps in 100% DMSO starting from 10 mM highest concentration, unless otherwise requested for very potent compounds, which started from 5mM or 1mM stock. Compounds are further diluted 1/500 in assay medium.

A negative control (vehicle: 24 wells with 0.2% DMSO) and positive control (24 wells with 100nM Myrcludex B (Cas no.: 406461-66-3), 0.2% DMSO and in 10 point DR) are included in the experiments.

On the second day, 40μl medium is removed from the cells with the plate washer (Select 405 microplate washer, Biotek). 15 μL of diluted compound series is transferred to the assay plate and cells are infected with 15 μL of HBV mix (HBV virus, 4%PEG, 1%DMSO in assay medium). The infection is performed in assay medium containing final concentration of 1% DMSO. The assay plate is incubated at 37°C, 5% $CO_2$ overnight.

The next day, 30μl compound and HBV mix are removed from the assay plate with the plate washer and 30 μL of compound series diluted 1/500 in assay medium containing final concentration of 2.5% DMSO is added to the cell assay plate. The assay plate is incubated at 37°C, 5% $CO_2$ for 5 days.

After 5 days of incubation, supernatant is removed from the cells with the plate washer. Cells are fixed with 50 μL of 3.7% formaldehyde added with the Well mate (Matrix technologies) and incubated for 30 min at room temperature. After fixation, cells are washed four times with phosphate buffer saline (1xPBS, 137mM NaCl, 10mM na2HPO4, 2.7mM KCl, 1.8mM $KH_2PO_4$ at pH7.4). The cells are finally subjected to HBc staining.

First, the assay plate is blocked with blocking buffer (2%FBS, 3% BSA, 0.2% TritonX100 in PBS) for 1 h at room temperature (optional to block overnight at 4°C).

After 4 washes of PBST (1XPBS + 0.05% Tween) with the plate washer, the assay plate is incubated with 30 μL diluted primary antibody (1/700 Rabbit anti-HBV core antigen, Dako, B0586 or 1/350 Rabbit anti HBV core antigen, Invitrogen, PA516368) in blocking buffer and incubated for 90 min at room temperature.

After 4 washes of PBST with the plate washer, the assay plate is incubated with 50 μL of secondary antibody (Donkey-anti-rabbit Alexa 488 antibody, Lifetechnologies A21206) 1/500 diluted in blocking buffer and incubated for 60 min at room temperature and in the dark.

The assay plate is washed 4 times with PBST on the plate washer and incubated with 50μL of DAPI (Sigma D9542, stock 5mg/mL) 1/10000 diluted in PBS for 15-30 min at room temperature and in the dark. The assay plate is now ready for read out on the InCell Analyzer 2200 (GE Healthcare).

### *2.1.3. Data Analysis*

Raw data are generated following read-out performed on InCell 2200 and are imported into the galapagos LIMS for automated calculation of percentage inhibition (PIN) and toxicity by nuclei count, using the control wells mentioned above, and creation of Dose Response curves.

**Table V. Hepg2 NTCP assay activity of illustrative compounds of the invention**

| Log | $EC_{50}$ |
|---|---|
| **** | 0.001 - 1 nM |
| *** | > 1 - 20 nM |
| ** | > 20 - 100 nM |
| * | > 100 nM |
| **Cpd #** | **$IC_{50}$ (nM)** |
| 1 | **** |
| 2 | **** |
| 3 | *** |
| 4 | **** |
| 5 | **** |
| 6 | **** |

(continued)

| Cpd # | $IC_{50}$ (nM) |
|---|---|
| 7 | **** |
| 8 | *** |
| 9 | *** |
| 10 | *** |
| 11 | **** |
| 12 | *** |
| 13 | **** |
| 14 | *** |
| 15 | ** |
| 16 | **** |
| 17 | **** |
| 18 | ** |
| 19 | **** |
| 20 | *** |
| 21 | *** |
| 22 | **** |
| 23 | *** |
| 24 | **** |
| 25 | **** |
| 26 | **** |
| 27 | **** |
| 28 | *** |
| 29 | *** |
| 30 | *** |
| 31 | *** |
| 32 | *** |
| 33 | *** |
| 34 | *** |
| 35 | ** |
| 36 | *** |
| 37 | *** |
| 38 | *** |
| 39 | *** |
| 40 | *** |
| 41 | * |
| 42 | *** |
| 43 | *** |
| 44 | **** |

(continued)

| Cpd # | $IC_{50}$ (nM) |
|---|---|
| 45 | * |
| 46 | **** |
| 47 | *** |
| 48 | **** |
| 49 | **** |
| 50 | **** |
| 51 | **** |
| 52 | **** |
| 53 | **** |
| 54 | **** |
| 55 | **** |
| 56 | **** |
| 57 | *** |
| 58 | **** |
| 59 | * |
| 60 | * |
| 61 | *** |
| 62 | **** |
| 63 | *** |
| 64 | *** |
| 65 | **** |
| 66 | *** |
| 67 | **** |
| 68 | **** |
| 69 | **** |
| 70 | *** |
| 71 | **** |
| 72 | *** |
| 74 | * |

### *2.2. iCell hepatocytes assay*

#### *2.2.1. Principle of the assay*

The principle of the iCell hepatocyte 2.0 96 well assay is to evaluate the inhibitory effect of a test compound on hepatitis virus B (HBV) infection. In this assays the HBV core antigen (HBcAg) and/or extracellular antigen (HBeAg) and/or surface antigen (HBsAg) are used as a marker for HBV infection. Nuclei count may be used as an indication for compound toxicity.

In the standard assay set-up HBV infection and compound addition are performed on the same day, in the delayed assay the compounds are added three days after HBV infection. iCell hepatocytes 2.0 are human hepatocytes derived from induced pluripotent stem cells (iPSc) purchased from Cellular Dynamics international (CDI, R1027).

### *2.2.2. Assay procedural description*

At day 0, iCell hepatocytes 2.0 (CDI, R1027) are thawed and seeded in collagen I coated 96well plates at 112 000c/well (100μL) in plating medium (RPMI supplemented with B27 supplement, 20 ng/mL recombinant human oncostatin M (OSM), 25μg/mL gentamicin, 0.1μM Dexamethasone and iCell medium supplement provided by CDI). Plates are incubated at 37°C, 5% $CO_2$ and daily refreshed with plating medium for the following four days.

On day 5 and 6, plates are refreshed with 100μL maintenance medium (RPMI supplemented with B27 supplement, 25μg/mL gentamicin, 0.1μM Dexamethasone, and iCell medium supplement provided by CDI) and incubated at 37°C, 5% $CO_2$ until the following day.

#### *2.2.2.1. Standard assay*

On day 7 (day of infection) the supernatant is removed from the assay plates and 25 μL of 1/250 diluted compound in maintenance medium and 25 μL of HBV mix (HBV virus, 8% PEG, 0% or 4% DMSO in maintenance medium) are added to the assay plates. Compounds series are either prepared manually as a 6 point serial dilution using 1/5 dilution steps in 100% DMSO or as an 8 point serial dilution using 1/3 dilution steps in 100% DMSO or as an 8 point serial dilution using ¼ dilution steps, normalized for 0.2% DMSO. The assay plates were incubated overnight at 37°C, 5% $CO_2$.

On day 1 post infection (day 8) 50 μL of HBV/cpd mix is removed from the assay plates and refreshed with 100 μL of compound series diluted 1/500 in maintenance medium. The assay plates are incubated at 37°C, 5% $CO_2$ and refreshed with compound series diluted 1/500 in maintenance medium every other day (at day 3 and day 5 post infection) or after three days of incubation (at day 4 post infection).

At day 7 post infection (day 14), 100μL of supernatant is harvested and frozen at -20°C. The cells were fixed with 50 μL of 3.7% formaldehyde and incubated for 30 min at room temperature. After fixation, cells are washed four times with phosphate buffer saline (PBS) with the plate washer (Select 405, microplate washer, Biotek) and subjected to HBc staining. The harvested supernatant is used for HBe and/or HBs ELISA.

#### *2.2.2.2. Delayed assay*

On day 7 (day of infection), the maintenance medium is removed from the assay plates and cells are infected with 50 μL of HBV mix (HBV virus, 4% PEG, 0% or 2% DMSO in maintenance medium). The assay plates were incubated overnight at 37°C, 5% $CO_2$.

At day 1 post infection (day8), 50μL of HBV mix is removed from the assay plates and refreshed with 100 μL of maintenance medium. The assay plates are incubated at 37°C, 5% $CO_2$ for three days.

At day 4 post infection (day 11) and day 7 post infection (day14), the supernatant is removed and 100 μL of compound series diluted 1/500 in maintenance medium is added to the assay plates (8 point serial dilution using 1/3 dilution steps in 100% DMSO or 8 point serial dilution using ¼ dilution steps, normalized for 0.2% DMSO. The assay plates were incubated at 37°C, 5% $CO_2$ for three days.

At day 10 post infection (day 17), 100μL of supernatant is harvested and frozen to -20°C. The cells are fixed with 50 μL of 3.7% formaldehyde and incubated for 30 min at room temperature. After fixation, cells are washed four times with phosphate buffer saline (PBS) with the plate washer (Select 405, microplate washer, Biotek) and subjected to HBc staining. harvested supernatant is used for HBe and/or HBs ELISA.

### *2.2.3. HBc staining*

The assay plates are blocked with blocking buffer (2% FBS, 3% BSA, 0.2% TritonX100 in PBS) for 1 h at room temperature.

After blocking, the assay plates are tapped manually and incubated with 50 μL diluted primary antibody (1/700 Rabbit-anti-HBc Ag, Dako, B0586 or 1/350 Rabbit-anti-HBc Ag, Invitrogen, PA516368) in blocking buffer and incubated for 90 min at room temperature.

After 4 washes of PBST (1XPBS + 0.05% Tween) with the plate washer, the assay plates are incubated with 100 μL of secondary antibody (Donkey-anti-rabbit Alexa 488, Lifetechnologies, A21206) 1/500 diluted in blocking buffer and incubated for 60 min at room temperature in the dark, then washed 4 times with PBST on the plate washer and incubated with 100μL of DAPI (Sigma D9542, stock 5mg/mL) 1/10000 diluted in PBS for 15-30 min at room temperature in the dark.

Finally, the assay plates are read out on a InCell Analyzer 2200 (GE Healthcare).

### *2.2.4. HBe ELISA*

A white lumitrac 600 384 well plate iss coated with 40μL of capture antibody (monoclonal Mouse-anti-HBe Ag, Fitzgerald, 10-H10) diluted 1/2000 in coating buffer (0.1M sodium carbonate pH 9.5) and incubated overnight at 4°C.

The next day the capture Ab is removed by tapping and the plate is blocked for 1h at room temperature with 80μL of blocking buffer (1% BSA, 0.01% alkali-soluble casein in PBS).

After blocking, 30 μL of sample and HBe Ag standard (recombinant hepatitis B virus E antigen protein, 4mg/mL, Abcam, ab91273) are added to the plate. The HBe Ag standard is 1/3 diluted in blocking buffer starting from 1.25μg/mL. A vehicle control is also included. The plate is incubated for 30 min at room temperature.

After sample incubation, the plates are washed 4 times with PBST on a platewasher followed by 2 washed of PBS. The plate is tapped in between both washing steps and after the last wash with PBS.

After washing the plate, 40μL of detection antibody (monoclonal Mouse-anti-HBe Ag HRP, Fitzgerald, 61-H10K) 1/16000 diluted in blocking buffer is added to the plate and incubated for 30 min at room temperature in the dark.

The assay plate is washed 4 times with PBST on the platewasher followed by 2 washed of PBS. The plate is tapped in between both washing steps and after the last washing step with PBS.

For the read out, 50μL of luminol substrate (SuperSignal ELISA pico chemiluminescent substrate kit, Thermo Fisher, Scientific, 37070) is added to the plate and incubated for 1 min at room temperature, protected from light. Luminescence read out is performed on the envision (PerkinElmer).

### *2.2.5. HBs ELISA*

HBs ELISA is performed according to the HBs ELISA kit (Diasource, KAPG4SGE3). A HBs Ag standard (active hepatitis B surface antigen (adw) full length protein, 4mg/mL, Abcam, ab91276) 1/3 diluted in blocking buffer (1% BSA, 0.01% alkali-soluble casein in PBS) starting from 1.25μg/mL and a vehicle control are included in the assay. Absorption read out is performed on the envision (Perkin Elmer).

### *2.2.6. Data analysis*

A negative (vehicle: 6 wells with 0.2% DMSO) and positive (6 wells with 100nM Myrcludex B (Cas n°:406461-66-3), 0.2% DMSO and in 8 point DR and/or 6 wells non infected cells, 0.2% DMSO) controls are included in the experiments.

Raw data are generated following read-out performed on the InCell Analyzer 2200 (HBc Ag and nuclei count) and on the envision (HBe and HBs Ag) and are used to calculate percentage inhibition (PIN) and toxicity, using the control wells mentioned above, and to create Dose Response curves.

**Table VI. Hepatocyte assay activity of illustrative compounds of the invention**

| | EC50(nM) % | | inhibition | |
|---|---|---|---|---|
| | *** | 0.001 - 20 nM | +++ | 75-100% |
| | ** | >22 - 50 nM | ++ | 50-75% |
| | * | > 50 nM | + | < 50% |
| Cpd # | Standard assay HBs ELISA | | Delayed assay HBs ELISA | |
| | $EC_{50}$(nM) | % inhibition | $EC_{50}$(nM) | % inhibition |
| 1 | *** | ++ | *** | ++ |
| 2 | - | - | *** | ++ |
| 3 | - | - | * | +++ |
| 4 | | | *** | ++ |
| 6 | *** | +++ | ** | ++ |
| 16 | ** | ++ | ** | ++ |
| 17 | - | - | *** | +++ |
| 19 | ** | ++ | ** | ++ |

(continued)

| Cpd # | Standard assay HBs ELISA | | Delayed assay HBs ELISA | |
|---|---|---|---|---|
| | $EC_{50}$(nM) | % inhibition | $EC_{50}$(nM) | % inhibition |
| 44 | ** | ++ | * | ++ |
| 46 | *** | +++ | *** | ++ |
| 47 | - | - | ** | ++ |

**Example 3. In vivo assays**

***3.1. Humanized Mouse model of HBV infection in human-murine liver chimeric mice***

***3.1.1. Animals***

Six to ten weeks old male or female FRG KO mice (Fah-/-/Rag2-/-/Il2rg-/-triple knock-out - Yecuris Corporation P.O. Box 4645, Tualatin, OR 97062, USA) are used. They are provided with filtered tap water and standard chow *ad libitum* and maintained at 22 ± 2°C in 55 ± 10% humidity on a 12h light/dark cycle.

***3.1.2. Humanisation process***

Each mouse is repopulated by intra splenic injection of 1 to 2 $10^6$ human hepatocytes (Life Technologies ref HMCS2SA). To allow a progressive and efficacious repopulation, drinking water is regularly supplemented with Nitisinone (Yecuris, lot TNAK0002). The repopulation rate is controlled by hAlb dosage in serum (Euromedex ref E88-129). About 3 to 4 months after the human hepatocyte injection, mice for which repopulation is successful reach an hAlb level >1g/L and can be used for HBV infection.

***3.1.3. Compounds preparation***

The test compounds for *p.o* administration are formulated in Solutol at 2% (Sigma : Kolliphor hS15 12966-1kg lot BCBM0868V) and methylcellulose 0.5% at 98%

***3.1.4. HBV infection***

Each mouse is injected with HBV-2015 batch (6.6 106 copies/μl) with an inoculum of 1.38 $10^8$ GE HBV (0.2 mL) via tail vein.

Six to seven weeks after infection, blood is sampled under isoflurane anesthesia by retro-orbital puncture. HBsAg is dosed in serum by Elisa assay (Immunodiagnostic, ref HBSAG EIA). HBV DNA level is measured in the mice serum using qPCR (SYBR green). Only mice reaching HBsAg level above 1000UI/mL are kept and randomized.

Treatment is given for 5 weeks according to body weight.

Body weight is recorded every week.

Between Day 7 and Day 11, a steady state PK is performed with sampling of blood under isoflurane anesthesia by retro-orbital puncture at T=0, 1h, 3h, 6h post treatment. Plasma are frozen before decontamination with ethanol absolute 80% and transfer to bioanalysis laboratory for compound dosage by LCMS-MS.

At D8, D8, D15, D22 and D28, blood is sampled under isoflurane anesthesia by retro-orbital puncture. To follow compounds efficacy, HBsAg is dosed in serum by Elisa assay (Immunodiagnostic, ref HBSAG EIA) and HBV DNA level is measured in serum using qPCR (SYBR green).

After 5 weeks of treatment, mice are euthanized 2 hours after the last dosing for liver and blood collection.

In blood HBsAg is measured by Elisa assay, HBV DNA by qPCR and compound level by LCMS-MS.

In liver HBV DNA is measured by qPCR, compound level by LCMS-MS, pgRNA by RT-qPCR, HBcAg by immunohistochemistry (Dako ref B0586).

***3.1.5. Results***

When subjected to this protocol, the following results for illustrative compounds of the invention are obtained.

**Table VII. Infected mice HBsAg blood level variation**

| Group (6 mouse/group) | D1 | D8 | D15 | D22 | D28 |
|---|---|---|---|---|---|
| Disease vehicle<br>*sem* | 4.4<br>*0.24* | 4.3<br>*0.14* | 4.2<br>*0.20* | 4.4<br>*0.18* | 4.2<br>*0.17* |
| Cpd 46 0.3 mg/kg p.o q.d<br>*sent (p)* | 4.4<br>*0.22* | 3.7<br>*0.17* | 3.8<br>*0.11* | 3.9<br>*0.25* | 3.7<br>*0.09 (*)* |
| Cpd 46 3 mg/kg p.o q.d<br>*sem (p)* | 4.4<br>*0.12* | 3.4<br>*0.13 (*)* | 3.4<br>*0.17 (*)* | 3.4<br>*0.17 (**)* | 3.4<br>*0.14 (**)* |
| Cpd 46 30 mg/kg p.o q.d<br>*sem (p)* | 4.4<br>*0.13* | 3.5<br>*0.17 (*)* | 3.5<br>*0.19 (*)* | 3.4<br>*0.16 (**)* | 3.5<br>*0.18 (**)* |
| Cpd 44 1 mg/kg p.o q.d<br>*sem (p)* | 4.5<br>*0.14* | 3.6<br>*0.18 (*)* | 3.5<br>*0.16 (*)* | 3.5<br>*0.12 (*)* | 3.6<br>*0.10 (*)* |
| Cpd 44 10 mg/kg p.o q.d<br>*sem (p)* | 4.4<br>*0.13* | 3.4<br>*0.14 (**)* | 3.5<br>*0.15 (*)* | 3.4<br>*0.12 (**)* | 3.4<br>*0.09 (**)* |
| Mean + sem, one way ANOVA + Dunetts vs Control - *p<0.05, **p<0.01***p<0.001 - n=6/gpe | | | | | |

**Example 4. Pharmacokinetic, ADME and Toxicity Assays**

***4.1. Thermodynamic solubility***

The test compound is added to 0.2M phosphate buffer pH 7.4 or 0.1M citrate buffer pH 3.0 at a concentration of 1 mg/mL in a glass vial.

In a 20 mL glass vial, 1-2 mg of dry matter of compound are added and stirred with the suitable buffer (Fed State Simulated Intestine Fluid FeSSIF, or Fasted State Simulated Intestine Fluid FaSSIF, or Fasted State Simulated gastric Fluid FaSSGF, or phosphate buffer pH7.4) for 24h at room temperature (for the buffer pH7.4) or 37°C (for the 3 physiological buffers) under magnetic stirring (200tr/min). The concentration of the mixture is 1mg/mL.

After 24 h, a volume of 500μL is sampled, centrifuged for 10 min at 10 000 rpm and filtered. The samples are diluted in duplicates in DMSO (F100 and F10). Then, a final dilution (factor 100) in 80/20 water/acetonitrile containing the internal standard (warfarin) is used for LCMS-MS analysis.

A standard curve is made starting from a 200,000 ng/mL stock in DMSO, freshly prepared from dry matter. Then, successive concentrations at 15,000, 10,000, 2,500, 1,000, 200 and 75 ng/mL in DMSO are prepared by using the Tecan robot.

Two quality control samples are made: one of 10,000ng/mL and one of 500 ng/mL in DMSO, also starting from the DMSO working stock solution at 200,000 ng/mL.

The standard curve and quality controls are diluted a factor 100 in 80/20 water/acetonitrile (with internal standard) and analyzed on LC-MS/MS (API4000 or API5500).

The samples are analyzed on LC-MS with a flow rate of 0.6mL/min. The mobile phase A is 0.1% formic acid in water and the mobile phase B is 0.1% formic acid in acetonitrile. The sample is run under positive or negative ion spray on Pursuit C18-5μm (2.0 x 20mm) column, from Agilent.

The peak areas of the standard curve are plotted in a graph and a linear or polynomial of the second order equation is used to calculate the unknown concentrations of the test compound.

Solubility values are reported in μM and μg/mL.

***4.2. Aqueous Solubility***

Starting from a 10 mM stock solution of compound in DMSO, a second concentration in DMSO of 3mM is prepared.

Both DMSO concentrations will be diluted in 0.1 M phosphate buffer pH 7.4, by adding 200 μL of buffer to 2 μL stock solutions in DMSO. The final concentrations are 100 and 30 μM with a final DMSO concentration of 1%. Measurements are done in duplicate.

As a positive control for precipitation, Pyrene is added to the corner points of each 96 well plate and serves as a reference point for calibration of Z-axis on the microscope. As a negative control, DMSO is added to the 12 wells on

columns between positive control wells.

The assay plates are sealed and incubated for 1 h at 37°C while shaking at 230rpm.

The plates are then scanned under a white light microscope using a Nikon microscope, yielding individual pictures (20x) of the precipitate per concentration.

The precipitate is analyzed visually:

- If a precipitate is observed at 100μM and at 30μM, the data generated will be : <30μM
- If a precipitate is observed at 100μM but not at 30μM, the data generated will be : >30μM
- If no precipitate is observed (neither at 30 or at 100μM), the data generated will be : >100μM

Solubility values measured according to this protocol are reported in μM and in μg/mL.

### *4.3. Plasma Protein Binding PPB (Equilibrium Dialysis)*

Prior to the start of the experiment, dialysis membranes (membrane strips, MW cut-off 12-14kDa, HTDialysis, Cat.No.#1101) are soaked in deionized water for 60 min, transferred and left overnight in 20% ethanol.

A 10mM stock solution of the compound in DMSO is diluted with a factor 10 in DMSO. This solution is further diluted in freshly thawed human, rat, mouse or dog plasma (BioReclamation INC) with a final concentration of 5 μM and final DMSO concentration of 0.5%.

Equilibrium Dialysis Device (96-well, model HTD96b, HTDialysis, Cat.No.#1006) is assembled according to manufacturer's instructions. Immediately after assembly, a volume of 100μL of plasma (spiked with compound) is placed on one side of the well and another 100μL of blank PBS buffer are added to the other side, respectively. Each compound is tested in duplicate.

Acebutolol and Nicardipine are used as low and very high binding controls.

The plate is incubated for 4 h at 37°C while shaking at 230rpm.

Thereafter, an aliquot is taken from each side of the well and matrix matched (mix of equal volumes of spiked plasma with blank PBS buffer and samples from buffer compartment with blank plasma).

Matrix matched samples are further mixed with 4 volumes of STOP solution (acetonitrile with warfarin as internal standard). After brief mixing and centrifugation (at 2400 rpm for 15 min, at +4°C), the supernatant is filtered and transferred into new 96-well plates for analysis on LC-MS/MS (systems API4000 or API5500).

The samples are analyzed on LC/MS-MS with a flow rate of 0.6mL/min. The mobile phase A is 0.1% formic acid in water and the mobile phase B is 0.1% formic acid in acetonitrile. The sample is run under positive or negative ion spray on Pursuit C18-5μm (2.0 x 20mm) column, from Agilent. The solvent gradient has a total run time of 1.2 min with a gradient profile as followed:

| Time (min) | % B |
|---|---|
| 0.0 | 5 |
| 0.2 | 100 |
| 0.8 | 100 |
| 0.9 | 5 |
| 1.2 | 5 |

The percentage bound in plasma (PPB) is determined using the following equation:

$$PPB = ((Cplasma\text{-}Cbuffer)/Cplasma)*100$$

Cplasma = compound concentration in plasma
Cbuffer = compound concentration in buffer
"Concentration" is the ratio between compound and internal standard peak areas.

The solubility of the compound in the final test concentration in PBS is checked by microscope to indicate whether precipitation is observed or not. If a precipitate is observed, no data of PPB is generated.

### *4.4. Liver microsomal stability*

A 10 mM stock solution of compound in DMSO is diluted a factor 3 in DMSO. This pre-dilution in DMSO is further diluted to 2 μM in a 100 mM phosphate buffer pH 7.4 and pre-warmed at 37°C. This compound dilution is mixed factor 2 with microsomal/cofactor mix at 37°C under shaking at 300 rpm.

Each compound is tested in duplicate. Warfarin and Verapamil are included in the assay as reference molecules.

A glucose-6-phosphate-dehydrogenase (G6PDH, Sigma Aldrich) working stock solution of 700U/mL is diluted with a factor 1:700 in a 100mM phosphate buffer, pH7.4. A co-factor mix containing 0.528 M $MgCl_2.6H_2O$ (Sigma, M2670), 0.528M glucose-6-phosphate (Sigma, G-7879) and 0.208M NADP+ (Sigma,N-0505) is diluted with a factor 1:8in a 100mM phosphate buffer, pH 7.4.

A working solution is made containing 1 mg/mL liver microsomes of the species of interest (human, mouse, rat, dog), 0.6U/mL G6PDH and co-factor mix (6.6mM $MgCl_2$, 6.6 mM glucose-6-phosphate, 2.6mM NADP+). This mix is pre-incubated for 15 min, but never more than 20 min, at room temperature.

After pre-incubation, compound dilution and the mix containing the microsomes, are added together in equal amount and incubated for 30 min at 300 rpm and 37°C.

The final concentrations during incubation are: 1μM test compound or control compound, 0.5 mg/mL microsomes, 0.6U/mL G6PDH, 3.3mM $MgCl_2$, 3.3mM glucose-6-phosphate and 1.3mM NaDP+.

After 30 min of incubation, the reaction is stopped with 2 volumes of stop solution (containing acetonitrile with Diclofenac as internal standard). For the time point of 0 min, two volumes of stop solution are added to the compound dilution before the microsome mix is added.

Samples of both time points are centrifuged, filtered and the supernatant is harvested for analysis on LC-MS/MS (API5500 system).

The samples are analyzed on LC/MS-MS with a flow rate of 0.6mL/min. The mobile phase A is 0.1% formic acid in water and the mobile phase B is 0.1% formic acid in acetonitrile. The sample is run under positive or negative ion spray on Pursuit C18-5μm (2.0 x 20mm) column, from Agilent. The solvent gradient has a total run time of 2.2 min with a gradient profile as followed:

| Time (min) | % B |
|---|---|
| 0.0 | 5 |
| 0.6 | 5 |
| 1 | 100 |
| 1.9 | 100 |
| 2.0 | 5 |
| 2.2 | 5 |

The instrument responses (peak areas/IS peak area) are referenced to the zero time-point samples (considered as 100%) in order to determine the percentage of compound remaining.

The data of microsomal stability are expressed as a percentage of the total amount of compound remaining after 30 min.

The solubility of the compound in the final test concentration in 100mM buffer pH 7.4 is checked by microscope to indicate whether precipitation is observed or not. If a precipitate is observed, no data of microsomal stability is generated.

### *4.5. Hepatocyte stability*

A 10 mM DMSO stock solution is first diluted in DMSO (0.33 mM and 3.3 mM) and then in modified Krebs-Henseleit buffer (KHB, Sigma). In addition, third compound dilution was obtained by diluting 10 mM DMSO stock dilution directly in KHB. These compound dilutions in KHB (0.5 μM, 5 μM and 50 μM) were further added to a suspension of pooled cryopreserved hepatocytes (Bioreclamation IVT) at 37°C with gentle shaking.

Final reaction conditions were 0.1μM, 1 μM and 10 μM of compound (n=2 per concentration), 0.03% DMSO (0.1% for 10 μM concentration), 0.5 million viable hepatocytes/mL, 75 μL incubation volume.

Testosterone (1 μM) and 7-Hydroxycoumarin (1 μM) were used as Phase I and Phase II reaction controls, while caffeine was used as a negative control.

A full time curve (with: 0, 10, 20, 45, 90, 120 and 180 min of incubation) is done with hepatocytes from species of interest (mouse, rat, dog, minipig, monkey or human).

After the respective time of incubation, the reaction (75 μL) was terminated by adding 225 μL of acetonitrile:methanol (2:1) containing 100 ng/mL of diclofenac as analytical internal standard. Samples were mixed, centrifuged and the supernatant analyzed by LC-MS/MS.

The instrument responses (peak areas/IS peak area) are referenced to the zero time-point samples (considered as 100%) in order to determine the percentage of compound remaining.

### *4.6. MDCKII-MDR1 Permeability*

MDCKII-MDR1 cells are Madin-Darby canine kidney epithelial cells, over-expressing human multi-drug resistance (MDR1) gene, coding for P-glycoprotein (P-gp). Cells are obtained from Netherlands Cancer Institute and used after a 3-4 day cell culture in 24-well Millicell cell culture insert plates (Millipore, PSRP010R5). Bi-directional MDCKII-MDR1 permeability assay is performed as described below.

3x105 cells/mL (1.2x105 cells/well) are seeded in plating medium consisting of DMEM + 1% glutamax-100 + 1% Antibiotic/Antimycotic + 10% FBS (Biowest, S1810). Cells are left in $CO_2$ incubator for 3-4 days. The medium is changed 24h after seeding and on the day of experiment.

Test and reference compounds (amprenavir and propranolol) are prepared in Dulbecco's phosphate buffer saline (D-PBS, pH7.4) and added to either the apical (400μL) or basolateral (800μL) chambers of the Millicell cell culture insert plates assembly at a final concentration of 10 μM (0.5 μM in case of amprenavir) with a final DMSO concentration of 1%.

100μM Lucifer Yellow (Sigma) is added to the all donor buffer solutions, in order to assess integrity of the cell monolayers by monitoring Lucifer Yellow permeation. Lucifer yellow is a fluorescent marker for the paracellular pathway and it is used as an internal control in every monolayer to verify tight junction integrity during the assay.

After a 1 h incubation at 37°C while shaking at an orbital shaker at 150rpm, 75μL aliquots are taken from both apical (A) and basal (B) chambers and added to 225μL acetonitrile:water solution (2:1) containing analytical internal standard (10 ng/mL warfarin) in a 96 well plate. Aliquoting is also performed at the beginning of the experiment from donor solutions to obtain initial (Co) concentration.

Concentration of compound in the samples is measured by high performance liquid-chromatography/mass spectroscopy (LC-MS/MS).

Lucifer yellow is measured with a Fluoroscan Ascent FL Thermo Scientific (Ex 485nm and Em 530nm) in a 96 well plate containing 150μL of liquid from all receiver wells (basolateral or apical side).

### *4.7. Pharmacokinetic study in rodents*

#### *4.7.1. Animals*

Sprague-Dawley rats (male, 5-6 weeks old) and CD1(male, 18-20g) mice are obtained from Janvier (France). Rats are acclimatized for at least 4 days before treatment and are kept on a 12 h light/dark cycle (0700 - 1900). Temperature is maintained at approximately 22°C, and food and water are provided ad libitum. Rats and mice are deprived of food for at least 16 h before oral dosing and 4 h after. Water is provided ad libitum.

#### *4.7.2. Pharmacokinetic study*

Compounds are formulated in PEG200/water (60/40) or DMSO/PBS (1/99) +/-2eq NaOH for the intravenous route and in Solutol/0.5% methylcellulose (2/98) for the oral route. Test compounds are orally dosed as a single esophageal gavage at 5 mg/kg under a dosing volume of 5 mL/kg and intravenously dosed as a bolus via the caudal vein at 0.1 mg/kg under a dosing volume of 5 mL/kg. Each group consisted of 3 rats or 3 to 6 mice (if composite design). Blood samples are collected at the retro-orbital sinus under light anaesthesiausing lithium heparin as anti-coagulant at the following time points: 0.05, 0.25, 0.5, 1, 3, 6 and 24 h (intravenous route), and 0.25, 0.5, 1, 3, 5, 6 and 24 h (oral route). Alternatively, blood samples are collected at the retro-orbital sinus with lithium heparin as anti-coagulant at the following time points 0.25, 1, 3 and 6 h (oral route). Whole blood samples are centrifuged at 2,800 x g for 10 min and the resulting plasma samples are stored at -20°C pending analysis

#### *4.7.3. Quantification of compound levels in plasma*

Plasma concentrations of each test compound are determined by an LC-MS/MS method in which the mass spectrometer is operated in positive electrospray mode.

*4.7.4. Determination ofpharmacokinetic parameters*

Pharmacokinetic parameters are calculated using Phoenix® (Certara®).

*4.8. Liability for QT prolongation*

Potential for QT prolongation is assessed in the hERG patch clamp assay.

The method used is the IonFlux HT automated whole cell patch-clamp instrument to record outward potassium currents.

HEK-293 cells stably transfected with human hERG cDNA are used. The cells are harvested by trypsinization and maintained in Serum Free Medium at room temperature before recording. The cells are washed and re-suspended in Extracellular Solution before being applied to the automated patch-clamp instrument.

The test solutions are prepared in the Extracellular Solution on the day of patch-clamp assay. The assay can tolerate up to 1% DMSO.

The intracellular Solution contained (in mM): 70 KF, 60 KCl, 15 NaCl, 5 HEPES, 5 EGTA, 4 MgATP (pH 7.3 by KOH)

The extracellular Solution contained (in mM): 137 NaCl, 4 KCl, 1 $MgCl_2$, 1.8 $CaCl_2$, 10 HEPES, 10 glucose (pH 7.35 by NaOH)

After whole cell configuration is achieved, the cell is held at -80 mV. A 50 ms pulse to -50 mV is delivered to measure the leaking current, which is subtracted from the tail current. Then the cell is depolarized to +30 mV for 800 ms, followed by a 1200 ms pulse to -50 mV to reveal the hERG tail current. This paradigm is delivered once every 5 s to monitor the current amplitude. The assay is conducted at room temperature. The Extracellular Solution (control) is applied first and the cell is stabilized in the solution for 3 min. Then the test compound is applied from low to high concentrations sequentially on the same cell, the cells are perfused with each test concentration for 3 min.

A reference compound (E-4031) is tested concurrently at multiple concentrations to obtain an IC50 value.

The percent inhibition of hERG channel is calculated by comparing the tail current amplitude before and after application of the compound (the current difference is normalized to control).

## FINAL REMARKS

It will be appreciated by those skilled in the art that the foregoing descriptions are exemplary and explanatory in nature, and intended to illustrate the invention and its preferred embodiments. Through routine experimentation, an artisan will recognize apparent modifications and variations that may be made without departing from the scope of the invention. All such modifications coming within the scope of the appended claims are intended to be included therein. Thus, the invention is intended to be defined not by the above description, but by the following claims.

It should be understood that factors such as the differential cell penetration capacity of the various compounds can contribute to discrepancies between the activity of the compounds in the *in vitro* biochemical and cellular assays.

At least some of the chemical names of compound of the invention as given and set forth in this application, may have been generated on an automated basis by use of a commercially available chemical naming software program, and have not been independently verified. Representative programs performing this function include the Lexichem naming tool sold by Open Eye Software, Inc. and the Autonom Software tool sold by MDL, Inc. In the instance where the indicated chemical name and the depicted structure differ, the depicted structure will control.

## REFERENCES

Buti, M., Tsai, N., Petersen, J., Flisiak, R., Gurel, S., Krastev, Z., Aguilar Schall, R., Flaherty, J.F., Martins, E.B., Charuworn, P., Kitrinos, K.M., Mani Subramanian, G., Gane, E., Marcellin, P., 2015. Seven-Year Efficacy and Safety of Treatment with Tenofovir Disoproxil Fumarate for Chronic Hepatitis B Virus Infection. Dig. Dis. Sci. 60, 1457-1464. https://doi.org/10.1007/s10620-014-3486-7

Chevaliez, S., Hézode, C., Bahrami, S., Grare, M., Pawlotsky, J.-M., 2013. Long-term hepatitis B surface antigen (HBsAg) kinetics during nucleoside/nucleotide analogue therapy: Finite treatment duration unlikely. J. Hepatol. 58, 676-683. https://doi.org/10.1016/j.jhep.2012.11.039

Dawood, A., Abdul Basit, S., Jayaraj, M., Gish, R.G., 2017. Drugs in Development for Hepatitis B. Drugs 77, 1263-1280. https://doi.org/10.1007/s40265-017-0769-2

Gómez-Moreno, A., Garaigorta, U., 2017. Hepatitis B Virus and DNA Damage Response: Interactions and Consequences for the Infection. Viruses 9, 304. https://doi.org/10.3390/v9100304

Ni, Y., Lempp, F.A., Mehrle, S., Nkongolo, S., Kaufman, C., Fälth, M., Stindt, J., Königer, C., Nassal, M., Kubitz, R., Sültmann, H., Urban, S., 2014. Hepatitis B and D viruses exploit sodium taurocholate co-transporting polypeptide for species-specific entry into hepatocytes. Gastroenterology 146, 1070-1083. https://doi.org/10.1053/j.gastro.2013.12.024

Ruan, P., Xu, S.-Y., Zhou, B.-P., Huang, J., Gong, Z.-J., 2013. Hepatitis B surface antigen seroclearance in patients with chronic hepatitis B infection: A clinical study. J. Int. Med. Res. 41, 1732-1739. https://doi.org/10.1177/0300060513487643

Sussman, N.., 2009. Treatment of hepatitis B virus infection. John Hopkins Adv. Stud. Med. 9, 89-95. Tu, T., Budzinska, M.A., Shackel, N.A., Urban, S., 2017. HBV DNA Integration: Molecular Mechanisms and Clinical Implications. Viruses 9. https://doi.org/10.3390/v9040075

van Zonneveld, M., Honkoop, P., Hansen, B.E., Niesters, H.G.M., Murad, S.D., de Man, R.A., Schalm, S.W., Janssen, H.L.A., 2004. Long-term follow-up of alpha-interferon treatment of patients with chronic hepatitis B. Hepatology 39, 804-810. https://doi.org/10.1002/hep.20128

**Claims**

**1.** A compound according to Formula I:

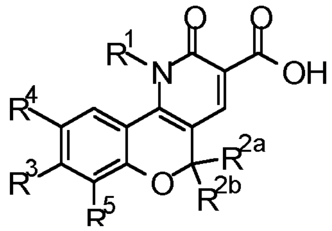

I

wherein

$R^1$ is $C_{3-6}$ cycloalkyl optionally substituted with one or more independently selected halo;
Each $R^{2a}$ and $R^{2b}$ is independently selected from:

- H,
- $C_{1-4}$ alkyl optionally substituted with one or more independently selected
    - halo,
    - -OH,
    - -CN,
    - $C_{1-4}$ alkoxy,
    - $C_{1-4}$ thioalkoxy, or
    - $-SO_2-C_{1-4}$ alkyl;
- $C_{3-7}$ cycloalkyl optionally substituted with one or more independently selected
    - -CN,
    - halo,
    - -OH,
    - $-SO_2-C_{1-4}$ alkyl,
    - $C_{1-4}$ alkyl optionally substituted with one or more independently selected halo, or
    - $C_{1-4}$ alkoxy optionally substituted with one or more independently selected halo; and
- 4-7 membered monocyclic heterocycloalkyl comprising one or more independently selected O, N, or S heteroatoms, optionally substituted with one or more independently selected
    - -CN,
    - halo,

◦ -OH,
◦ $-SO_2-C_{1-4}$ alkyl,
◦ =O,
◦ $C_{1-4}$ alkyl optionally substituted with one or more independently selected halo, or
◦ $C_{1-4}$ alkoxy optionally substituted with one or more independently selected halo;

$R^3$ is

- $C_{1-4}$ alkyl optionally substituted with one or more independently selected

  ◦ halo,
  ◦ CN,
  ◦ -OH,
  ◦ $SO_2-C_{1-4}$ alkyl,
  ◦ $C_{1-4}$ alkoxy, or
  ◦ 4-7 membered monocyclic heterocycloalkyl comprising one or more independently selected O, N, or S heteroatoms,

- halo,
- -CN, or
- $C_{1-4}$ alkoxy optionally substituted with one or more independently selected $R^7$ groups;

$R^4$ is

- H,
- $-SO_2-C_{1-4}$ alkyl,
- halo,
- CN,
- $C_{1-4}$ alkyl optionally substituted with one or more independently selected halo,
- $C_{1-4}$ alkoxy

$R^5$ is H, -CN, halo, or $C_{1-4}$ Alkyl optionally substituted with one or more independently selected halo; and each $R^{6a}$, $R^{6b}$, $R^{6c}$, and $R^{6d}$ is independently H or $C_{1-4}$ alkyl.
each $R^7$ is independently

- halo,
- -CN,
- -OH,
- $-SO_2-C_{1-4}$ alkyl,
- $C_{1-4}$ alkoxy,
- phenyl,
- 5-6 membered monocyclic heteroaryl comprising one or more independently selected O, N, or S heteroatoms,
- $C_{3-7}$ cycloalkyl optionally substituted with one or more independently selected $R^{8a}$,
- monocyclic 4-7 membered heterocycloalkyl comprising one or more independently selected O, N, or S, optionally substituted with one or more independently selected $R^{8b}$, and
- fused/spiro/bridged 4-10 membered heterocycloalkyl comprising one or more independently selected O, N, or S, optionally substituted with one or more independently selected $R^{8c}$;

each $R^{8a}$, $R^{8b}$, and $R^{8c}$ is independently selected from:

- halo,
- -CN,
- $-SO_2-C_{1-4}$ alkyl,
- =O,
- alkyl optionally substituted with one or more independently selected halo, or -OH,
- $-C(=O)NR^{6a}R^{6b}$,
- $-C_{1-4}$ alkoxy, or

- -C(=O) $C_{1-4}$ alkyl;

or a pharmaceutically acceptable salt thereof, solvate, or pharmaceutically acceptable salt of the solvate.

**2.** A compound or pharmaceutically acceptable salt according to claim 1, wherein $R^1$ is cyclopropyl, or cyclobutyl.

**3.** A compound or pharmaceutically acceptable salt according to claim 1 or 2, wherein $R^{2a}$ is $-CH_3$, $-CH_2CH_3$, $-CH(CH_3)_2$ or $-C(CH_3)_3$.

**4.** A compound or pharmaceutically acceptable salt according to claim 1, wherein the compound is according to Formula III:

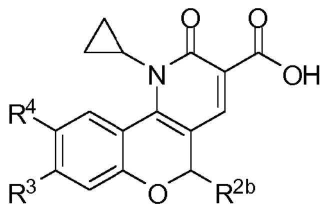

III

**5.** A compound or pharmaceutically acceptable salt according to any one of claims 1-4, wherein $R^{2b}$ is $C_{1-4}$ alkyl.

**6.** A compound or pharmaceutically acceptable salt according to any one of claims 1-4, wherein $R^{2b}$ is oxetanyl substituted with one or more independently selected -CN, halo, -OH, $-SO_2-C_{1-4}$ alkyl, =O, $C_{1-4}$ alkyl optionally substituted with one or more independently selected halo, or $C_{1-4}$ alkoxy optionally substituted with one or more independently selected halo.

**7.** A compound or pharmaceutically acceptable salt according to any one of claims 1-4, wherein $R^{2b}$ is oxetanyl substituted with one or more independently selected -CN, F, -OH, $-SO_2-CH_3$, =O, $-CH_3$, $-CH_2CH_3$, $-CF_3$, $-CHF_2$, $-CH_2CF_3$, $-OCH_3$, $-OCH_2CH_3$, or $-OCF_3$.

**8.** A compound or pharmaceutically acceptable salt according to claim 1, wherein the compound is according to Formula IVa or IVb:

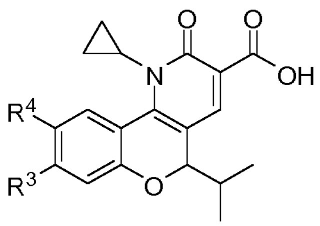

IVa

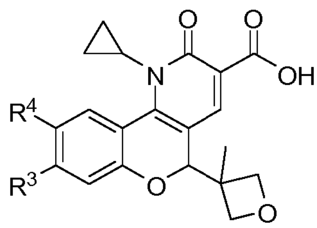

IVb

**9.** A compound or pharmaceutically acceptable salt according to any one of claims 1-8, wherein $R^4$ is halo, -CN, $-CH_3$, $-CH_2CH_3$, $-CH(CH_3)_2$, $-CF_3$, $-OCH_3$, or $-OCH_2CH_3$.

**10.** A compound or pharmaceutically acceptable salt according to any one of claims 1-9, wherein $R^3$ is F, Cl, -CN, $-OCH_3$, $-OCH_2CH_3$, $-OCH_2CH_2CH_3$, $-OCH_2CH(CH_3)_2$, or $-OCH(CH_3)_2$.

**11.** A compound or pharmaceutically acceptable salt according to any one of claims 1-9, wherein $R^3$ is $-OCH_3$, $-OCH_2CH_3$, $-OCH_2CH_2CH_3$, $-OCH_2CH(CH_3)_2$, or $-OCH(CH_3)_2$, each of which substituted with one, two or three independently selected $R^7$ groups.

**12.** A compound or pharmaceutically acceptable salt according to claim 11, wherein $R^7$ is F, -CN, -OH, $-SO_2CH_3$, $-SO_2CH_2CH_3$, $-OCH_3$, $-OCH_2CH_3$, $-OCH_2CH_2CH_3$, $-OCH_2CH(CH_3)_2$, $-OCH(CH_3)_2$, phenyl, oxetanyl, oxazolidinyl,

azetidinyl, or pyrrolyl.

**13.** A pharmaceutical composition comprising a pharmaceutically acceptable carrier and a pharmaceutically effective amount of a compound or pharmaceutically acceptable salt thereof according to any one of claims 1-12.

**14.** A compound or pharmaceutically acceptable salt thereof according to any one of claims 1-12, or a pharmaceutical composition according to claim 13, for use in medicine.

**15.** A compound or pharmaceutically acceptable salt thereof according to any one of claims 1-12, or a pharmaceutical composition according to claim 13, for use in the prophylaxis and/or treatment of hepatitis B.

## Patentansprüche

**1.** Verbindung der Formel I:

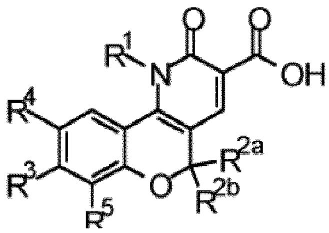

I

wobei

$R^1$ $C_{3-6}$-Cycloalkyl ist, gegebenenfalls substituiert mit einem oder mehreren unabhängig ausgewählten Halogen; jedes $R^{2a}$ und $R^{2b}$ unabhängig ausgewählt ist aus:

- H,
- $C_{1-4}$-Alkyl, gegebenenfalls substituiert mit einem oder mehreren unabhängig ausgewählten
  - Halogen,
  - -OH,
  - -CN,
  - $C_{1-4}$-Alkoxy,
  - $C_{1-4}$-Thioalkoxy, oder
  - $-SO_2-C_{1-4}$-Alkyl;
- $C_{3-7}$-Cycloalkyl, gegebenenfalls substituiert mit einem oder mehreren unabhängig ausgewählten
  - -CN,
  - Halogen,
  - -OH,
  - $-SO_2-C_{1-4}$-Alkyl,
  - $C_{1-4}$-Alkyl, gegebenenfalls substituiert mit einem oder mehreren unabhängig ausgewählten Halogen, oder
  - $C_{1-4}$-Alkoxy, gegebenenfalls substituiert mit einem oder mehreren unabhängig ausgewählten Halogen; und
- 4-7-gliedrigem monocyclischem Heterocycloalkyl, das ein oder mehrere unabhängig ausgewählte O-, N-, oder S-Heteroatome umfasst, gegebenenfalls substituiert mit einem oder mehreren unabhängig ausgewählten
  - -CN,
  - Halogen,

◦ -OH,
◦ $-SO_2-C_{1-4}$-Alkyl,
◦ =O,
◦ $C_{1-4}$-Alkyl, gegebenenfalls substituiert mit einem oder mehreren unabhängig ausgewählten Halogen, oder
◦ $C_{1-4}$-Alkoxy, gegebenenfalls substituiert mit einem oder mehreren unabhängig ausgewählten Halogen;

$R^3$

- $C_{1-4}$-Alkyl, gegebenenfalls substituiert mit einem oder mehreren unabhängig ausgewählten

◦ Halogen,
◦ CN,
◦ -OH,
◦ $SO_2-C_{1-4}$-Alkyl,
◦ $C_{1-4}$-Alkoxy, oder
◦ 4-7-gliedrigem monocyklischem Heterocycloalkyl, das ein oder mehrere unabhängig ausgewählte O-, N- oder S-Heteroatome umfasst,

- Halogen,
- -CN, oder
- $C_{1-4}$-Alkoxy, gegebenenfalls substituiert mit einer oder mehreren unabhängig ausgewählten $R^7$-Gruppen, ist;

$R^4$

- H,
- $-SO_2-C_{1-4}$-Alkyl,
- Halogen,
- CN,
- $C_{1-4}$-Alkyl, gegebenenfalls substituiert mit einem oder mehreren unabhängig ausgewählten Halogen,
- $C_{1-4}$-Alkoxy, ist;

$R^5$ H, -CN, Halogen, oder $C_{1-4}$-Alkyl, gegebenenfalls substituiert mit einem oder mehreren unabhängig ausgewählten Halogen, ist; und
jedes $R^{6a}$, $R^{6b}$, $R^{6c}$ und $R^{6d}$ unabhängig H oder $C_{1-4}$-Alkyl ist.
jedes $R^7$ unabhängig

- Halogen,
- -CN,
- -OH,
- $-SO_2-C_{1-4}$-Alkyl,
- $C_{1-4}$-Alkoxy,
- Phenyl,
- 5-6-gliedriges monocyclisches Heteroaryl, das ein oder mehrere unabhängig ausgewählte O-, N- oder S-Heteroatome umfasst,
- $C_{3-7}$-Cycloalkyl, gegebenenfalls substituiert mit einem oder mehreren unabhängig ausgewählten $R^{8a}$,
- monocyclisches 4-7-gliedriges Heterocycloalkyl, das ein oder mehrere unabhängig ausgewählte O, N oder S umfasst, gegebenenfalls substituiert mit einem oder mehreren unabhängig ausgewählten $R^{8b}$, und
- 4-10-gliedriges kondensiertes/Spiro-/verbrücktes Heterocycloalkyl, das ein oder mehrere unabhängig ausgewählte O, N oder S umfasst, gegebenenfalls substituiert mit einem oder mehreren unabhängig ausgewählten $R^{8c}$, ist;

jedes $R^{8a}$, $R^{8b}$ und $R^{8c}$ unabhängig ausgewählt ist aus:

- Halogen,
- -CN,

- $-SO_2-C_{1-4}$-Alkyl,
- =O,
- Alkyl, gegebenenfalls substituiert mit einem oder mehreren unabhängig ausgewählten Halogen, oder -OH,
- $-C(=O)NR^{6a}R^{6b}$,
- $-C_{1-4}$-Alkoxy, oder
- $-C(=O)C_{1-4}$-Alkyl;

oder ein pharmazeutisch verträgliches Salz davon, Solvat, oder pharmazeutisch verträgliches Salz des Solvats.

**2.** Verbindung oder pharmazeutisch verträgliches Salz nach Anspruch 1, wobei $R^1$ Cyclopropyl oder Cyclobutyl ist.

**3.** Verbindung oder pharmazeutisch verträgliches Salz nach Anspruch 1 oder 2, wobei $R^{2a}$ $-CH_3$, $-CH_2CH_3$, $-CH(CH_3)_2$ oder $-C(CH_3)_3$ ist.

**4.** Verbindung oder pharmazeutisch verträgliches Salz nach Anspruch 1, wobei die Verbindung der Formel III entspricht:

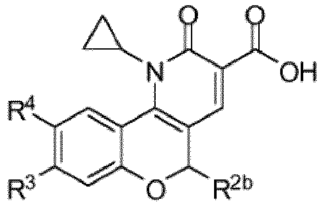

III

**5.** Verbindung oder pharmazeutisch verträgliches Salz nach einem der Ansprüche 1-4, wobei $R^{2b}$ $C_{1-4}$-Alkyl ist.

**6.** Verbindung oder pharmazeutisch verträgliches Salz nach einem der Ansprüche 1-4, wobei $R^{2b}$ Oxetanyl, substituiert mit einem oder mehreren unabhängig ausgewählten -CN, Halogen, -OH, $-SO_2-C_{1-4}$-Alkyl, =O, $C_{1-4}$-Alkyl, gegebenenfalls substituiert mit einem oder mehreren unabhängig ausgewählten Halogen, oder $C_{1-4}$-Alkoxy, gegebenenfalls substituiert mit einem oder mehreren unabhängig ausgewählten Halogen, ist.

**7.** Verbindung oder pharmazeutisch verträgliches Salz nach einem der Ansprüche 1-4, wobei $R^{2b}$ Oxetanyl, substituiert mit einem oder mehreren unabhängig ausgewählten -CN, F, -OH, $-SO_2-CH_3$, =O, $-CH_3$, $-CH_2CH_3$, $-CF_3$, $-CHF_2$, $-CH_2CF_3$, $-OCH_3$, $-OCH_2CH_3$, oder $-OCF_3$ ist.

**8.** Verbindung oder pharmazeutisch verträgliches Salz nach Anspruch 1, wobei die Verbindung Formel IVa oder IVb entspricht:

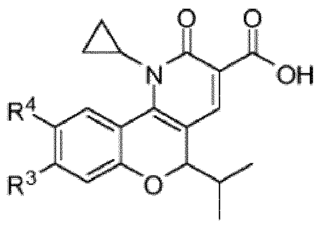

IVa

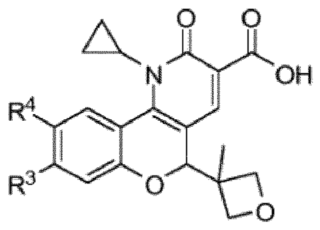

IVb

**9.** Verbindung oder pharmazeutisch verträgliches Salz nach einem der Ansprüche 1-8, wobei $R^4$ Halogen, -CN, $-CH_3$, $-CH_2CH_3$, $-CH(CH_3)_2$, $-CF_3$, $-OCH_3$, oder $-OCH_2CH_3$ ist.

**10.** Verbindung oder pharmazeutisch verträgliches Salz nach einem der Ansprüche 1-9, wobei $R^3$ F, Cl, -CN, $-OCH_3$, $-OCH_2CH_3$, $-OCH_2CH_2CH_3$, $-OCH_2CH(CH_3)_2$, oder-$OCH(CH_3)_2$ ist.

**11.** Verbindung oder pharmazeutisch verträgliches Salz nach einem der Ansprüche 1-9, wobei $R^3$ $-OCH_3$, $-OCH_2CH_3$, $-OCH_2CH_2CH_3$, $-OCH_2CH(CH_3)_2$, oder-$OCH(CH_3)_2$, jedes davon substituiert mit einer, zwei oder drei unabhängig

ausgewählten $R^7$-Gruppen, ist.

**12.** Verbindung oder pharmazeutisch verträgliches Salz nach Anspruch 11, wobei $R^7$ F, -CN, -OH, $-SO_2CH_3$, $-SO_2CH_2CH_3$, $-OCH_3$, $-OCH_2CH_3$, $-OCH_2CH_2CH_3$, $-OCH_2CH(CH_3)_2$,$-OCH(CH_3)_2$, Phenyl, Oxetanyl, Oxazolidinyl, Azetidinyl, oder Pyrrolyl ist.

**13.** Pharmazeutische Zusammensetzung, die einen pharmazeutisch verträglichen Träger und eine pharmazeutisch wirksame Menge einer Verbindung oder eines pharmazeutisch verträglichen Salzes davon nach einem der Ansprüche 1-12 umfasst.

**14.** Verbindung oder pharmazeutisch verträgliches Salz davon nach einem der Ansprüche 1-12, oder pharmazeutische Zusammensetzung nach Anspruch 13, zur Verwendung in Medizin.

**15.** Verbindung oder pharmazeutisch verträgliches Salz davon nach einem der Ansprüche 1-12, oder pharmazeutische Zusammensetzung nach Anspruch 13, zur Verwendung in der Vorbeugung und/oder Behandlung von Hepatitis B.

## Revendications

**1.** Composé selon la formule I :

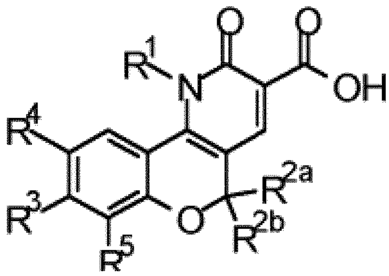

I

dans lequel

$R^1$ est un cycloalkyle en $C_{3-6}$ éventuellement substitué par un ou plusieurs, indépendamment sélectionnés, halo ;
chaque $R^{2a}$ et $R^{2b}$ est indépendamment sélectionné parmi :

- H,
- alkyle en $C_{1-4}$ éventuellement substitué par un ou plusieurs, indépendamment sélectionnés,
  - halo,
  - -OH,
  - -CN,
  - alcoxy en $C_{1-4}$,
  - thioalcoxy en $C_{1-4}$, ou
  - $-SO_2$-alkyle en $C_{1-4}$ ;
- cycloalkyle en $C_{3-7}$ éventuellement substitué par un ou plusieurs, indépendamment sélectionnés,
  - -CN,
  - halo,
  - -OH,
  - $-SO_2$-alkyle en $C_{1-4}$,
  - alkyle en $C_{1-4}$ éventuellement substitué par un ou plusieurs, indépendamment sélectionnés, halo, ou
  - alcoxy en $C_{1-4}$ éventuellement substitué par un ou plusieurs, indépendamment sélectionnés, halo ; et
- hétérocycloalkyle monocyclique à 4-7 membres comprenant un ou plusieurs, indépendamment sélectionnés, hétéroatomes O, N ou S, éventuellement substitués par un ou plusieurs, indépendamment sélectionnés,

- ◦ -CN,
- ◦ halo,
- ◦ -OH,
- ◦ -$SO_2$-alkyle en $C_{1-4}$,
- ◦ =O,
- ◦ alkyle en $C_{1-4}$ éventuellement substitué par un ou plusieurs, indépendamment sélectionnés, halo, ou
- ◦ alcoxy en $C_{1-4}$ éventuellement substitué par un ou plusieurs, indépendamment sélectionnés, halo ;

$R^3$ est

- alkyle en $C_{1-4}$ éventuellement substitué par un ou plusieurs, indépendamment sélectionnés,

  - ◦ halo,
  - ◦ CN,
  - ◦ -OH,
  - ◦ $SO_2$-alkyle en $C_{1-4}$,
  - ◦ alcoxy en $C_{1-4}$, ou
  - ◦ hétérocycloalkyle monocyclique à 4-7 membres comprenant un ou plusieurs, indépendamment sélectionnés, hétéroatomes O, N ou S,

- halo,
- -CN ou
- alcoxy en $C_{1-4}$ éventuellement substitué par un ou plusieurs, indépendamment sélectionnés, groupes $R^7$;

$R^4$ est

- H,
- -$SO_2$-alkyle en $C_{1-4}$,
- halo,
- CN,
- alkyle en $C_{1-4}$ éventuellement substitué par un ou plusieurs, indépendamment sélectionnés, halo,
- alcoxy en $C_{1-4}$

$R^5$ est H, -CN, halo ou un alkyle en $C_{1-4}$ éventuellement substitué par un ou plusieurs, indépendamment sélectionnés, halo ; et
chaque $R^{6a}$, $R^{6b}$, $R^{6c}$ et $R^{6d}$ est indépendamment H ou un alkyle en $C_{1-4}$,
chaque $R^7$ est indépendamment

- halo,
- -CN,
- -OH,
- -$SO_2$-alkyle en $C_{1-4}$,
- alcoxy en $C_{1-4}$,
- phényle,
- hétéroaryle monocyclique à 5-6 membres comprenant un ou plusieurs, indépendamment sélectionnés, hétéroatomes O, N ou S,
- cycloalkyle en $C_{3-7}$ éventuellement substitué par un ou plusieurs, indépendamment sélectionnés, $R^{8a}$,
- hétérocycloalkyle monocyclique à 4-7 membres comprenant un ou plusieurs, indépendamment sélectionnés, O, N ou S, éventuellement substitués par un ou plusieurs, indépendamment sélectionnés, $R^{8b}$, et
- hétérocycloalkyle à 4-10 membres condensé/spiro/ponté comprenant un ou plusieurs, indépendamment sélectionnés, O, N ou S, éventuellement substitués par un ou plusieurs, indépendamment sélectionnés, $R^{8b}$ ;

chaque $R^{8a}$, $R^{8b}$ et $R^{8c}$ est indépendamment sélectionné parmi :

- halo,
- -CN,
- $-SO_2$-alkyle en $C_{1-4}$,
- =O,
- alkyle éventuellement substitué par un ou plusieurs, indépendamment sélectionnés, halo ou -OH,
- $-C(=O)NR^{6a}R^{6b}$,
- alcoxy en $C_{1-4}$, ou
- -C(=O)-alkyle en $C_{1-4}$ ;

ou un sel pharmaceutiquement acceptable de celui-ci, un solvate ou un sel pharmaceutiquement acceptable du solvate.

**2.** Composé ou sel pharmaceutiquement acceptable selon la revendication 1, dans lequel $R^1$ est un cyclopropyle ou un cyclobutyle.

**3.** Composé ou sel pharmaceutiquement acceptable selon la revendication 1 ou 2, dans lequel $R^{2a}$ est $-CH_3$, $-CH_2CH_3$, $-CH(CH_3)_2$ ou $-C(CH_3)_3$.

**4.** Composé ou sel pharmaceutiquement acceptable selon la revendication 1, dans lequel le composé est selon la formule III :

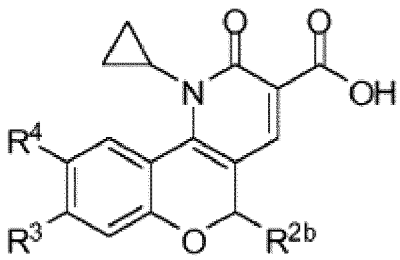

III

**5.** Composé ou sel pharmaceutiquement acceptable selon l'une quelconque des revendications 1-4, dans lequel $R^{2b}$ est un alkyle en $C_{1-4}$.

**6.** Composé ou sel pharmaceutiquement acceptable selon l'une quelconque des revendications 1-4, dans lequel $R^{2b}$ est un oxétanyle substitué par un ou plusieurs, indépendamment sélectionnés, -CN, halo, -OH, -SO2-alkyle en $C_{1-4}$, =O, alkyle en $C_{1-4}$ éventuellement substitué par un ou plusieurs, indépendamment sélectionnés, halo, ou alcoxy en $C_{1-4}$ éventuellement substitué par un ou plusieurs, indépendamment sélectionnés, halo.

**7.** Composé ou sel pharmaceutiquement acceptable selon l'une quelconque des revendications 1-4, dans lequel $R^{2b}$ est un oxétanyle substitué par un ou plusieurs, indépendamment sélectionnés, -CN, F, -OH, $-SO_2-CH_3$, =O, $-CH_3$, $-CH_2CH_3$, $-CF_3$, $-CHF_2$, - $CH_2CF_3$, $-OCH_3$, $-OCH_2CH_3$ ou $-OCF_3$.

**8.** Composé ou sel pharmaceutiquement acceptable selon la revendication 1, dans lequel le composé est selon la formule IVa ou IVb :

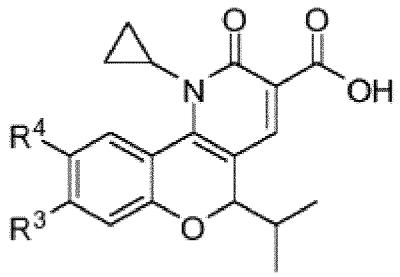

IVa

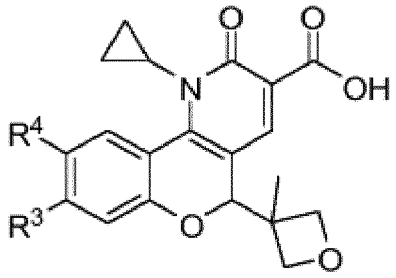

IVb

**9.** Composé ou sel pharmaceutiquement acceptable selon l'une quelconque des revendications 1-8, dans lequel $R^4$ est halo, -CN, $-CH_3$, $-CH_2CH_3$, $-CH(CH_3)_2$, $-CF_3$, $-OCH_3$ ou $-OCH_2CH_3$.

**10.** Composé ou sel pharmaceutiquement acceptable selon l'une quelconque des revendications 1-9, dans lequel $R^3$ est F, Cl, -CN, $-OCH_3$, $-OCH_2CH_3$, $-OCH_2CH_2CH_3$, - $OCH_2CH(CH_3)_2$ ou $-OCH(CH_3)_2$.

**11.** Composé ou sel pharmaceutiquement acceptable selon l'une quelconque des revendications 1-9, dans lequel $R^3$ est $-OCH_3$, $-OCH_2CH_3$, $-OCH_2CH_2CH_3$, $-OCH_2CH(CH_3)_2$ ou $-OCH(CH_3)_2$, chacun étant substitué par un, deux ou trois, indépendamment sélectionnés, groupes $R^7$.

**12.** Composé ou sel pharmaceutiquement acceptable selon la revendication 11, dans lequel $R^7$ est F, -CN, -OH, $-SO_2CH_3$, $-SO_2CH_2CH_3$, $-OCH_3$, $-OCH_2CH_3$, $-OCH_2CH_2CH_3$, - $OCH_2CH(CH_3)_2$, $-OCH(CH_3)_2$, phényle, oxétanyle, oxazolidinyle, azétidinyle ou pyrrolyle.

**13.** Composition pharmaceutique comprenant un support pharmaceutiquement acceptable et une quantité pharmaceutiquement efficace d'un composé ou d'un sel pharmaceutiquement acceptable de celui-ci selon l'une quelconque des revendications 1-12.

**14.** Composé ou sel pharmaceutiquement acceptable de celui-ci selon l'une quelconque des revendications 1-12, ou composition pharmaceutique selon la revendication 13, destiné à être utilisé en médecine.

**15.** Composé ou sel pharmaceutiquement acceptable de celui-ci selon l'une quelconque des revendications 1-12, ou composition pharmaceutique selon la revendication 13, destiné à être utilisé dans la prophylaxie et/ou le traitement de l'hépatite B.

FIGURE 1

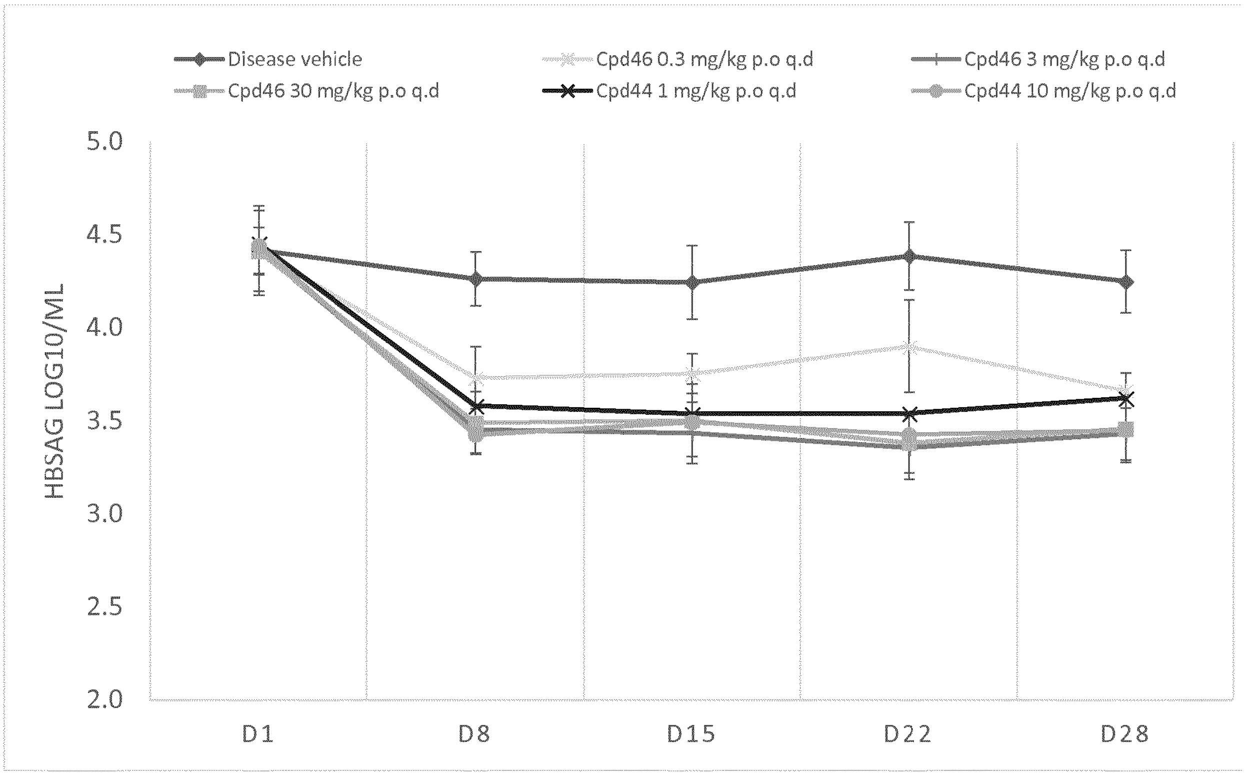
Disease vehicle
Cpd46 0.3 mg/kg p.o q.d
Cpd46 3 mg/kg p.o q.d
Cpd46 30 mg/kg p.o q.d
Cpd44 1 mg/kg p.o q.d
Cpd44 10 mg/kg p.o q.d
HBSAG LOG10/ML
5.0
4.5
4.0
3.5
3.0
2.5
2.0
D1
D8
D15
D22
D28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017205115 A
- WO 2017140821 A
- WO 2017114812 A
- WO 2017108630 A
- WO 2017102648 A
- WO 2017017043 A
- WO 2017013046 A


### Non-patent literature cited in the description


- Remington's Pharmaceutical Sciences. Mack Publishing Company, 1985
- **BUTI, M. ; TSAI, N. ; PETERSEN, J. ; FLISIAK, R. ; GUREL, S. ; KRASTEV, Z. ; AGUILAR SCHALL, R. ; FLAHERTY, J.F. ; MARTINS, E.B. ; CHARUWORN, P.** Seven-Year Efficacy and Safety of Treatment with Tenofovir Disoproxil Fumarate for Chronic Hepatitis B Virus Infection. *Dig. Dis. Sci.,* 2015, vol. 60, 1457-1464, https://doi.org/10.1007/s10620-014-3486-7
- **CHEVALIEZ, S. ; HÉZODE, C. ; BAHRAMI, S. ; GRARE, M. ; PAWLOTSKY, J.-M.** Long-term hepatitis B surface antigen (HBsAg) kinetics during nucleoside/nucleotide analogue therapy: Finite treatment duration unlikely. *J. Hepatol.,* 2013, vol. 58, 676-683, https://doi.org/10.1016/j.jhep.2012.11.039
- **DAWOOD, A. ; ABDUL BASIT, S. ; JAYARAJ, M. ; GISH, R.G.** Drugs in Development for Hepatitis B. *Drugs,* 2017, vol. 77, 1263-1280, https://doi.org/10.1007/s40265-017-0769-2
- **GÓMEZ-MORENO, A. ; GARAIGORTA, U.** Hepatitis B Virus and DNA Damage Response: Interactions and Consequences for the Infection. *Viruses,* 2017, vol. 9, 304, https://doi.org/10.3390/v9100304
- **NI, Y. ; LEMPP, F.A. ; MEHRLE, S. ; NKONGOLO, S. ; KAUFMAN, C. ; FÄLTH, M. ; STINDT, J. ; KÖNIGER, C. ; NASSAL, M. ; KUBITZ, R.** Hepatitis B and D viruses exploit sodium taurocholate co-transporting polypeptide for species-specific entry into hepatocytes. *Gastroenterology,* 2014, vol. 146, 1070-1083, https://doi.org/10.1053/j.gastro.2013.12.024
- **RUAN, P. ; XU, S.-Y. ; ZHOU, B.-P. ; HUANG, J., GONG, Z.-J.** Hepatitis B surface antigen seroclearance in patients with chronic hepatitis B infection: A clinical study. *J. Int. Med. Res.,* 2013, vol. 41, 1732-1739, https://doi.org/10.1177/0300060513487643
- **SUSSMAN, N.** Treatment of hepatitis B virus infection. *John Hopkins Adv. Stud. Med.,* 2009, vol. 9, 89-95
- **TU, T. ; BUDZINSKA, M.A. ; SHACKEL, N.A. ; URBAN, S.** HBV DNA Integration: Molecular Mechanisms and Clinical Implications. *Viruses,* 2017, vol. 9, https://doi.org/10.3390/v9040075
- **VAN ZONNEVELD, M. ; HONKOOP, P. ; HANSEN, B.E. ; NIESTERS, H.G.M. ; MURAD, S.D. ; MAN, R.A. ; SCHALM, S.W. ; JANSSEN, H.L.A.** Long-term follow-up of alpha-interferon treatment of patients with chronic hepatitis B. *Hepatology,* 2004, vol. 39, 804-810, https://doi.org/10.1002/hep.20128